(19) Europäisches Patentamt
European Patent Office
Office européen des brevets

(11) EP 1 317 459 B1

(12) **EUROPEAN PATENT SPECIFICATION**

(45) Date of publication and mention of the grant of the patent:
**07.04.2004 Bulletin 2004/15**

(21) Application number: **01961577.2**

(22) Date of filing: **31.08.2001**

(51) Int Cl.[7]: **C07D 495/04**, C07D 491/04, C07D 513/04, C07D 487/04, A61K 31/407, A61P 3/10, A61P 9/10

(86) International application number:
**PCT/SE2001/001880**

(87) International publication number:
**WO 2002/020530 (14.03.2002 Gazette 2002/11)**

(54) **BICYCLIC PYRROLYL AMIDES AS GLUCOGEN PHOSPHORYLASE INHIBITORS**

BIZYKLISCHE PYRROLYL-AMIDE ALS GLUKOGEN-PHOSPHORYLASE-HEMMER

AMIDES DE PYRROLYLE BICYCLIQUES SERVANT D'INHIBITEURS DE GLYCOGENE PHOSPHORYLASE

(84) Designated Contracting States:
**AT BE CH CY DE DK ES FI FR GB GR IE IT LI LU MC NL PT SE TR**
Designated Extension States:
**AL LT LV MK RO SI**

(30) Priority: **06.09.2000 GB 0021831**

(43) Date of publication of application:
**11.06.2003 Bulletin 2003/24**

(73) Proprietor: **AstraZeneca AB**
**151 85 Södertälje (SE)**

(72) Inventors:
- **BARTLETT, Julie, B., AstraZeneca R & D Alderley Macclesfield, Cheshire SK10 4TG (GB)**
- **FREEMAN, Sue Macclesfiel, Cheshire SK10 4TG (GB)**
- **KENNY, Peter, AstraZeneca R & D Alderley Macclesfield, Cheshire SK10 4TG (GB)**
- **MORLEY, Andrew, AstraZeneca R & D Alderley Macclesrfield, Cheshire SK10 4TG (GB)**
- **WHITTAMORE, Paul, AstraZeneca R & D Alderley Macclesfield, Chreshire SK10 4TG (GB)**

(74) Representative: **Tinsley, Rachel Maria et al**
**AstraZeneca**
**R & D Headquarters**
**Global Intellectual Property, Patents**
**151 85 Sodertalje (SE)**

(56) References cited:
**EP-A2- 0 846 464**
**WO-A1-96/39384**
**ES-A1- 2 081 747**
**EP-A2- 1 088 824**
**WO-A1-99/46268**
**US-A- 5 998 463**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 317 459 B1

Printed by Jouve, 75001 PARIS (FR)

## Description

The present invention relates to heterocyclic amide derivatives, pharmaceutically acceptable salts and *in vivo* hydrolysable esters thereof. These heterocyclic amides possess glycogen phosphorylase inhibitory activity and accordingly have value in the treatment of disease states associated with increased glycogen phosphorylase activity and thus are potentially useful in methods for the treatment of a warm-blooded animal such as man. The invention also relates to processes for the manufacture of said heterocyclic amide derivatives, to pharmaceutical compositions containing them and to their use in the manufacture of medicaments to inhibit glycogen phosphorylase activity in a warm-blooded animal such as man.

The liver is the major organ regulating glycaemia in the post-absorptive state. Additionally, although having a smaller role in the contribution to post-prandial blood glucose levels, the response of the liver to exogenous sources of plasma glucose is key to an ability to maintain euglycaemia. An increased hepatic glucose output (HGO) is considered to play an important role in maintaining the elevated fasting plasma glucose (FPG) levels seen in type 2 diabetics; particularly those with a FPG >140mg/dl (7.8mM). (Weyer et al, (1999), J Clin Invest 104: 787-794; Clore & Blackgard (1994), Diabetes 43: 256-262; De Fronzo, R. A., et al, (1992) Diabetes Care 15; 318 - 355; Reaven, G.M. (1995) Diabetologia 38; 3-13).

Since current oral, anti-diabetic therapies fail to bring FPG levels to within the normal, non-diabetic range and since raised FPG (and glycHbAlc) levels are risk factors for both macro- (Charles, M.A. et al (1996) Lancet. 348,1657-1658; Coutinho, M. et al (1999) Diabetes Care 22; 233-240; Shaw, J.E. et al (2000) Diabetes Care 23, 34-39) and micro-vascular disease (DCCT Research Group (1993) New. Eng. J. Med. 329; 977-986); the reduction and normalisation of elevated FPG levels remains a treatment goal in type 2 diabetes.

It has been estimated that, after an overnight fast, 74% of HGO is derived from glycogenolysis with the remainder derived from gluconeogenic precursors (Hellerstein et al (1997) Am J Physiol, 272: E163). Glycogen phosphorylase is a key enzyme in the generation by glycogenolysis of glucose-1-phosphate, and hence glucose in liver and also in other tissues such as muscle and neuronal tissue.

Liver glycogen phosphorylase activity is elevated in diabetic animal models including the db/db mouse and the fa/fa rat (Aiston S et al (2000). Diabetalogia 43, 589-597).

Inhibition of hepatic glycogen phosphorylase with chloroindole inhibitors (CP91149 and CP320626) has been shown to reduce both glucagon stimulated glycogenolysis and glucose output in hepatocytes (Hoover et al (1998) J Med Chem 41, 2934-8; Martin et al (1998) PNAS 95, 1776-81, WO 96/39384 and WO 96/39385). Additionally, plasma glucose concentration is reduced, in a dose related manner, in db/db and ob/ob mice following treatment with these compounds.

Studies in conscious dogs with glucagon challenge in the absence and presence of another glycogen phosphorylase inhibitor, Bay K 3401, also show the potential utility of such agents where there is elevated circulating levels of glucagon, as in both Type 1 and Type 2 diabetes. In the presence of Bay R 3401, hepatic glucose output and arterial plasma glucose levels following a glucagon challenge were reduced significantly (Shiota et al, (1997), Am J Physiol, 273: E868).

ES 2,081,747 discloses that certain amide derivatives of 4*H*-thieno[3,2-b]pyrroles and 4*H*-thieno[2,3-b]pyrroles are CCK antagonists and are useful in the treatment of gastric secretion disorders and in the regulation of appetite. The compounds disclosed in this document are disclaimed from the compound claims of the present invention.

US 3,706,810 discloses that certain N-(aminoalkyl) derivatives of thieno [3,2-b]pyrrole-5-carboxamide are useful as analgesic and anti-depressant agents. The compounds disclosed in this document are disclaimed from the compound claims of the present invention.

US 4,751,231 discloses that certain thieno[2,3-*b*]pyrrole-5-sulfonamides are useful in the treatment of elevated intraocular pressure and glaucoma. Certain amides are disclosed as intermediates. The compounds disclosed in this document are disclaimed from the compound claims of the present invention.

US 4,794,120 discloses 4*H*-thieno[3,2-*b*]pyrrole-5-carboxylic acid hydrazide and *6H*-thieno[2,3-b]pyrrole-5-carboxylic acid hydrazide as intermediates in the preparation of corresponding (5-nitro-2-furanyl)methylenehydrazides which are antibacterials, fungicides and protozoacides. The compounds disclosed in this document are disclaimed from the compound claims of the present invention.

Co-pending application EP 1088824 discloses that a compound of Formula A: a stereoisomer, pharmaceutically acceptable salt or prodrug thereof, or a pharmaceutically

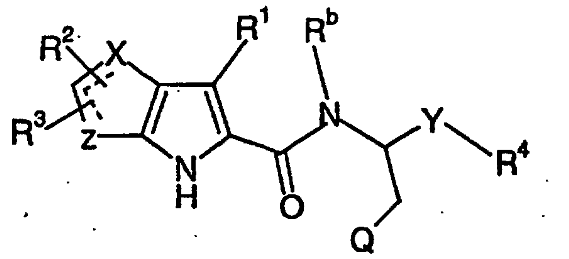

A

acceptable salt of the prodrug,
wherein

Q is aryl, substituted aryl, heteroaryl, or substituted heteroaryl;

each z and X are independently (C, CH or $CH_2$), N, O or S;

$X^1$ is $NR^a$, $-CH_2-$, O or S;

each — is independently a bond or is absent, provided that both — are not simultaneously bonds;

$R^1$ is hydrogen, halogen, $-OC_1$-$C_8$alkyl, $-SC_1$-$C_8$alkyl, $-C_1$-$C_8$alkyl, $-CF_3$, $-NH_2$-, $-NHC_1$-$C_8$alkyl, $-N(C_1$-$C_8alkyl)_2$, $-NO_2$, -CN, $-CO_2H$, $-CO_2C_1$-$C_8$alkyl, $-C_2$-$C_8$alkenyl, or $-C_2$-$C_8$alkynyl;

each $R^a$ and $R^b$ is independently hydrogen or—$C_1$-$C_8$alkyl;

Y is

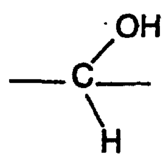

or absent;

$R^2$ and $R^3$ are independently hydrogen, halogen, $-C_1$-$C_8$alkyl, -CN, $-C{\equiv}C-Si(CH_3)_3$, $-OC_1$-$C_8$alkyl, $-SC_1$-$C_8$alkyl, $-CF_3$, $-NH_2$,$-NHC_1$-$C_8$alkyl, $-N(C_1$-$C_8alkyl)_2$, $-NO_2$, $-CO_2H$, $-CO_2C_1$-$C_8$alkyl, $-C_2$-$C_8$alkenyl, or —$C_2$-$C_8$alkynyl, or $R^2$ and $R^3$ together with the atoms on the ring to which they are attached form a five or six membered ring containing from 0 to 3 heteroatoms and from 0 to 2 double bonds;

$R^4$ is —C(=O)-A;

A is $-NR^dR^d$,$-NR^aCH_2CH_2OR^a$,

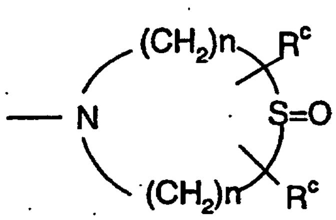 , 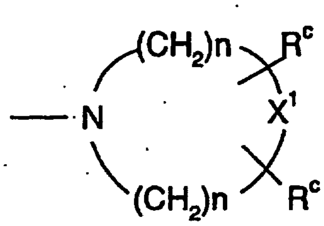 , or 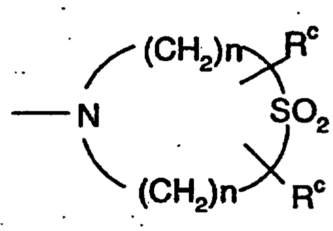 ;

each $R^d$ is independently hydrogen, $C_1$-$C_8$alkyl, $C_1$-$C_8$alkoxy, aryl, substituted aryl, heteroaryl, or substituted heteroaryl;

each $R^c$ is independently hydrogen, $-C(=O)OR^a$,$-OR^a$, $-SR^a$, or $-NR^aR^a$; and each n is independently 1-3,
are useful in treating diabetes, insulin resistance, diabetic neuropathy, diabetic nephropathy, diabetic retinopathy, cataracts, hyperglycemia, hypercholesterolemia, hypertension, hyperinsulinemia, hyperlipidemia, atherosclerosis, or tissue ischemia. These compounds are disclaimed from the present application.

The heterocyclic amides of the present invention possess glycogen phosphorylase inhibitory activity and accordingly are expected to be of use in the treatment of type 2 diabetes, insulin resistance, syndrome X, hyperinsulinaemia, hyperglucagonaemia, cardiac ischaemia and obesity, particularly type 2 diabetes.

The present invention provides a compound of formula (**I**):

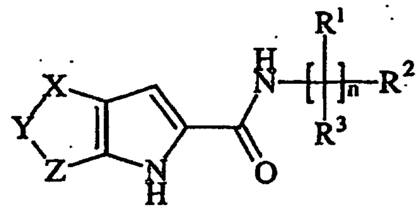

**(I)**

wherein:

**-X-Y-Z-** is selected from -S-$CR^4$=$CR^5$-, -$CR^4$=$CR^5$-S-, -O-$CR^4$=$CR^5$-, -$CR^4$=$CR^5$-O-, -N=$CR^4$-S-, -S-$CR^4$=N-, -$NR^6$-$CR^4$=$CR^5$- and -$CR^4$=$CR^5$-$NR^6$-;

wherein **$R^4$** and **$R^5$** are independently selected from hydrogen, halo, nitro, cyano, hydroxy, fluoromethyl, difluoromethyl, trifluoromethyl, trifluoromethoxy, amino, carboxy, carbamoyl, mercapto, sulphamoyl, ureido, $C_{1-6}$alkyl, $C_{2-6}$alkenyl, $C_{2-6}$alkynyl, $C_{1-6}$alkoxy, $C_{1-6}$alkanoyl, $C_{1-6}$alkanoyloxy, *N*-($C_{1-6}$alkyl)amino, *N,N*-($C_{1-6}$alkyl)$_2$amino, $C_{1-6}$alkanoylamino, *N*-($C_{1-6}$alkyl)carbamoyl, *N,N*-($C_{1-6}$alkyl)$_2$carbamoyl, $C_{1-6}$alkylS(O)$_a$ wherein a is 0 to 2, $C_{1-6}$alkoxycarbonyl, $C_{1-6}$alkoxycarbonylamino, *N*-($C_{1-6}$alkyl)sulphamoyl, *N,N*-($C_{1-6}$alkyl)$_2$sulphamoyl, $C_{1-6}$alkylsulphonylamino and $C_{1-6}$alkylsulphonyl-*N*-($C_{1-6}$alkyl)amino;

**$R^6$** is hydrogen or $C_{1-6}$alkyl;

**$R^1$** is selected from hydrogen, halo, nitro, cyano, hydroxy, amino, carboxy, carbamoyl, mercapto, sulphamoyl, ureido, $C_{1-6}$alkyl, $C_{2-6}$alkenyl, $C_{2-6}$alkynyl, $C_{1-6}$alkoxy, $C_{1-6}$alkanoyl, $C_{1-6}$alkanoyloxy, *N*-($C_{1-6}$alkyl)amino, *N,N*-($C_{1-6}$alkyl)$_2$amino, $C_{1-6}$alkanoylamino, *N*-($C_{1-6}$alkyl)carbamoyl, *N,N*-($C_{1-4}$alkyl)$_2$carbamoyl, $C_{1-6}$alkylS(O)$_a$ wherein a is 0 to 2, $C_{1-6}$alkoxycarbonyl, $C_{1-6}$alkoxycarbonylamino, *N*-($C_{1-6}$alkyl)sulphamoyl, *N,N*-($C_{1-6}$alkyl)$_2$sulphamoyl, $C_{1-6}$alkylsulphonylamino, $C_{1-6}$alkylsulphonyl-*N*-($C_{1-6}$alkyl)amino, $C_{3-8}$cycloalkyl, $C_{3-8}$cycloalkyl$C_{1-6}$alkyl, aryl, aryl$C_{1-6}$alkyl, heterocyclic group and (heterocyclic group)$C_{1-6}$alkyl; wherein $R^1$ may be optionally substituted on carbon by one or more groups selected from P and wherein if said heterocyclic group contains an -NH- moiety that nitrogen may be optionally substituted by a group selected from R;

**$R^2$** is selected from hydrogen, halo, nitro, cyano, hydroxy, fluoromethyl, difluoromethyl, trifluoromethyl, trifluoromethoxy, amino, carboxy, carbamoyl, mercapto, sulphamoyl, ureido, $C_{1-6}$alkyl, $C_{2-6}$alkenyl, $C_{2-6}$alkynyl, $C_{1-6}$alkoxy, $C_{1-6}$alkanoyl, $C_{1-6}$alkanoyloxy, *N*-($C_{1-6}$alkyl)amino, *N,N*-($C_{1-6}$alkyl)$_2$amino, $C_{1-6}$alkanoylamino, *N*-($C_{1-6}$alkyl)carbamoyl, *N,N*-($C_{1-4}$alkyl)$_2$carbamoyl, *N*-($C_{1-6}$alkyl)-*N*-($C_{1-6}$alkoxy)carbamoyl, $C_{1-6}$alkylS(O)$_a$ wherein a is 0 to 2, $C_{1-6}$alkoxycarbonyl, $C_{1-6}$alkoxycarbonylamino, *N*-($C_{1-6}$alkyl)sulphamoyl, *N,N*-($C_{1-6}$alkyl)$_2$sulphamoyl, sulphamoylamino, *N*-($C_{1-6}$alkyl)sulphamoylamino, *N,N*-($C_{1-6}$alkyl)$_2$sulphamoylamino, $C_{1-6}$alkylsulphonylamino, $C_{1-6}$alkylsulphonylaminocarbonyl, $C_{1-6}$alkylsulphonyl-*N*-($C_{1-6}$alkyl)amino and a group -E-F-G-H;

wherein **E** and **G** are independently selected from a direct bond, -O-, -S-, -SO-, -$SO_2$-, -OC(O)-, -C(O)-, -C(O)-, -$NR^a$-, -$NR^a$C(O)-, -C(O)$NR^a$-, -$SO_2NR^a$-, -$NR^aSO_2$--$NR^a$C(O)$NR^b$-, -OC(O)$NR^a$-, -$NR^a$C(O)O-, -$NR^aSO_2NR^b$-, -$SO_2NR^a$C(O)- and -C(O)$NR^aSO_2$-; wherein $R^a$ and $R^b$ are independently selected from hydrogen or $C_{1-6}$alkyl which is optionally substituted by a group V;

**F** is $C_{1-6}$alkylene optionally substituted by one or more Q or a direct bond;

**H** is selected from aryl, $C_{3-8}$cycloalkyl and heterocyclic group; wherein H may be optionally substituted on carbon by one or more groups selected from S and wherein if said heterocyclic group contains an -NH- moiety that nitrogen may be optionally substituted by a group selected from T;

**$R^3$** is hydrogen or $C_{1-6}$alkyl;

**n** is selected from 0-4; wherein the values of $R^1$ may be the same or different; and wherein the values of $R^3$ may be the same or different;

**P, S** and **Q** are independently selected from halo, nitro, cyano, hydroxy, trifluoromethyl, trifluoromethoxy, amino, carboxy, carbamoyl, mercapto, sulphamoyl, ureido, $C_{1-6}$alkyl, $C_{2-6}$alkenyl, $C_{2-6}$alkynyl, $C_{1-6}$alkoxy, $C_{1-6}$alkanoyl, $C_{1-6}$alkanoyloxy, *N*-$C_{1-6}$alkyl)amino. *N,N*-($C_{1-6}$alkyl)$_2$amino, $C_{1-6}$alkanoylamino, *N*-($C_{1-6}$alkyl)carbamoyl, *N, N*-($C_{1-6}$alkyl)$_2$carbamoyl,-*N*-($C_{1-6}$alkyl)-*N*-($C_{1-6}$alkoxy)carbamoyl, $C_{1-6}$alkylS(O)$_a$ wherein a is 0 to 2, $C_{1-6}$alkoxycarbonyl, $C_{1-6}$alkoxycarbonylamino,N-($C_{1-6}$alkyl)sulphamoyl, *N,N*-($C_{1-6}$alkyl)$_2$sulphamoyl, $C_{1-6}$alkylsulphonylamino, $C_{1-6}$alkylsulphonyl-*N*-($C_{1-6}$balkyl)amino, $C_{3-8}$cycloalkyl, aryl and heterocyclic group; wherein P, S and Q may be optionally and independently substituted on carbon by one or more groups selected from V and

wherein if said heterocyclic group contains an -NH- moiety that nitrogen may be optionally substituted by a group selected from U;

**V** is selected from halo, nitro, cyano, hydroxy, trifluoromethoxy, trifluoromethyl, amino, carboxy, carbamoyl, mercapto, sulphamoyl, methyl, ethyl, methoxy, ethoxy, acetyl, acetoxy, methylamino, ethylamino, dimethylamino, diethylamino, *N*-methyl-*N*-ethylamino, acetylamino, *N*-methylcarbamoyl, *N*-ethylcarbamoyl, *N,N*-dimethylcarbamoyl, *N,N*-diethylcarbamoyl, *N*-methyl-*N*-ethylcarbamoyl, methylthio, ethylthio, methylsulphinyl, ethylsulphinyl, mesyl, ethylsulphonyl, methoxycarbonyl, ethoxycarbonyl, *N*-methylsulphamoyl, *N*-ethylsulphamoyl, *N,N*-dimethylsulphamoyl, *N,N*-diethylsulphamoyl, *N*-methyl-*N*-ethylsulphamoyl, morpholino, morpholinocarbonyl, *N*-benzylcarbamoyl, and 4-hydroxypiperidinocarbonyl;

**R, T** and **U** are independently selected from $C_{1-4}$alkyl, $C_{1-4}$alkanoyl, $C_{1-4}$alkylsulphonyl, $C_{1-4}$alkoxycarbonyl, carbamoyl, *N*-($C_{1-4}$alkyl)carbamoyl, *N,N*-($C_{1-4}$alkyl)carbamoyl, phenyl, benzyl, benzyloxycarbonyl, benzoyl and phenylsulphonyl wherein R, T and U may be optionally and independently substituted on carbon by one or more groups selected from V;

or a pharmaceutically acceptable salt or an *in vivo* hydrolysable ester thereof; with the provisos:

i) when -X-Y-Z- is -S-CH=CH-, $R^2$-($CR^1R^3$)$_n$- cannot be amino, 1-phenyl-5-methyl-1H-1,5-benzodiazepine-2,4(3H, 5H)dion-3-yl, 1-methyl-5-phenyl-2-oxo-2,3-dihydro-1H-benzo(E)(1,4)diazepin-3-yl, 2-(4-phenyl-1,2,5,6-tehahydropyrid-1-yl)ethyl, 3-(4-phenyl-1,2,5,6-tetrahydropyrid-1-yl)propyl, 2-(4-phenylpiperazin-1-yl)ethyl, 2-(*N*-methylamino)ethyl, 2-morpholinoethyl or 2-(*N*-methyl-*N*-benzylamino)ethyl;

ii) when -X-Y-Z- is -CH=CH-S-, $R^2$-($CR^1R^3$)$_n$- cannot be amino or 1-methyl-5-phenyl-2-oxo-2,3-dihydro-1H-benzo (E)(1,4)diazepin-3-yl;

iii) when -X-Y-Z- is -CE=C($SO_2NH_2$)-S-, $R^2$-($CR^1R^3$)$_n$- cannot be methyl or isobutyl; and

iv) when -X-Y-Z- is as initially defined, n is 1, $R^1$ is arylmethyl, substituted arylmethyl, (heterocyclic group)methyl and substituted (heterocyclic group)methyl and $R^3$ is hydrogen then $R^2$ is not a group -C(=O)-A or a group -CH (OH)-C(=O)-A in which A is $NR^dR^d$, $-NR^aH_2CH_2OR^a$, or

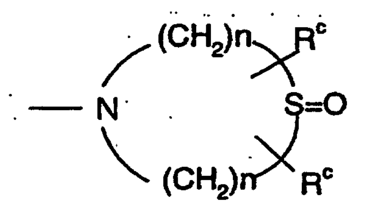 , 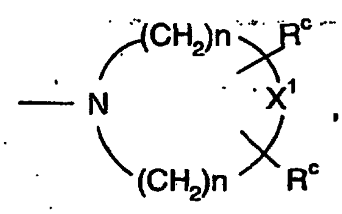 , or 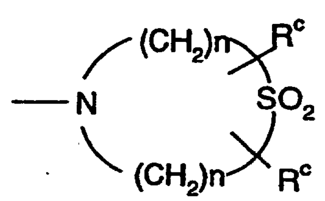 ;

each $R^a$ and $R^b$ is independently hydrogen or $-C_1$-$C_8$alkyl;
each $R^d$ is independently hydrogen, $C_1$-$C_8$alkyl, $C_1$-$C_8$alkoxy, aryl, substituted aryl, heteroaryl, or substituted heteroaryl;
each $R^c$ is independently hydrogen, $-C(=O)OR^a$, $-OR^a$, $-SR^a$, or $-NR^aR^a$; and each n is independently 1-3, and
$X^1$ is $NR^a$, $-CH_2$-, O or S.

In another aspect the present invention provides a compound of formula (**I**):

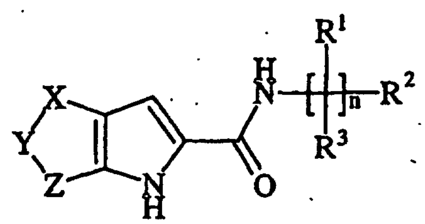

**(I)**

wherein:

**-X-Y-Z-** is selected from $-S-CR^4=CR^5-$, $-CR^4=CR^5-S-$, $-O-CR^4=CR^5-$, $-CR^4=CR^5-O-$, $-N=CR^4-S-$,$-S-CR^4N-$,

$-NR^6-CR^4=CR^5-$ and$-CR^4=CR^5-NR^6-$;

wherein $\mathbf{R^4}$ and $\mathbf{R^5}$ are independently selected from hydrogen, halo, nitro, cyano, hydroxy, fluoromethyl, difluoromethyl, trifluoromethyl, trifluoromethoxy, amino, carboxy, carbamoyl, mercapto, sulphamoyl, ureido, $C_{1-6}$alkyl, $C_{2-6}$alkenyl, $C_{2-6}$alkynyl, $C_{1-6}$alkoxy; $C_{1-6}$alkanoyl, $C_{1-6}$alkanoyloxy, *N*-($C_{1-6}$alkyl)amino, *N,N*-($C_{1-6}$alkyl)$_2$amino, $C_{1-6}$alkanoylamino, *N*-($C_{1-6}$alkyl)carbamoyl, *N,N*-($C_{1-6}$alkyl)$_2$carbamoyl, $C_{1-6}$alkylS(O)$_a$ wherein a is 0 to 2, $C_{1-6}$alkoxycarbonyl, $C_{1-6}$alkoxycarbonylamino, *N*-($C_{1-6}$alkyl)sulphamoyl, *N,N*-($C_{1-6}$alkyl)$_2$sulphamoyl, $C_{1-6}$alkylsulphonylamino and $C_{1-6}$alkylsulphonyl-*N*-($C_{1-6}$alkyl)amino;

$\mathbf{R^6}$ is hydrogen or $C_{1-6}$alkyl;

$\mathbf{R^1}$ is selected from hydrogen, halo, nitro, cyano, hydroxy, amino, carboxy, carbamoyl, mercapto, sulphamoyl, ureido, $C_{1-6}$alkyl, $C_{2-6}$alkenyl, $C_{2-6}$alkynyl, $C_{1-6}$alkoxy, $C_{1-6}$alkanoyl, $C_{1-6}$alkanoyloxy, *N*-($C_{1-6}$alkyl)amino, *N,N*-($C_{1-6}$alkyl)$_2$amino, $C_{1-6}$alkanoylamino, *N*-($C_{1-6}$alkyl)carbamoyl, *N,N* ($C_{1-4}$alkyl)$_2$carbamoyl, $C_{1-6}$alkylS(O)$_a$ wherein a is 0 to 2, $C_{1-6}$alkoxycarbonyl, $C_{1-6}$alkoxycarbonylamino, *N*-($C_{1-6}$alkyl)sulphamoyl, *N,N*-($C_{1-6}$ alkyl)$_2$sulphamoyl, $C_{1-6}$alkylsulphonylamino, $C_{1-6}$alkylsulphonyl-*N*-($C_{1-6}$alkyl)amino, $C_{3-8}$cycloalkyl, $C_{3-8}$ cycloalkyl$C_{1-6}$alkyl, aryl, aryl$C_{1-6}$alkyl, heterocyclic group and (heterocyclic group)$C_{1-6}$alkyl; wherein $R^1$ may be optionally substituted on carbon by one or more groups selected from P and wherein if said heterocyclic group contains an -NH- moiety that nitrogen may be optionally substituted by a group selected from R;

$\mathbf{R^2}$ is selected from hydrogen, halo, nitro, cyano, hydroxy, fluoromethyl, difluoromethyl, trifluoromethyl, trifluoromethoxy, amino, carboxy, carbamoyl, mercapto, sulphamoyl, ureido, $C_{1-6}$alkyl, $C_{2-6}$alkenyl, $C_{2-6}$alkynyl, $C_{1-6}$alkoxy, $C_{1-6}$alkanoyl, $C_{1-6}$alkanoyloxy, *N*-($C_{1-6}$alkyl)amino, *N,N*-($C_{1-6}$alkyl)$_2$amino, $C_{1-6}$alkanoylamino, *N*-($C_{1-6}$alkyl)carbamoyl, *N,N* ($C_{1-4}$alkyl)$_2$carbamoyl, *N*-($C_{1-6}$alkyl)-*N*-($C_{1-6}$alkoxy)carbamoyl, $C_{1-6}$alkylS(O)$_a$ wherein a is 0 to 2, $C_{1-6}$alkoxycarbonyl, $C_{1-6}$alkoxycarbonylamino, *N*-($C_{1-6}$alkyl)sulphamoyl, *N,N* ($C_{1-6}$alkyl)$_2$sulphamoyl, sulphamoylamino, *N*-($C_{1-6}$alkyl)sulphamoylamino, *N,N*-($C_{1-6}$alkyl)$_2$sulphamoylamino, $C_{1-6}$alkylsulphonylamino, $C_{1-6}$alkylsulphonylaminocarbonyl, $C_{1-6}$alkylsulphonyl-*N*-($C_{1-6}$alkyl)amino and a group -E-F-G-H;

wherein **E** and **G** are independently selected from a direct bond, -O-, -S-, -SO-, $-SO_2-$, -OC(O)-,-C(O)O-,-C(O)-, $-NR^a-$,$-NR^aC(O)-$,$-C(O)NR^a-SO_2NR^a-$,$-NR^aSO_2-$, $-NR^aC(O)NR^b-$, $-CC(O)NR^a-$, $-NR^aC(O)O-$, $-NR^aSO_2NR^b-$, $-SO_2NR^aC(O)-$ and $-C(O)NR^aSO_2-$; wherein $R^a$ and $R^b$ are independently selected from hydrogen or $C_{1-6}$alkyl;

**F** is $C_{1-6}$alkylene optionally substituted by one or more Q or a direct bond;

**H** is selected from aryl, $C_{3-8}$cycloalkyl and heterocyclic group; wherein H may be optionally substituted on carbon by one or more groups selected from S and wherein if said heterocyclic group contains an -NH- moiety that nitrogen may be optionally substituted by a group selected from T;

$\mathbf{R^3}$ is hydrogen or $C_{1-6}$alkyl;

**n** is selected from 0-4; wherein the values of $R^1$ may be the same or different; and wherein the values of $R^3$ may be the same or different;

**P, S** and **Q** are independently selected from halo, nitro, cyano, hydroxy, trifluoromethyl, trifluoromethoxy, amino, carboxy, carbamoyl, mercapto, sulphamoyl, ureido, $C_{1-6}$alkyl, $C_{2-6}$alkenyl, $C_{2-6}$alkynyl, $C_{1-6}$alkoxy, $C_{1-6}$alkanoyl, $C_{1-6}$alkanoyloxy, *N*-($C_{1-6}$alkyl)amino, *N,N*-($C_{1-6}$alkyl)$_2$amino, $C_{1-6}$alkanoylamino, *N*-($C_{1-6}$alkyl)carbamoyl, *N,N*-($C_{1-6}$alkyl)$_2$carbamoyl, *N*-($C_{1-6}$alkyl)-*N*-($C_{1-6}$alkoxy)carbamoyl, $C_{1-6}$alkylS(O)$_a$ wherein a is 0 to 2, $C_{1-6}$alkoxycarbonyl, $C_{1-6}$alkoxycarbonylamino, *N*-($C_{1-6}$alkyl)sulphamoyl, *N,N*-($C_{1-6}$alkyl)$_2$sulphamoyl, $C_{1-6}$alkylsulphonylamino, $C_{1-6}$alkylsulphonyl-*N*-($C_{1-6}$alkyl)amino, $C_{3-8}$cycloalkyl, aryl and heterocyclic group; wherein P, S and Q may be optionally and independently substituted on carbon by one or more groups selected from V and wherein if said heterocyclic group contains an -NH- moiety that nitrogen may be optionally substituted by a group selected from U;

**V** is selected from halo, nitro, cyano, hydroxy, trifluoromethoxy, trifluoromethyl, amino, carboxy, carbamoyl, mercapto, sulphamoyl, methyl, ethyl, methoxy, ethoxy, acetyl, acetoxy, methylamino, ethylamino, dimethylamino, diethylamino, *N*-methyl-*N*-ethylamino, acetylamino, *N*-methylcarbamoyl, *N*-ethylcarbamoyl, *N,N*-dimethylcarbamoyl, *N,N*-diethylcarbamoyl, *N*-methyl-*N*-ethylcarbamoyl, methylthio, ethylthio, methylsulphinyl, ethylsulphinyl, mesyl, ethylsulphonyl, methoxycarbonyl, ethoxycarbonyl, *N*-methylsulphamoyl, *N*-ethylsulphamoyl, *N,N*-dimethylsulphamoyl, *N,N*-diethylsulphamoyl or *N*-methyl-*N*-ethylsulphamoyl;

**R, T** and **U** are independently selected from $C_{1-4}$alkyl, $C_{1-4}$alkanoyl, $C_{1-4}$alkylsulphonyl, $C_{1-4}$alkoxycarbonyl, carbamoyl, *N*-($C_{1-4}$alkyl)carbamoyl, *N,N* ($C_{1-4}$alkyl)carbamoyl, phenyl, benzyl, benzyloxycarbonyl, benzoyl and phenylsulphonyl; or a pharmaceutically acceptable salt or an *in vivo* hydrolysable ester thereof; with the provisos:i) when -X-Y-Z- is -S-CH=CH-, $R^2$-($CR^1R^3$)$_n$- cannot be amino, 1-phenyl-5-methyl-1H-1,5-benzodiazepine-2,4(3H, 5H)dion-3-yl, 1-methyl-5-phenyl-2-oxo-2,3-dihydro-1H-benzo(E)(1,4)diazepin-3-yl, 2-(4-phenyl-1,2,5,6-tetrahydropyrid-1-yl)ethyl, 3-(4-phenyl-1,2,5,6-tetrahydropyrid-1-yl)propyl, 2-(4-phenylpiperazin-1-yl)ethyl, 2-(*N*-methylamino)ethyl, 2-morpholinoethyl or 2-(*N*-methyl-*N*-benzylamino)ethyl; ii)when -X-Y-Z- is -CH=CH-S-, $R^2$-($CR^1R^3$)$_n$- cannot be amino or 1-methyl-5-phenyl-2-oxo-2,3-dihydro-1H-benzo(E)(1,4)diazepin-3-yl; iii)when -X-Y-Z- is -CH=C($SO_2NH_2$)-S-, $R^2$-($CR^1R^3$)$_n$- cannot be methyl or isobutyl; and iv) when -X-Y Z- is as initially defined, n is 1, $R^1$ is arylmethyl, substituted arylmethyl,(heterocyclic group)methyl and substituted heterocyclic group) methyl

and $R^3$ is hydrogen then $R^2$ is not a group —C(=O)-A or a group-CH(OH)-C(=O)-A in which A is $NR^dR^d$, $-NR^aCH_2CH_2OR^a$, or

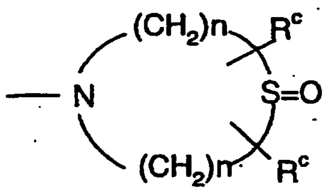 , 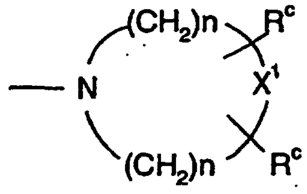 , or 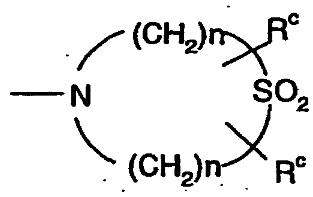 ;

each $R^a$ and $R^b$ is independently hydrogen or $-C_1-C_8$alkyl;
each $R^d$ is independently hydrogen, $C_1-C_8$alkyl, $C_1-C_8$alkoxy, aryl, substituted aryl, heteroaryl, or substituted heteroaryl;
each $R^c$ is independently hydrogen, $-C(=O)OR^a$ $-OR^a$, $-SR^a$, or $-NR^aR^a$; and each n is independently 1-3, and
$X^1$ is $NR^a$,$-CH_2-$, O or S.

In this specification the term "alkyl" includes both straight and branched chain alkyl groups but references to individual alkyl groups such as "propyl" are specific for the straight chain version only. For example, "$C_{1-6}$alkyl" includes $C_{1-4}$alkyl, propyl, isopropyl and *t*-butyl: However, references to individual alkyl groups such as 'propyl' are specific for the straight chained version only and references to individual branched chain alkyl groups such as 'isopropyl' are specific for the branched chain version only. A similar convention applies to other radicals, for example "aryl$C_{1-6}$alkyl" includes aryl$C_{1-4}$alkyl, benzyl, 1-phenylethyl and 2-phenylethyl. The term "halo" refers to fluoro, chloro, bromo and iodo.

Where optional substituents are chosen from "one or more" groups it is to be understood that this definition includes all substituents being chosen from one of the specified groups or the substituents being chosen from two or more of the specified groups.

A "heterocyclic group" is a saturated, partially saturated or unsaturated, mono or bicyclic ring containing 4-12 atoms of which at least one atom is chosen from nitrogen, sulphur or oxygen, which may, unless otherwise specified, be carbon or nitrogen linked, wherein a $-CH_2-$ group can optionally be replaced by a -C(O)-and a ring sulphur atom may be optionally oxidised to form the S-oxide(s). Examples and suitable values of the term "heterocyclic group" are morpholino, piperidyl, pyridyl, pyranyl, pyrrolyl, imidazolyl, thiazolyl, indolyl, quinolyl, thienyl, 1,3-benzodioxolyl, 1,3-dioxolanyl, thiadiazolyl, piperazinyl, isothiazolidinyl, 1,3,4-triazolyl, tetrazolyl, pyrrolidinyl, 2-oxazolidinonyl, 5-isoxazolonyl, benz-3-azepinyl, 1,4-benzodioxanyl, thiomorpholino, pyrrolinyl, homopiperazinyl, 3,5-dioxapiperidinyl, 3-pyrazolin-5-onyl, tetrahydropyranyl, benzimidazolyl, benzthiazolyl, imidazo[1,2-a]pyridyl, pyrimidyl, pyrazinyl, pyridazinyl, isoxazolyl, 4-pyridone, 1-isoquinolone, 2-pyrrolidone, 4-thiazolidone 2,3-dihydro-1,5-benzothiazepin-4(5H)-one. Preferably a "heterocyclic group" is pyridyl, imidazolyl, thiazolyl, quinolyl, thienyl, 1,3-benzodioxolyl, 1,3-dioxolanyl, isothiazolidinyl, 1,3,4-triazolyl, tetrazolyl, 2-oxazolidinonyl, 5-isoxazolonyl, benz-3-azepinyl, hydantoinyl, 1,4-benzodioxanyl, thiomorpholino, 3-pyrazolin-5-onyl, benzimidazolyl, benzthiazolyl, imidazo[1,2-a]pyridyl, pyrimidyl, pyrazinyl, and 2,3-dihydro-1,5-benzothiazepin-4(5H)-one

"Aryl" is a partially saturated or unsaturated, mono or bicyclic ring containing 4-12 carbon atoms, wherein a $-CH_2-$ group can optionally be replaced by a-C(O)-. Preferably aryl is phenyl, naphthyl, 1,2,3,4-tetrahydronaphthyl (tetralinyl) or indanyl. More preferably aryl is phenyl, naphthyl or 1,2,3,4-tetrahydronaphthyl. Most preferably aryl is phenyl, or naphthyl.

An example of "$C_{1-6}$alkanoyloxy" is acetoxy. Examples of "$C_{1-6}$alkoxycarbonyl" include $C_{1-4}$alkoxycarbonyl, methoxycarbonyl, ethoxycarbonyl, *n*- and *t*-butoxycarbonyl. Examples of "$C_{1-6}$alkoxycarbonylamino" include methoxycarbonylamino, ethoxycarbonylamino, *n*- and *t*-butoxycarbonyl amino. Examples of "$C_{1-6}$alkoxy" include methoxy, etboxy and propoxy. Examples of "$C_{1-6}$alkanoylamino" include formamido, acetamido and propionylamino. Examples of "$C_{1-6}$alkyls$(O)_a$ wherein a is 0 to 2" include $C_{1-4}$alkylsulphonyl, methylthio, ethylthio, methylsulphinyl, ethylsulphinyl, mesyl and ethylsulphonyl. Examples of "$C_{1-6}$alkylsulphonylamino" include methylsulphonylamino, ethylsulphonylamino and propylsulphonylamino. Examples of "$C_{1-6}$alkylsulphonyl-*N*-($C_{1-6}$alkyl)amino" include methylsulphonyl-*N*-methylamino, ethylsulphonyl-*N*-methylamino and propylsulphonyl-*N*-ethylamino. Examples of "$C_{1-6}$alkanoyl" include $C_{1-4}$alkanoyl, propionyl and acetyl. Examples of "*N*-($C_{1-6}$alkyl)amino" include methylamino and ethylamino. Examples of "*N*,*N*-$(C_{1-6}alkyl)_2$amino" include di-*N*-methylamino, di-(*N*-ethyl)amino and *N*-ethyl-*N*-methylamino. Examples of "$C_{2-6}$alkenyl" are vinyl, allyl and 1-propenyl. Examples of "$C_{2-6}$alkynyl" are ethynyl, 1-propynyl and 2-propynyl. Examples of "*N*-($C_{1-6}$alkyl)sulphamoyl" are *N*-(methyl)sulphamoyl and *N* (ethyl)sulphamoyl. Examples of "*N* ($C_{1-6}$alkyl)sulphamoyl" are *N*,*N*-(dimethyl)sulphamoyl and *N*-(methyl)-*N*-(ethyl)sulphamoyl. Examples of "*N*-($C_{1-6}$alkyl)carbamoyl" are *N*-($C_{1-4}$alkyl)carbamoyl, methylaminocarbonyl and ethylaminocarbonyl. Examples of *N*, *N*-$(C_{1-6}alkyl)_2$carbamoyl" are *N*,*N*-($C_{1-4}$alkyl)carbamoyl,dimethylaminocarbonyl and methylethylaminocarbonyl. Examples of "$C_{3-8}$cycloalkyl ring" are cyclopropyl and cyclohexyl. Examples of "(heterocyclic group)$C_{1-6}$alkyl" include pyridylmethyl, 3-morpholinopropyl and 2-pyrimid-2-ylethyl. Examples of "$C_{3-8}$cycloalkyl$C_{1-6}$cycloalkyl" include cyclopro-

pylmethyl and 2-cyclohexylpropyl. "*N*-($C_{1-6}$alkyl)sulphamoylamino" are *N*-(methyl)sulphamoylamino and *N*-(ethyl)sulphamoylamino. Examples of "*N*-($C_{1-6}$alkyl)sulphamoylamino" are *N*,*N*-(dimethyl)sulphamoylamino and *N*-(methyl)-*N*-(ethyl)sulphamoylamino. Examples of "$C_{1-6}$alkylsulphonylaminocarbonyl" include methylsulphonylaminocarbonyl, ethylsulphonylaminocarbonyl and propylsulphonylaminocarbonyl.

A suitable pharmaceutically acceptable salt of a compound of the invention is, for example, an acid-addition salt of a compound of the invention which is sufficiently basic, for example, an acid-addition salt with, for example, an inorganic or organic acid, for example hydrochloric, hydrobromic, sulphuric, phosphoric, trifluoroacetic, citric or maleic acid. In addition a suitable pharmaceutically acceptable salt of a compound of the invention which is sufficiently acidic is an alkali metal salt, for example a sodium or potassium salt, an alkaline earth metal salt, for example a calcium or magnesium salt, an ammonium salt or a salt with an organic base which affords a physiologically-acceptable cation, for example a salt with methylamine, dimethylamine, trimethylamine, piperidine, morpholine or tris-(2-hydroxyethyl) amine.

An *in vivo* hydrolysable ester of a compound of the formula **(I)** containing carboxy or hydroxy group is, for example, a pharmaceutically acceptable ester which is hydrolysed in the human or animal body to produce the parent acid or alcohol. Suitable pharmaceutically acceptable esters for carboxy include $C_{1-6}$alkoxymethyl esters for example methoxymethyl, $C_{1-6}$alkanoyloxymethyl esters for example pivaloyloxymethyl, phthalidyl esters, $C_{3-8}$cycloalkoxycarbonyloxy$C_{1-6}$alkyl esters for example 1-cyclohexylcarbonyloxyethyl; 1,3-dioxolen-2-onylmethyl esters for example 5-methyl-1,3-dioxolen-2-onylmethyl; and $C_{1-6}$alkoxycarbonyloxyethyl esters for example 1-methoxycarbonyloxyethyl and may be formed at any carboxy group in the compounds of this invention.

An *in vivo* hydrolysable ester of a compound of the formula **(I)** containing a hydroxy group includes inorganic esters such as phosphate esters and α-acyloxyalkyl ethers and related compounds which as a result of the *in vivo* hydrolysis of the ester breakdown to give the parent hydroxy group. Examples of α-acyloxyalkyl ethers include acetoxymethoxy and 2,2-dimethylpropionyloxy-methoxy. A selection of in *vivo* hydrolysable ester forming groups for hydroxy include alkanoyl, benzoyl, phenylacety and substituted benzoyl and phenylacetyl, alkoxycarbonyl (to give alkyl carbonate esters), dialkylcarbamoyl and *N*-(dialkylaminoethyl)-*N*-alkylcarbamoyl (to give carbamates), dialkylaminoacetyl and carboxyacetyl. Examples of substituents on benzoyl include morpholino and piperazino linked from a ring nitrogen atom via a methylene group to the 3- or 4- position of the benzoyl ring.

Some compounds of the formula **(I)** may have chiral centres and/or geometric isomeric centres (E- and Z-isomers), and it is to be understood that the invention encompasses all such optical, diastereoisomers and geometric isomers that possess glycogen phosphorylase inhibitory activity.

The invention relates to any and all tautomeric forms of the compounds of the formula **(I)** that possess glycogen phosphorylase inhibitory activity.

It is also to be understood that certain compounds of the formula **(I)** can exist in solvated as well as unsolvated forms such as, for example, hydrated forms. It is to be understood that the invention encompasses all such solvated forms which possess glycogen phosphorylase inhibitory activity.

Preferred values of $R^1$, $R^2$, $R^3$, -X-Y-Z-and n are as follows. Such values may be used where appropriate with any of the definitions, claims or embodiments defined hereinbefore or hereinafter.

Preferably -X-Y-Z- is selected from -S-$CR^4$=$CR^5$-,-$CR^4$=$CR^5$-S-,-O-$CR^4$ =$CR^5$-and -N=$CR^4$-S-,

More preferably -X-Y-Z- is selected from -S-$CR^4$=$CR^5$- and -$CR^4$-$CR^5$-S-.

In one aspect of the invention preferably -X-Y-Z- is selected from -S-$CR^4$-$CR^5$-.

In another aspect of the invention preferably -X-Y-Z- is selected from -$CR^4$=$CR^5$-S-.

Preferably $R^4$ and $R^5$ are independently selected from hydrogen, halo or $C_{1-6}$alkyl.

More preferably $R^4$ and $R^5$ are independently selected from hydrogen, chloro, bromo or methyl.

Particularly $R^4$ and $R^5$ are independently selected from hydrogen or chloro.

More particularly $R^4$ and $R^5$ are both chloro.

Preferably -X-Y-Z- is selected from -S-C(l)=C(Cl)-, -S-C(Cl)=CH , -S-CH=C(Cl)-, -S-C(Br)=CH-,-S-CH=CH-, -CH=CH-S-, -O-CH=CH- , -N=C(Me)-S- and -S-CH=CCl-.More preferably -X-Y-Z- is selected from -S-C(Cl)=C(Cl)-, -S-C(Cl)=CH-, -S-CH=C(Cl)-, -S-C(Br)=CH-, -S-CH=CH-, -CH=CH-S-, -O-CH=CH- and -N=C(Me)-S-.

Most preferably-X-Y-Z-is selected from -S-C(Cl)=C(Cl)-, -S-C(Cl)=CH and S-CH-C(Cl)-.

Particularly -X-Y-Z- is selected from-S-C(Cl)=C(Cl)-.

In one aspect of the invention, preferably $R^6$ is hydrogen.

In another aspect of the invention, preferably $R^6$ is $C_{1-6}$alkyl.

Preferably $R^1$ is selected from hydrogen, hydroxy, $C_{1-6}$alkyl, $C_{1-6}$alkoxycarbonyl, aryl$C_{1-6}$alkyl and (heterocyclic group)$C_{1-6}$alkyl; wherein $R^1$ may be optionally substituted on carbon by one or more groups selected from P; and

P is selected from hydroxy and $C_{1-6}$alkylsulphonyl-*N*-($C_{1-6}$alkyl)amino.

More preferably $R^1$ is selected from hydrogen, hydroxy, methyl, methoxycarbonyl, benzyl and imidazol-4-ylmethyl; wherein $R^1$ may be optionally substituted on carbon by one or more groups selected from P; and

P is selected from hydroxy and mesyl-*N*-(methyl)amino.

Particularly $R^1$ is selected from hydrogen, hydroxy, methyl, methoxycarbonyl, mesyl-*N*-(methyl)aminomethyl, benzyl, hydroxymethyl and imidazol-4-ylmethyl.

More particularly $R^1$ is selected from hydrogen, mesyl-*N*-(methyl)aminomethyl or benzyl.

Preferably $R^2$ is selected from *N,N*-($C_{1-4}$alkyl)$_2$carbamoyl, *N,N*-($C_{1-6}$akyl)$_2$sulphamoylamino, $C_{1-6}$alkylsulphonylaminocarbonyl and a group -E-F-G-H;

wherein E and G are independently selected from a direct bond, -O-, -S-, -C(O)-, -$NR^a$-, -C(O)$NR^a$-, -$NR^aSO_2$- and -$NR^a$C(O)O-; wherein $R^a$ is hydrogen;

F is $C_{1-6}$salkylene optionally substituted by one or more Q or a direct bond;

H is selected from aryl, $C_{3-8}$cycloalkyl and heterocyclic group; wherein H may be optionally substituted on carbon by one or more groups selected from S and wherein if said heterocyclic group contains an -NH- moiety that nitrogen may be optionally substituted by a group selected from T;

S is selected from halo, hydroxy, trifluoromethyl, sulphamoyl, ureido, $C_{1-6}$alkyl, $C_{1-6}$alkoxy, *N*,*N*-($C_{1-6}$alkyl)$_2$amino, $C_{1-6}$alkanoylamino and aryl; wherein S may be optionally substituted on carbon by one or more groups selected from V;

Q is hydroxy;

V is carbamoyl; and

T is independently selected from $C_{1-4}$alkyl or phenyl.

More preferably $R^2$ is selected from *N*,*N*-dimethylcarbamoyl, *N*,*N*-dimethylsulphamoylamino, mesylaminocarbonyl and a group -E-F-G-H;

wherein E and G are independently selected from a direct bond, -O-, -S-, -C(O)-, -$NR^a$-, -C(O)$NR^a$-, -$NR^aSO_2$- and -$NR^a$C(O)O-; wherein $R^a$ is hydrogen;

F is methylene optionally substituted by one or more Q or a direct bond;

H is selected from phenyl, naphthyl, cyclopropyl, thiomorpholino, pyridyl, thiazolyl, isothiazolyl, morpholinyl, 2,3-dihydro-1,5-benzothiazepin-4(5H)-onyl, 5-oxo-3-pyrazolinyl, 2-oxazolidinonyl, 5-hydroxy-1,3,4,5-tetrahydro-benzo[b]azepin-2-onyl, 5-oxo-2-isoxazolinyl, imidazo[1,2-a]pyridinyl, benzothiazolyl, 2,5-dioxoimidazolidinyl, pyrazinyl, pyridazinyl, imidazolyl, benzimidazolyl, tetrazolyl, quinolyl, 1,3-dioxolanyl and thienyl; wherein H may be optionally substituted on carbon by one or more groups selected from S and wherein if a heterocyclic group contains an -NH- moiety that nitrogen may be optionally substituted by a group selected from T;

S is selected from fluoro, chloro, hydroxy, trifluoromethyl, sulphamoyl,. ureido, methyl, ethyl, methoxy, *N,N* dimethylamino, acetamido and phenyl; wherein S may be optionally substituted on carbon by one or more groups selected from V;

Q is hydroxy;

V is carbamoyl; and

T is independently selected from methyl or phenyl.

Particularly $R^2$ is selected from *N,N*-dimethylcarbamoyl, *N,N*-dimethylsulphamoylamino, mesylaminocarbonyl, 2-methoxyphenyl, phenoxy, 2-phenylcyclopropyl, thien-2-yl, 4-fluorophenyl, benzoyl, thiomorpholino, anilinocarbonyl, pyrid-2-ylamino, thiazol-2-yl, benzylsulphonylamino, 2,3-dihydro-l,5-benzothiazepin-4(5H)-one-3-yl, 1-phenyl-2,3-dimethyl-5-oxo-3-pyrazolin-4-yl, 3-phenyl-2-oxazolidinon-5-yl, 5-hydroxy-1,3,4,5-tetrahydrobenzo[b]azepin-2-onyl, 3-phenyl-5-oxo-2-isoxazolin-4-yl, imidazo[1,2-a]pyridin-2-yl, benzothiazol-2-yl, 2,5-dioxoimidazolidin-3-yl, naphth-2-ylaminocarbonyl, phenyl, 4-sulphamoylphenethyl, 4-(*N*,*N*-dimethylamino)phenyl, 4-sulphamoylphenyl, anilino, 4-hydroxyphenyl, quinolin-3-yl, 4-chlorophenyl, 2-methoxypyrid-5-yl, 3-methylisothiazol-5-yl, 3-trifluoromethylpyrid-2-yl, tetrazol-5-yl, benzyloxycarbonylamino, benzimidazol-2-yl, 2-. trifluoromethylpyrid-5-yl, pyridazin-2-yl, pyridazin-3-yloxy, pyrid-2-yl, imidazol-5-yl, 4-acetamidophenoxy, 2-ureidothiazol-4-yl, benzylthio; 2-phenyl-1,3-dioxolan-2-yl, 4-carbamoylmethylphenoxy, (*N*-benzylcarbamoylmethyl), phenethyl, 3-phenylpropyl, [2-(2-hydroxyphenyl)ethyl], -($\alpha$,$\alpha$-dimethylphenethyl);(1-phenylcyclobutyl)methyl), ($\beta$-methylphenethyl), (1,2,3,4-tetrahydronaphth-2-yl), benzyl, (*N*-benzyl-*N*-methylcarbamoylmethyl), (*N*-methyl-*N*-phenylcarbamoylmethyl), [*N*-(2-cyanoethyl)-*N*-phenylcarbamoylmethyl], [*N*-(4-methoxyphenyl)carbamoylmethyl], [*N*-(4-fluorophenyl)carbamoylmethyl], [*N*-(4-nitrophenyl)carbamoylmethyl], [*N*-(2,*6*-dimethylphenyl)carbamoylmethyl], [*N*-methyl-*N*-(4-methylphenyl)carbamoylmethyl], [*N*-methyl-*N*-(3-methylphenyl)carbamoylmethyl], [*N*-(3-chlorophenyl) *N*-methylcarbamoylmethyl], [*N*-(2-hydroxyethyl)-*N*-phenylcarbamoylmethyl], [*N*-(1,1-dimethyl-2-hydroxyethyl)carbamoylmethyl], [*N*-(2-hydroxyethyl)-*N*-methylcarbamoyl-methyl], [*N*-(2-hydroxyethyl)carbamoylmethyl) , [*N*-(3-hydroxypropyl)carbamoylmethyl], [*N*-(4-hydroxybutyl)carbamoylmethyl], {*N*-[bis(hydroxymethyl)methyl]carbamoylmethyl}. [*N*-(2,3-dihydroxypropyl)carbamoylmethyl], [*N*-(4-hydroxymethylphenyl)-carbamoylmethyl], [*N*-(5-isoquinolyl)carbamoylmethyl], [*N*-(3-hydroxymethy)lphenyl]-carbamoylmethyl], {*N*-[4 (2-hydroxyethyl)phenyl]carbamoyl-methyl}, [*N*,(2,4-difluorophenyl)-*N*-methyl-carbamoylmethyl], [(1,2,3,4-tetrahydro-1-quinolyl)carbonyl-methyl], [*N*-(2-cyanoethyl)-*N*-methylcarbamoylmethyl], [*N*-(4-hydroxypiperidino)-carbamoylmethyl], (*N*-cyclopentylcarbamoylmethyl), (*N*-isopropyl-carbamoylmethyl),(*N*-isopropyl-N-methylcarbamoylmethyl), (thiomorpholin-carbonylmethyl), (morpholino-carbonylmethyl), [(1,1-dioxothiomorpholino)carbonyl-methyl], [(1-oxothiomorpholino)-carbonylmethyl], (2-indanyl), (benz[1,2]oxazol-3-ylmethyl), {2-[2-(hydroxymethyl)phenyl)-ethyl}, (4-phenylisoxazol-3-ylmethyl), {2-[2-(2-morpholino-ethoxy)phenyl]ethyl},{2-[2-(methoxycarbonyl-methoxy)phenyl]ethyl}, {2-[2-(carboxy-methoxy)phenyl]ethyl}, [2-(3-methoxyphenyl)ethyl], (2-oxo-1,2,3,4-tetrahydroquinol-3-yl), {2-[2-(2-methoxyethoxy)phenyl]ethyl}, {2-[2-(carbamoylmethoxy)phenyl]ethyl}, (2-[2-(N-methylcarbamoylmethoxy) phenyl]ethyl}, {2-[2-(N,N-dimethylcarbamoylmethoxy)phenyl]ethyl}, 2-[2-(morpholinocarbonylmethoxy)-phenyl]ethyl, 2-[2-(N-benzylcarbamoylmethoxy)phenyl]ethyl, 2-[2-(4-hydroxypiperidinocarbonylmethoxy)-phenyl]ethyl,[1-(5-ethoxycarbonyl-1,3,4-oxadiazo-2-yl)-2-phenylethyl], [1-(4-methoxycarbonyl-oxazo-5-yl)-2-phenylethyl], [2-phenylethyl-1-(pyrid-3-yl)], [2-phenylethyl-1-(3-phenyl-1,2,4,-oxadiazo-5-yl) ], (1-hydroxyindan-2-yl), [(1S,2S)-2-indan-1-ol], [(1R,2R)-2-indan-1-ol], (3-indanyl), (1-hydroxy-1,2,3,4-tetrahydronaphth 2-yl), (6-fluoro-1-hydroxyindan-2-yl), (7-methoxy-1-oxo-1,2,3,4-tetrahydronapht 2-yl), -(3-methylisoxazol-5-yl)metyl], (4-hydroxy-1,1-dioxotetrahydrothiophen-3-yl),-{*N*-metyl-*N*-[{1-oxo-1,2,3,4-tetrahydronaphth-2-yl)methyl]carbamoylmethyl}, (3-methylisoxazol-5-yl)methyl],(4-tetrahydronaphth-2-yl)methyl]carbamoylmethyl}, (3-methylisoxazol-5-yl)methyl], (4-hydroxy-1,1-dioxotetrahydrothiophen-3-yl), {*N*-methyl-N-[(1-oxo-1,2,3,4-tetrahydronaphth-2-yl)methyl]carbamoylmethyl}, (2-oxo-1,2,3,4-tetrahydroquinol-3-yl), (1,2,3,4-tetrahydroquinol-3-yl), (1-methyl-2-oxo-1,2,3,4-tetrahydroquinol-3-yl), (3-oxo-2,3,4,5-tetrahydro-1*H*-benz[2]azepin-4-yl), (1-methoxyindan-2-yl), {1-[*N*-(1,1-dimethylethoxy)carbonylamino]indan-2-yl}, (1-aminoindan-2-yl)], (1-acetamidoindan-2-yl), [1-(methanesulphonamido)indan-2-yl], [1-(methylamino)indan-2-yl], or [1-(*N*-methylacetamido)indan-2-yl].

More particularly $R^2$ is selected from *N,N*-dimethylcarbamoyl, *N,N*-dimethylsulphamoylamino, mesylaminocarbonyl, 2-methoxyphenyl, phenoxy, 2-phenylcyclopropyl, thien-2-yl, 4-fluorophenyl, benzoyl, thiomorpholino, anilinocarbonyl, pyrid-2-ylamino, thiazol-2-yl, benzylsulphonylamino, 2,3-dihydro-1,5-benzothiazepin-4(5H)-one-3-yl, 1-phenyl-2,3-dimethyl-5-oxo-3-pyrazolin4-yl,3-phenyl-2-oxazolidinon-5-yl, 5-hydroxy-1,3,4,5-tetrahydro-benzo[b]azepin-2-onyl, 3-pitenyl-5-oxo-2-isoxazolin-4-yl. imidazo[1,2-a]pyridin-2-yl,benzothiazol-2-yl, 2,5-dioxoimidazolidin-3-yl, naphth-2-ylaminocarbonyl, phenyl, 4-sulphamoylphenethyl, 4-(N,N-dimethylamino)phenyl, 4-sulphamoylphenyl, anilino, 4-hydroxyphenyl, quinolin-3-yl, 4-chlorophenyl, 2-methoxypyrid-5-yl, 3-methylisothiazol-5-yl, 3-trifluoromethylpyrid-2-yl, tetrazol-5-yl, benzyloxycarbonylamino, benzimidazol-2-yl, 2-trifluoromethylpyrid-5-yl, pyridazin-2-yl, pyridazin-3-yloxy, pyrid-2-yl, imidazol-5-yl, 4-acetamidophenoxy, 2-ureidothiazol-4-yl, benzylthio, 2-phenyl-1,3-dioxolan-2-yl and 4-carbamoylmethylphenoxy.

Most particularly $R^2$ is selected from *N,N*-dimethylcarbamoyl, phenoxy, 2-phenylcyclopropyl, thien-2-yl, 4-fluorophenyl, benzoyl, thiomorpholino, anilinocarbonyl, pyrid-2-ylamino or thiazol-2-yl.

Preferably $R^3$ is hydrogen.

Preferably n is selected from 0-3; wherein the values of $R^1$ may be the same or different; and wherein the values of $R^3$ may be the same or different.

More preferably n is selected from 0-2; wherein the values of $R^1$ may be the same or different; and wherein the values of $R^3$ may be the same or different.

In one aspect of the invention preferably n is 2; wherein the values of $R^1$ may be the same or different; and wherein the values of $R^3$ may be the same or different.

In one aspect of the invention preferably n is 1.

In one aspect of the invention preferably n is 0.

In another aspect of the invention, preferred compounds of the invention are any one of the Examples or a pharmaceutically acceptable salt or an *in vivo* hydrolysable ester thereof.

In another aspect the present invention provides a compound of formula (**I**) (as depicted above) wherein:

-X-Y-Z- is selected from $-S-CR^4=CR^5-$, $-CR^4=CR^5-S-$, $-O-CR^4=CR^5-$ and $-N=CR^4-S-$;
$R^4$ and $R^5$ are independently selected from hydrogen, halo or $C_{1-6}$alkyl;
$R^1$ is selected from hydrogen, hydroxy, $C_{1-6}$alkyl, $C_{1-6}$alkoxycarbonyl, aryl$C_{1-6}$alkyl and (heterocyclic group) $C_{1-6}$alkyl; wherein $R^1$ may be optionally substituted on carbon by one or more groups selected from P; and
P is selected from hydroxy and $C_{1-6}$alkylsulphonyl-*N*-($C_{1-6}$alkyl)amino;
$R^2$ is selected from *N,N* ($C_{1-4}$alkyl)$_2$Carbamoyl, *N,N*-($C_{1-6}$alkyl)$_2$sulphamoylamino, $C_{1-6}$alkylsulphonylaminocarbonyl and a group -E-F-G-H;
wherein E and G are independently selected from a direct bond, -O-, -S-, -C(O)-, $-NR^a-$, $-C(O)NR^a-$, $-NR^aSO_2-$ and $-NR^aC(O)O-$; wherein $R^a$ is hydrogen;
F is $C_{1-6}$alkylene optionally substituted by one or more Q or a direct bond;
H is selected from aryl, $C_{3-8}$cycloalkyl and heterocyclic group; wherein H may be optionally substituted on carbon by one or more groups selected from S and wherein if said heterocyclic group contains an -NH- moiety that nitrogen may be optionally substituted by a group selected from T;
S is selected from halo, hydroxy, trifluoromethyl, sulphamoyl, ureido, $C_{1-6}$alkyl, $C_{1-6}$alkoxy, *N,N*-($C_{1-6}$alkyl)$_2$amino, $C_{1-6}$alkanoylamino and aryl; wherein S may be optionally substituted on carbon by one or more groups selected from V;
Q is hydroxy;

V is carbamoyl; and
T is independently selected from $C_{1-4}$alkyl or phenyl;
$R^3$ is hydrogen;
n is selected from 0-3; wherein the values of $R^1$ may be the same or different; or a pharmaceutically acceptable salt or an *in vivo* hydrolysable ester thereof; with the provisos:

i) when -X-Y-Z- is -S-CH=CH-, $R^2$-$(CR^1R^3)_n$- cannot be 1-phenyl-5-methyl-1H-1,5-benzodiazepine-2,4(3H, 5H)dion-3-yl, 1-methyl-5 phenyl-2-oxo-2,3-dihydro-1H-benzo(E)(1,4)diazepin-3-yl,2-(4-phenyl-1,2,5,6-tetrahydropyrid-1-yl)ethyl,3-(4-phenyl-1,2,5,6-tetrahydropyrid-1-yl)propyl,2-(4-phenylpiperazin-1-yl)ethyl or 2-morpholinoethyl;
ii) when -X-Y-Z- is -CH=CH-S-, $R^2$-$(CR^1R^3)_n$- cannot be 1-methyl-5-phenyl-2-oxo-2,3-dihydro-1H-benzo(E)(1,4)diazepin-3-yl;and
iii) when -X-Y-Z- is as initially defined, n is 1, $R^1$ is arylmethyl, substituted arylmethyl, (heterocyclic group)methyl and substituted (heterocyclic group)methyl and $R^3$ is hydrogen then $R^2$ is not a group -C(=O)-A or a group -CH(OH)-C(=O)-A in which A is $NR^dR^d$,-$NR^aCH_2CH_2OR^a$, or

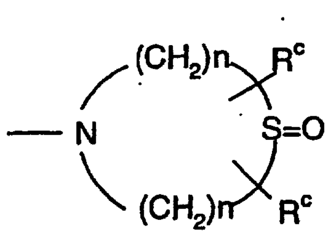 , 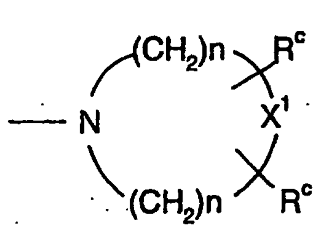 , or 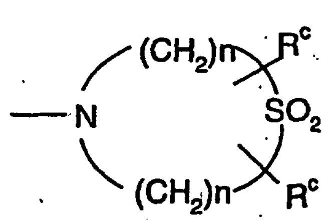 ;

each $R^a$ and $R^b$ is independently hydrogen or -$C_1$-$C_8$alkyl;
each $R^d$ is independently hydrogen, $C_1$-$C_8$alkyl, $C_1$-$C_8$alkoxy, aryl, substituted aryl, heteroaryl, or substituted heteroaryl;
each $R^c$ is independently hydrogen, -C(=O)$OR^a$, -$OR^a$, -$SR^a$, or -$NR^aR^a$; and each n is independently 1-3, and
$X^1$ is $NR^a$, -$CH_2$-, O or S.

In yet another aspect the present invention provides a compound of formula **(I)** (as depicted above) wherein:

-X-Y-Z is selected from -S-C(Cl)=C(Cl)-, -S-C(Cl)=CH-, -S-CH=C(Cl)-, -S-C(Br)=CH-, -S-CH=CH-, -CH=CH-S-, -O-CH=CH- and -N=C(Me)-S-;
$R^1$ is selected from hydrogen, hydroxy, methyl, methoxycarbonyl, mesyl-*N*-(methyl)aminomethyl and hydroxymethyl;
$R^2$ is selected from *N*,*N*-dimethylcarbamoyl, *N*,*N*-dimethylsulphamoylamino, mesylaminocarbonyl, 2-methoxyphenyl, phenoxy, 2-phenylcyclopropyl, thien-2-yl, 4-fluorophenyl, benzoyl, thiomorpholino, anilinocarbonyl, pyrid-2-ylamino, thiazol-2-yl, benzylsulphonylamino, 2,3-dihydro-1,5-benzothiazepin-4(5H)one-3-yl, 1-phenyl-2,3-dimethyl-5-oxo-3-pyrazolin-4-yl, 3-phenyl-2-oxazolidinon-5-yl, 5-hydroxy-1,3,4,5-tetrahydrobenzo[B]azepin-2-onyl, 3-phenyl-5-oxo-2-isoxazolin-4-yl, imidazo[1,2-a]pyridin-2-yl, benzothiazol-2-yl, 2,5-ditoxoimidazolidin-3-yl, naphth-2-ylaminocarbonyl, phenyl, 4-sulphamoylphenethyl, 4-(*N*,*N*-dimethylamino)phenyl, 4-sulphamoylphenyl, anilino, 4-hydroxyphenyl, quinolin-3-yl, 4-chlorophenyl, 2-methoxypyrid-5-yl, 3-methylisothiazol-5-yl, 3-trifluoromethylpyrid-2-yl, tetrazol-5-yl, benzyloxycarbonylamino, benzimidazol-2-yl, 2-triffuoromethylpyrid-5-yl, pyridazin-2-yl, pyndazin-3-yloxy, pyrid-2-yl, imidazol-5-yl, 4-acetamidophenoxy, 2-ureidothiazol-4-yl, benzylthio, 2-phenyl-1,3-dioxolan-2-yl and 4-carbamoylmethylphenoxy;
$R^3$ is hydrogen; and
n is selected from 0-3; wherein the values of $R^1$ may be the same or different; or a pharmaceutically acceptable salt or an *in vivo* hydrolysable ester thereof.

In a first preferred aspect the present invention provides a compound of formula (**I**) (as depicted above) wherein:

-X-Y-Z- is selected from -S-$CR^4$=$CR^5$- or -$CR^4$=$CR^5$-S-;
wherein $R^4$ and $R^5$ are independently selected from hydrogen, halo, nitro, cyano, hydroxy, fluoromethyl, difluoromethyl, trifluoromethyl, trifluoromethoxy, amino, carboxy, carbamoyl, mercapto, sulphamoyl, ureido, $C_{1-6}$alkyl, $C_{2-6}$alkenyl, $C_{2-6}$alkynyl, $C_{1-6}$alkoxy, $C_{1-6}$alkanoyl, $C_{1-6}$alkanoyloxy, *N*-($C_{1-6}$alkyl)amino, *N,N*-$(C_{1-6}alkyl)_2$amino, $C_{1-6}$alkanoylamino, *N*-($C_{1-6}$alkyl)carbamoyl, *N,N* $(C_{1-6}alkyl)_2$carbamoyl, $C_{1-6}alkylS(O)_a$ wherein a is 0 to 2, $C_{1-6}$alkoxycarbonyl, $C_{1-6}$alkoxycarbonylamino, *N*-($C_{1-6}$alkyl)sulphamoyl, *N,N*-$(C_{1-6}alkyl)_2$sulphamoyl, $C_{1-6}$alkyl-

sulphonylamino and $C_{1-6}$alkylsulphonyl-*N*-($C_{1-6}$alkyl)amino;

n is 0;

$R^2$ is a group -E-F-G-H;

wherein E, F and G are each a direct bond;

H is a $C_{3-12}$cycloalkyl which is optionally fused to a benz ring wherein H may be optionally substituted on carbon by one or more groups S which are independently selected from halo, nitro, cyano, hydroxy, trifluoromethyl, trifluoromethoxy, amino, carboxy, carbamoyl, mercapto, sulphamoyl, ureido, $C_{1-6}$alkyl, $C_{2-6}$alkenyl, $C_{2-6}$alkynyl, $C_{1-6}$alkoxy, $C_{1-6}$alkanoyl, $C_{1-6}$alkanoyloxy, *N*-($C_{1-6}$alkyl)amino, *N*,*N*-($C_{1-6}$alkyl)$_2$amino, $C_{1-6}$alkanoylamino, *N*-($C_{1-6}$alkyl)$_2$carmoyl, *N*-($C_{1-6}$alkyl)-*N*-($C_{1-6}$alkoxy)carbamoyl, $C_{1-6}$alkylS(O)$_a$ wherein a is 0 to 2, $C_{1-6}$alkoxycarbonyl, $C_{1-6}$alkoxycarbonylamino, *N*-($C_{1-6}$alkyl)sulphamoyl, *N,N*-($C_{1-6}$alkyl)$_2$sulphamoyl, $C_{1-6}$alkylsulphonylamino, $C_{1-6}$alkylsulphonyl-*N*-($C_{1-6}$alkyl)amino, $C_{3-8}$cycloalkyl, aryl and heterocyclic groups; wherein S may be optionally substituted on carbon by one or more groups selected from V;

V is selected from halo, nitro, cyano, hydroxy, trifluoromethoxy, trifluoromethyl, amino, carboxy, carbamoyl, mercapto, sulphamoyl, methyl, ethyl, methoxy, ethoxy, acetyl, acetoxy, methylamino, ethylamino, dimethylamino, diethylamino, *N*-methyl-*N*-ethylamino, acetylamino, *N*-methylcarbamoyl, *N*-ethylcarbamoyl, *N*,*N*-dimethylcarbamoyl, *N*,*N*-diethylcarbamoyl, *N*-methyl-*N*-ethylcarbamoyl, methylthio, ethylthio, methylsulphinyl, ethylsulphinyl, mesyl, ethylsulphonyl, methoxycarbonyl, ethoxycarbonyl, *N*-methylsulphamoyl, *N*-ethylsulphamoyl, *N*,*N*-dimethylsulphamoyl, *N*,*N*-diethylsulphamoyl, *N*-methyl-*N*-ethylsulphamoyl, morpholino , morpholinocarbonyl, *N*-benzylcarbamoyl, and 4-hydroxypiperidinocarbonyl;

or a pharmaceutically acceptable salt thereof.

Preferred values of $R^2$, $R^4$, and $R^5$ are as follows. Such values may be used where appropriate with any of the definitions, claims or embodiments defined hereinbefore or hereinafter.

In this first preferred aspect preferably $R^4$ and $R^5$ are independently selected from hydrogen, halo or $C_{1-6}$alkyl.

In this first preferred aspect preferably H is indanyl, 1,2,3,4-tetrahydronaphthyl or cyclopropyl. More preferably H is indanyl or 1,2,3,4-tetrahydronaphthyl. Most preferably H is indanyl.

In this first preferred aspect preferably S is independently selected from from halo, nitro, cyano, hydroxy, amino, carboxy, carbamoyl, $C_{1-6}$alkyl, $C_{1-6}$alkoxy, $C_{1-6}$alkanoyl, $C_{1-6}$alkanoyloxy, *N*-($C_{1-6}$alkyl)amino, *N*, *N*-($C_{1-6}$alkcyl)$_2$amino, $C_{1-6}$alkanoylamino, *N*-($C_{1-6}$alkyl)carbamoyl, *N,N*-($C_{1-6}$alkyl)$_2$carbamoyl, *N*-($C_{1-6}$alkyl) -*N*-($C_{1-6}$alkoxy)carbamoyl, $C_{1-6}$alkylS(O)$_a$ wherein a is 0 to 2, $C_{1-6}$alkoxycarbonyl, $C_{1-6}$alkoxycarbonylamino, $C_{3-8}$cycloalkyl and aryl. More preferably S is selected from hydroxy; amino, $C_{1-6}$alkoxy and $C_{1-6}$alkoxycarbonylamino.

In a second preferred aspect the present invention provides a compound of formula (**I**) (as depicted above) wherein:

-X-Y-Z- is selected from-S-CR$^4$=CR$^5$- or -CR$^4$=CR$^5$-S-;

wherein $R^4$ and $R^5$ are independently selected from hydrogen, halo, nitro, cyano, hydroxy, fluoromethyl, difluoromethyl, trifluoromethyl, trifluoromethoxy, amino, carboxy, carbamoyl, mercapto, sulphamoyl, ureido, $C_{1-6}$akyl, $C_{2-6}$alkenyl, $C_{2-6}$alkynyl, $C_{1-6}$alkoxy, $C_{1-6}$alkanoyl, $C_{1-6}$alkanoyloxy, *N*-($C_{1-6}$alkyl)amino, *N,N*-($C_{1-6}$alkyl)$_2$amino, $C_{1-6}$alkanoylamino, *N*-($C_{1-6}$alkyl)carbamoyl,*N,N*-($C_{1-6}$alkyl)$_2$carbamoyl, $C_{1-6}$alkylS(O)$_a$ wherein a is 0 to 2, $C_{1-6}$alkoxycarbonyl, $C_{1-6}$alkoxycarbonylamino, *N*-($C_{1-6}$alkyl)sulphamoyl, *N,N*-($C_{1-6}$alkyl)$_2$sulphamoyl, $C_{1-6}$alkylsulphonylamino and $C_{1-6}$alkylsulphonyl-*N*-($C_{1-6}$alkyl)amino;

n is 0;

$R^2$ is a group -E-F-G-H;

wherein E, F and G are each a direct bond; and

H is a cyclic amide of formula

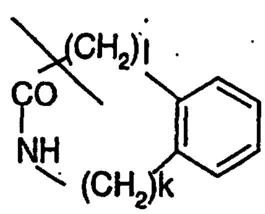

in which k is 0, 1, 2 or 3 and 1 is 0, 1, 2 or 3 such that the sum of k and 1 is 2 or 3 and wherein one of the carbon atoms governed by k or 1 may be replaced by sulphur and wherein H is optionally substituted on carbon by one

or more groups selected from S and may be independently optionally substituted on nitrogen by a group selected from T;

S is selected from halo, nitro, cyano, hydroxy, trifluoromethyl, trifluoromethoxy, amino, carboxy, carbamoyl, mercapto, sulphamoyl, ureido, $C_{1-6}$alkyl, $C_{2-6}$alkenyl, $C_{2-6}$alkynyl, $C_{1-6}$alkoxy,$C_{1-6}$alkanoyl, $C_{1-6}$alkanoyloxy, *N*-($C_{1-6}$ alkyl)amino, *N,N* ($C_{1-6}$alkyl)$_2$amino, $C_{1-6}$alkanoylamino, *N*-($C_{1-6}$alkyl)carbamoyl, *N,N*-($C_{1-6}$alkyl)$_2$carbamoyl, *N*-($C_{1-6}$alkyl)-*N*-($C_{1-6}$alkoxy)carbamoyl, $C_{1-6}$alkylS(O)$_a$ wherein a is 0 to 2, $C_{1-6}$alkoxycarbonyl, $C_{1-6}$alkoxycarbonylamino, *N*-($C_{1-6}$alkyl)sulphamoyl, *N,N*-($C_{1-6}$alkyl)$_2$sulphamoyl, $C_{1-6}$alkylsulphonylamino, $C_{1-6}$alkylsulphonyl-*N*-($C_{1-6}$alkyl)amino, $C_{3-8}$cycloalkyl, aryl and heterocyclic group; wherein S may be optionally and independently substituted on carbon by one or more groups selected from V and wherein if said heterocyclic group contains an -NH- moiety that nitrogen may be optionally substituted by a group selected from U;

T and U are independently selected from $C_{1-4}$akyl, $C_{1-4}$alkanoyl,$C_{1-4}$alkylsulphonyl, $C_{1-4}$alkoxycarbonyl, carbamoyl, *N*-($C_{1-4}$alkyl)carbamoyl, *N,N*-($C_{1-4}$alkyl)carbamoyl, phenyl, benzyl, benzyloxycarbonyl, benzoyl and phenylsulphonyl wherein T and U may be optionally and independently substituted on carbon by one or more groups selected from V;

V is selected from halo, nitro, cyano, hydroxy, trifluoromethoxy, trifluoromethyl, amino, carboxy, carbamoyl, mercapto, sulphamoyl, methyl, ethyl, methoxy, ethoxy, acetyl, acetoxy, methylamino, ethylamino, dimethylamino, diethylamino, *N*-methyl-*N*-ethylamino, acetylamino, *N*-methylcarbamoyl, *N*-ethylcarbamoyl, *N,N*-dimethylcarbamoyl, *N,N*-diethylcarbamoyl, *N*-methyl-*N*-ethylcarbamoyl, methylthio, ethylthio, methylsulphinyl, ethylsulphinyl, mesyl, ethylsulphonyl, methoxycarbonyl, ethoxycarbonyl, *N*-methylsulphamoyl, *N*-ethylsulphamoyl, *N,N*-dimethylsulphamoyl, *N,N*-diethylsulphamoyl, *N*-methyl-*N*-ethylsulphamoyl, morpholino, morpholinocarbonyl, *N*-benzylcarbamoyl and 4-hydroxypiperidinocarbonyl ;

or a pharmaceutically acceptable salt or an *in vivo* hydrolysable ester thereof.

Preferred values of $R^4$, $R^5$ and H are as follows. Such values may be used where appropriate with any of the definitions, claims or embodiments defined hereinbefore or hereinafter.

In this second preferred aspect preferably $R^4$ and $R^5$ are independently selected from hydrogen, halo or $C_{1-6}$alkyl.

In this second preferred aspect preferably H is 1,2,3,4-tetrahydroquinolyl, 2-oxo-1,2,3,4-tetrahydroquinolyl, 4-oxo-2,3,4,5-tetrahydrobenz[1,5]thiazepin-3-yl, 2-oxo-2,3,4,5-tetrahydro-1H-benz[*b*]azepinyl, 2,3,4,5-tetrahydro-1H-benz[*b*]azepinyl or 3-oxo-2,3,4,5-tetrahydro-1H-benz[*c*]azepinyl each optionally substituted on carbon by one or more groups selected from S wherein S is selected from hydroxy, $C_{1-6}$alkyl or $C_{1-6}$alkoxy, and each independently optionally substituted on nitrogen by a group selected from T wherein T is selected from $C_{1-4}$alkyl or $C_{1-4}$alkanoyl.

More preferably H is 2-oxo-1,2,3,4-tetrahydroquinol-3-yl, 1-methyl-2-oxo-1,2,3,4-tetrahydroquinol-3-yl, 4-oxo-2,3,4,5-tetrahydrobenz[1,5]thiazepin-3-yl, 5-hydroxy-2-oxo-2,3,4,5-tetrahydro-1H-benz[*b*]azepin-4-yl, 2-oxo-2,3,4,5-tetrahydro-1H-benz[*b*]azepin-3-yl or 3-oxo-2,3,4,5-tetrahydro-1H-benz[*c*]azepin-4-yl.

In a third preferred aspect the present invention provides a compound of formula (**I**) (as depicted above) wherein:

-X-Y-Z- is selected from -S-$CR^4$=$CR^5$- or -$CR^4$=$CR^5$-S-; wherein $R^4$ and $R^5$ are independently selected from hydrogen, halo or $C_{1-6}$alkyl.

n is 1;

$R^1$ is hydrogen or aryl$C_{1-6}$alkyl;

$R^2$ is selected from a group -E-F-G-H;

wherein E, F and G are each a direct bond;

H is an unsaturated five membered heterocyclic group containing at least one nitrogen atom and one or two ring atoms selected from oxygen and sulphur and wherein H may be optionally substituted on carbon by one or more groups S which are independently selected from halo, nitro, cyano, hydroxy, trifluoromethyl, trifluoromethoxy, amino, carboxy, carbamoyl, mercapto, sulphamoyl, ureido, $C_{1-6}$alkyl, $C_{2-6}$alkenyl, $C_{2-6}$alkynyl, $C_{1-6}$alkoxy, $C_{1-6}$alkanoyl, $C_{1-6}$alkanoyloxy, *N*-($C_{1-6}$alkyl)amino, *N,N*-($C_{1-6}$alkyl)$_2$amino, $C_{1-6}$alknoylamino, *N*-($C_{1-6}$alkyl)carbamoyl, *N*-*N*-($C_{1-6}$alkyl)$_2$carbamoyl, *N*-($C_{1-6}$alkyl)-*N*-($C_{1-6}$alkoxy)carbamoyl, $C_{1-6}$alkylS(O)$_a$ wherein a is 0 to 2, $C_{1-6}$alkoxycarbonyl, $C_{1-6}$alkoxycarbonylamino, *N*-($C_{1-6}$alkyl)sulphamoyl, *N,N*-($C_{1-6}$alkyl)$_2$sulphamoyl, $C_{1-6}$alkylsulphonylamino, $C_{1-6}$alkylsulphonyl-*N*-($C_{1-6}$alkyl)amino, $C_{3-8}$cycloalkyl and aryl groups;

$R^3$ is hydrogen or $C_{1-6}$alkyl;

or a pharmaceutically acceptable salt thereof.

Preferred values of $R^1$, $R^3$ and H are as follows. Such values may be used where appropriate with any of the definitions, claims or embodiments defined hereinbefore or hereinafter.

In this third preferred aspect preferably $R^1$ is selected from hydrogen or benzyl and more preferably benzyl.

In this third preferred aspect preferably $R^3$ is hydrogen.

In this third preferred aspect preferably H is 1,3,4-oxadiazolyl, isoxazolyl, oxazolyl or 1,2,4-oxadiazolyl. More preferably H is 5-ethoxycarbonyl-1,3,4-oxadiazol-2-yl, 4-phenylisoxazol-3-yl, 3-phenyl-1,2,4-oxadiazol-5-yl, 4-methoxycarbonyloxazol-5-yl or 3-methylisoxazol-5-yl.

In this third preferred aspect preferably H may be optionally substituted on carbon by one or more groups S which are independently selected from halo, carboxy, $C_{1-6}$alkyl, $C_{1-6}$alkoxy, $C_{1-6}$alkanoyloxy, *N*-($C_{1-6}$alkyl)amino, *N*,*N*-($C_{1-6}$alkyl)$_2$amino, $C_{1-6}$alkynoylamino, $C_{1-6}$alkoxycarbonyl, $C_{3-8}$cycloalkyl and aryl groups. Preferably S is $C_{1-6}$alkoxy, $C_{1-6}$alkoxycarbonyl or phenyl

In a fourth preferred aspect the present invention provides a compound of formula (I) (as depicted above) wherein:

-X-Y-Z-is selected from -S-$CR^4$=$CR^5$- or -$CR^4$=$CR^5$-S-;
wherein $R^4$ and $R^5$ are independently selected from hydrogen, halo or $C_{1-6}$alkyl.
n is 0;
$R^2$ is a group -E-F-G-H;
wherein E is a direct bond;
F is methylene;
wherein G is -C(O)$NR^a$-, wherein $R^a$ is selected from hydrogen or $C_{1-6}$alkyl which is optionally substituted by a group V ;
H is aryl which may be optionally substituted on carbon by one or more groups selected from S;
S is selected from halo, nitro, cyano, hydroxy, trifluoromethyl, trifluoromethoxy, amino, carboxy, carbamoyl, mercapto, sulphamoyl, ureido, $C_{1-6}$alkyl, $C_{2-6}$alkenyl, $C_{2-6}$alkynyl, $C_{1-6}$alkoxy, $C_{1-6}$alkanoyl, $C_{1-6}$alkanoyloxy, *N*-($C_{1-6}$alkyl)amino, *N,N*-($C_{1-6}$alkyl)$_2$amino, $C_{1-6}$alkanoylamino, *N*-($C_{1-6}$alkyl)carbamoyl, *N,N*-($C_{1-6}$alkyl)$_2$carbamoyl, *N*-($C_{1-6}$alkyl)-*N*-($C_{1-6}$alkoxy)carbamoyl, $C_{1-6}$alkylS(O)$_a$ wherein a is 0 to 2, $C_{1-6}$alkoxycarbonyl, $C_{1-6}$alkoxycarbonylamino, *N*-($C_{1-6}$alkyl)sulphamoyl, *N,N*-($C_{1-6}$alkyl)$_2$sulphamoyl, $C_{1-6}$alkylsulphonylamino, $C_{1-6}$alkylsulphonyl-*N*-($C_{1-6}$alkyl)amino, $C_{3-8}$cycloallryl, aryl and heterocyclic group; wherein S may be optionally and independently substituted on carbon by one or more groups selected from V ;
V is selected from halo, nitro, cyano, hydroxy, trifluoromethoxy, trifluoromethyl, amino, carboxy, carbamoyl, mercapto, sulphamoyl, methyl, ethyl, methoxy, ethoxy, acetyl, acetoxy, methylamino, ethylamino, dimethylamino, diethylamino, *N*-methyl-*N*-ethylamino, acetylamino, *N*-methylcarbamoyl, *N*-ethylcarbamoyl, *N*,*N*-dimethyicarbamoyl, *N*,*N*-diethylcarbamoyl, *N*-methyl-*N*-ethylcarbamoyl, methylthio, ethylthio, methylsulphinyl, ethylsulphinyl, mesyl, ethylsulphonyl, methoxycarbonyl, ethoxycarbonyl, *N*-methylsulphamoyl, *N*-ethylsulphamoyl, *N*,*N*-dimethylsulphamoyl, *N*,*N*-diethylsulphamoyl, *N*-methyl-*N*-ethylsulphamoyl, morpholino, morpholinocarbonyl, *N*-benzylcarbamoyl , and 4-hydroxypiperidinocarbonyl,

or a pharmaceutically acceptable salt thereof.

Preferred values of $R^1$, $R^2$, $R^3$, -X-Y-Z-and n are as follows. Such values may be used where appropriate with any of the definitions, claims or embodiments defined hereinbefore or hereinafter.

In this fourth preferred aspect preferably H is aryl.

In this fourth preferred aspect preferably V is cyano or hydroxy.

Specific compounds of the present invention are:

2,3-dichloro-5-[*N*-(2-phenoxyethyl)carbamoyl]-4*H*-thieno[3,2-*b*]pyrrole;
2,3-dichloro-5-{*N*-[2-,(2-thienyl)ethyl]carbainoyl }-4*H*-thieno[3,2-*b*]pyrrole;
2,3-dichloro-5-{*N*-[2-(2-methoxyphenyl)ethyl]carbamoyl}-4*H*-thieno[3,2-*b*]pyrrole;
2,3-dichloro-5-[*N*-(2-phenyl-1-cyclopropyl)carbamoyl)-4*H*-thieno[3,2-*b*]pyrrole;
2,3-dichloro-5-{*N*-[2-(4-fluorophenyl)ethyl]carbamoyl}-4*H*-thieno[3,2-b]pyrrole;
2,3-dichloro-5-[*N*-{*N*-phenylcarbamoylmethyl)carbamoyl]-4*H*-thieno[3,2-*b*]pyrrole;
2,3-dichloro-5-(*N*-{2-[(2-pyridyl)amino]ethyl}carbamoyl)-4*H*-thieno[3,2-*b*]pyrrole;
2,3-dichloro-5-{*N*-[2-(*N*-methylmethanesulphonamido)-1-(thiazol-2-yl)ethyl]carbamoyl}-4*H*-Ehieno[3,2-*b*]pyrrole;
2,3-dichloro-5-{*N*-[2-(thiomorpholino)ethyl]carbamoyl}-4*H*-thieno[3,2-*b*]pyrrole;
5-[*N*-(benzoylmethyl)carbamoyl]-2,3-dichloro-4*H*-thieno[3,2-*b*]pyrrole;
3-chloro-5-[*N*-(*N*-phenylcarbamoylmethyl)carbamoyl]-4*H*-thieno[3,2-b]pyrrole;
3-chloro-5-{*N*-[2-(thiomorpholino)ethyl]carbamoyl}-4*H*-thieno[3,2-*b*]pyrrole;
3-chloro-5-{*N*-[2-(*N*-methylmethanesulphonamido)-1-(thiazol-2-yl)ethyl]carbamoyl}-4*H*-thieno[3,2-*b*]pyrrole;
3-chloro-5-[*N*-(benzoylmethyl)carbamoyl]-4*H*-thieno[3,2-*b*]pyrrole;
3-chloro-5-(*N*-[2-(2-methoxyphenyl)ethylcarbamoyl)-4*H*-thieno[3,2-*b*]pyrrole;
3-chloro-5-{*N*-[2-(2-thienyl)ethyl]carbamoyl}-4*H*-thieno[3,2-*b*]pyrrole;

3-chloro-5-[*N*-(2-phenyl-1-cyclopropyl)carbamoyl]-4*H*-thieno[3,2-*b*]pyrrole;
3-chloro-5-{*N*-[2-(4-fluorophenyl)ethyl]carbamoyl)-4*H*-thieno[3,2-*b*]pyrrole;
3-chloro-5-[*N*-(2-phenoxyethyl)carbamoyl]-4*H*-thieno[3,2-*b*]pyrrole;
3-chloro-5-{*N*-[2-(1-phenylmethanesulphonamido)ethyl]carbamoyl}-4*H*-thieno[3,2-*b*]pyrtole;
3-chloro-5-[*N*-(4-oxo-2,3,4,5-tetrahydrobenz[1,5]thiazepin-3-yl)carbamoyl]-4*H*-thieno[3,2-*b*]pyrrole;
2-chloro-5-[*N*-(benzoylmethyl)carbamoyl]-4*H*-thieno[3,2-*b*]pyrrole;
2-choro-5-([*N*-(2phenyl-1-cyclopropyl)carbamoyl]-4*H*-thieno[3,2-*b*]pyrrole;
2-chloro-5-[*N*-(*N*-phenylcarbamoylmethyl)carbamoyl]-4*H*-thieno[3,2-*b*]pyrrole;
2-chloro-5-(*N*-{2-[(2-pyridyl)amino]ethyl }carbamoyl)-4*H*-thieno[3,2-*b*]pyrrole;
2-chloro-5-(*N*-[2-(2-methoxyphenyl)ethyl]carbamoyl}-4*H*-thieno[3,2-*b*]pyrrole;
2-chloro-5-[*N*-(2-phenoxyethyl)carbamoyl]-4*H*-thieno[3,2-*b*]pyrrole;
2-chloro-5-{*N*-[2-(2-thienyl)ethyl]carbamoyl}-4*H*-thieno[3,2-*b*]pyrrole;
2-chloro-5-{*N*-[2-(4-fluorophenyl)ethyl]carbamoyl}-4*H*-thieno[3,2-*b*]pyrrole;
2-chloro-5-{*N*-[2-(*N*-methylmethanesulphonamido)-1-(thiazol-2-yl)ethyl]carbamoyl}-4*H*-thieno[3,2-*b*]pyrrole;
2-chloro-5-{*N*-[2-(thiomorpholino)ethyl]carbamoyl}-4*H*-thieno[3,2-*b*]pyrrole;
2,3-dichloro-5-[*N*-(2,3-dimethyl-5-oxo-1-phenyl-2,5-dihydro-1*H*-pyrazol-4-yl)carbamoyl]-4*H*-thieno[3,2-*b*]pyrrole;
2,3-dichloro-5-[*N*-(4-sulphamoylphenylmethyl)carbamoyl]-4*H*-thieno[3,2-*b*]pyrrole;
2,3-dichloro-5-[*N*-(2-hydroxy-1-phenethyl)carbamoyl]-4*H*-thieno[3,2-*b*]pyrrole;
2,3-dichloro-5-{*N*-*(2*-[(3-trifluoromethylpyrid-2-yl)amino]ethyl)carbamoyl}-4*H*-thieno[3,2-*b*]pyrrole;
2,3-dichloro-5-{*N*-[3-(5-tetrazolyl)propyl]carbamoyl}-4*H*-thieno[3,2-b]pyrrole;
2,3-dichloro-5-[*N*-(5-oxo-3-phenyl-4,5-dihydroisoxazol-4-yl)carbamoyl]-4*H*-thieno[3,2-*b*]pyrrole;
2,3-dichloro-5-[*N*-(5-hydroxy-2-oxo-2,3,4,5-tetrahydro-1*H*-benz[*b*]azepin-4-yl)carbamoyl]-4*H*-thieno[3,2-b]pyrrole;
2-chloro-5-{N-[3-(benzyloxycarbonylamino)propyl]carbamoyl)-4*H*-thieno[3,2]pyrrole;
2,3-dichloro-5-{*N*-[(4-dimethylaminophenyl)methyl]carbamoyl}-4*H* thieno[3,2-b]pyrrole;
5-[*N*-(1-benzyl-2-hydroxyethyl)carbamoyl]-2,3-dichloro-4*H*-thieno[3,2-*b*]pyrrole;
2,3-dichloro-5-{*N*-[2-(phenylamino)ethyl]carbamoyl}-4*H*-thieno[3,2-*b*]pyrrole;
2,3-dichloro-5-[*N*-(β-*(R)*-hydroxy-α methylphenethyl)carbamoyl]-4*H*-thieno[3,2-*b*]pyrrole;
2,3-dichloro-5-[*N*-(N-(β-hydroxyphenethyl)carbamoyl)-4*H*-thieno[3,2-*b*]pyrrole;
2,3-dichloro-5-{*N*-[2-(4-hydroxyphenyl)ethyl}carbamoyl}4*H*-thieno[3,2-*b*]pyrrole;
2,3-dichloro-5-{*N*-[(benzimidazol-2-yl)methyl]carbamoyl}-4*H*-thieno[3,2-*b*]pyrrole;
2,3-dichloro-5-{*N*-[2-(4-chlorophenyl)-2-hydroxy-1-(methoxycarbonyl)ethyl]carbamoyl}-4*H*-thieno[3,2-*b*]pyrrole;
2,3-dichloro-5-{*N*-(imidazo[1,2-*a*]pyrid-2 yl)carbamoyl}-4*H*-thieno[3,2-*b*]pyrrole;
5-{*N*-[(benzthiazol-2-yl)methyl]carbamoyl}-2,3-dichloro-4*H*-thieno[3,2-*b*]pyrrole;
2,3-dichloro-5-{*N*-(2-[ (2-pyridazinyl)methyl]carbamoyl}-4*H*-thieno[3,2-*b*]pyrrole;
2,3-dichloro-5-{*N* [*N*-(2-hydroxy-3-phenoxypropyl)carbamoylmethyl]carbamoyl}-4*H*-thieno[3,2-*b*]pyrrole;
2,3-dichloro-5-{*N*-[*N*-(3-methylisothiazol-5-yl)carbamoylmethyl]carbamoyl}4*H*-thieno[3,2-*b*]pyrrole;
2,3-dichloro-5-{*N*-[2-(pyridazin-3-yloxy)ethyl]carbamoyl)-4*H*-thieno[3,2-*b*]pyrrole;
2-chloro-5-(*N*-{2-[(3-trifluoromethylpyrid-2-yl)amino]ethyl}carbamoyl}-4*H*-thieno[3,2-*b*]pyrrole;
2,3-dichloro-5-{*N*-[2-(4-sulphamoylphenyl)ethyl]carbamoyl}-4*H*-thieno[3,2-*b*]pyrrole;
2,3-dichloro-5-{*N*-[2-(2-pyridyl)ethyl]carbamoyl}-4*H*-thieno[3,2-*b*]pyrrole;
2,3-dichloro-5-{*N*-(2-[1-hydroxymethyl-2-(4-imidazolyl)ethyl]carbamoyl}-4*H*-thieno[3,2-*b*]pyrrole;
2,3-dichloro-5-{*N* (2-[(3-quinolyl)methyl]carbamoyl}-4*H*-thieno[3,2-*b*]pyrrole;
5-{*N* [3-(4-acetamidophenoxy)-2-hydroxypropyl]carbamoyl}-2,3-dichloro-4*H*-thieno[3,2-b]pyrrole;
2,3-dichloro-5-{*N*-[3-(*N*-methylsulphonylcarbamoyl)propyl]carbamoyl}-4*H*-thieno[3,2-*b*]pyrrole;
2,3-dichloro-5-[*N*-(2-{ [2-(guanidino)thiazol-4-yl]methylthio}ethyl)carbamoyl]-4*H*-thieno[3,2-*b*]pyrrole;
2,3-dichloro-5-(*N* [2-(2,4-dioxoimidazolidin-1-yl)ethyl]carbamoyl}-4*H*-thieno[3,2-*b*]pyrrole;
5-{*N*-[2-benzylthio-1-(hydroxymethyl)ethyl]carbamoyl}-2,3-dichloro-4*H*-thieno[3,2-*b*]pyrrole;
2,3-dichloro-5-{*N*-[2-(dimethyaminosulphonylamino)ethyl]carbamoyl}-4*H*-thieno[3,2-*b*]pyrrole;
2.3-dichloro-5-{*N*-[(6-methoxypyrid-3-yl)methyl]carbamoyl)-4*H*-thieno[3,2-*b*]pyrrole;
(*S*)-2,3-dichloro-5-{*N*-[(2-oxo-3-phenyl-2,3,4,5-tetrahydrooxazol-5-yl)methyl]carbamoyl }-4*H*-thieno[3,2-*b*]pyrrole;
2,3-dichloro-5-(*N*-{2-[3-(carbamoylmethyl)phenoxy]ethyl}carbamoyl)-4*H*-thieno[3,2-*b*]pyrrole;
5-(*N*-{[6-(benzo[ 1,3]dioxal-5-yl)-4-methylmorpholin-2-yl]methyl }carbamoyl)-2,3-dichloro-4*H*-thieno[3,2-b]pyrrole;
5-(*N*-benzylcarbamoyl)-2,3-dichloro-4*H*-thieno[3,2-*b*] pyrrole,
2,3-dichloro-5-(*N*-phenethylcarbamoyl}-4*H*-thieno[3,2-b]pyrrole;
2,3-dichloro-5-[*N*-(3-phenylpropyl)carbamoyl]-4*H*-thieno[3,2-*b*]pyrrole;
2,3-dichloro-5-{*N* [2-(2-hydroxyphenyl)ethyl]carbamoyl}-4*H*-thieno[3,2-*b*]pyrrole;

2,3-dichloro-5-[*N*-(α,α-dimethylphenethyl)carbamoyl]-4*H*-thieno[3,2-*b*]pyrrole;
2,3-dichloro-5-[*N*-(1-phenylcyclobutyl)methyl)carbamoyl]-4*H*-thieno[3,2-*b*]pyrrole;
2,3-dichloro-5-[*N*-(β-methylphenethyl)carbamoyl]-4*H*-thieno[3,2-*b*]pyrrole;
2,3-dichloro-5-[*N*-( 1,2,3,4-tetrahydronaphth-2-yl)carbamoyl]-4*H*-thieno[3,2-*b*]pyrrole;
*5*-[*N-(N*-benzylcarbamoylmethyl)carbamoyl]-2,3-dichloro-4*H*-thieno[3,2-*b*]pyrrole;
*5*-[*N*-(*N*-benzyl-*N*-methylcarbamoylmethyl)carbamoyl]-2,3-dichloro-4*H*-thieno[3,2-*b*]pyrrole;
2,3-dichloro-5-[*N*-(*N*-methyl-*N*-phenylcarbamoylmethyl)carbamoyl]-4*H*-thieno[3,2-*b*]pyrrole;
2,3-dichloro-5-{*N*-[*N*-(2-cyanoethyl)-*N*-phenylcarbamoylmethyl]carbamoyl}-4*H*-thieno[3,2-*b*]pyrrole;
2,3-dichloro-5-{*N*-[*N*-(4-methoxyphenyl)carbamoylmethyl]carbamoyl}-4*H* thieno[3,2-*b*]pyrrole;
2,3-dichloro-5-{*N*-[*N*-(4-ffuorophenyl)carbamoylmethyl]carbamoyl}-4*H*-thieno[3,2-*b*]pyrrole;
2,3-dichloro-5-{*N*-[*N*-(4-nitrophenyl)carbamoylmethyl]carbamoyl}-4*H*-thieno[3,2-*b*]pyrrole;
2,3-dichloro-5-{*N*-{*N*-(2,6-dimethylphenyl)carbamoylmethyl]carbamoyl}-4*H*-thieno[3,2-*b*]pyrrole;
2,3-dichloro-5-{*N*-[*N*-methyl-*N*-(4-methylphenyl)carbamoylmethyl]carbamoyl}-4*H*-thieno[3,2-*b*]pyrrole;
2,3-dichloro-5-{*N*-[*N*-methyl-*N*-(3-methylphenyl)carbamoylmethyl]carbamoyl]-4*H*-thieno[3,2-*b*]pyrrole;
2,3-dichloro-5-{*N*-[*N*-(3-chlorophenyl) *N*-methylcarbamoylmethyl]carbamoyl)-4*H*-thieno[3,2-*b*]pyrrole;
2,3-dichloro-5-{*N*-[*N*-(2-hydroxyethyl)-*N*-phenylcarbamoylmethyl]carbamoyl}-4*H*-thieno[3,2-*b*]pyrrole;
2,3-dichloro-5-{*N-[N*-{1,1-dimethyl-2-hydroxyethyl)carbamoylmethyl]carbamoyl}-4*H*-thieno[3,2-*b*]pyrrole;
2,3-dichloro-5-{*N*-[*N*-(2-hydroxyethyl)-*N*-methylcarbamoylmethyl]carbamoyl}-4*H* thieno[3,2-*b*]pyrrole;
2,3-dichloro-5-{*N*-[*N*-(2-hydroxyethyl)carbamoylmethyl]carbamoyl}-4*H*-thieno[3,2-*b*]pyrrole;
2,3-dichloro-5-{*N*-[*N*-(3-hydroxypropyl)carbamoylmethyl]carbamoyl)-4*H*-thieno[3,2-*b*]pyrrole;
2,3-dichloro-5-{*N*[*N*-(4-hydroxybutyl)carbamoylmethyl]carbamoyl}-4*H* thieno[3,2-*b*]pyrrole;
2,3-dichloro-5-(*N*-{*N*-[bis(hydroxymethyl)methyl]carbamoylmethyl}carbamoyl)-4*H*-thieno[3,2-*b*]pyrrole;
2,3-dichloro-5-{*N*-[*N*-(2,3-dihydroxypropyl)carbamoylmethyl]carbamoyl}-4*H*-thieno[3,2-*b*]pyrrole;
2,3-dichloro-5-{*N*-[*N*-(4-hydroxymethylphenylcarbamoylmethyl]carbamoyl}-4*H*-thieno[3,2-*b*]pyrrole;
2,3-dichloro-5-{*N*-[*N*-(5-isoquinolyl)carbamoylmethyl]carbamoyl}-4*H*-thieno[3,2-*b*]pyrrole;
2,3-dichloro-5-{*N*-[*N*-(3-hydroxymethyl)phenyl]carbamoylmethyl]carbamoyl}-4*H*-thieno[3,2-*b*]pyrrole
2,3-dichloro-5-(*N*-{*N*-[4-(2-hydroxyethyl)phenyl]carbamoylmethyl}carbamoyl)-4*H*-thieno[3,2-*b*]pyrrole;
2,3-dichloro-5-{*N*-[*N*-(2,4-difluorophenyl)-*N*-methyl-carbamoylmethyl]carbamoyl}-4*H*-thieno[3,2-*b*]pyrrole;
2,3-dichloro-5-{*N*-[(1,2,3,4-tetrahydro-1-quinolyl)carbonylmethyl]carbamoyl}-4*H*-thieno[3,2-*b*]pyrrole;
2,3-dichloro-5-{*N*-[*N*-(2-cyanoethyl)-*N*-methylcarbamoylmethyl]carbamoyl)-4*H*-thieno[3,2-*b*]pyrrole;
2,3-dichloro-5-{*N*-[*N*-(4-hydroxypiperidino)carbamoylmethyl]carbamoyl}-4*H*-thieno[3,2-*b*)pyrrole;
2,3-dichloro-5-[*N*-(*N*-cyclopentylcarbamoylmethyl)carbamoyl]-4*H*-thieno[3,2-*b*]pyrrole;
2,3-dichloro-5-[*N*-(*N*-isopropylcarbamoylmethyl)carbamoyl]-4*H*-thieno[3,2-*b*]pyrrole;
2,3-dichloro-5-[*N*-(*N*-isopropyl-*N*-methylcarbamoylmethyl)carbamoyl]-4*H*-thieno[3,2-*b*]pyrrole;
2,3-dichloro-5-[*N*-(thiomorpholinocarbonylmethyl)carbamoyl]-4*H*-thieno[3,2-*b*]pyrrole;
2,3-dichloro-5-[*N*-(morpholinocarbonylmethyl)carbamoyl]-4*H*-thieno[3,2-*b*]pyrrole;
2,3-dichloro-5-{*N*-[(1,1-dioxothiomorpholino)carbonylmethyl]carbamoyl}-4*H*-thieno[3,2*b*]-pyrrole;
2,3-dichloro-5-{*N*-[(1-oxothiomorpholino)carbonylmethyl]carbamoyl}-4*H*-thieno[ 3,2-*b*]pyrrole;
2-chloro-5-[*N*-(2-indanyl)carbamoyl]-6*H*-thieno[2,3-*b*]pyrrole;
5-[*N*-(benz[1,2]oxazol-3-ylmethyl)carbamoyl]-2,3-dichloro-4*H*-thieno[3,2-*b*]pyrrole;
2,3-dichloro-5-(*N*-{2-[2-(hydroxymethyl)phenyl]ethyl}carbamoyl)-4*H*-thieno[3,2-*b*]pyrrole;
2,3-dichloro-5-[*N*-(4-phenylisoxazol-3-ylmethyl)carbamoyl]-4H-thieno[3,2-b]pyrrole;
2,3-dichloro-5-(*N*-{2-[2-(2-morpholinoethoxy)phenyl]ethyl}carbamoyl)-4*H*-thieno[3,2-*b*]pyrrole;
2,3-dichloro-5-(*N*-{2-[2-(methoxycarbonylmethoxy)phenyl]ethyl}carbamoyl)-4*H*-thieno[3,2-*b*]pyrrole;
5-(*N*-{2-[2-(carboxymethoxy)phenyl]ethyl}carbamoyl}-2,3-dichloro-4*H*-thieno[3,2-*b*]pyrrole;
2,3-dichloro-5-{*N*-[2-(3-methoxyphenyl)ethyl]carbamoyl}-4*H*-thieno[3,2-*b*]pyrrole;
2,3-dichloro-5-[*N*-(2-oxo-1,2,3,4-tetrahydroquinol-3-yl)carbamoyl]-4*H*-thieno[3,2-*b*]pyrrole;
2,3-dichloro-5-(*N*-{2-[2-(2-methoxyethoxy)phenyl]ethyl}carbamoyl)-4*H*-thieno[3,2-*b*]pyrrole;
5-(*N*-{2-[2-(carbamoylmethoxy)phenyl]ethyl}carbamoyl)-2,3-dichloro-4*H*-thieno[3,2-*b*]pyrrole;
2,3-dichloro-5-(*N*-{2-[2-(*N*-methylcarbamoylmethoxy)phenyl]ethyl}carbamoyl)-4*H*-thieno[3,2-*b*]pyrrole;
2,3-dichloro-5-(*N*-{2-[2-(*N*,*N*-dimethylcarbamoylmethoxy)phenyl]ethyl }carbamoyl)-4*H*-thieno[3,2-*b*]pyrrole;
2,3-dichloro-5-(*N*-(2-[2-(morpholinocarbonylmethoxy)phenyl]ethyl)carbamoyl)-4*H*-thieno[3,2-*b*]pyrrole;
5-(*N*-(2-[2-(*N*-benzylcarbamoylmethoxy)phenyl]ethyl)carbamoyl)-2,3-dichloro-4*H*-thieno[3,2-*b*]pyrrole;
2,3-dichloro-5-(*N*-{2-[2-(4-hydroxypiperidinocarbonylmethoxy)phenyl]ethyl}carbamoyl)-4*H*-thieno[3,2-*b*]pyrrole;
(*S*)-2-chloro-5-{*N*-[α-(5-ethoxycarbonyl-1,3,4-oxadiazol-2-yl)phenethyl]carbamoyl}-6*H*-thieno[2,3-*b*]pyrrole;
(*S*)-2-chloro-5-{*N*-[α(4-methoxycarbonyloxazol-5-yl)phenethyl]carbamoyl}-6*H*-thieno[2,3-*b*]pyrrole;
2-chloro-5-{*N*-[α-(3-pyridyl)phenethyl]carbamoyl}-6*H*-thieno[2,3-*b*]pyrrole;
2,3-dichloro-5-{*N*-[α-(3-pyridyl)phenethyl)]carbamoyl)-4*H*-thieno[3,2-*b*]pyrrole;

(*S*)-2-chloro-5-{*N*-[α-(3-phenyl-1,2,4-oxadiazol-5-yl)phenethyl]carbamoyl}-6*H*-thieno[2,3-*b*]pyrrole;
2,3-dichloro-5-[*N*-(1-hydroxyindan-2-yl)carbamoyl]-4*H*-thieno[3,2-*b*]pyrole;
2,3-dichloro-5-[*N* ((1S,2S)-1-hydroxyindan-2-yl)carbamoyl]-4*H*-thieno[3,2-*b*]pyrrole;
2,3-dichloro-5-[*N*-((*1R*,*2R*)-1-hydroxyindan-2-yl)carbamoyl]-4*H*-thieno[3,2-*b*]pyrrole;
2-chloro-5-[*N*-(1-hydroxyindan-2-yl)carbamoyl]-6*H*-thieno[2,3-*b*]pyrrole;
2,3-dichloro-5-[*N*-(1-hydroxy-1,2,3,4-tetrahydronaphth-2-yl)carbamoyl]-4*H*-thieno[3,2-*b*]pyrrole;
2,3-dichloro-5-[*N*-(6-fluoro-1-hydroxyindan-2 yl)carbamoyl]-4*H*-thieno[3,2-*b*]pyrole;
2,3-dichloro-5-[*N*-(7-methoxy-1-oxo-1,2,3,4-tetrahydronaphth-2-yl)carbamoyl]-4*H*-thieno[3,2-b]pyrrole;
2,3-dichloro-5-[*N*-(2-indanyl)carbamoyl]-4*H*-thieno[3,2-*b*]pyrrole; 2,3-dichloro-5-[*N*-(3-methylisoxazol-5-yl)methyl] carbamoyl]-4*H*-thieno[3,2-*b*]pyrrole;
2,3-dichloro-5-[*N*-(4-hydroxy-1,1-dioxotetrahydrothiophen-3-yl)carbamoyl]-4*H* thieno[3,2-*b*]pyrrole;
2,3-dichloro-5-(*N*-{*N*methyl-*N*-[(1-oxo-1,2,3,4-tetrahydronaphth-2-yl)methyl]carbamoylmethyl}carbamoyl)-4*H*-thieno[3,2-*b*]pyrrole;
2-chloro-5-[*N*-(2-oxo-1,2,3,4-tetrahydroquinol-3-yl)carbamoyl)-6*H* thieno[2,3-b]pyrrole;
2-chloro-5-[*N* (1,2,3,4-tetrahydroquinol-3-yl)carbamoyl]-6*H* thieno[2,3-*b*]pyrrole;
2-chloro-5-[*N*-(1-methyl-2-oxo-1,2,3,4-tetrahydroquinol-3-yl)carbamoyl]-6*H* thieno[2,3-*b*]pyrrole;
2-chloro-5-[*N*-(3-oxo-2,3,4,5-tetrahydro-1*H*-benz[2]azepin-4-yl)carbamoyl]-6*H*-thieno[2,3-*b*]pyrrole;
2,3-dichloro-5-[*N*-(1-methoxyindan-2-yl)carbamoyl]-4*H* thieno[3,2-*b*]pyrrole;
2,3-dichloro-5-(*N* {1-[*N*-(1,1-dimethylethoxy)carbonylamino]indan-2-yl}carbamoyl)-4*H*-thieno[3,2-*b*]pyrrole;
5-[*N*-(1-aminoindan-2-yl)carbamoyl]-2,3-dichloro-4*H*-thieno[3,2-*b*]pyrrole;
5-[*N*-(1-acetamidoindan-2-yl)carbamoyl]-2,3-dichloro-4*H*-thieno[3,2-*b*]pyrrole;
2,3-dichloro-5-{N-[1-(methanesulphonamido)indan-2-yl]carbamoyl}-4*H*-thieno[3,2-b]pyrrole;
2,3-dichloro-5-{*N*-[1-(methylamino)indan-2-yl]carbamoyl}-4*H*-thieno[3,2-*b*]pyrrole; and
2,3-dichloro-5-{*N* [1-(*N*-methylacetamido)indan-2-yl]carbamoyl}-4*H*-thieno[3,2-*b*]pyrrole.

Preferred aspects of the invention are those which relate to the compound of formula **(I)** or a pharmaceutically acceptable salt thereof.

Another aspect of the present invention provides a process for preparing a compound of formula (**I**) or a pharmaceutically acceptable salt or an *in vivo* hydrolysable ester thereof which process (wherein $R^1$, $R^2$, $R^3$, -X-Y-Zrand n are, unless otherwise specified, as defined in formula (**I**)) comprises of:

a) reacting an acid of the formula (II):

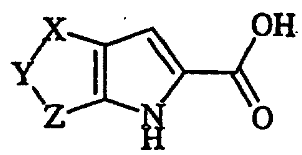

**(II)**

or an activated derivative thereof; with an amine of formula (III):

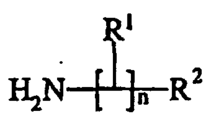

**(III)**

and thereafter if necessary:

i) converting a compound of the formula (**I**) into another compound of the formula (**I**);
ii) removing any protecting groups;
iii) forming a pharmaceutically acceptable salt *or in vivo* hydrolysable ester.

Specific reaction conditions for the above reaction are as follows.

*Process a)* Acids of formula **(II)** and amines of formula **(III)** may be coupled together in the presence of a suitable coupling reagent. Standard peptide coupling reagents known in the art can be employed as suitable coupling reagents, or for example carbonyldiimidazole, 1-ethyl-3-(3-dimethylaminopropyl)carbodi-imide hydrochloride and dicyclohexyl-carbodiimide, optionally in the presence of a catalyst such as 1-hydroxybenzotriazole, dimethylaminopyridine or 4-pyrrolidinopyridine, optionally in the presence of a base for example triethylamine, di-isopropylethylamine, pyridine, or 2,6-di-*alkyl*-pyridines such as 2,6-lutidine or 2,6-di-*tert*-butylpyridine. Suitable solvents include dimethylacetamide, dichloromethane, benzene, tetrahydrofuran and dimethylformamide. The coupling reaction may conveniently be performed at a temperature in the range of -40 to 40°C.

Suitable activated acid derivatives include acid halides, for example acid chlorides, and active esters, for example pentafluorophenyl esters. The reaction of these types of compounds with amines is well known in the art, for example they may be reacted in the presence of a base, such as those described above, and in a suitable solvent, such as those described above. The reaction may conveniently be performed at a temperature in the range of -40 to 40°C.

The acids of formula (**II**) may be prepared according to *Scheme 1:*

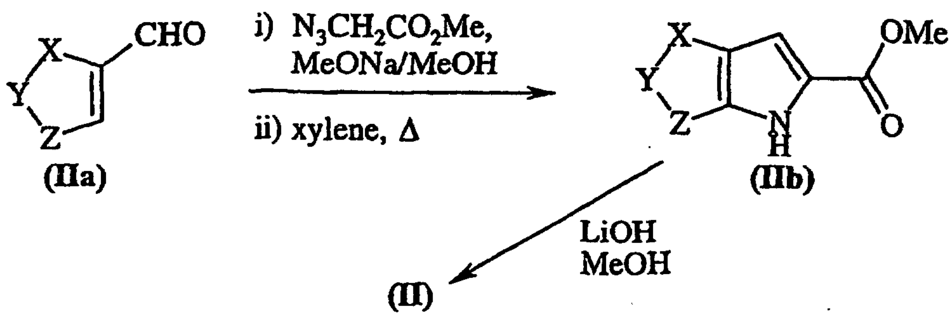


*Scheme 1*

Compounds of formula (**IIa**) and amines of formula (**III**) are commercially available or they are known compounds or they are prepared by processes known in the art.

It will be appreciated that certain of the various ring substituents in the compounds of the present invention may be introduced by standard aromatic substitution reactions or generated by conventional functional group modifications either prior to or immediately following the processes mentioned above, and as such are included in the process aspect of the invention. Such reactions and modifications include, for example, introduction of a substituent by means of an aromatic substitution reaction, reduction of substituents, alkylation of substituents and oxidation of substituents. The reagents and reaction conditions for such procedures are well known in the chemical art. Particular examples of aromatic substitution reactions include the introduction of a nitro group using concentrated nitric acid, the introduction of an acyl group using, for example, an acyl halide and Lewis acid (such as aluminium trichloride) under Friedel Crafts conditions; the introduction of an alkyl group, using an alkyl halide and Lewis acid (such as aluminium trichloride) under Friedel Crafts conditions; and the introduction of a halogeno group. Particular examples of modifications include the reduction of a nitro group to an amino group by for example, catalytic hydrogenation with a nickel catalyst or treatment with iron in the presence of hydrochloric acid with heating; oxidation of alkylthio to alkylsulphinyl or alkylsulphonyl.

It will also be appreciated that in some of the reactions mentioned herein it may be necessary/desirable to protect any sensitive groups in the compounds. The instances where protection is necessary or desirable and suitable methods for protection are known to those skilled in the art. Conventional protecting groups may be used in accordance with standard practice (for illustration see T.W. Green, Protective Groups in Organic Synthesis, John Wiley and Sons, 1991). Thus, if reactants include groups such as amino, carboxy or hydroxy it may be desirable to protect the group in some of the reactions mentioned herein.

A suitable protecting group for an amino or alkylamino group is, for example, an acyl group, for example an alkanoyl group such as acetyl, an alkoxycarbonyl group, for example a methoxycarbonyl, ethoxycarbonyl or t-butoxycarbonyl group, an arylmethoxycarbonyl group, for example benzyloxycarbonyl, or an aroyl group, for example benzoyl. The deprotection conditions for the above protecting groups necessarily vary with the choice of protecting group. Thus, for example, an acyl group such as an alkanoyl or alkoxycarbonyl group or an aroyl group may be removed for example, by hydrolysis with a suitable base such as an alkali metal hydroxide, for example lithium or sodium hydroxide. Alternatively an acyl group such as a *t*-butoxycarbonyl group may be removed, for example, by treatment with a suitable acid as hydrochloric, sulphuric or phosphoric acid or trifluoroacetic acid and an arylmethoxycarbonyl group such as a

benzyloxycarbonyl group may be removed, for example, by hydrogenation over a catalyst such as palladium-on-carbon, or by treatment with a Lewis acid for example boron tris(trifluoroacetate). A suitable alternative protecting group for a primary amino group is, for example, a phthaloyl group which may be removed by treatment with an alkylamine, for example dimethylaminopropylamine, or with hydrazine.

A suitable protecting group for a hydroxy group is, for example, an acyl group, for example an alkanoyl group such as acetyl, an aroyl group, for example benzoyl, or an arylmethyl group, for example benzyl. The deprotection conditions for the above protecting groups will necessarily vary with the choice of protecting group. Thus, for example, an acyl group such as an alkanoyl or an aroyl group may be removed, for example, by hydrolysis with a suitable base such as an alkali metal hydroxide, for example lithium or sodium, hydroxide. Alternatively an arylmethyl group such as a benzyl group may be removed, for example, by hydrogenation over a catalyst such as palladium-on-carbon.

A suitable protecting group for a carboxy group is, for example, an esterifying group, for example a methyl or an ethyl group which may be removed, for example, by hydrolysis with a base such as sodium hydroxide, or for example a *t*-butyl group which may be removed, for example, by treatment with an acid, for example an organic acid such as trifluroacetic acid, or for example a benzyl group which may be removed, for example, by hydrogenation over a catalyst such as palladium-on-carbon.

The protecting groups may be removed at any convenient stage in the synthesis using conventional techniques well known in the chemical art.

As stated hereinbefore the compounds defined in the present invention possesses glycogen phosphorylase inhibitory activity. This property may be assessed, for example, using the procedure set out below.

## Assay

The activity of the compounds is determined by measuring the inhibitory effect of the compounds in the direction of glycogen synthesis, the conversion of glucose-1-phosphate into glycogen with the release of inorganic phosphate, as described in EP 0 846 464 A2. The reactions were in 96well microplate format in a volume of 100µl. The change in optical density due to inorganic phosphate formation was measured at 620nM in a Labsystems iEMS Reader MF by the general method of (Nordlie R.C and Arion WJ, Methods of Enzymology, 1966, 619-625). The reaction is in 50mM HEPES, 2.5mM $MgCl_2$, 2.25mM ethylene glycol-bis(b-aminoethyl ether) *N,N,N;N',* tetraacetic acid, 100mM KCl, 2mM D-(+)-glucose pH7.2, containing 0.5mM dithiothreitol, the assay buffer solution, with 0.1mg type III glycogen, 0.15ug glycogen phosphorylase *a (*GP*a)* from rabbit muscle and 0.5mM glucose-1-phosphate. GPa is pre-incubated in the assay buffer solution with the type III glycogen at 2.5 mg $ml^{-1}$ for 30 minutes. 40µl of the enzyme solution is added to 25µl assay buffer solution and the reaction started with the addition of 25µl 2mM glucose-1-phosphate. Compounds to be tested are prepared in 10µl 10% DMSO in assay buffer solution, with final concentration of 1% DMSO in the assay. The non-inhibited activity of GPa is measured in the presence of 10µl 10% DMSO in assay buffer solution and maximum inhibition measured in the presence of 30µM CP320626 (Hoover et al (1998) J Med Chem 41, 2934-8; Martin et al (1998) PNAS 95, 1776-81). The reaction is stopped after 30min with the addition of 50µl acidic ammonium molybdate solution, 12ug $ml^{-1}$ in 3.48% $H_2SO_4$ with 1% sodium lauryl sulphate and 10ug $ml^{-1}$ ascorbic acid. After 30 minutes at room temperature the absorbency at 620nm is measured.

The assay is performed at a test concentration of inhibitor of 10µM or 100µM. Compounds demonstrating significant inhibition at one or both of these concentrations may be further evaluated using a range of test concentrations of inhibitor to determine an $IC_{50}$, a concentration predicted to inhibit the enzyme reaction by 50%.

Activity is calculated as follows:-

$$\% \text{ inhibition} = (1 - (\text{compound OD620} - \text{fully inhibited OD620}) / (\text{non-inhibited rate OD620} - \text{fully inhibited OD620})) * 100.$$

OD620 = optical density at 620nM.

Typical $IC_{50}$ values for compounds of the invention when tested in the above assay are in the range 100µM to 1nM.

The activity of the compounds is alternatively determined by measuring the inhibitory effect of the compounds on glycogen degradation, the production of glucose-1-phosphate from glycogen is monitored by the multienzyme coupled assay, as described in EP 0 846 464 A2, general method of Pesce et al ( Pesce, M A, Bodourian, S H, Harris, R C, and Nicholson, J F (1977) Clinical Chemistry 23, 1171- 1717). The reactions were in 384well microplate format in a volume of 50µl. The change in fluorescence due to the conversion of the co-factor NAD to NADH is measured at 340nM excitation, 465nm emission in a Tecan Ultra Multifunctional Microplate Reader. The reaction is in 50mM HEPES, 3.5mM $KH_2PO_4$,2.5mM $MgCl_2$, 2.5mM ethylene glycol-bis(b-aminoethyl ether) *N,N,N,N'*-tetraacetic acid, 100mM KCl,

8mM D-(+)-glucose pH7.2, containing 0.5mM dithiothreitol, the assay buffer solution. Human recombinant liver glycogen phosphorylase *a* (hrl GPa) 20nM is pre-incubated in assay buffer solution with 6.25mM NAD, 1.25mg type III glycogen at 1.25 mg $ml^{-1}$ the reagent buffer, for 30 minutes. The coupling enzymes, phosphoglucomutase and glucose-6-phosphate dehydrogenase ( Sigma) are prepared in reagent buffer, final concentration 0.25Units per well. 20µl of the hrl GPa solution is added to 10µl compound solution and the reaction started with the addition of 20µl coupling enzyme solution. Compounds to be tested are prepared in 10µl 5% DMSO in assay buffer solution, with final concentration of 1% DMSO in the assay. The non-inhibited activity of GP*a* is measured in the presence of 10µl 5% DMSO in assay buffer solution and maximum inhibition measured in the presence of 5mgs $ml^{-1}$ N-ethylmaleimide. After 6 hours at 30°C Relative Fluoresence Units (RFUs) are measured at 340nM excitation, 465nm emission.

The assay is performed at a test concentration of inhibitor of 10µM or 100µM. Compounds demonstrating significant inhibition at one or both of these concentrations may be further evaluated using a range of test concentrations of inhibitor to determine an $IC_{50}$, a concentration predicted to inhibit the enzyme reaction by 50%.

Activity is calculated as follows:-

% inhibition = (1 - (compound RFUs - fully inhibited RFUs)/ (non-inhibited rate RFUs - fully inhibited RFUs)) * 100.

Typical $IC_{50}$ values for compounds of the invention when tested in the above assay are in the range 100µM to 1nM.

The inhibitory activity of compounds was further tested in rat primary hepatocytes. Rat hepatocytes were isolated by the collagenase perfusion technique, general method of Seglen (P.O. Seglen, Methods Cell Biology (1976) 13 29-83). Cells were cultured on Nunclon six well culture plates in DMEM with high level of glucose containing 10% foetal calf serum, NEAA, Glutamine, penicillin /streptomycin ((100units/100ug)/ml) for 4 to 6 hours. The hepatocytes were then cultured in the DMEM solution without foetal calf serum and with 10nM insulin and 10nM dexamethasone. Experiments were initiated after 18-20 hours culture by washing the cells and adding Krebs-Henseleit bicarbonate buffer containing 2.5mM $CaCl_2$ and 1% gelatin. The test compound was added and 5 minutes later the cells were challenged with 25nM glucagon. The Krebs-Henseleit solution was removed after 60 min incubation at 37°C, 95%$O_2$/ 5%$CO_2$ and the glucose concentration of the Krebs-Henseleit solution measured.

According to a further aspect of the invention there is provided a pharmaceutical composition which comprises a compound of the formula (I), or a pharmaceutically acceptable salt or *in vivo* hydrolysable ester thereof, as defined hereinbefore in association with a pharmaceutically-acceptable diluent or carrier.

The composition may be in a form suitable for oral administration, for example as a tablet or capsule, for parenteral injection (including intravenous, subcutaneous, intramuscular, intravascular or infusion) as a sterile solution, suspension or emulsion, for topical administration as an ointment or cream or for rectal administration as a suppository.

In general the above compositions may be prepared in a conventional manner using conventional excipients.

The compound of formula (**I**) will normally be administered to a warm-blooded animal at a unit dose within the range 5-5000 mg per square meter body area of the animal, i.e. approximately 0.1-100 mg/kg, and this normally provides a therapeutically-effective dose. A unit dose form such as a tablet or capsule will usually contain, for example 1-250 mg of active ingredient. Preferably a daily dose in the range of 1-50 mg/kg is employed. However the daily dose will necessarily be varied depending upon the host treated, the particular route of administration, and the severity of the illness being treated. Accordingly the optimum dosage may be determined by the practitioner who is treating any particular patient.

According to a further aspect of the present invention there is provided a compound of the formula (**I**), or a pharmaceutically acceptable salt or *in vivo* hydrolysable ester thereof, as defined hereinbefore, for use in a method of treatment of a warm-blooded animal such as man by therapy.

According to an additional aspect of the invention there is provided a compound of the formula (**I**), or a pharmaceutically acceptable salt or in vivo hydrolysable ester thereof, as defined hereinbefore, for use as a medicament.

According to an another aspect of the invention there is provided the use of a compound of the formula (**I'**):

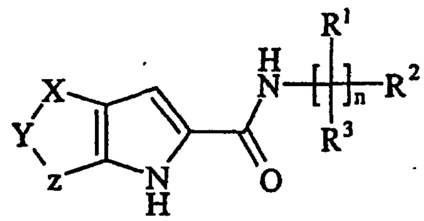

**(I')**

wherein:

**-X-Y-Z-** is selected from -S-$CR^4$=$CR^5$-, -$CR^4$=$CR^5$-S-, -O-$CR^4$=$CR^5$-, -$CR^4$=$CR^5$-O-, -N=$CR^4$-S-, -S-$CR^4$=N-, -$NR^6$-$CR^4$=$CR^5$- and -$CR^4$=$CR^5$-$NR^6$-;

wherein $\mathbf{R^4}$ and $\mathbf{R^5}$ are independently selected from hydrogen, halo, nitro, cyano, hydroxy, fluoromethyl, difluoromethyl, trifluoromethyl, trifluoromethoxy, amino, carboxy, carbamoyl, mercapto, sulphamoyl, ureido, $C_{1-6}$alkyl, $C_{2-6}$alkenyl, $C_{2-6}$alkynyl, $C_{1-6}$alkoxy, $C_{1-6}$alkanoyl, $C_{1-6}$alkanoyloxy, *N*-($C_{1-6}$alkyl)amino, *N,N*-($C_{1-6}$alkyl)$_2$amino, $C_{1-6}$alkanoylamino, *N*-($C_{1-6}$alkyl)carbamoyl, *N,N*-($C_{1-6}$alkyl)$_2$carbamoyl, $C_{1-6}$alkylS(O)$_a$ wherein a is 0 to 2, $C_{1-6}$alkoxycarbonyl, $C_{1-6}$alkoxycarbonylamino, N-($C_{1-6}$alkyl)sulphamoyl, *N,N*-($C_{1-6}$alkyl)$_2$sulphamoyl, $C_{1-6}$alkylsulphonylamino and $C_{1-6}$alkylsulphonyl-*N*-($C_{1-6}$alkyl)amino;

$\mathbf{R^6}$ is hydrogen or $C_{1-6}$alkyl;

$\mathbf{R^1}$ is selected from hydrogen, halo, nitro, cyano, hydroxy, amino, carboxy, carbamoyl, mercapto, sulphamoyl, ureido, $C_{1-6}$alkyl, $C_{2-6}$alkenyl, $C_{2-6}$alkynyl, $C_{1-6}$alkoxy, $C_{1-6}$alkanoyl, $C_{1-6}$alkanoyloxy, *N*-($C_{1-6}$alkyl)amino, *N,N*-($C_{1-6}$alkyl)$_2$amino, $C_{1-6}$alkanoylamino, *N*-($C_{1-6}$alkyl)carbamoyl, *N,N*-($C_{1-6}$alkyl)$_2$carbamoyl, $C_{1-6}$alkylS(O)$_a$ wherein a is 0 to 2, $C_{1-6}$alkoxycarbonyl, $C_{1-6}$alkoxycarbonylamino, *N*-($C_{1-6}$alkyl)sulphamoyl, *N,N*-($C_{1-6}$alkyl)$_2$sulphamoyl, $C_{1-6}$alkylsulphonylamino, $C_{1-6}$alkylsulphonyl-*N*-($C_{1-6}$alkyl)amino, $C_{3-8}$cycloalkyl, $C_{3-8}$cycloalkyl$C_{1-6}$alkyl, aryl, aryl$C_{1-6}$alkyl, heterocyclic group and (heterocyclic group)$C_{1-6}$alkyl; wherein $R^1$ may be optionally substituted on carbon by one or more groups selected from P and wherein if said heterocyclic group contains an -NH- moiety that nitrogen may be optionally substituted by a group selected from R;

$\mathbf{R^2}$ is selected from hydrogen, halo, nitro, cyano, hydroxy, fluoromethyl, difluoromethyl, trifluoromethyl, trifluoromethoxy, amino, carboxy, carbamoyl, mercapto, sulphamoyl, ureido, $C_{1-6}$alkyl, $C_{2-6}$alkenyl, $C_{2-6}$alkynyl, $C_{1-6}$alkoxy, $C_{1-6}$alkanoyl, $C_{1-6}$alkanoyloxy, *N*-($C_{1-6}$alkyl)amino, *N,N*-($C_{1-6}$alkyl)$_2$amino, $C_{1-6}$alkanoylamino, *N*-($C_{1-6}$alkyl)carbamoyl, *N,N* ($C_{1-4}$alkyl)$_2$carbamoyl, *N*-($C_{1-6}$alkyl)-*N*-($C_{1-6}$alkoxy)carbamoyl, $C_{1-6}$alkylS(O)$_a$ wherein a is 0 to 2, $C_{1-6}$alkoxycarbonyl, $C_{1-6}$alkoxycarbonylamino, *N*-($C_{1-6}$alkyl)sulphamoyl, *N,N*-($C_{1-6}$alkyl)$_2$sulphamoyl, sulphamoylamino, *N*-($C_{1-6}$alkyl)sulphamoylamino, *N,N*-($C_{1-6}$alkyl)$_2$sulphamoylamino, $C_{1-6}$akylsulphonylamino, $C_{1-6}$-alkylsulphonylaminocarbonyl, $C_{1-6}$alkylsulphonyl-*N*-($C_{1-6}$alkyl)amino and a group -E-F-G-H;

wherein **E** and **G** are independently selected from a direct bond, -O-, -S-, -SO-, -$SO_2$-, -OC(O)-, -C(O)O-, -C(O)-, -$NR^a$-, -$NR^a$C(O)-, -C(O)$NR^a$-, -$SO_2NR^a$-, -$NR^aSO_2$-, -$NR^a$C(O)$NR^b$-, -OC(O)$NR^a$-, -$NR^a$C(O)O-, -$NR^aSO_2NR^6$-, -$SO_2NR^a$C(O)- and -C(O)$NR^aSO_2$-; wherein $R^a$ and $R^b$ are independently selected from hydrogen or $C_{1-6}$alkyl which is optionally substituted by a group V;

**F** is $C_{1-6}$alkylene optionally substituted by one or more Q or a direct bond;

**H** is selected from aryl, $C_{3-8}$cycloalkyl and heterocyclic group; wherein H may be optionally substituted on carbon by one or more groups selected from S and wherein if said heterocyclic group contains an -NH- moiety that nitrogen may be optionally substituted by a group selected from T;

$\mathbf{R^3}$ is hydrogen or $C_{1-6}$alkyl;

**n** is selected from 0-4; wherein the values of $R^1$ may be the same or different; and wherein the values of $R^3$ may be the same or different;

**P, S** and **Q** are independently selected from halo, nitro, cyano, hydroxy, trifluoromethyl, trifluromethoxy, amino, carboxy, carbamoyl, mercapto,sulphamoyl, ureido, $C_{1-6}$alkyl, $C_{2-6}$alkenyl, $C_{2-6}$alkynyl, $C_{1-6}$alkoxy, $C_{1-6}$alkanoyl, $C_{1-6}$alkanoyloxy, *N*-($C_{1-6}$alkyl)amino, *N,N*-($C_{1-6}$alkyl)amino, $C_{1-6}$alkanoylamino, *N*-($C_{1-6}$alkyl)carbamoyl, *N, N*-($C_{1-6}$alkyl)$_2$carbamoyl, *N*-($C_{1-6}$alkyl)-*N*-($C_{1-6}$alkoxy)carbamoyl, $C_{1-6}$-alkylS(O)$_a$ wherein a is 0 to 2, $C_{1-6}$alkoxycarbonyl, $C_{1-6}$alkoxycarbonylamino, *N*-($C_{1-6}$alkyl)sulphamoyl, *N,N*-($C_{1-6}$alkyl)$_2$sulphamoyl, $C_{1-6}$alkylsulphonylamino, $C_{1-6}$alkylsulphonyl-*N*-($C_{1-6}$alkyl)amino, $C_{3-8}$cycloalkyl, aryl and heterocyclic group; wherein P, S and Q may be optionally and independently substituted on carbon by one or more groups selected from V and wherein if said heterocyclic group contains an -NH- moiety that nitrogen may be optionally substituted by a group selected from U;

**V** is selected from halo, nitro, cyano, hydroxy, trifluoromethoxy, trifluoromethyl, amino, carboxy, carbamoyl, mercapto, sulphamoyl, methyl, ethyl, methoxy, ethoxy, acetyl, acetoxy, methylamino, ethylamino, dimethylamino, diethylamino, *N*-methyl-*N*-ethylamino, acetylamino, *N*-methylcarbamoyl, *N*-ethylcarbamoyl, *N,N*-dimethylcarbamoyl, *N,N*-diethylcarbamoyl, *N*-methyl-*N*-ethylcarbamoyl, methylthio, ethylthio, methylsulphinyl, ethylsulphinyl, mesyl, ethylsulphonyl, methoxycarbonyl, ethoxycarbonyl, *N*-methylsulphamoyl, *N*-ethylsulphamoyl, *N,N*-dimethylsulphamoyl, *N,N*-diethylsulphamoyl, *N*-methyl-*N*-ethylsulphamoyl, morpholino, morpholinocarbonyl, *N*-benzylcarbamoyl, and 4-hydroxypiperidinocarbonyl;

**R, T** and **U** are independently selected from $C_{1-4}$alkyl, $C_{1-4}$alkanoyl; $C_{1-4}$alkylsulphonyl, $C_{1-4}$alkoxycarbonyl, carbamoyl, *N*-($C_{1-4}$alkyl)carbamoyl, *N,N*-($C_{1-4}$alkyl)carbamoyl, phenyl, benzyl, benzyloxycarbonyl, benzoyl and phenylsulphonyl; or a pharmaceutically acceptable salt or an *in* vivo hydrolysable ester thereof; with the proviso that

when -X-Y-Z- is as initially defined, n is 1, $R^1$ is arylmethyl, substituted arylmethyl, (heterocyclic group)methyl and substituted (heterocyclic group)methyl and $R^3$ is hydrogen then $R^2$ is not a group -C(=O)-A or a group -CH(OH)-C(=O)-A in which A is $NR^dR^d$, $-NR^aCH_2CH_2OR^a$, or

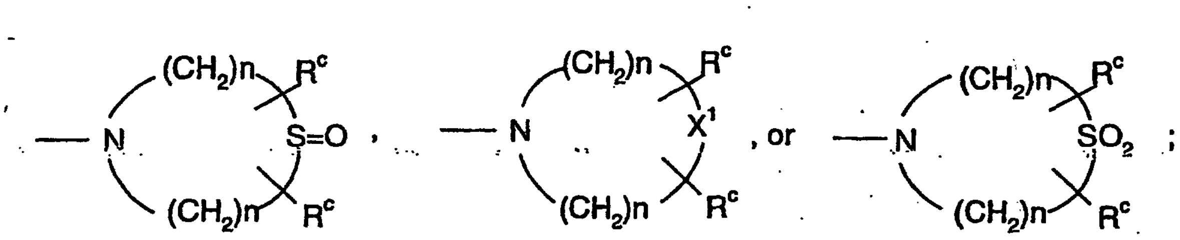

each $R^a$ and $R^b$ is independently hydrogen or $-C_1$-$C_8$alkyl;

each $R^d$ is independently hydrogen, $C_1$-$C_8$ alkyl, $C_1$-$C_8$alkoxy, aryl, substituted aryl; heteroaryl, or substituted heteroaryl;

each $R^c$ is independently hydrogen, $-C(=O)OR^a$, $-OR^a$, $-SR^a$, or $-NR^aR^a$; and each n is independently 1-3, and $X^1$ is $NR^a$, $-CH_2-$, O or S;

or a pharmaceutically acceptable salt or in *vivo* hydrolysable ester thereof, as defined hereinbefore in the manufacture of a medicament for use in the production of a glycogen phosphorylase inhibitory effect in a warm-blooded animal such as man.

According to this another aspect of the invention there is provided the use of a compound of the formula (**I'**), or a pharmaceutically acceptable salt or in vivo hydrolysable ester thereof, as defined hereinbefore in the manufacture of a medicament for use in the treatment of type 2 diabetes, insulin resistance, syndrome X, hyperinsulinaemia, hyperglucagonaemia, cardiac ischaemia or obesity in a warm-blooded animal, such as man.

According to this another aspect of the invention there is provided the use of a compound of the formula **(I'),** or a pharmaceutically acceptable salt or *in vivo* hydrolysable ester thereof, as defined hereinbefore in the manufacture of a medicament for use in the treatment of type 2 diabetes in a warm-blooded animal such as man.

According to a further feature of this aspect of the invention there is provided a method of producing a glycogen phosphorylase inhibitory effect in a warm-blooded animal, such as man, in need of such treatment which comprises administering to said animal an effective amount of a compound of formula **(I').**

According to this further feature of this aspect of the invention there is provided a method of treating type 2 diabetes, insulin resistance, syndrome **X**, hyperinsulinaemia, hyperglucagonaemia, cardiac ischaemia or obesity in a warm-blooded animal, such as man, in need of such treatment which comprises administering to said animal an effective amount of a compound of formula **(I').**

According to this further feature of this aspect of the invention there is provided a method of treating type 2 diabetes in a warm-blooded animal, such as man, in need of such treatment which comprises administering to said animal an effective amount of a compound of formula **(I').**

As stated above the size of the dose required for the therapeutic or prophylactic treatment of a particular cell-proliferation disease will necessarily be varied depending on the host treated, the route of administration and the severity of the illness being treated. A unit dose in the range, for example, 1-100 mg/kg, preferably 1-50 mg/kg is envisaged.

In addition to their use in therapeutic medicine, the compounds of formula **(I)** and their pharmaceutically acceptable salts are also useful as pharmacological tools in the development and standardisation of *in vitro* and *in vivo* test systems for the evaluation of the effects of inhibitors of cell cycle activity in laboratory animals such as cats, dogs, rabbits, monkeys, rats and mice, as part of the search for new therapeutic agents.

In the above other pharmaceutical composition, process, method, use and medicament manufacture features,

the alternative and preferred embodiments of the compounds of the invention described herein also apply.

## Examples

The invention will now be illustrated by the following non-limiting examples in which, unless stated otherwise:

(i) temperatures are given in degrees Celsius (°C); operations were carried out at room or ambient temperature, that is, at a temperature in the range of 18-25°C and under an atmosphere of an inert gas such as argon;
(ii) organic solutions were dried over anhydrous magnesium sulphate; evaporation of solvent was carried out using a rotary evaporator under reduced pressure (600-4000 Pascals; 4.5-30 mmHg) with a bath temperature of up to 60°C;
(iii) chromatography means flash chromatography on silica gel; thin layer chromatography (TLC) was carried out on silica gel plates; where a Bond Elut column is referred to, this means a column containing 10 g or 20 g or 50 g of silica of 40 micron particle size, the silica being contained in a 60 ml disposable syringe and supported by a porous disc, obtained from Varian, Harbor City, California, USA under the name "Mega Bond Elut Sr'; "Mega Bond Elut" is a trademark; where a Biotage cartridge is referred to this means a cartridge containing KP-SIL™ silica, 60 angstroms, particle size 32-63mM, supplied by Biotage, a division of Dyax Corp., 1500 Avon Street Extended, Charlottesville, VA 22902, USA;
(iv) in general, the course of reactions was followed by TLC and reaction times are given for illustration only;
(v) yields are given for illustration only and are not necessarily those which can be obtained by diligent process development; preparations were repeated if more material was required;
(vi) where given, NMR data is in the form of delta values for major diagnostic protons, given in parts per million (ppm) relative to tetramethylsilane (TMS) as an internal standard, determined at 300 MHz using perdeuterio dimethyl sulphoxide (DMSO-$\delta_6$) as solvent unless otherwise indicated, other solvents (where indicated in the text) include deuterated chloroform $CDCl_3$;
(vii) chemical symbols have their usual meanings; SI units and symbols are used;
(viii) reduced pressures are given as absolute pressures in Pascals (Pa); elevated pressures are given as gauge pressures in bars;
(ix) solvent ratios are given in volume: volume (v/v) terms;
(x) mass spectra (MS) were run with an electron energy of 70 electron volts in the chemical ionisation (CI) mode using a direct exposure probe; where indicated ionisation was effected by electron impact (EI), fast atom bombardment (FAB) or electrospray (ESP); values for m/z are given; generally, only ions which indicate the parent mass are reported and unless otherwise stated the value quoted is (M-H);
(xi) The following abbreviations are used:

| | |
|---|---|
| SM | starting material; |
| EtOAc | ethyl acetate; |
| MeOH | methanol; |
| DCM | dichloromethane |
| HOBT | 1-hydroxybenzotriazole |
| DIAD | diisopropyl azodicarboxylate |
| HATU | *O*-(7-azabenzotriazol-1-yl) *N, N, N', N',* tetramethyluronium hexafluorophosphate |
| TFA | trifluoroacetic acid |
| DIPEA | di-isopropylethylamine; and |
| EDAC | 1-ethyl-3-(3-dimethylaminopropyl)carbodi-imide hydrochloride |

**Example #1**

2.3-Dichloro-5-[*N*-(2-phenoxyethyl)carbamoyl)-4*H*-thieno[3,2-*b*]pyrrole

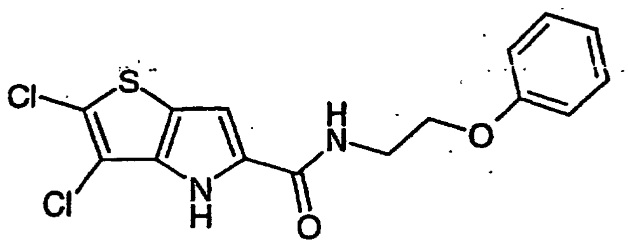

5-Carboxy-2,3-dichloro-4*H*-thieno[3,2-b]pyrrole (Method #9; 47 mg; 0:2 mmol) was dissolved in DCM (10 ml) containing 2-phenoxyethylamine (27 mg, 0.2 mmol), HOBT (27 mg, 0.2 mmol) and DIPEA (70 ml, 0.4 mmol). The mixture was stirred for 1 minute before the addition of EDAC (50 mg, 0.26 mmol). The mixture was stirred at ambient temperature for approximately 18 hours before being washed with water. The organic fraction was concentrated and was purified on a Bond Elut column (eluent 1:1 EtOAc/isohexane) to afford the title compound as an off white solid (32 mg). NMR: 12.4 (1H, br), 8.4 (1H, t), 7.3 (1H, d), 7.1 (1H, s), 6.9 (2H, m), 3.5 (2H, m), 3.0 (2H, t); m/z 353.2.

**Examples #2 - #9**

The following compounds were made by the process of Example #1 using 5-carboxy-2,3-dichloro-4*H*-thieno [3,2-*b*]pyrrole (Method #9) and the appropriate amine:

**Example #2:** 2,3-Dichloro-5-{*N*-[2-(2-thienyl)ethyllcarbamoyl-4*H*-thieno[3,2-*b*]pyrrole

**Example #3:** 2,3-Dichloro-5-{*N*-[2-(2-methoxyphenyl)ethyl]carbamoyl}-4*H*-thieno[3,2-*b*]pyrrole

**Example #4:** 2,3-Dichloro-5-[*N*-(2-phenyl-1-cycloprpryl)carbamoyl]-4*H*-thieno[3,2-*b*]pyrrole

**Example #5:** 2,3-Dichloro-5-{*N*-[2-(4-fluorophenyl)ethyl]carbamoyl}-4*H*-thieno[3,2-*b*]pyrrole

**Example #6:** 2,3-dichloro-5-[*N*-(*N*-phenylcarbamoylmethyl)carbamoyl]-4*H*-thieno[3,2-*b*]pyrrole

**Example #7:** 2,3-Dichloro-5-(*N*-{2-[(2-pyridyl)amino]ethyl}carbamoyl)-4*H*-thieno[3,2-*b*]pyrrole

**Example #8:** 2,3-Dichloro-5-{*N*-[2-(*N*-methylmethanesulphonamido)-1-(thiazol-2-yl)ethyl]carbamoyl}-4*H*-thieno [3,2-*b*]pyrrole

**Example #9:** 2,3-Dichloro-5-{*N*-[2-(thiomorpholino)ethyl]carbamoyl}-4*H* thieno[3,2-*b*]pyrrole

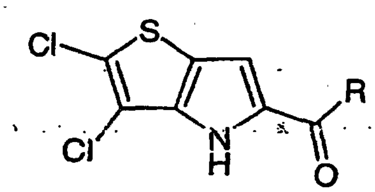

| Ex | R | NMR | M/z |
|---|---|---|---|
| #2 | 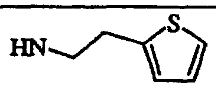 | 12.4 (1H, br), 8.4 (1H, t), 7.3 (1H, d), 7.1 (1H, s), 6.9 (2H, m), 3.5 (2H, m), 3.0 (2H, t) | 343.1 |
| #3 | 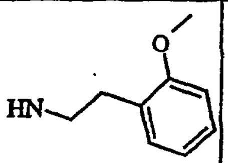 | 12.3 (1H, s), 8.3 (1H, t), 6.8-7.2 (5H, m), 3.8 (3H, s), 3.4 (2H, m), 2.8 (2H, t) | 367.3 |
| #4 | 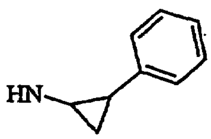 | 12.4 (1H, s), 8.5 (1H, d), 7.2 (5H, m), 7.1 (1H, s), 3.0 (1H, m), 2.1 (1H, m), 1.3 (2H, m) | 349.3 |
| | | | |
| #5 | 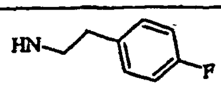 | 12.3 (1H, s), 8.4 (1H, t), 7.3 (2H, m), 7.1 (3H, m), 3.4 (2H, m), 2.8 (2H, t) | 355.3 |
| #6 [1] | 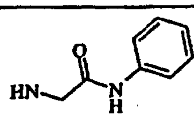 | 12.4 (1H, s), 10.0 (1H, s), 8.7 (1H, br), 7.1-7.6 (5H, m), 7.2 (1H, s), 4.1 (2H, d) | 366.2 |
| #7 [1, 2] | 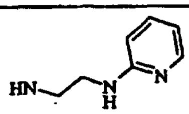 | 12.4 (1H, s), 8.4 (1H, s), 6.5-8.0 (5H, m), 6.6 (1H, s), 3.3 (4H, m) | 353.3 |
| #8 [3] | 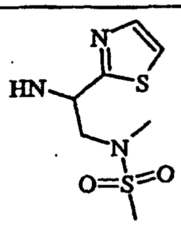 | ($CDCl_3$) 9.6 (1H, br), 7.8 (1H, m), 7.7 (1H, m), 7.3 (1H, m), 7.0 (1H, s), 5.6 (1H, m), 3.9 (1H, m), 3.7 (1H, m), 2.9 (3H, s), 2.8 (3H, s) | 451.2 |
| #9 | 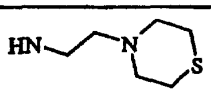 | ($CDCl_3$) 9.8 (1H, br), 6.9 (1H, br), 6.7 (1H, s), 4.1 (2H, m), 3.6 (2H, m), 2.8 (8H, m) | 362.2 |

[1] Water was added and the title compound precipitated as a yellow solid which was filtered off, washed with water, dried under reduced pressure and was not purified further.

[2] Amine: Eur J Med Chem, 1987, 22, 91.

[3] Amine: Method #15

**Example #10,**

5-[*N*-Benzoylmethyl)carbamoyl)-2,3-dichloro-4*H* thieno[3,2-*b*]pyrrole

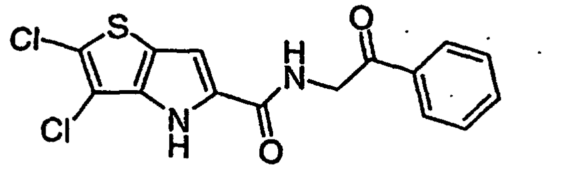

2,3-Dichloro-5-{*N* [(2-phenyl-1,3-dioxolan-2-yl)methyl]carbamoyl}-4*H* thieno[3,2-*b*]pyrrole (Method #17; 80 mg, 0.2 mmol) was dissolved in acetone (15 ml) containing aqueous hydrochloric acid (2.0 M, 1 ml). The mixture was heated under reflux for 90 minutes. The white precipitate formed was filtered off and washed with acetone. The filtrate was concentrated and the residue was triturated with water. The solid formed was filtered off and dried under reduced pressure to afford the title compound as a white solid (20 mg). NMR: 12.4 (1H, s), 8.6 (1H, t), 7.6-8.0 (5H, m), 7.2 (1H, s), 4.8 (2H, d); m/z 351.1.

**Example #11**

3-Chloro-5-[*N*-(*N*-phenylcarbamoylmethyl)carbamoyl]-4*H*-thieno[3,2-*b*]pyrrole

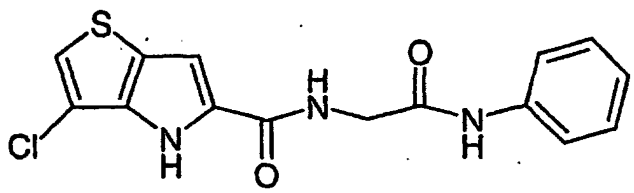

5-Carboxy-3-chloro-4*H* thieno[3,2-*b*]pyrrole (Method #7; 100 mg, 0.5 mmol) was dissolved in DCM (6 ml) containing HOBT (68 mg, 0.5 mmol), DIPEA (176 ml, 1.0 mmol) and N-glycylaniline (75 mg, 0.5 mmol). The mixture was allowed to stand for one minute before the addition of EDAC (150 mg, 0.7 mmol). The solution was allowed to stand for approximately 18 hours before being washed with water. The organic phase was dried, filtered and concentrated under reduced pressure to afford the title compound (112 mg, 67%). NMR ($CDCl_3$) 9.7 (1H, br), 8.6 (1H, br), 7.0-8.0 (8H, m), 4.3 (2H, d); m/z 332.1.

**Examples #12 - #21**

The following compounds were made by the processe of Exemple #11 using 5-carboxy 3-chloro-4*H*-thieno [3,2-*b*]pyrrole (Method #7) and the appropriate amine:

**Example #12:**3-Chloro-5-{*N*-[2-(thiomorpholino)ethyl]carbamoyl}-4*H*-thieno[3,2-*b*]pyrrole

**Example #13:** 3-Chloro-5-{*N*-[2-(*N*-methylmethanesulphonamido)-1-(thiazol-2-yl)ethyl]carbamoyl}-4*H* thieno[3,2-*b*] pyrrole

**Example#14:** 3-Chloro-5-[*N*-(benzoylmethyl)carbamoyl]-4*H*-thieno[3,2-*b*]pyrrole

**Example-#15:**3-Chloro-5-{*N*-[2-(2-methoxyphenyl)ethyl]carbamoyl}-4*H*-thieno[3.2-*b*]pyrrole

**Example #16:** 3-Chloro-5-{*N*-[2-(2-thienyl)ethyl]carbamoyl}-4*H*-thieno[3,2-*b*]pyrrole

**Example #17:** 3-Chloro-5-[*N*-(2-phenyl-1-cyclopropylcarbamoyl]-4*H*-thieno[3,2-*b*]pyrrole **Example #18:** 3-Chloro-5-{*N*-[2-(4-fluorophenyl)ethyl]carbamoyl}-4*H*-thieno[3,2-*b*]pyrrole

**Example #19** 3-Chloro-5-[-*N*-(2-phenoxyethyl)carbamoyl)-4*H*-thieno[3,2-*b*]pyrrole

**Example #20:** 3-Chloro-5-{*N*-[2-(1-phenylmethanesulphonamido)ethyl]carbamoyl}-4*H*-thieno[3,2-*b*]pyrrole

**Example#21:**3-chloro-5-[*N*-(4-oxo-2,3,4,5-tetrahydrobenz[1,5]thiazepin-3-yl)carbamoyl]-4*H*-thieno[3,2-*b*]pyrrole

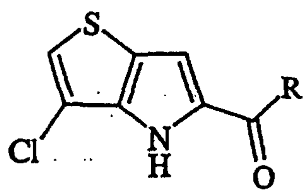

| Ex | R | NMR | M/z |
|---|---|---|---|
| #12 | 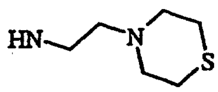 | ($CDCl_3$) 9.6 (1H, br), 7.0 (1H, s), 6.7 (1H, s), 6.6 (1H, br), 4.1 (2H, m), 3.5 (2H, m), 2.7 (8H, m) | 328.3 |
| #13 [1] | 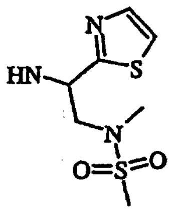 | ($CDCl_3$) 9.5 (1H, br), 7.8 (1H, m), 7.6 (1H, m), 7.3 (1H, m), 7.0 (2H, 2 x s), 5.6 (1H, m), 3.9 (1H, m), 3.7 (1H, m), 2.9 (6H, 2 x s) | 419.0 $(M+H)^+$ |
| #14 | 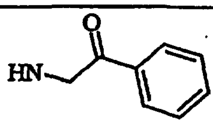 | ($CDCl_3$) 9.6 (1H, s), 7.0-8.0 (8H, m), 5.0 (2H, m) | 317.1 |
| #15 | 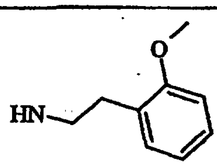 | ($CDCl_3$) 9.6 (1H, br), 7.2 (2H, m), 7.0 (1H, s), 6.9 (2H, m), 6.6 (1H, s), 6.3 (1H, br), 3.9 (3H, s), 3.7 (2H, m), 2.9 (2H, t) | 333.1 |
| #16 | 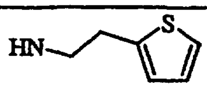 | ($CDCl_3$) 9.8 (1H, br), 6.9-7.2 (4H, m), 6.7 (1H, s), 6.2 (1H, br), 3.8 (2H, m), 3.2 (2H, t) | 309.1 |
| #17 | 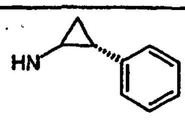 | ($CDCl_3$) 9.6 (1H, br), 7.0-7.3 (6H, m), 6.8 (1H, s), 6.3 (1H, br), 3.1 (1H, m), 2.2 (1H, m), 1.3 (2H, m) | 315.1 |
| #18 | 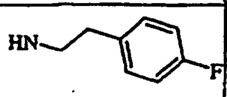 | ($CDCl_3$) 9.5 (1H, br), 7.0-7.2 (5H, m), 6.6 (1H, s), 5.9 (1H, br), 3.7 (2H, m), 2.9 ( 2H, t) | 321.1 |

| | | | |
|---|---|---|---|
| #19 | 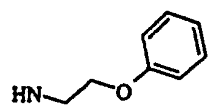 | ($CDCl_3$) 9.6 (1H, br), 6.9-7.3 (6H, m), 6.8 (1H, s), 6.5 (1H, m), 4.2 (2H, t), 3.8 (2H, m) | 319.1 |
| #20 | 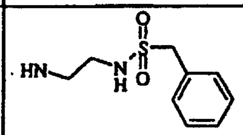 | ($CDCl_3$) 9.6 (1H, s), 6.8-7.4 (9H, m), 4.3 (2H, s), 3.4 (2H, m), 3.1 (2H, m) | 396.1 |
| #21 [2] | 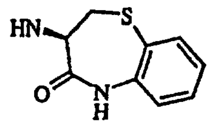 | ($CDCl_3$) 9.7 (1H, s), 8.0 (1H, s), 7.7 (1H, d), 7.1-7.4 (4H, m), 7.0 (1H, s), 6.9 (1H, s), 4.9 (1H, m), 4.0 (1H, m), 3.0 (1H, m) | 378.1 $(M+H)^+$ |

[1] Amine: Method #15

[2] Amine: J Med Chem, 1985, 28, 1517

**Example #22**

2-Chloro-5-[*N*-(benzoylmethyl)carbamoyl]-4*H*-thieno[3,2-*b*]pyrrole

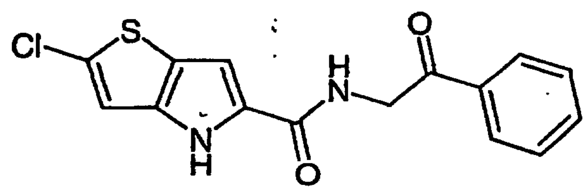

5-Carboxy-2-chloro-4*H*-thieno[3,2-*b*]pyrrole (Method #8; 50 mg, 0.25 mmol) was dissolved in DCM (7 ml) containing HOBT (34 mg, 0.25 mmol), DIPEA (49 ml, 0.28 mmol) and 2-amino-1-phenylethanone (43 mg, 0.25 mmol). The mixture was stirred for one minute and EDAC (63 mg, 0.33 mmol) was added. The mixture was stirred at room temperature for approximately 18 hours. Water was added to the solution and a solid precipitated out. This solid was filtered off and was washed with water and DCM before being dried under reduced pressure to afford the title compound as a white solid (45 mg, 61%). NMR: 11.9 (1H, s), 8.6 (1H, t), 7.1-8.1 (7H, m), 4.8 (2H, d); m/z 317.3.

**Examples #23 - #25**

The following compounds were made by the process of Example #22 using 5-carboxy-2-chloro-4*H* thieno [3,2-*b*]pyrrole (Method #8) and the appropriate amine:

**Example #23:** 2-Chloro-5-[*N*-(2-phenyl-1-cyclopropyl)carbamoy]-4*H* thieno[3 2-*b*]pyrrole

**Example #24:** 2-Chloro-5-[*N*-(*N*-phenylcarbamoylmethyl)carbarnoyl]-4*H*-thieno[3,2-*b*]pyrrole

**Example #25:** 2-Chloro-5-(*N*-{2-[(2-pyridyl)amino]ethyl}carbamoyl)-4*H* thieno[3,2-*b*]pyrrole

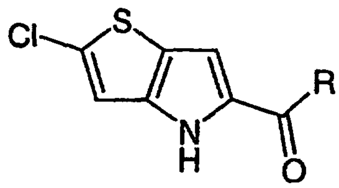

| Ex | R | NMR | M/z |
|---|---|---|---|
| #23 | 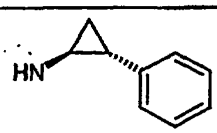 | 11.8 (1H, s), 8.5 (1H, d), 7.0-7.3 (7H, m), 3.0 (1H, m), 2.1 (1H, m), 1.3 (2H, m) | 315.3 |
| #24 | 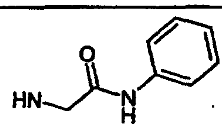 | 11.8 (1H, s), 10.0 (1H, br), 8.6 (1H, br), 7.0-7.6 (7H, m), 4.0 (2H, d) | 332.3 |
| #25 [1] | 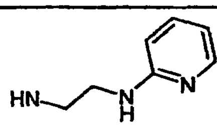 | 11.8 (1H, s), 8.4 (1H, s), 6.4-8.0 (6H, m), 6.6 (1H, br), 3.4 (4H, m) | 319.3 |

[1] Amine: Eur J Med Chem, 1987, 22, 91.

**Example #26**

2-Chloro-5-{*N*-[2-(2-methoxyphenyl)ethyl]carbamoyl}-4*H*-thieno[3,2-*b*]pyrrole

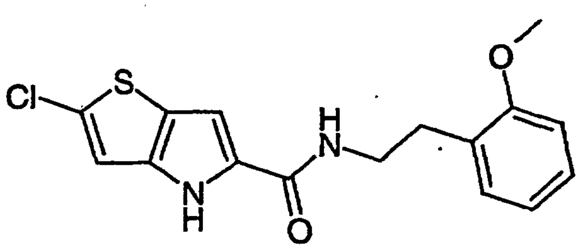

5-Carboxy-2-chloro-4*H* thieno[3,2-*b*]pyrrole (Method. #8; 50 mg, 0.25 mmol) was dissolved in DCM (7 ml) containing HOBT (34 mg, 0.25 mmol), DIPEA (49 ml, 0.28 mmol) and 2-(2-methoxyphenyl)ethylamine (43 mg, 0.25 mmol). The mixture was stirred for one minute and EDAC (63 mg, 0.33 mmol) was added. The mixture was stirred at

room temperature for approximately 18 hours. The reaction mixture was washed with water, the organic phase was dried, filtered and concentrated to afford the title compound as an off-white solid (84 mg, 100%). NMR ($CDCl_3$): 9.7 (1H, br), 6.9-7.2 (5H, m), 6.5 (1H. s), 6.3 (1H, br), 3.9 (3H, s), 3.7 (2H, m), 3.0 (2H, t); m/z 333.4.

<u>**Examples #27 - #31**</u>

The following compounds were made by the process of Example #26 using 5-carboxy-2-chioro-4*H*-thieno [3,2-*b*]pyrrole (Method #8) and the appropriate amine.

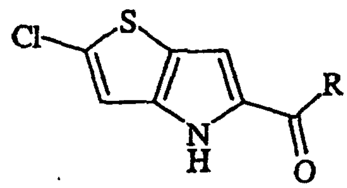

**Example #27:** 2-Chloro-5-[*N*-(2-phenoxyethyl)carbamoyl]-4*H* thieno[3.2-*b*]pyrrole

**Example #28:**2-Chloro-5-{*N*-[2-thienyl)ethy]carbamoyl}-4*H*-thieno[3.2-*b*]pyrrole

**Example #29:**2-Chloro-5-{*N*-[2-(4-fluorophenyl)ethyl]carbamoyl}-4*H*-thieno[3.2-*b*] pyrrole

**Example #30:**2-Chloro-5-{*N*-(2-(*N*-methylmethanesulphonamido)-1-(thiazol-2-yl)ethyl]carbamoyl}-4*H*-thieno[3,2-*b*] pyrrole

**Example #31:** 2-Chloro-5-{*N*-[2-(thiomorpholino}ethyl]carbamoyl}-4*H*-thieno [3,2-*b*]pyrrole

| Ex | R | NMR | M/z |
|---|---|---|---|
| #27 | 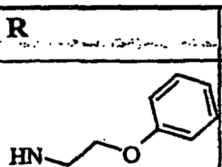 | ($CDCl_3$) 9.5 (1H, s), 6.9-7.3 (6H, m), 6.7 (1H, s), 6.4 (1H, br), 4.2 (2H, m), 3.9 (2H, m) | 319.3 |
| #28 | 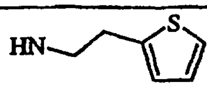 | ($CDCl_3$) 9.4 (1H, br), 6.6-7.2 (5H, m), 6.0 (1H, br), 3.7 (2H, m), 3.1 (2H, m) | 309.3 |
| #29 | 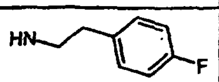 | ($CDCl_3$) 9.4 (1H, s), 7.0-7.2 (4H, m), 6.9 (1H, s), 6.6 (1H, s), 5.9 (1H, br), 3.7 (2H, m), 2.9 (2H, t) | 321.3 |

| | | | |
|---|---|---|---|
| #30 [1] | 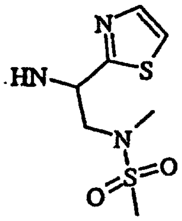 | 11.9 (1H, br), 9.0 (1H, d), 7.8 (1H, m), 7.7 (1H), 7.2 (1H, s), 7.0 (1H, s), 5.6 (1H, m), 3.9 (1H, m), 3.6 (1H, m), 2.9 (3H, s), 2.8 (3H, s) | 417.2 |
| #31 | 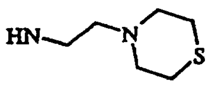 | 11.8 (1H, s), 8.1 (1H, t), 6.8-7.4 (2H, m), 2.5-4.0 (12H, m) | 328.3 |

[1] Amine: Method #15

**Examples #32 - #69**

The following compounds were made by the process of Example #1 using 5-carboxy-2,3-dichloro-4*H*-thieno[3,2-*b*]pyrrole (Method #9) or Example #22 using 5-carboxy-2-chloro-4*H*-thieno[3,2-*b*]pyrrole (Method #8) and the appropriate amine:

**Example #32:** 2.3-Dichloro-5-[*N*-(2,3-dimethyl-5-oxo-1-phenyl-2,5-dihydro-1*H*-pyrazol-4-yl)carbamoyl]-4*H*-thieno [3.2-*b*]pyrrole

**Example #33:** 2,3-Dichloro-5-[*N*-(4-sulphamoylphenylmethyl)carbamoyl-4*H*-thieno[3,2-*b*] pyrrole

**Example #34:** 2,3-Dichloro-5-[*N*-(2-hydroxy-1-phemethyl)carbamoyl]-4*H*-thieno[3,2-*b*]pyrrole

**Example #35:** 2,3-Dichloro-5-{*N*-(2-[(3-trifluoromethylpyrid-2yl)amino]ethyl)carbamoyl}-4*H*-thieno[3,2-*b*]pyrrole

**Example #36:** 2,3-Dichloro-5-{*N*-[3-(5-tetrazolyl)propyl]carbamoyl}-4*H*-thieno[3,2-*b*]pryrrole

**Example #37** 2,3-Dichloro-5-[*N*-(5-oxo-3-phenyl-4,5-dihydroisoxazol-4-yl)carbamoyl]-4*H*-thieno[3,2-*b*]pyrrole.

**Example #38:** 2,3-Dichloro-5-[*N*-(5-hydroxy-2-oxo-2,3,4,5-tetrahydro-1H-benz[*b*]azepin-4-yl)carbamoyl]-4*H*-thieno [3.2-*b*]pyrrole

**Example #39:** 2-Chloro-5-{*N*-[3-(benzyloxycarbonylamino)propyl]carbamoyl}-4*H*-thieno[3,2-*b*]pyrrole

**Example #40:** 2,3-Dichloro-5-{*N*-[(4-dimethylaminophenyl)methyl]carbamoy}-4*H*-thieno[3,2-*b*]pyrrole

**Example #41:** 5-[*N*-(1-Benzyl-2-hydroxyethyl)carbamoyl]-2,3-dichloro-4*H* thieno[3,2-*b*]pyrrole

**Example #42:** 2,3-Dichloro-5-{*N*-[2-(phenylamino)ethyl]carbamoyl}-4*H*-thieno[3,2-*b*]pyrrole

**Example #43:**x2,3-Dichloro-5-[*N*-(β-(*R*)-hydroxy-α-methylphenethyl)carbamoyl-4*H*-thieno[3,2-*b*]pyrrole

**Example #44:** 2,3-Dichloro-5-[*N*-(β-hydroxyphenethyl)carbamoyl)-4*H* thieno[3,2-*b*]pyrrole

**Example #45:** 2,3-Dichloro-5-{*N*-(2-(4-hydroxyphenyl)ethyl]carbamoyl}4*H*-thieno[3,2-*b*]pyrrole

**Example #46:** 2,3-Dichloro-5-{*N*-[(benzimidazol-2-yl)methyl]carbamoyl}-4*H*-thieno[3,2-*b*]pyrrole

**Example #47:** 2,3-Dichloro-5-{*N*-[2-(4-chlorophenyl)-2-hydroxy-1-(methoxycarbonyl)ethyl]carbamoyl}-4*H*-thieno [3,2-*b*]pyrrole

**Example #48:** 2,3-Dichloro-5-{*N*-(imidazo[1,2-*a*]pyrid-2-yl)carbamoyl}-4*H*-thieno[3,2-*b*]pyrrole

**Example #49:** 5-{N-(Benzthiazol-2-yl)methyl]carbamoyl}-2,3-dichloro-4*H* thieno[3,2-*b*]pyrrole

**Example #50:** 2,3-Dichloro-5-{*N*-[(6-trifluoromethylpyrid-3-yl)methyl]carbamoyl}-4*H* thieno [3,2-*b*]pyrrole

**Example #51**: 2,3-Dichloro-5-{*N*-(2-[(2-pyridazinyl)methyl]carbamoyl}-4*H*-thieno[3,2-*b*]pyrrole

**Example #52:** 2,3-Dichloro-5-{*N*-[*N*-(2-hydroxy-3-phenoxypropyl)carbamoylmethyl] carbamoyl}-4*H*-thieno[3,2-*b*] pyrrole

**Example #53:** 2,3-Dichloro-5-{*N*-[*N*-(3-methylisothiazol-5-yl)carbamoylmethyl]carbamoyl}-4*H*-thieno[3,2-*b*]pyrrole

**Example #54:** 2,3-Dichloro-5-{*N*-[2-(pyridazin-3-yloxy)ethyl]carbamoyl]-4*H*-thieno[3,2-*b*]pyrrole

**Example#55:** 2-Chloro-5-(*N*-[2-[(3-trifluoromethylpyrid-2-yl)amino]ethyl}carbamoyl)-4*H*-thieno[3,2-*b*]pyrrole

**Example#56:** 2,3-Dichloro-5-{*N*-[2-(4-sulphamoylphenyl)ethyl]carbamol}-4*H*-thieno[3,2-*b*]pyrrole

**Example#57:** 2,3-Dichloro-5-{*N*-(2-pyridyl)ethyl]carbamoyl)-4*H*-thieno[3 ,2-b]pyrrole

**Example#58:** 2,3-Dichloro-5-{*N*-(2-[1-hydroxymethyl-2-(4-imidazolyl)ethyl]carbamoyl}-4*H*-thieno[3,2-*b*]pyrrole

**Example#59:** 2,3-Dichloro-5-{*N*-(2-[(3-quinolyl)methyl]carbamoyl}-4*H*-thieno[3,2-*b*]pyrrole

**Example #60:** 5-{*N*-[3-(4-Acetamidophenoxy)-2-hydroxypropopyl]carbamoyl}-2,3-dichloro-4*H*-thieno[3,2-*b*]pyrrole

**Example #61:** 2,3-Dichloro-5-{*N*-[3-(*N* -methylsulphonylcarbamoyl)propyl]carbamoyl]-4*H*-thieno[3,2-*b*]pyrrole

**Example#62:** 2,3-Dichloro-5-[*N*-(2-{[2-guanidino)thiazol-4-yl]methylthio}ethyl)-carbamoyl]-4*H*-thieno[3,2-*b*]pyrrole

**Example#63:** 2,3-Dichloro-5-{*N*-[2-(2,4-dioxoimidazolidin-1-yl)ethyl]carbamoyl}-4*H*-thieno[3,2-*b*]pyrrole

**Example #64:** 5-{*N*-[2-Benzylthio-1-(hydroxymethyl)ethyl]carbamoyl}-2,3-dichloro-4*H*-thieno[3,2-*b*]pyrrole

**Example #65:** 2,3-Dichloro-5-{*N*-[2-(dimethyaminosulphonylamino)ethyl]carbamoyl}-4*H*-thieno[3,2-*b*]pyrrole

**Example #66:** 2,3-Dichloro-5-{*N*-[(6-methoxypyrid-3-yl)methyl]carbamoyl]-4*H*-thieno[3,2-*b*]pyrrole

**Example #67:** (*S*)-2,3-Dichloro-5-{*N*-[(2-oxo-3-phenyl-2,3,4,5-tetrahydrooxazol-5-yl)-methyl]carbamoyl}-4*H*-thieno [3,2-*b*]pyrrole

**Example #68:** 2,3-Dichloro-5-(*N*-{2-[3-(carbamoylmethyl)phenoxy]ethyl}carbamoyl)-4*H*-thieno[3,2-*b*]pyrrole

**Example #69:** 5-(*N*-{(6-(Benzo[1,3]dioxol-5-yl)-4-methylmorpholin-2-yl]methyl}-carbamoyl)-2,3-dichloro-4*H*-thieno [3,2-*b*]pyrrole

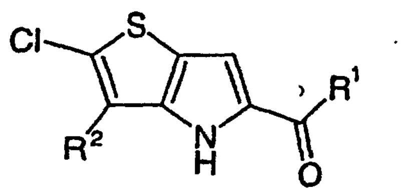

| Ex | $R^1$ | $R^2$ | M/z $(M+H)^+$ | Amine |
|---|---|---|---|---|

| | | | | |
|---|---|---|---|---|
| 32 | 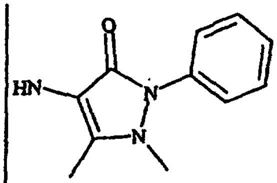 | Cl | 421 | commercially available |
| 33 | 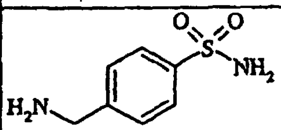 | Cl | 404 | commercially available |
| 34 | 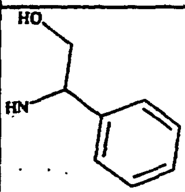 | Cl | 355 | commercially available |
| 35 | 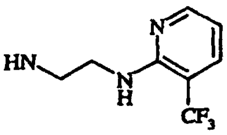 | Cl | 423 | commercially available |
| 36 | 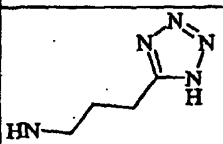 | Cl | 345 | Meth #19 |
| 37 | 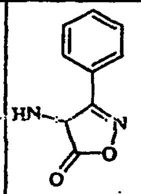 | Cl | 394 | Gazz. Chim. Ital., 1981, 111, 167 |
| 38 | 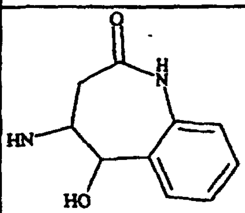 amine and hydroxy group are trans to one another | Cl | 410 | Method #26 |
| 39 | 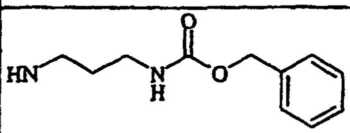 | H | 393 | commercially available |

| | | | | |
|---|---|---|---|---|
| 40 | 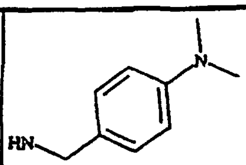 | Cl | 368 | commercially available |
| 41 | 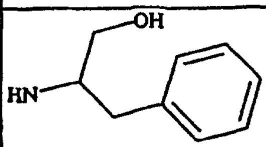 | Cl | 369 | commercially available |
| 42 | 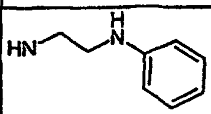 | Cl | 354 | commercially available |
| 43 | 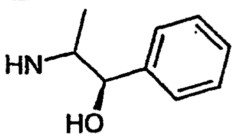 | Cl | 369 | commercially available |
| 44 | 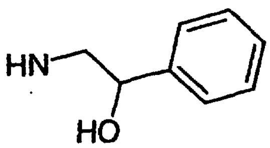 | Cl | 355 | commercially available |
| 45 | 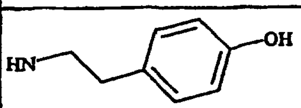 | Cl | 355 | commercially available |
| 46 | 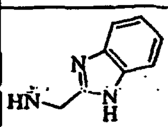 | Cl | 365 | commercially available |
| 47 | 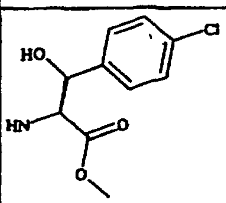 | Cl | 448 | commercially available |
| 48 | 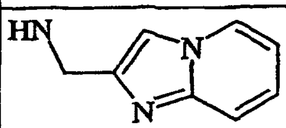 | Cl | 365 | commercially available |
| 49 | 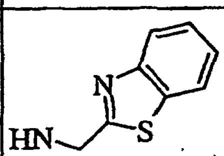 | Cl | 382 | commercially available |

| | | | | |
|---|---|---|---|---|
| 50 | 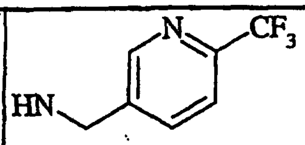 | Cl | 394 | commercially available |
| 51 | 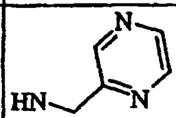 | Cl | 327 | commercially available |
| 52 | 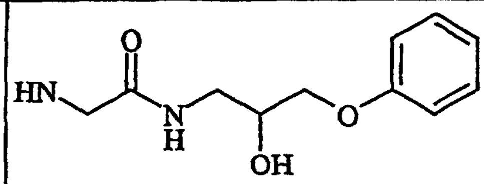 | Cl | 442 | Method #20 |
| 53 | 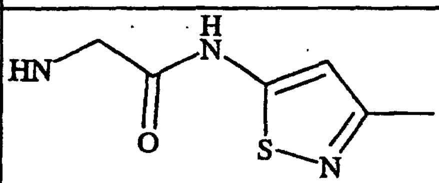 | Cl | 389 | Method #21 |
| 54 | 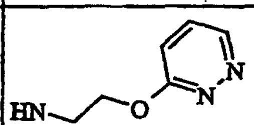 | Cl | 357 | Method #22 |
| 55 | 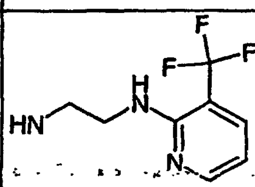 | H | 388 | commercially available |
| 56 | 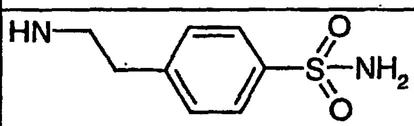 | Cl | 418 | commercially available |
| 57 | 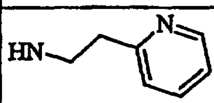 | Cl | 340 | commercially available |
| 58 | 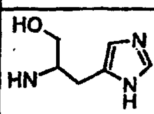 | Cl | 359 | commercially available |
| 59 | 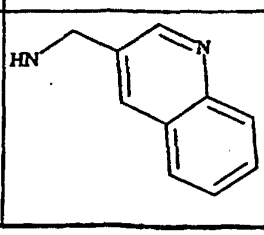 | Cl | 376 | commercially available |

| | | | | |
|---|---|---|---|---|
| 60 | 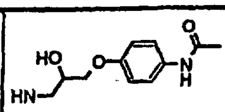 | Cl | 442 | commercially available |
| 61 | 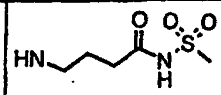 | Cl | 398 | Method #24 |
| 62 | 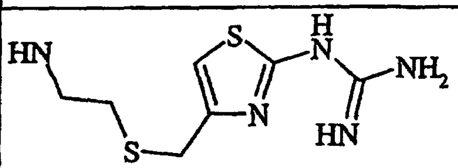 | Cl | 449 | Eur. J. Med. Chem., 1993, 28, 601 |
| 63 | 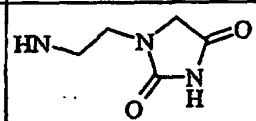 | Cl | 361 | Method #23 |
| 64 | 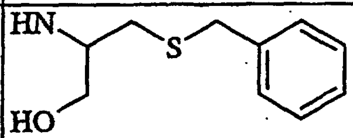 | Cl | 415 | commercially available |
| 65 | 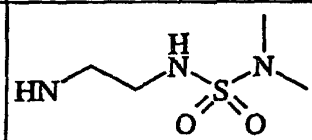 | Cl | 385 | commercially available |
| 66 | 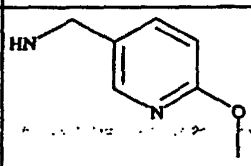 | Cl | 356 | WO 9518097 |
| 67 | 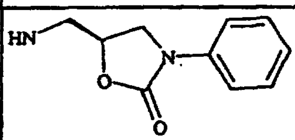 | Cl | 410 | J. Med. Chem., 1989, 32, 1673 |
| 68 | 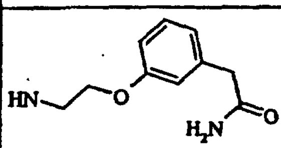 | Cl | 412 | Method #25 |
| 69 | 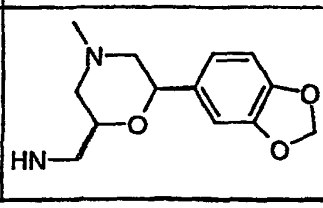 | Cl | 468 | Method #27 |

**Example #70**

*5-(N*-Benzylcarbamoyl)- 2,3-dichloro-4*H*-thieno[3,2-*b*]pyrrole

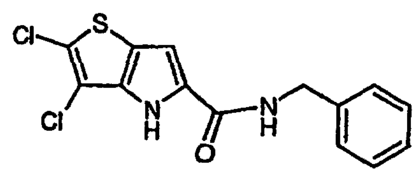

5-Carboxy-2,3-dichloro-4*H*-thieno[3,2-*b*]pyrrole (Method # 9,118mg, 0.5mmol) was dissolved in dichloromethane (10ml) containing benzylamine (55 mg, 0.5 mmol), 1-HOBT (68mg, 0.5mmol) and DIPEA (258µl, 2mmol). The mixture was stirred for one minute before the addition of EDAC (125mg, 0.65mmol). The mixture was stirred at ambient temperature for approximately 16hours before being washed with water. The organic fraction was concentrated and was purified using Bond-Elut silica column chromatography (eluent : dichloromethane-dichloromethane/methanol 5% gradient) to afford the *title compound* as a white solid (121mg, 75%).
NMR: 12.4 (1H, br), 8.8 (1H, t), 7.3 (5H, m), 7.1 (1H, s), 4.5 (2H, d); m/z 323.27

The following compounds were made by the process of Example #70 using 5-carboxy-2,3-dichloro-4*H*-thieno [3,2-*b*]pyrrole (Method #9) and the appropriate amine:

**Example #71 :** 2,3-Dichloro-5-(*N*-phenethylcarbamoyl)-4*H*-thieno[3,2-*b*]pyrrole

**Example #72 :** 2,3-Dichloro-5-[*N* (3-phenylpropyl)carbamoyl]-4*H* thieno[3.2-*b*]pyrrole

**Example #73:** 2,3-Dichloro-5-{*N*-(2-hydroxyphenyl)ethyl]carbamoyl-4*H* thieno[3,2-*b*]Pyrrole

Example #74: 2,3-Dichloro-5-[*N*-((1-phenylcyclobutyl)methyl)carbamoyl]-4*H*-thieno[3,2-*b*]pyrrole

Example #75: 2,3-Dichloro-5-[*N*(α,α-dimethylphenethyl)carbamoyl]-4*H*-thieno[3,2-*b*]pyrrole

**Example #76:** 2.3-Dichloro-5-[*N*-(β-methylphenetyl)carbamoyl]-4*H*-thieno[3,2-*b*]pyrrole

**Example #77:** 2.3-Dichloro-5-[*N*-(1,2,3,4-tetrahydronaphth-2-yl)carbamoyl]-4*H*-thieno[3,2-*b*]pyrrole

**Example #78:** 5-[*N*-(*N*-Benzylcarbamoyl]methyl)carbamoyl]-2,3-dichloro-4*H* thieno[3,2-*b*]pyrrole

**Example #79:** 5-[*N*-(*N*-Benzyl-*N*-methylcarbamoylmethyl)carbamoyl]-2,3-dichloro-4*H*-thieno[3,2-*b*]pyrrole

**Example #80:** 2,3-dichloro-5-[*N-(N*-methyl-*N*-phenylcarbamoylmethyl)carbamoyl]-4*H*-thieno[3,2-*b*] pyrrole

**Example #81:** 2,3-dichloro-5-{*N*-[*N*-(2-cyanoethyl)-*N*-phenylcarbamoylmethyl]carbamoyl}-4*H*-thieno[3,2-*b*]pyrrole

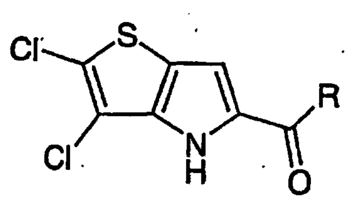

| Ex | R | NMR | M/z |
|---|---|---|---|
| #71 | 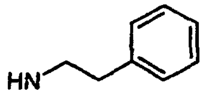 | 12.33(1H, br), 8.4(1H, t), 7.3(5H, m), 7.1(1H, s), 3.5(2H, dd), 2.8(2H, t). | 337.38 |
| #72 | 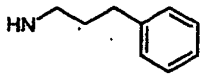 | 12.35 (1H, br), 8.3 (1H, t), 7.2 (5H, m), 7.1 (1H, s), 3.3 (2H, q), 2.6 (2H, t), 1.8 (2H, m). | 351.40 |
| #73 | 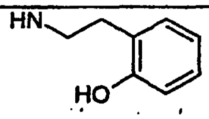 | 12.3 (1H, br), 8.3 (1H, t), 7.0 (3H, m), 6.8 (1H, d), 6.7 (1H, t), 3.4 (2H, q), 2.8 (2H, t) | 353.39 |
| #74[1] | 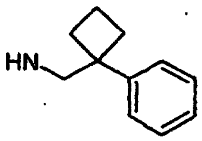 | 12.3 (1H, br), 8.1 (1H, t), 7.3 (2H, t), 7.15 (3H, m), 7.05 (1H, s), 3.6 (2H, d), 2.4 (2H, m), 2.2 (2H, m), 2.0 (1H, m), 1.8 (1H, m). | 377.44 |
| #75[2] | 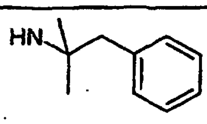 | 12.25 (1H, br), 7.4 (1H, s), 7.1 (6H, m), 3.1 (2H, s), 1.3 (6H, s) | 365.41 |
| #76[3] | 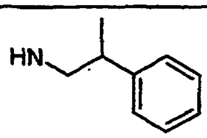 | 12.3 (1H, br), 8.3 (1h, t), 7.2 (5H, m), 7.0 (1H, s), 3.4 (2H, m), 3.0 (1H, q), 1.2 (3H, d). | no mass ion |
| #77 | 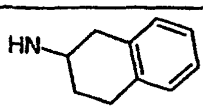 | 12.35 (1H, br), 8.2 (1H, d), 7.1 (1H, s), 7.05 (4H, s), 4.2 (1H, br), 3.1 (1H, dd), 2.8 (3H, m), 2.0 (1H, m), 1.8 (1H, m). | 363.35 |

| | | | |
|---|---|---|---|
| #78[4] | 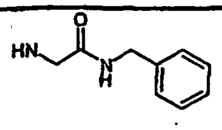 | 12.4 (1H, br), 8.6 (1H, t), 8.4 (1H, t), 7.3 (5H, m), 4.3 (2H, d), 3.9 (2H, d). | 380.41 |
| #79[5] | 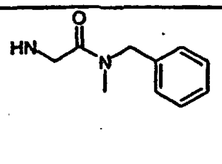 | 12.45 (1h, br), 8.4(1H, m), 7.3 (5H, m), 7.2(1H, m), 4.6 (0.6, s), 4.5(1.4H, s), 4.2(2H, m), 3.0(2H, s), 2.8(1H, s) | 394.44 |
| #80[6] | 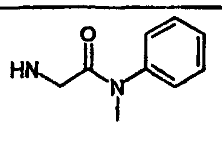 | 12.4 (1H, br), 8.4(1H, t), 7.4(5H, m), 7.1(1H, s), 3.8(2H, br), 3.2(3H, s). | 380.41 |
| #81[7] | 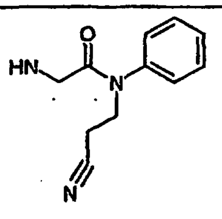 | 12.4 (1H, br), 8.4 (1H, t), 7.5 (5H, m), 7.1(1H, s), 3.9(2H, t), 3.7(2H, br), 2.7(2H, t). | 419.41 |

[1] Amine: J. Org. Chem.; 1976, 41(14), 2502-2503

[2] Amine: J. Med. Chem .; 1993, 36(22), 3300-3307

[3] Amine: J. Am. Chem. Soc.; 1960, 82, 2577

[4] Amine: Method #6

[5] Amine: Method #31

[6] Amine: Method #30

[7] Amine: Method #14

**Example #82**

2.3-dichloro-5-{*N*-[*N*-(4-methoxyphenyl)carbamoylmethyl]carbamoyl}-4*H*-thieno[3,2-*b*]pyrrole

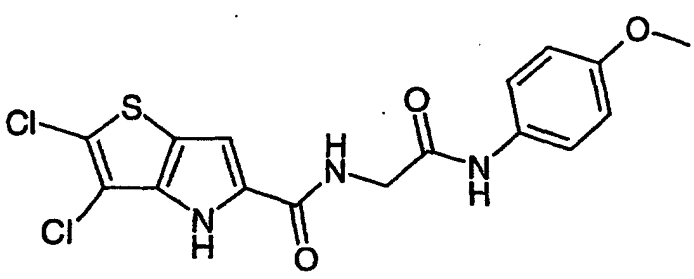

A solution of 5-(*N*-carboxymethylcarbamoyl)- 2,3-dichloro-4*H*-thieno[3,2-*b*]pyrrole (Method #12, 150mg, 0.51mmol) and 4-methoxyaniline (69mg, 0.56mmol) in tetrahydrofuran (THF) (6ml) was stirred at ambient temperature for 30 minutes. 4-(4,6-dimethoxy-1,3,5-triazin-2-yl)-4-methylmorpholinium chloride (DMTMM) (142mg, 0.51mmol) was added and the reaction mixture stirred at ambient temperature overnight, poured into water (15ml) and extracted with ethyl acetate (3x15ml). The organic extracts were combined and washed with 1N citric acid solution (15ml), sodium bicarbonate solution (15ml), dried over magnesium sulphate, filtered and concentrated to give the *title product* as a

white solid NMR: 12.4 (1H, s), 9.9 (1H, s), 8.6 (1H, t), 7.5 (2H, d), 7.2 (1H, s), 6.85 (2H, d), 4.0 (2H,d), 3.7 (3H, s); m/z 396.38

The following compounds were made by the process of Example #82 using 2,3-dichloro-5-[*N*-carboxymethylcarbamoyl]-4*H*-thieno[3,2-*b*]pyrrole (Method #12) and the appropriate commercially available amine:

**Example #83:** 2.3-dichloro-5-{*N*-[*N*-(4-fluorophenyl)carbamoymethyl]carbamoyl}-4*H*-thieno[3,2-*b*]pyrrole

**Example #84:** 2.3-dichloro-5-{*N*-[*N*-(4-nitrophenyl)carbamoylmethyl]carbamoyl)-4*H*-thieno[3,2-*b*]pyrrole

**Example #85:** 2,3-dichloro-5-{*N* [*N*-(2,6-dimethylphenyl)carbamoylmethyl]carbamoyl}-4*H*-thieno[3,2-*b*]pyrrole

**Example #86:** 2,3-dichloro-5-{*N*-[*N*-methyl-*N*-(4-methylphenyl)carbamoylmethyl]-carbamoyl}-4*H*-thieno[3,2-*b*] pyrrole

**Example #87:** 2,3-dichloro-5-{*N*-{*N*-methyl-*N*-(3-methylphenyl}carbamoy]methyl} carbamoy}-4*H*-thieno[3,2-*b*]pyrrole

**Example #88**: 2,3-dichloro-5-{*N*-[*N*-(3-chlorophenyl)*N*-methylcarbamoylmethyl carbamoyl}-4*H*-thieno[3,2-*b*]pyrrole

**Example #89:** 2,3-dichloro-5-{*N*-[*N*-(2-hydroxyethyl)-*N*-phenylcarbamoylmethyl]-carbamoyl}-4*H*-thieno[3,2-*b*]pyrrole

**Example #90:** 2.3-dichloro-5-{*N*-[*N*-(1,1-dimethyl-2-hydroxyethyl)carbamoylmethyl]-carbamoyl}-4*H*-thieno[3,2-*b*] pyrrole

**Example #91:** 2,3-dichloro-5-[*N*-[*N*-(2-hydroxyethyl)-*N*-methylcarbamoylmethyl]-carbamoyl]-4*H*-thieno[3,2-*b*]pyrrole

**Example #92**: 2,3-dichloro-5-{*N*-[*N*-(2-hydroxyethyl)carbamoymethyl]carbamoyl}-4*H*-thieno[3,2-*b*] pyrrole

**Example #93:** 2,3-dichloro-5-{*N*-[*N*-(3-hydroxypropyl)carbamoylmethyl]carbamoyl}-4*H*-thieno[3.2-*b*]pyrrole

**Example #94:** 2,3-dichloro-5-{*N*-[*N* -(4-hydroxybutylcarbamoylmethyl]carbamoyl}-4*H* thieno[3,2-*b*]pyrrole

**Example #95:** 2,3-dichloro-5-(*N*-{*N*-[bis(hydroxymethyl)methyl]carbamoylmethyl}-carbamoyl)-4*H*-thieno[3-2-*b*]pyrrole

**Example #96:** 2,3-dichloro-5-{*N*-[*N*-(2,3-dihydroxypropyl)carbamoylmethyl]carbamoyl}-4*H*-thieno[3,2-*b*]pyrrole

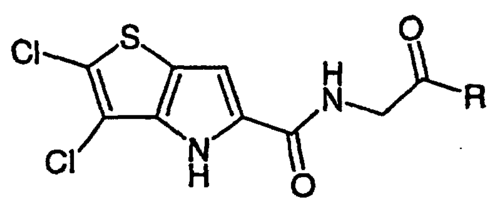

| Ex | R | NMR | m/z |
|---|---|---|---|
| #83 | 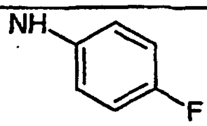 | 12.42 (1H, br), 10.08 (1H, br), 8.6 (1H, t), 7.6 (2H, m), 7.1 (3H, m), 4.0 (2H, d) | 384.20 |
| #84 | 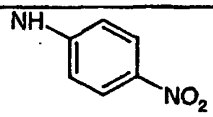 | 12.5 (1H, br), 10.74 (1H, br), 8.8 (1H, t), 8.3 (2H, d), 7.9 (2H, d), 7.2 (1H, s), 4.2 (2H, d). | 411.36 |
| #85 | 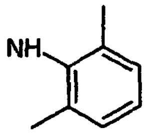 | 12.45 (1H, br), 9.3 (1H, br), 8.7 (1H, t), 7.2 (1H, s), 7.1 (3H, s), 4.1 (2H, d), 2.1 (6H, s). | 394.39 |
| #86 | 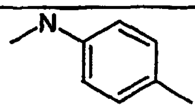 | 12.4 (1H, br), 8.4 (1H, t), 7.3 (4H, s), 7.1 (1H, s), 3.7 (2H, br), 3.2 (3H, s), 2.3 (3H, s) | 394.17 |
| #87 | 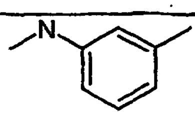 | 12.4 (1H, br), 8.4 (1H, t), 7.4 (1H, t), 7.2 (3H, m), 7.1 (1H, s), 3.8 (2H, br), 3.3 (3H, s), 2.3 (3H, s) | 394.28 |
| #88 | 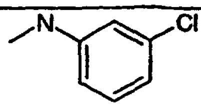 | 12.4 (1H, br), 8.4 (1H, t), 7.5 (4H, m), 7.1 (1H, s), (3.9, br), 3.2 (3H, s). | no mass ion |

| | | | |
|---|---|---|---|
| #89 | 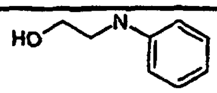 | 12.4 (1H, br), 8.4 (1H, t), 7.4 (5H, m), 7.1 (1H, s), 4.7 (1H, br), 3.7 (4H, m), 3.5 (2H, d). | 410.54 |
| #90 | 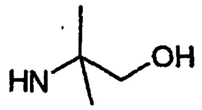 | 12.4 (1H, br), 8.4 (1H, t), 7.3 (1H, br), 7.1 (1H, s), 4.8 (1H, t), 3.8 (2H, d), 3.4 (1.3H, d), 3.1 (0.7H, d), 1.2 (6H, s) | 362.33 |
| #91 | 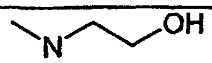 | 12.44 (1H, br), 8.3 (1H, br), 7.1 (1H, s), 4.9 (0.5H, t), 4.6 (0.5H, t), 4.1 (2H, dd), 3.4 (4H, m), 3.0 (1.5H, s), 2.8 (0.5H, s). | 348.30 |
| #92 | 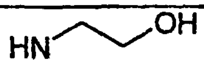 | 12.42 (1H, br), 8.5 (1H, t), 7.8 (1H, t), 7.1 (1H, s), 4.6 (1H, br), 3.8 (2H, d), 3.4 (2H, br), 3.1 (2H, q). | 334.34 |
| #93 | 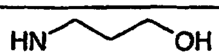 | 12.41 (1H, br), 8.5 (1H, t), 7.8 (1H, t), 7.1 (1H, s), 4.4 (1H, t, br), 3.8 (2H, d), 3.4 (2H, q, br), 3.1 (2H, q), 1.5 (2H, m). | 348.38 |
| #94 | 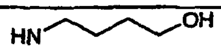 | 12.4 (1H, br), 8.5 (1H, t), 7.9 (1H, t), 7.1 (1H, s), 4.3 (1H, t), 3.8 (2H, d), 3.4 (2H, d), 3.1 (2H, q, br), 1.4 (4H, m). | 362.36 |
| #95 | 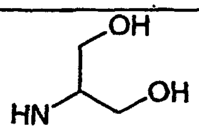 | 12.42 (1H, br), 8.5 (1H, t), 7.6 (1H, d), 7.1 (1H, s), 4.6 (2H, br), 3.9 (2H, d), 3.7 (1H, m), 3.4 (4H, br). | 364.36 |
| #96 | 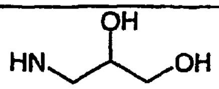 | 12. 4 (1H, br), 8.5 (1H, br), 7.8 (1H, t), 7.1 (1H, s), 4.7 (1H, br) 4.5 (1H, br), 3.8 (2H, d), 3.5 (1H, m), 3.2 (3H, m), 3.0 (1H, m). | 364.35 |

**Example #97**

2,3-dichloro-5-{*N-[N*-(4-hydroxymethylphenyl)carbamoylmethyl]carbamol}-4*H*-thieno[3,2-*b*]pyrrole

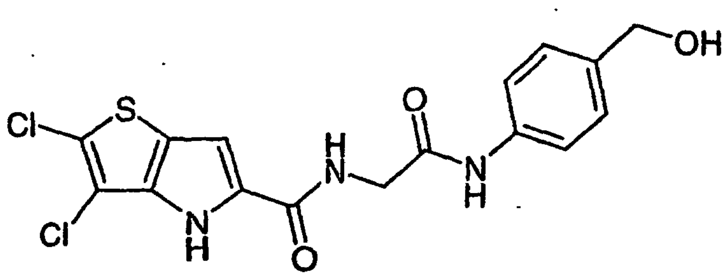

A solution of 5-(*N*-carboxymethylcarbamoyl)-2,3-dichloro-4*H*-thieno[3,2-*b*]pyrrole (Method #12) (150mg, 0.51mmol), and 4-aminobenzyl alcohol (70.5mg, 0.56mmol) in THF (6ml) was stirred, at ambient temperature for 30 minutes. 4-(4,6-dimethoxy-1,3,5-triazin-2-yl)-4-methylmorpholinium chloride (DMTMM) (142mg, 0.5 1mmol) was added and the reaction mixture stirred at ambient temperature overnight, then poured into water (15ml). The resultant precipitate was isolated by filtration, washed with water, ether and dried *in vacuo* to give the *title product* as a white solid (149mg, 73%)

NMR: 12.42 (1H, br), 9.9 (1H, s), 8.6 (1H, t), 7.5 (2H, d), 7.2 (2H, d), 7:1 (1H, s), 5.0 (1H, br), 4.4 (2H, s), 4.0 (2H, d); m/z 396.21

The following compounds were made by the process of Example #97 using 5-(*N*-carboxymethylcarbamoyl)-2,3-dichloro-4*H*-thieno[3,2-*b*]pyrrole (Method #12) and the appropriate commercially available amine:

**Example #98:** 2,3-dichloro-5-{*N*-[*N*-(5-isoquinolyl)carbamoylmethyl]carbamoyl}-4*H*-thieno(3,2-*b*]pyrrole

**Example #99:** 2,3-dichloro-5-{*N*-[*N*-(3-hydroxymethyl)phenyl]carbamoylmethyl]carbamoyl}-4*H*-thieno[3,2-*b*]pyrrole

**Example #100:** 2,3-dichloro-5-(*N*-{*N*-[4-(2-hydroxyethyl)phenyl]carbamoylmethyl]carbamoyl)-4*H*-thieno[3,2-*b*]pyrrole

**Example #101:** 2-3-dichloro-5-{*N*-[*N*-(2,4-difluorophenyl)-*N*-methyl-carbamoylmethyl]-carbamoyl}-4*H*-thieno[3,2-*b*] pyrrole

**Example #102:** 2,3-dichloro-5-{*N*-[(1,2,3,4-tetrahydro-1-quinolyl)carbamoylmethyl]-carbamoyl}-4*H*-thieno[3,2-*b*] pyrrole

**Example #103:** 2,3-dichloro-5-{*N*-[*N*-(2-cyanoethyl)-*N*-methylcarbamoylmethyl]-carbamoyl}-4*H*-thieno[3,2-*b*]pyrrole

**Example #104:** 2,3-dichloro-5-{*N*-[*N*-(4-hydroxypiperidino)carbamoylmethyl]carbamoyl}-4*H*-thieno[3,2-*b*]pyrrole

**Example #105:** 2,3-dichloro-5-[*N*-(*N* -cyclopentylcarbamoylmethyl)carbamoyl]-4*H*-thieno[3,2-*b*]pyrrole

**Example #106:** 2,3-dichloro-5-[*N*-(*N*-isopropylcarbamoylmethyl)carbamoyl]-4*H*-thieno[3,2-*b*]pyrrole

**Example #107:** 2,3-dichloro-5-[*N*-(*N*-isopropyl-*N*-methylcarbamoylmethyl)carbamoyl]-4*H*-thieno[3,2-*b*]pyrrole

**Example #108:** 2,3-dichloro-5-[*N*-(thiomorpholinocarbonylmethyl)carbamoyl]-4*H*-thieno[3,2-*b*]pyrrole

**Example #109:** 2,3-dichloro-5-[*N*-(morpholinocarbonylmethyl)carbamoyl]-4*H*-thieno[3,2-*b*]pyrrole

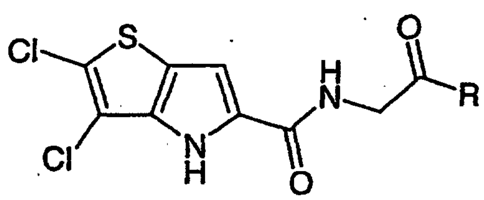

| Ex | R | NMR | m/z |
|---|---|---|---|
| #98 | 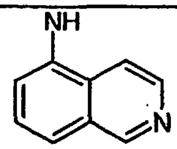 | 12.5 (1H, s), 8.7 (1H, t), 8.5 (1H, d), 8.0 (3H, m), 7.7 (1H, t), 7.1 (1H, s), 4.2 (2H, d). | |
| #99 | 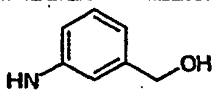 | 12.42 (1H, br), 9.9 (1H, s), 8.6 (1H, t), 7.55 (1H, s), 7.45 (1H, d), 7.2 (1H, t), 7.1 (1H, s), 7.0 (1H, d), 5.1 (1H, br), 4.4 (2H, s), 4.0 (2H, d) | 396.19 |
| #100 | 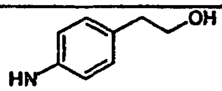 | 12.41 (1H, br), 9.9 (1H, s), 8.6 (1H, t), 7.5 (1H, m), 7.1 (3H, m), 4.6 (1H, br), 4.0 (2H, d), 3.6 (2H, br), 2.6 (2H, t) | 410.16 |
| #101 | 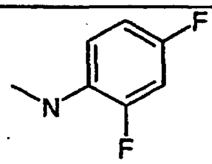 | 12.4 (1H, br), 8.5 (1H, t), 7.7 (1H, q), 7.5 (1H, dt), 7.2 (1H, t, br), 7.1 (1H, s), 3.8 (1H, m), 3.6 (1H, m), 3.1 (3H, s). | No mass ion |
| #102 | 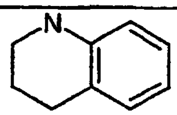 | 12.41 (1H, br), 8.5 (1H, t), 7.6 (1H, d), 7.1 (5H, m), 4.2 (2H, d), 3.7 (2H, t), 2.7 (2H, t), 1.9 (2H, m). | 406.18 |

| | | | |
|---|---|---|---|
| **#103** | 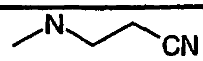 | 12.42 (1H, br), 8.4 (1H, m), 7.1 (1H, s), 4.1( 2H, m), 3.7 (0.5H, t), 3.6 (1.5H, t), 3.1 (2H, s), 2.9 (1.5H, m), 2.7 (1.5H, t). | 357.27 |
| **#104** | 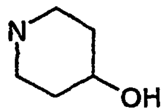 | 12.42 (1H, br), 8.4 (1H, t), 7.1 (1H, s), 4.7 (1H, br), 4.1 (2H, d), 3.9 (1H, m), 3.7 (2H, m), 3.2 (1H, m), 3.0 (1H, m), 1.7 (2H, m), 1.3 (2H, m). | 376.26 |
| **#105** | 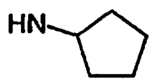 | 12.4 (1H, br), 8.45 (1H, t), 7.8 (1H, d), 7.1 ( 1H, s), 4.0 (1H, m), 3.8 (2H, d), 1.8 (2H, m), 1.6 (2H, m), 1.5 (2H, m), 1.4 (2H, m). | 358.35 |
| **#106** | 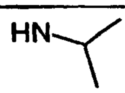 | 12.4 (1H, br), 8.5 (1H, t), 7.7 (1H, d), 7.1 (1H, s), 3.9 (1H, m), 3.8 (2H, d), 1.1 (6H, d). | 332.34 |
| **#107** | 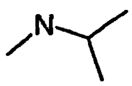 | 12.42 (1H, br), 8.3 (1H, m), 7.1 (1H, s), 4.6 ( 0.66H, m), 4.1 (2.33H, m), 2.8 (2H, s), 2.7 (1H, s), 1.15 (2.4H, d), 1.05 (3.6H, d). | 346.34 |
| **#108** | 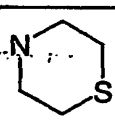 | 12.42 (1H, br), 8.4 (1H, t), 7.1 (1H, s), 4.2 (1H, d), 3.7 (4H, m), 2.7 (2H, br), 2.5 (2H, br). | no mass ion |
| **#109** | 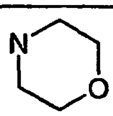 | 12.42 (1H, br), 8.4 (1H, t), 7.1 (1H, s), 4.2 (2H, d), 3.6 (4H, br), 3.5 (4H, br). | 360.30 |

**Example #110**

2,3-dichloro-5-{N[(1,1-dioxothiomorpholino)carbonylmethyl]carbamoyl}-4*H*-thieno[3,*2b*]-pyrrole and

**Example #111**

2,3-dichloro-5-{*N*-[(1-oxothiomorpholino)carbonylmethyl]carbamoyl}-4*H*-thieno[3,2-*b*]pyrrole

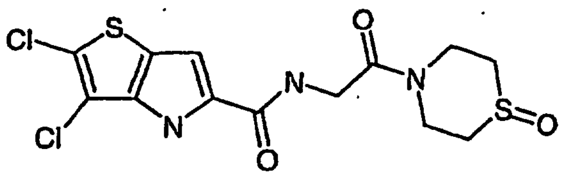

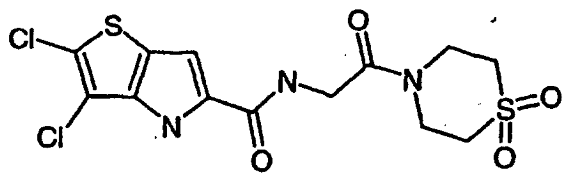

A solution of *m*-chloroperbenzoic acid (mCPBA) (14mg, 0.85mmol) in dichloromethane (5ml) was added dropwise to a suspension of 2,3-dichloro-5-[*N*-(thiomorpholinocarbonylmethyl)carbamoyl)-4*H*-thieno[3,2-*b*]pyrrole (example **# 108**) in dichloromethane and the reaction mixture stirred at ambient temperature for one hour. 5% sodium metabisulfite solution (5ml) was added and the mixture stirred for several minutes. The aqueous layer was extracted with ethyl acetate (2x15ml) and the combined organic extracts washed with sodium bicarbonate solution (2x15ml) and concentrated. The two component mixture was separated using bond-elute silica column chromatography (eluent: dichloromethane-dichloromethane/methanol 5% gradient) to afford the less polar product (sulfone) as a white powder (57mg 33%) and the more polar product (sulfoxide) as a white solid (62mg, 37%).
NMR: (sulfone) 12.43 (1H, br), 8.4 (1H, t), 7.1 (1H, s), 4.2 (2H, d), 3.9 (4H, br), 3.3 (2H, br), 3.1 (2H, br); m/z 408.33
NMR: (sulfoxide) 12.42 (1H, br), 8.4 (1H, t), 7.1 (1H, s), 4.2 (3H, m), 3.9 (2H, d), 3.6 (1H, m), 2.9 (4H, m).

**Example #112**

2-Chloro-5-[*N*-(2-indanyl)carbamoyl]-6*H*-thieno[2,3-*b*]pyrrole

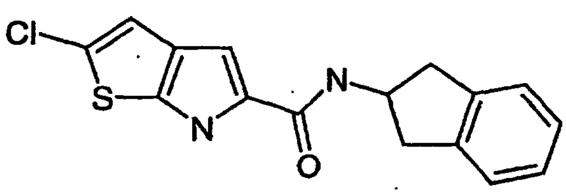

5-Carboxy 2-chloro-6*H* thieno[2,3-*b*]pyrrole (Method #10; 101mg, 0.5mmol) was dissolved in dichloromethane (6ml) containing 2-aminoindane (68mg, 0.5mmol), 1-Hydroxybenotriazole (HOBT) (68mg, 0.5mmol) and DIPEA (355µl, 2.0mmol). The mixture was stirred for one minute before the addition of EDAC (125mg, 0.65mmol). The mixture was stirred at ambient temperature for approximately 16 hours before being washed with water. The organic fraction was concentrated and was purified using bon-elute silica-column chromatography (eluent: dichloromethane-dichloromethane/methanol 2.5% gradient) to afford the *title compound* as a beige solid (96mg, 61 %).
NMR: 11.80 (1H, br), 8.3 (1H, t), 7.2 (5H, m), 7.0 (1H, s), 4.6 (1H, m), 4.7 (1H, d), 3.2 (2H, m), 2.9 (2H, m); m/z 315.46

**Example #113**

5-[*N*-(Benz[1,2]oxazol-3-ylmethyl)carbamoyl]-2,3-dichloro-4*H*-thieno[3,2-*b*]pyrrole

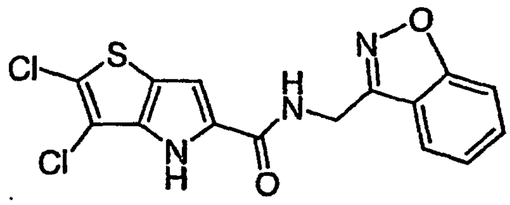

5-Carboxy-2,3-dichloro-4*H*-thieno[3,2-*b*]pyrrole (Method #9; 118 mg, 0.5 mmol) was dissolved in DMF (5 ml) containing HOBT (83 mg, 0.55 mmol), DIPEA (52 ul, 0.30 mmol) and 1,2-Benzisoxazole-3-methylamine (Eur.J.Med. Chem-Chimica Therapeutica, Jan.-Feb.-10, No. 1 p32) (89 mg, 0.6 mmol). The mixture was stirred for one minute and EDAC (106 mg, 0.55 mmol) was added. The mixture was stirred at room temperature for approximately 18 hours. Water was added to the solution and a solid precipitated out. This solid was filtered off and was washed with water before being dried under reduced pressure to afford the title compound as a white solid (174mg) 1H NMR: 4.9(2H,s), 7.1(1H,s),7.4(1H,t),7,6(1H,t) (7.8(1H,d), 8.0(1H,d), 9.2(1H,s), 12.5(1H,s); m/z 366 (M+H);
HPLC Hichrome C 18 column Acetonitrile/water/0.1 %TFA 5-95% over 7.5 min Rt 4.9min m/z 366 (M+H)

The following compounds were made by the process of Example #113 using 5-carboxy-2,3-dichloro-4*H*-thieno [3,2-*b*]pyrrole (Method #9) and the appropriate amine:

**Example#114:** 2,3-Dichloro-5-(*N*-{2-[2-(hydroxymethyl)phenyl]carbamoyl)-4*H*-thieno[3,2-*b*]pyrrole

**Example #115:** 2,3-Dichloro-5-[*N*-(4-phenylisoxazol-3-ylmethyl)carbamoyl]-4*H*-thieno[3,2-*b*]pyrrole

**Example #116:** 2,3-Dichloro-5-(*N*-{2-[2-(2-morpholinoethoxy)phenyl]ethyl}carbamoyl)-4*H*-thieno[3,2-*b*]pyrrole

**Example #117:** 2,3-Dichloro-5-(*N*-{2-[2-(methoxycarbonylmethoxy)phenyl)]ethyl}-carbamoyl)-4*H*-thieno[3,2-*b*]pyrrole

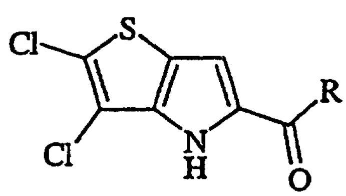

| Ex | R | NMR | Rt | M/z |
|---|---|---|---|---|
| #114 [1] | 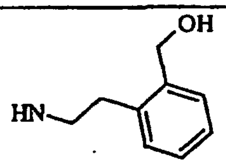 | 1H NMR : 2.9(2H,q), 3.4(2H,q), 4.6(2H,d), 5.1(1H,t), 7.1(1H,s), 7.2(3H,m), 7.4(1H,m), 8.4(1H,m) | 4.55 | 369 |
| #115 [2] | 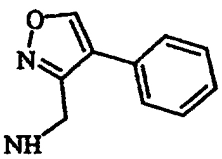 | 1H NMR : 4.7(2H,s), 7.1(1H,s), 7.4(3H,m), 7.6(2H,m), 8.8(1H,t), 9.2(1H,s) | 5.01 | 392 |
| #116 [3] | 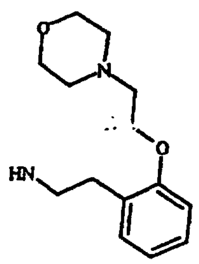 | 1H NMR (CDCl3) : 2.6(4H,m), 2.8(2H,t), 3.0(2H,t), 3.7(6H,m), 4.1(2H,t), 6.3(1H,m), 6.6(1H,s), 6.9(2H,m), 7.2(2H,m), 9.8(1H,b) | 4.26 | 468 |
| #117 [4] | 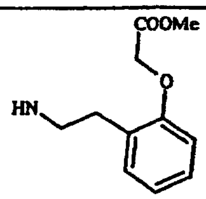 | (CDCl3) : 3.0(2H,q), 3.8(2H,q), 3.9(3H,s), 4.7(2H,s), 6.6(1H,s), 6.7(1H,s), 6.8(1H,d), 7.0(1H,t), 7.2(2H,m) | 4.47 | 413 |

[1] Amine: EP86-300884

[2] Amine: Method #33

[3] Amine: Method #34

[4] Amine: Method #35

**Example #118**

5-(*N*-{2-[2-(Carboxymethoxy)phenyl]ethyl}carbamoyl)-2,3-dichloro-4*H*-thieno[3,2-*b*]pyrrole

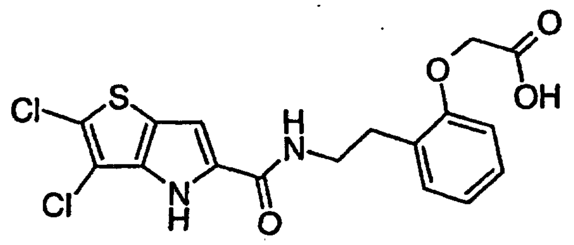

2,3-Dichloro-5-(*N*-{2-[2-(methoxycarbonylmethoxy)phenyl]ethyl}carbamoyl)-4*H*-thieno[3,2-*b*]pyrrole (Example #117, 100mg,0.25mmol) dissolved in 2:1 THP: methanol (2ml), was treated with 1N Lithium hydroxide solution (0.25ml, 0.25mmol), followed by the addition of water till the solution was just opalescent and then stirred for 2 hours at room temperature. The organic solvents were removed by evaporation under reduced pressure, the solution filtered then acidified with 2N HCl to give a thick white precipitate which was isolated by filtration, washed with water and dried under reduced pressure over phosphorous pentoxide to give the title compound (83mg)
1H NMR : 2.9(2H,q), 3.5(2H,q), 4.7(2H,s), 6.8(2H,m), 7.1(1H,s), 7.2(2H,m), 8.4(1H,m), 12.4(1H,s)
HPLC Hichrome C18 column Acetonitrile/water/0.1 %TFA 5-95% over 7.5 min Rt 4.47min m/z 413 (M+H)

**Example #119**

2,3-Dichloro-5-{*N*-[2-(3-methoxyphenyl)ethyl]carbamoyl}-4*H*-thieno[3, 2-*b*]pyrrole

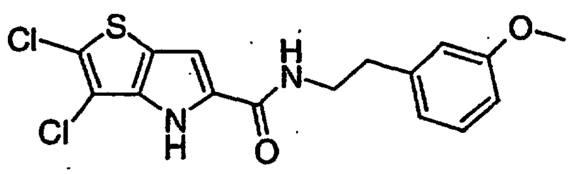

5-Carboxy-2,3-dichloro-4*H*-thieno[3,2-*b*]pyrrole (Method #9; 100mg; 0.42mmol) was suspended in DCM 10ml; 2M oxalyl chloride solution in DCM (750ul, 1.5mmol) was added dropwise followed by one drop of DMF and the resultant mixture stirred overnight at room temperature. The suspension was filtered, the residue washed with DCM and the filtrate evaporated to dryness under reduced pressure and then azeotroped with toluene to give a yellow solid which was dissolved in DCM (6.9ml) under nitrogen. Calcium carbonate (60mg, 0.6mmol) was added followed by a solution of 3-methoxyphenethylamine; 90mg, 0.4mmol) and the mixture was stirred at room temperature overnight. The mixture was filtered, the residue washed with DCM followed by 0.1 M HCl then water to give the title compound as a white solid (98mg) 1H NMR (CDCl3) : 2.9(2H,t), 3.7(2H,t), 3.9(3H,s), 6.0(1H,m), 6.6(1H,s), 6.8(3H,m), 7.2(2H, m), 9.9(1H,s)
m/z 369 (M+H)

**Example #120**

2,3-Dichloro-5-[*N*-(2-oxo-1,2,3,4-tetrahydroquinol-3-yl)carbamoyl]-4*H*-thieno[3,2-*b*]pyrrole

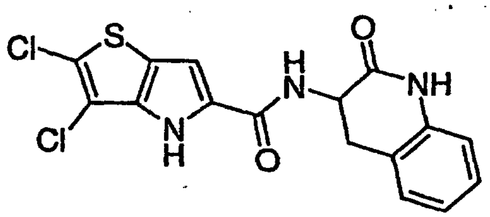

This was prepared following the procedure described in Example #97 using 3-aminohydrocarbostyril (Arch. Biochem&Biophys. 1963 109 48) was used in place of 4-aminobenzyl alcohol and 5-carboxy-2,3-dichloro-4*H*-thieno [3,2-b]pyrrole (Method #9) was used in place of 5-(*N*-carboxyrnethylcarbamoyl)-2,3-dichloro-4*H*-thieno[3,2-*b*]pyrrole to give the title compound: 1H NMR: 3.0(2H,d), 4.7(1H,q), 6.9(3H,m), 7.2(2H,m), 8.5(1H,d), 10.35(1H,s)
HPLC Hichrome C18 column Acetonitrile/water/0.1 %TFA 5-95% over 7.5 min Rt 4.44min m/z 380 (M+H)

**Example #121**

2,3-Dichloro-5-(*N*-{2-[2-(2-methoxyethoxy)phenyl]ethyl}carbamoyl)-4*H*-thieno[3,2-*b*]pyrrole

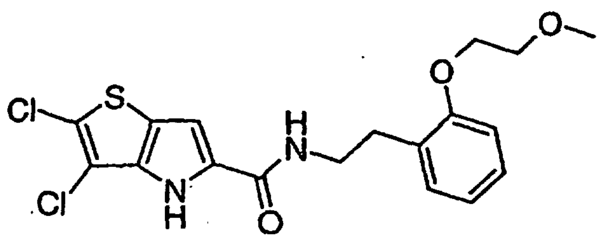

2,3-Dichloro-5-{*N*-[2-(2-hydroxyphenyl)ethyl)carbamoyl}-4*H*-thieno[3,2-*b*]pyrrole ( Example 73, 177.5mg, 0.5mmol) was dissolved in THF 3ml under nitrogen together with methoxyethanol (38µl, 0.5mmol) and triphenylphosphine (131mg, 0.5mmol), the resultant stirred solution was cooled to 0C and treated with DIAD (98µl,0.5mmol) dropwise over 30 mins, then allowed to warm to room temperature overnight. After evaporation to dryness the mixture was purified by chromatography on a 20g Bond Elute silica column eluting with DCM. The product was taken up in diethyl ether 20ml, washed with 2N NaOH (3x5ml), water (5ml) and saturated brine (5ml) then dried over magnesium sulphate and evaporated under reduced pressure to give the title compound (45mg) 1H NMR (CDCl3): 3.0(2H,t), 3.5(3H,s), 3.7(2H, m), 3.9(2H,m), 4.2(2H,m), 6.6(1H,m), 6.8(1H,s), 6.9(2H,m), 7.2(2H,m), 9.9(1H,s) HPLC Hichrome C18 column Acetonitnle/water/0.1%TFA 5-95% over 7.5 min Rt 5.02min m/z 411 (M-H)

**Example #122**

5-(*N*-2-[2-(Carbamoylmethoxy)phenyl]ethyl}carbamoyl)-2,3-dichloro-4*H*-thieno[3,2-*b*]pyrrole

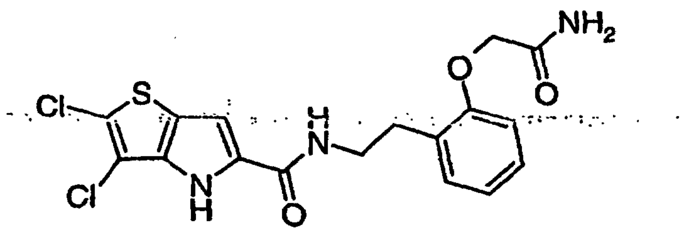

5-(*N* {2-[2-(Carboxymethoxy)phenyl]ethyl)carbamoyl)-2,3-dichloro-4*H*-thieno[3,2-*b*]pyrrole (Example #118,124mg, 0.3 mmol) was dissolved in DMF (3 ml) containing HOBT (50 mg, 0.33 mmol), DIPEA (140 µl, 0.69 mmol) and ammonium chloride (18mg, 0.36 mmol). The mixture was stirred for one minute and EDAC (64mg, 0.33 mmol) was added. The mixture was stirred at room temperature for approximately 18 hours. Water was added to the solution and a solid precipitated out. This solid was filtered off and was washed with water before being dried under reduced pressure to afford the title compound as a white solid (111mg)1H NMR: 2.9(2H,q), 3.5(2H,q), 4.5(2H,s), 6.8(2H,m), 7.0 (1H,s), 7.2(2H,m), 7.5(1H,s), 7.6(1H,s), 8.4(1H,m), 12.4(1H,s)
HPLC Hichrome C18 column Acetonitrilelwater/0.1%TFA 5-95% over 7.5 min Rt 4.37min
m/z 412 (M+H)

The following compounds were made by the process of Example #122 using 5-*(N*-{2-[2-(Carboxymethoxy) phenyl]ethyl}carbamoyl)-2,3-dichloro-4*H*-thieno[3,2-*b*]pyrrole (Example #118) and the appropriate amine:

**Example #123:** 2,3-Dichloro-5-(*N*-{2-[2-(*N*-methylcarbamoylmethoxy)phenyl]ethyl}-carbamoyl)-4*H*-thieno[3,2-*b*] pyrrole

**Example #124:** 2,3-Dichloro-5-(*N*-{2-[2-(*N*,*N*-dimethylcarbamoylmethoxy)phenyl]-ethyl}carbamoyl)-4*H*-thieno[3,2-*b*] pyrrole

**Example #125:**2,3-Dichloro-5-(*N*-{2-[2-(morpholinocarbonylmethoxy)phenyl]ethyl}-carbamoyl)-4*H*-thieno[3,2-*b*] pyrrole

**Example #126:** 5-(*N*-(2-{2-(*N*-Benzylcarbamoylmethoxy)phenyl]ethyl}carbamoyl)2,3-dichloro-4*H*-thieno[3,2-*b*]pyrrole

**Example #127:** 2,3-Dichloro-5-(*N*-{2-[2-(4-hydroxypiperidinocarbonylmethoxy)phenyl]- ethyl}carbamoyl)-4*H*-thieno [3,2-*b*]pyrrole

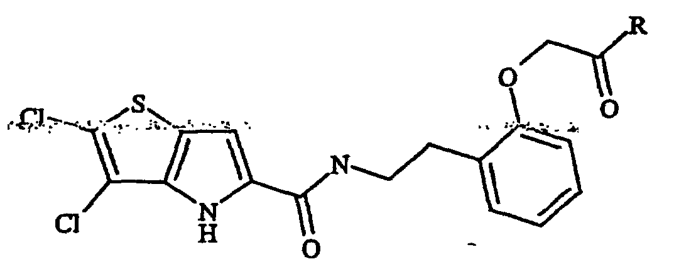

| Ex | R | NMR | Rt | M/z |
|---|---|---|---|---|
| #123 | 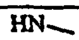 | 1H NMR : 2.8(3H,d), 2.9(2H,q), 3.5(2H,q), 4.5(2H,s), 6.8(2H,m), 7.1(1H,s), 7.2(2H,m), 8.0(1H,m), 8.5(1H,m), 12.2(1H,s) | 4.65 | 426 |
| #124 | 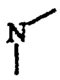 | 1H NMR : 2.9(5H,m), 3.0(3H,s), 3.5(2H,q), 4.8(2H,s), 6.8(2H,m), 7.0(1H,s), 7.1(2H,m), 8.4(1H,m), 12.4(1H,s) | 4.57 | 440 |
| #125 | 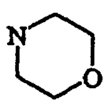 | 1H NMR : 2.9(2H,q), 3.4-3.7(10H,m), 4.8(2H,s), 6.8(2H,m), 7.0(1H,s), 7.1(2H,m), 8.3(1H,m), 12.4(1H,s) | 4.5 | 484 |
| #126 | 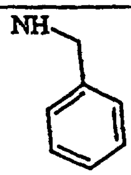 | 1H NMR : 2.9(2H,q), 3.5(2H,q), 4.4(2H,d), 4.6(2H,s), 6.9(2H,m), 7.0(1H,s), 7.2-7.4(7H,m), 8.4(1H,m), 8.6(1H,m), 12.4(1H,s) | 5.26 | 502 |
| #127 | 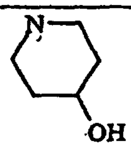 | 1H NMR : 1.2-1.5(2H,m), 1.6-1.8(2H,m), 2.9(2H,q), 3.1(1H,q), 3.2(1H,q), 3.5(2H,q), 3.7(2H,q), 3.9(1H,m), 4.7(1H,d), 4.8(2H,s), 6.9(2H,m), 7.1(1H,s), 7.2(2H,m), 8.0(1H,s), 8.4(1H,m), 12.4(1H,s) | 4.2 | 496 |

Example #128

(S)-2-Chloro-5-{*N*-[α-(5-ethoxycarbonyl-1,3,4-oxadiazol-2-yl)phenethyl]carbamoyl)-6*H*-thieno[2,3-*b*]pyrrole

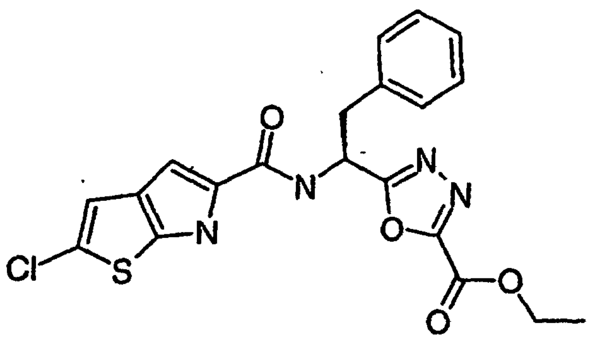

5-Carboxy-2-chloro-6*H*-thieno[2,3-*b*]pyrrole (Method #10 172mg, 0.86mmol) was dissolved in DCM (10ml) containing HOBT ( 115mg, 0.86.mmol), DIPEA (331mg,2.57mmol) and ethyl (*S*)-5-((α-aminophenethyl)-1,3,4-oxadiazole,2-carboxylate trifluoroacetate (Method #16; 322mg, 0.86mmol). EDAC (205mg,1.07mmol) was added and the mixture stirred at ambient temperature for 16 hours. The reaction mixture was filtered and the filtrate washed with dilute hydrochloric acid and water. After drying over magnesium sulphate and concentration the crude material was purified by bond elute silica column chromatography (eluent - DCM/Ethyl acetate gradient 0-20%) to give the title compound (104mg, 27%)

NMR 1.3(3H, t); 3.3-3.5(2H, m); 4.4(2H,q); 5.5-5.6(1H,m); 7.05(1H,s); 7.15(lH,s); 7.2-7.35(5H,m); 8.95(1H,d); 11.84 (1H,s)

m/z 444.9

Example #129

(*S*)-2-chloro-5-{*N*-[α-(4-methoxycarbonyloxazol-5-yl)phenethyl]carbamoyl}-6*H*-thieno[2,3-*b*]pyrrole

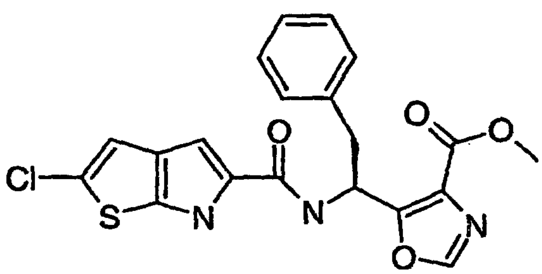

5-Carboxy-2-chloro-6*H*-thieno[2,3-*b*]pyrrole (Method #10, 100mg, 0.5mmol) was dissolved in DCM (10ml) containing HOBT (68mg, 0.5mmol), DIPEA (193mg, 1.5mmol) and methyl (*S*)-5-(α-aminophenethyl)oxazole-4-carboxylate trifluoroacetate (Method #32, 230mg, 0.5mmol). EDAC (143mg, 0.75mmol) was added and the mixture stirred at ambient temperature for 4 hours. The reaction mixture was diluted with ethyl acetate (75ml) washed temperature for 4 hours. The reaction mixture was diluted with ethyl acetate (75ml) washed with dilute citric acid, water and brine, dried over magnesium sulphate and concentrated. The crude material was purified by bond elute silica column chromatography (eluent - DCM/Ethyl acetate gradient 0-50%) to give the title compound (147mg, 34%).

NMR 3.05-3.15(1H,m); 3.2-3.25(1H,m); 3.8(3H,s); 5.85-5.95(1H,m); 7.1(1H,s); 7.15(1H,s); 7.15-7.25(5H,m); 7.75(1H, d) 8.4(1H,s); 12.74(1H,s); m/z 429

**Example #130**

2-Chloro-5-{*N*-[α-(3-pyridyl)phenethyl]carbamoyl}-6*H*-thieno[2,3-*b*]pyrrole

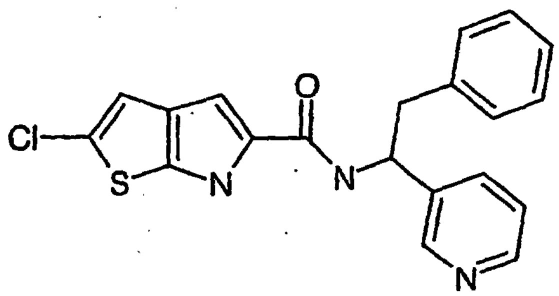

5-Carboxy 2-chloro-6*H* thieno[2,3-*b*] pyrrole (Method #10, 100mg, 0.5mmol) was dissolved in DCM (10ml) containing HOBT (68mg, 0.5mmol), DIPEA (193mg,1.5mmol) and α-(3-pyridyl)phenethylamine dihydrochloride (J. Am. Chem. Soc., 1950,72,1988; 135mg, 0.5mmol). EDAC (143mg,0.75mmol) was added and the mixture stirred at ambient temperature for 16 hours. The reaction mixture was diluted with ethyl acetate (50ml), water (20ml) was added and the pH adjusted to 7 with dilute hydrochloric acid. The organic fraction was separated washed with water and brine, dried over magnesium sulphate and concentrated. The crude material was purified by bond elute silica column chromatography (eluent - Ethyl acetate) to give the title compound as a solid (128mg, 67%).
NMR 3.0-3.2(2H,m); 5.2-5.3(1H,m); 7.05(1H,s); 7.1-7.2(2H,m); 7.2-7.4(5H,m); 7.8(1H,d); 8.4(1H,d); 8.55-8.65(2H,m); 11.71(1H,s); m/z 381

**Example #131**

2,3-dichloro-5-{*N*-[α(3-pyridyl)phenethy)]carbamoyl}-4*H*-thieno[3,2-*b*]pyrrole

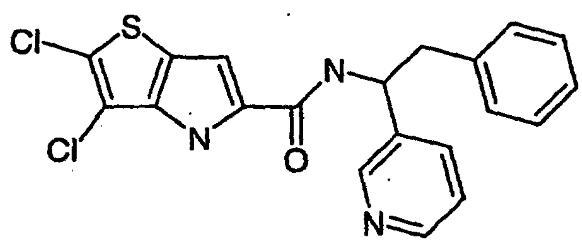

5-carboxy-2,3-dichloro-4*H*-thieno[3,2-*b*]pyrrole (Method #9, 118mg,0.5mmol) was dissolved in DCM (10ml) containing HOBT (68mg, 0.5mmol), DIPEA (193mg,1.5mmol), α-(3-pyridyl)phenethyl)amine dihydrochloride (J. Am. Chem. Soc.,1950, 72,1988; 135mg, 0.5mmol). EDAC (143mg,0.75mmol) was added and the mixture stirred at ambient temperature for 16 hours. The reaction mixture was diluted with ethyl acetate (100ml), water (20ml) was added and the pH adjusted to 7 with dilute hydrochloric acid. The organic fraction was separated washed with water and brine, dried over magnesium sulphate and concentrated. The crude material was purified by bond elute silica column chromatography (eluent - Ethyl acetate) to give the title compound as a solid (72mg, 34%)
NMR 3.0-3.3(2H,m); 5.2-5.3(1H,m); 7.1-7.2(2H,m); 7.2-7.4(5H,m); 7.85(1H,dt); 8.4(1H,dd); 8.6(1H,s); 8.75(1H,d); 12.28(1H,s); m/z 417

**Example #132**

(*S*)-2-Chloro-5-{*N*-[α-(3-phenyl-1,2,4-oxadiazol-5-yl)phenethyl]carbamoyl}-6*H*-thieno[2,3-*b*]pyrrole

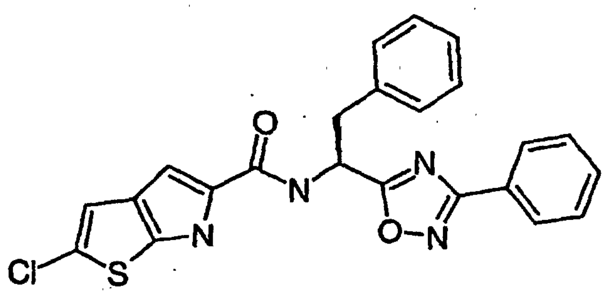

5-Carboxy-2-chloro-6*H*-thieno[2,3-*b*] pyrrole (Method #10, 201mg, 1mmol) was dissolved in DCM (15ml) containing HOBT (148mg, 1.1mmol), DIPEA (387mg,3mmol) and (S)-5 (α-aminophenethyl)-3-phenyl-1,2,4-oxadiazole trifluoroacetate (Method #18, 379mg, 1mmol). EDAC (238mg,1.25mmol) was added and the mixture stirred at ambient temperature for 16 hours. The reaction mixture was filtered and the filtrate diluted with DCM (50ml) washed with dilute hydrochloric acid and water. After drying over magnesium sulphate and concentration the crude material was purified by bond elute silica column chromatography (eluent - DCM/Ethyl acetate gradient 0-20%) to give the title compound (120mg, 26%);
NMR 3.3-3.6(2H,m); 5.5-5.7(1H,m); 7.1(1H,s); 7.15-7.4(6H,m); 7.55-7.65(3H,m); 8.05(2H,d); 9.05(1H,d); 11.9(1H,s); m/z 449

**Example #133**

2,3-Dichloro-5-[*N*-(1-hydroxyindan-2-yl)carbamoyl]-4*H*-thieno[3,2-*b*]pyrrole

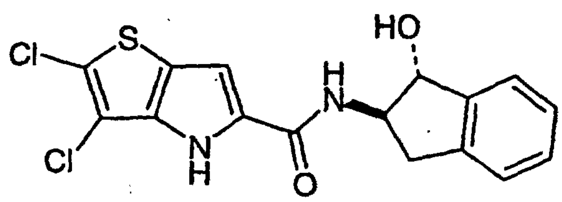

5-Carboxy-2,3-dichloro-4*H*-thieno[3,2-*b*]pyrrole (Method #9,329 mg, 1.4 mmol) was dissolved in DCM (20 ml) containing HOBT (189 mg, 1.4 mmol), DIPEA (0.5 ml, 2.8 mmol) and 2-aminoindan-1-ol (Method #36, 250 mg, 1.4 mmol). The reaction mixture was stiired for one minute and EDAC (306 mg, 1.6 mmol) was added. The reaction mixture was stirred at room temperature for approximately 18 hours. The resulting solution was washed with water (20 ml) and the aqueous layer extracted with DCM (2 x 20 ml). The organic extracts were combined, dried over magnesium sulphate and concentrated under reduced pressure to give the title compound as a white solid (70 mg, 14%).
NMR: 2.8(1H,dd), 3.2(1H,dd), 4.4(1H,quin), 5.1(1H,d), 7.1(1H,s), 7.2-7.4(4H.m), 8.7(1H,d), 12.4(1H,s); m/z 366 (M-H)

**Example #134**

2,3-Dichloro-5-[*N*-((*1S,2S*)-1-hydroxyindan-2-yl)carbamoyl]-4*H*-thieno[3,2-*b*]pyrrole

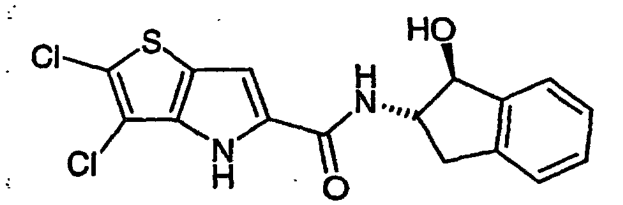

and

**Example #135**

2,3-Dichloro-5-[*N*-((*1R,2R*)-1-hydroxyindan-2-yl)carbamoyl]-4*H* thieno[3,2-*b*]pyrrole

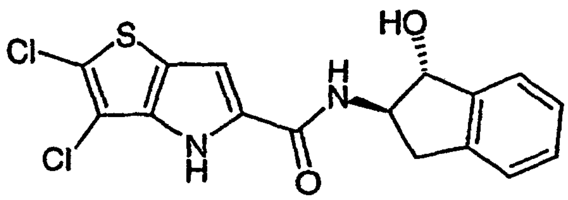

2,3-Dichloro-5-[*N*-(1-hydroxyindan-2-yl)carbamoyl]-4*H*-thieno[3,2-*b*]pyrrole (Example #133) was subject to preparative HPLC under the following conditions to give 2,3-dichloro-5-[*N*-( (1*S*,2*S*)-1-hydroxyindan-2-yl)carbamoyl]-4*H*-thieno[3,2-*b*]pyrrole as a white solid (9 mg) and 2,3-dichloro-5-[*N*-((1*R*,2*R*)-1-hydroxyindan-2-yl)carbamoyl]-4*H*-thieno[3,2-*b*]pyrrole as a white solid (12 mg).

| | |
|---|---|
| Instrument | P.E Series 200 system 1 |
| Column | Chiralpak AD (250mm x 4.6 mm) No.ADOOCE-AJ052 |
| Eluent | MeOH |
| Oven Temperature | Ambient |
| Flow | 1ml/min |
| Wavelength | 254 nm |
| Sample concentration | 1mg/ml in EtOH+sonication |

**Example #136**

2-Chloro-5-[*N*-(1-hydroxyindan-2-yl)carbamoyl]-6*H*-thieno[2,3-*b*]pyrrole

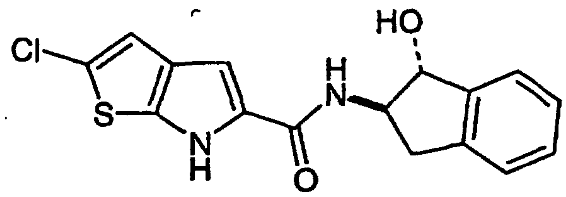

5-Carboxy-2-chloro-6*H*-thieno[2,3-*b*]pyrrole (Method #10; 280 mg, 1.4 mmol) was dissolved in DCM (20 ml) containing HOBT (189 mg, 1.4 mmol), DIPEA (0.5 ml, 2.8 mmol) and 2-aminoindan-1-ol (Method #36, 250 mg, 1.4 mmol). The reaction mixture was stirred for one minute and EDAC (306 mg, 1.6 mmol) was added. The reaction mixture was stirred at room temperature for approximately 18 hours. The resulting solution was washed with water (20 ml) and the aqueous layer extracted with DCM (2 x 20 ml). The organic extracts were combined, dried over magnesium sulphate and concentrated under reduced pressure to give the title compound as a white solid (78 mg, 17%).
NMR: 2.8(1H,dd), 3.2(1H,dd), 4.4(1H,quin), 5.1(1H,t), 5.6(1H,d), 7.1(1H,s), 7.2-7.4(5H,m), 8.4(1H,d), 11.8(1H,s); m/z 331 (M-H)

**Example #137**

2,3-Dichloro-5-[*N*-(1-hydroxy-1,2,3,4-tetrahydronaphth-2-yl)carbamoyl]-4*H*-thieno[3,2-*b*]pyrrole

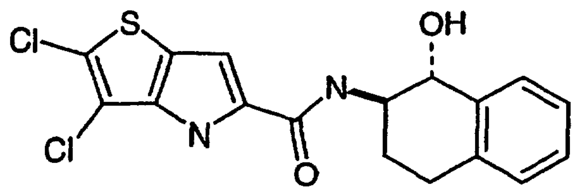

5-Carboxy-2,3-dichloro-4*H*-thieno[3,2-*b*]pyrrole (Method #9; 216 mg, 0.9 mmol) was dissolved in DCM (15 ml) containing HOBT (122 mg, 0.9 mmol), DIPEA (0.3 ml, 1.8 mmol) and 2-amino-1,2,3,4-tetrahydronaphth-1-ol (Method #39,150 mg, 0.9 mmol). The reaction mixture was stirred for one minute and EDAC (206 mg, 1.1 mmol) was added. The reaction mixture was stirred at room temperature for approximately 18 hours. The resulting solution was washed with water (20 ml) and the aqueous layer extracted with DCM (2x20 ml). The organic fractions were combined and concentrated under reduced pressure to give the title compound as a white solid (70 mg, 14%). NMR 1.8(1H,m), 2.0 (1H,qd), 2.9(1H,m), 3.3(1H,qd), 4.4(1H,quin), 4.9(1H,m), 5.2(1H,s), 5.8(1H,brs), 6.8(1H;s), 7.0(1H,dd), 7.1(1H,d), 7.2 (1H,s), 7.5(1H,d) 10.1(1H,brs).
m/Z 380 (M-H).

**Example #138**

2,3-Dichloro-5-[*N*-(6-fluoro-1-hydroxyindan-2 yl)carbamoyl]-4*H*-thieno[3,2-*b*]pyrrole

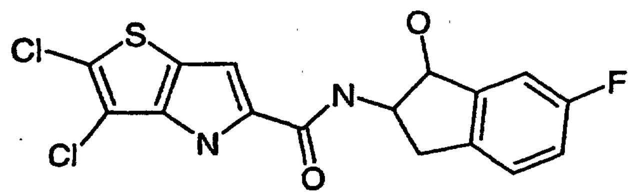

5-Carboxy-2,3-dichloro-4*H*-thieno[3,2-*b*]pyrrole (Method #9; 141 mg, 0.6 mmol) was dissolved in DCM (10 ml) containing HOBT (81 mg, 0.6 mmol), DIPEA (0.2 ml, 1.2 mmol) and 2-amino-6-fluoro-1-indanol (Method #38,100 mg,0.6 mmol).The reaction mixture was stirred for one minute and EDAC (138 mg, 0.7 mmol) was added. The reaction mixture was stirred at room temperature for approximately 18 hours. The resulting solution was washed with water (20 ml) and the aqueous layer extracted with DCM (2x20 ml). The organic fractions were combined and concentrated under reduced pressure to give a white solid. Purification by flash column chromatography (Isohexane:ethyl acetate 1:1) gave the title compound as a white solid (70 mg, 14%). NMR 3.0(1H,dd), 3.3(1H,m), 4.6(1H,q), 4.9(1H,t), 5.5(1H,d), 4.9(1H,m), 6.9-7.2(4H,m), 8.1(1H,d), 12.4(1H,brs); m/Z 383 (M-H)

**Example #139**

2,3-Dichloro-5-[*N*-(7-methoxy-1-oxo-1,2,3,4-tetrahydronaphth-2-yl)carbamoyl]-4*H*thieno[3,2-*b*]pyrrole

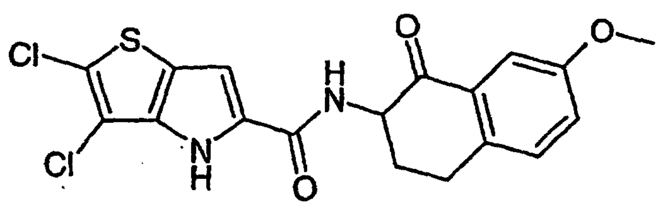

5-Carboxy-2,3-dichloro-4*H*-thieno[3,2-*b*]pyrrole (Method #9; 216 mg, 0.9 mmol) was dissolved in DCM (20 ml) containing HOBT (122 mg, 0.9 mmol), DIPEA (0.3 ml, 1.8 mmol) and 2-amino-7-methoxy-1-oxo-1,2,3,4-tetrahydronaphthalene ((Farmaco, Ed. Sci.(1985), 40(6) , 422-428), 204 mg, 0.9 mmol). The reaction mixture was stirred for one minute and EDAC (206 mg, 1.0 mmol) was added. The reaction mixture was stirred at room temperature for approximately 18 hours. The resulting solution was washed with water (20 ml) and the aqueous layer extracted with DCM (2x20 ml). The organic fractions were combined and concentrated under reduced pressure to give the title compound as a brown solid (100 mg, 27%). NMR 3.0(1H,dd), 3.1(1H,dd), 3.3(2H,m), 4.8(1H,q), 5.8(1H,s), 6.9-7.2(4H,m), 8.5(1H,d), 12.2(1H,brs); m/z 408 (M-H).

**Example #140**

2,3-Dichloro-5-[*N*-(2-indanyl)carbamoyl]-4*H*-thieno[3,2-*b*]pyrrole

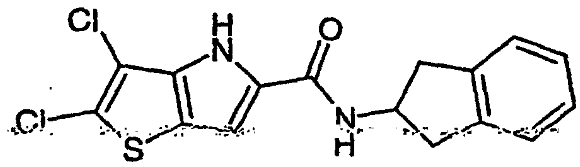

5-Carboxy-2,3-dichloro-4*H*-thieno[3,2-*b*]pyrrole (Method #9, 45mg, 0. 19 mmol) was dissolved in DMF (10 ml) with 2-aminoindan (28 mg, 0.21 mmol), di-isopropylethylamine (0.1 ml, 0.57 mmol) and HATU (80 mg, 0.21 mmol). The mixtures were stirred at ambient temperature for approximately 16 hours. The mixture was partitioned between water and ethyl acetate and was washed with water (x 5). The aqueous phase was dried, filtered and concentrated, and the residue purified by silica bond-elut chromatography using a gradient of ethyl acetate in iso-hexane (0-50%) *as* eluent to give the title compound, m/z 351.

Following a similar procedure to the process of Example #140 the following examples were prepared:

**Example #141:** 2,3-Dichloro-5-[*N*-(3-methylisoxazol-5-yl)methyl]carbamoyl]-4*H*-thieno[3,2-*b*]pyrrole

**Example #142:** 2,3-Dichloro-5-[*N*-(4-hydroxy-1,1-dioxotetrahydrothiophen-3-yl)carbamoyl]-4*H* thieno[3,2-*b*]pyrrole

**Example #143**: 2,3-dichloro-5-(*N*-{(*N*-methyl-*N*-[(1-oxo-1,2,3,4-tetrahydronaphth-2-yl)methyl]carbamoylmethyl] carbamoyl)-4*H*-thieno[3,2-*b*]pyrrole

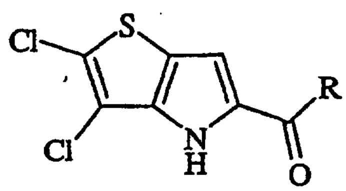

| Example | R | M/z |
|---|---|---|
| #141 | 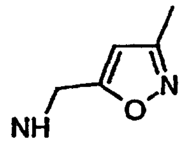 | 329 |
| #142 | 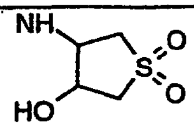 | 365 |
| #143 [1] | 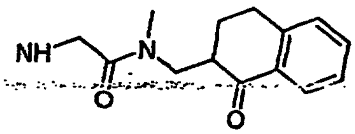 | 491 |

[1] Amine: Method #37

Example #144

2-Chloro-5-[*N*-(2-oxo-1,2,3,4-tetrahydroquinol-3-yl)carbamoyl]-6*H*-thieno[2,3-*b*]pyrrole

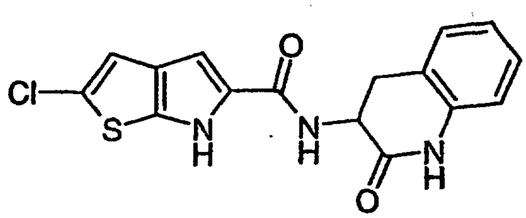

5-Carboxy-2-chloro-6*H*-thieno[2,3-*b*]pyrrole (Method #10; 101 mg, 0.5 mmol) was dissolved in DMF (2.5 ml) containing 3-amino-3,4-dihydro-2(1*H*)-quinolinone [J Med Chem (1986) 29 (12) 2427-32] (99 mg,0.5 mmol), HOBT (68 mg, 0.5 mmol) and $Et_3N$ (55 mg, 0.5 mmol). The mixture was stirred for I minute before the addition of EDAC (96 mg, 0.5 mmol). The mixture was stirred at ambient temperature for approximately 18 hours before being poured into water (50 ml), stirred vigorously and filtered. The recovered solid was washed with water, ether and dried to give the title compound as a amorphous solid.(161 mg). NMR ($DMSOd_6$): 11.96 (1H, s), 10.36 (1H, s), 8.50 (1H, d), 7.20 (2H, m), 7.19 (1H, s), 7.09 (1H, s), 6.96 (1H, m), 6.91 (1H,m), 4.72 (1H, m), 3.08 (2H, m); $MH^+$ 346.14.

**Example #145**

2-Chloro-5-[*N*-(1,2,3,4-tetrahydroquinol-3-yl)carbamoyl]-6*H*-thieno[2,3-*b*]pyrrole

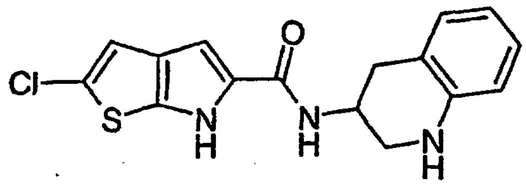

5-Carboxy-2-chloro-6*H*-thieno[2,3-*b*]pyrrole (Method #10; 157 mg, 0.78 mmol) was dissolved in DMF (4 ml) containing 3-amino-1,2,3,4-tetrahydroquinoline [J Med Chem (1982) 25 (1) 68-70] (115 mg, 0.78 mmol) and HOBT (105 mg, 0.78 mmol). The mixture was stirred for 1 minute before the addition of EDAC (149 mg, 0.78 mmol). The mixture was stirred at ambient temperature for approximately 64 hours before being partitioned between water and EtOAc. The organics were washed with water, saturated aqueous $NaHCO_3$, water, saturated brine and dried. The organics were filtered, concentrated and chromatographed on Fluorochem silica 40-63µ 60A (eluent 40:60 EtOAc/isohexane) to afford the title compound as an amorphous solid (44 mg). NMR ($DMSOd_6$): 11.94 (1H, s), 8.04 (1H, d), 7.16 (1H, s), 7.06 (1H, s), 6.90 (2H, m), 6.48 (2H, m), 5.8 (1H, br), 4.18 (1H, m}, 3.05 (1H, t), 2.85 (2H, m); $MH^+$ 332.17.

**Example #146**

2-Chloro-5-[*N*-(1-methyl-2-oxo-1,2,3,4-tetrahydroquinol-3-yl)carbamoyl]-6*H* -thieno[2,3-*b*]pyrrole

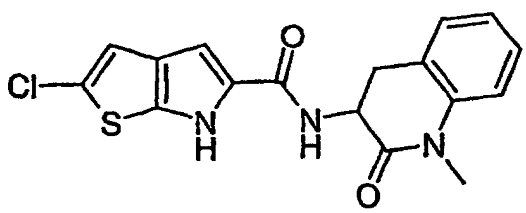

5-Carboxy-2-chloro-6*H*-thieno[2,3-*b*]pyrrole (Method #10; 80 mg, 0.4 mmol) was dissolved in DMF (2 ml) containing 3-amino-3,4-dihydro-1-carbostyril [JCS 1965 1080-1087] (71 mg, 0.4 mmol) and HOBT (54 mg, 0.4 mmol). The mixture was stirred for 1 minute before the addition of EDAC (77 mg, 0.4 mmol). The mixture was stirred at ambient temperature for approximately 18 hours before being partitioned between water and EtOAc. The organics were washed with water, saturated aqueous $NaHCO_3$, water, saturated brine and dried. The organics were filtered, concentrated and recrystalised from EtOAc to afford the title compound as an amorphous solid (66 mg). NMR 11.96 (1H, s), 8.54 (1H, d), 7.30 (2H,m), 7.17 (2H, m), 7.08 (2H, m), 4.68 (1H, m), 3.32 (3H, s), 3.14 (1H, m), 3.04 (1H, m); m/z ($NBH^{+)}$ 360.14

**Example #147**

2-Chloro-5-[*N*-(3-oxo-2,3,4,5-tetrahydro-1*H*-benz[2]azepin-4-yl)carbamoyl]-6*H* -thieno[2,3-*b*]pyrrole

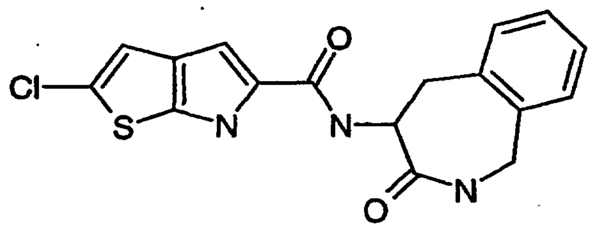

5-Carboxy-2-chloro-6*H*-thieno[2,3-*b*]pyrrole (Method #10; 101 mg 0.5mmol) was dissolved in DMF (2.5 ml) containing 4-amino-1,2,4,5-tetrahydro-3*H*-2-benzazepin-3-one hydrochloride [CAS Reg No 148842-85-7] (107 mg, 0.5 mmol), HOBT (68 mg, 0.5 mmol) and $Et_3N$ (101 mg, 1.0 mmol). The mixture was stirred for 1 minute before the addition of EDAC (96 mg, 0.5 mmol), then at ambient temperature for approximately 18 hours before being partitioned between water and EtOAc. The organics were washed with water, saturated aqueous $NaHCO_3$ water, saturated brine and dried; filtered and evaporated to afford the title compound as an amorphous solid (26 mg). NMR:11.92 (1H, s), 8.33 (1H, t), 8.29 (1H, d), 7.2 (6H, m), 5.30 (1H, m), 4.83 (1H, dd), 3.98 (1H, dd), 3.20 (2H, m); m/z ($MH^{+}$) 360.19.

**Example #148**

2,3-Dichloro-5-[*N*-(1-methoxyindan-2-yl)carbamoyl]-4*H*-thieno[3,2-*b*]pyrrole

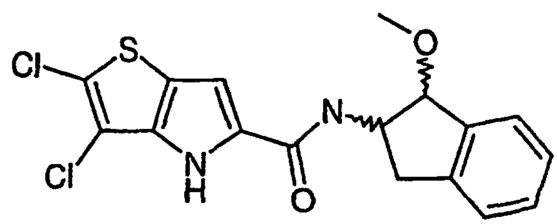

5-Carboxy-2,3-dichloro-4*H*-thieno[3,2-*b*]pyrrole (Method #9, 145mg, 0.613mmol), *trans*-2-amino-1-methoxyindan (Method #40, 100mg, 0.163mmol), DIPEA (0.105ml, 0.613mmol), and HOBT (83mg, 0.613mmol) was stirred in dichloromethane (5ml) for one minute. EDAC (147mg, 0.766mmol) was added and the mixture stirred at room temperature for 20 hours. The reaction mixture was evaporated, ethyl acetate (25ml) added and then washed with water. The organic solution was dried over magnesium sulphate and evaporated to give the title compound as a white powder (180mg, 77%).
NMR2.8(1H, dd), 3.3(1H, dd), 3.35(3H, s), 4.1-4.2(1H, m), 5.35-5.45(1H, m), 7.1-7.3(4H, m), 7.15(1H, s), 8.7(1H, d); m/z 380.9/382.9 (M+H).

**Example #149**

2.3-Dichloro-5-(*N*-{1-[*N*-(1,1-dimethylethoxy)carbonylamino]indan-2-yl}carbamoyl)-4*H*-thieno[3.2-*b*]pyrrole

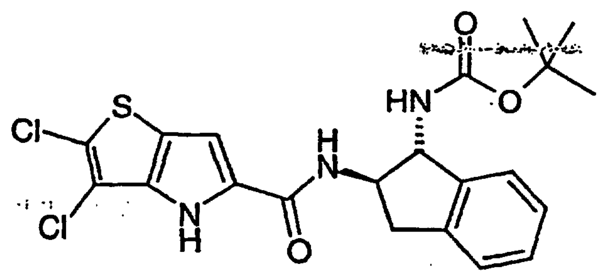

5-Carboxy-2,3-dichloro-4*H*-thieno[3,2-*b*]pyrrole (Method #9, 1.18g, 5.0mmol), (1*R*, 2*R*)-2-amino-1-[(1,1-dimethylethoxy)carbonylamino]indan (Method #43, 1.25g, 5.0mol), DIPEA (0.855ml, 5.0mmol), and HOBT (675mg, 5.0mmol) was stirred in dichloromethane (50ml) for one minute. EDAC (1.2g; 6.25mmol) was added and the mixture stirred at room temperature for 20 hours. The reaction mixture was diluted with dichloromethane (50ml), filtered and dried to give the title compound as a pale green powder (1.95g, 85% ). NMR1.4(9H, s), 2.8(1H, dd), 3.2(1H, dd), 4.5-4.7(1H, m), 5.1-5.2(1H, m), 7.05-7.3(5H, m), 7.4(1H, d), 8.6(1H, d), 12.4(1H, s).

**Example #150**

5-[*N*-(1-Aminoindan-2-yl)carbamoyl]-2,3-dichloro-4*H*-thieno[3,2-*b*]pyrrole

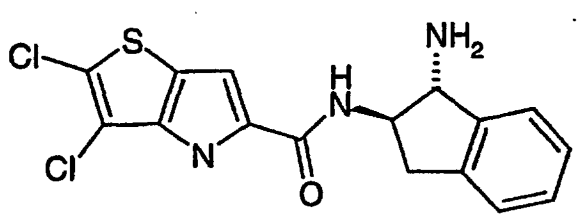

2,3-Dichloro-5-(*N*-{1-[(1,1-dimethylethoxy)carbonylamino]indan-2-yl}carbamoyl)-4*H*-thieno[3,2-b]pyrrole (Example #149, 1.0g, 2.15mmol) was dissolved in dichloromethane (20ml). Trifluoroacetic acid (2ml) was added and the mixture stirred at room temperature for 24 hours. The reaction was filtered and the isolated solid washed with dichloromethane to give the trifluoroacetic acid salt of the title compound as a pale green powder (800mg, 78%). NMR3.05(1H, dd), 3.4(1H, dd), 4.6-4.85(2H, m), 7.2(1H, d), 7.3-7.45(3H, m), 7.55(1H, d), 8.6(3H, broad s), 8.8(1H, d), 12.5(1H, s)

**Example #151**

5-[*N*-(1-Acetamidoindan-2-yl)carbamoyl]-2,3-dichloro-4*H*-thienol[3,2-*b*]pyrrole

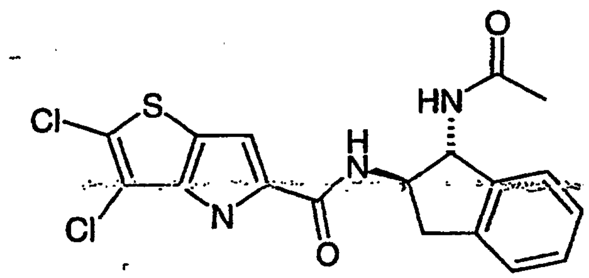

Triethylamine (101mg, 1.0mmol was added to a suspension 5-[*N* (1-aminoindan-2-yl)carbamoyl]-2,3-dichloro-4*H*-thieno[3,2-*b*]pyrrole trifluoroacetic acid salt (Example #150, 240mg, 0.5mmol) in dichloromethane (4ml), followed by acetyl chloride (47mg, 0.6mmol) dissolved in dichloromethane (1ml) and the reaction stirred at room temperature for 6 hours during which a white solid precipitated. The reaction was filtered and the crude material purified by silica chromatography with hexane : ethyl acetate to give the title compound as a white solid (50mg, 25%). NMR 1.87(3H, s), 2.82(1H, dd), 3.22(1H, dd), 4.45-4.62(1H, m), 5.38-5.5(1H, m), 7.02-7.27(4H, m), 7.1(1H, s), 835(1H, d), 8.59(1H, d), 12.36(1H, broad s); m/z 406.13/408.8 (M-H).

**Example #152**

2,3-Dichloro-5-{*N*-[1-(methanesulphonamido)indan-2-yl]carbamoyl]-4*H* -thieno[3,2-*b*]pyrrole

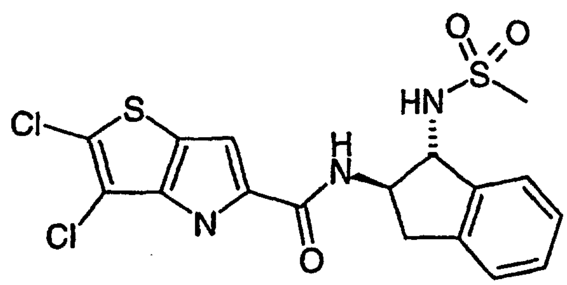

5-Carboxy-2,3-dichloro-4*H*-thieno[3,2-*b*]pyrrole, (Method #9, 236mg, 1.0mmol), (1*R*,2*R*)-2-amino-1-methanesulphonamidoindan (Method #42, 226mg, 1.0mol), DIPEA. (0.174ml, 1.0mmol), and HOBT (135mg, 1.0mmol) was stirred in dichloromethane (10ml) for one minute. EDAC (240mg, 1.25mmol) was added and the mixture stirred at room temperature for 20 hours. The mixture was diluted with ethyl acetate, washed with water (2 x 25ml), dried over magnesium sulphate and evaporated to give the title compound as a foam (400mg, 90%). NMR 2.84(1H, dd), 2.99(3H, s), 3.22(1H, dd), 4.44-4.64(1H, m), 4.89-5.0(1H, m), 7.14(1H, s), 7.16-7.36(4H, m), 7.84(1H, d), 8.64(1H, d), 12.43(1H, broad s); m/z 442.2/444.0 (M-H).

**Example #153**

2,3-Dichloro-5-{*N*-[1-(methylamino)indan-2-yl]carbamoyl}-4*H* -thieno(3,2-*b*]pyrrole

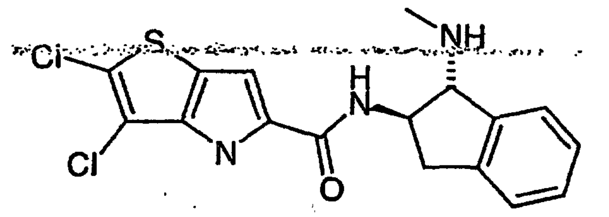

2,3-Dichloro-5-[*N*-(1-{*N*-[(1,1-dimethylethoxy)carbonyl]-*N*-methylamino}indan-2-yl)carbannoyl]-4*H*-thieno [3,2-*b*]pyrrole (Method #44, 900mg, 1.87mmol)in dichloromethane (20ml) treated with trifluoroacetic acid (2ml) at room temperature for 1 hour. Evaporation followed by co-evaporation with chloroform and drying gave the trifluoroacetic acid salt of the title compound as a pale brown foam (850mg, 92%). NMR 2.75(3H,:s), 3.02(1H, dd), 3.5(1H, dd), 4.7-4.95 (2H, m), 7.15(1H, s), 7.28 7.48(3H, m), 7.6(1H, d), 8.68(1H, d), 9.1(2H, broad s); m/z 380.4/382.4 (M+H).

**Example #154**

2,3-Dichloro-5-{*N*-[1-(*N*-methylacetamido)indan-2-yl]carbamoyl}-4*H*-thieno[3,2-*b*]pyrrole

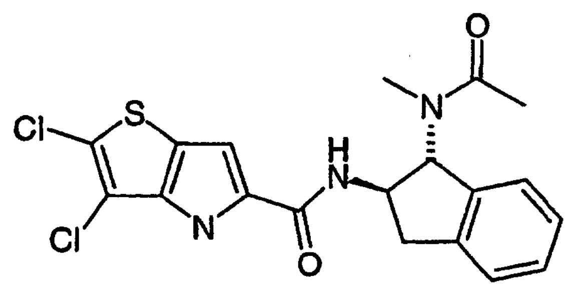

2,3-Dichloro-5-{*N* [1-(methylamino)indan-2-yl]carbamoyl)-4*H*-thieno[3,2-*b*]pyrrole trifluoroacetic acid salt (Example #153, 390mg, 0.79mmol) in dichloromethane (5ml) at 5°C was treated with triethylamine (0.33ml, 2.37mmol) and acetyl chloride (68mg, 0.86mmol). After stirring at 5°C for 15 minutes the reaction was allowed to warm to room temperature and stirred for a further 2 hours. The mixture was diluted with ethyl acetate (25ml) and washed with saturated sodium bicarbonate and water. Drying over magnesium sulphate followed by evaporation gave the title compound as a pale brown foam (270mg, 80%). NMR: Indicates an approximate 1:1 ratio of rotamers of the title compound; 2.05(1.5H, s), 2.1(1.5H, s), 2.6(1.5H, s), 2.8(1.5H, s), 2.9-3.08(1H, m), 3.12-3.3(1H, m), 4.7-4.9(1H, m), 5.24(0.5H, d), 6.14(0.5H, d), 6.94-7.35(5H, m), 8.6(0.5H, d), 8.68(0.5H, d), 12.38(0.5H, broad s), 12.46(0.5H, broad s); m/z 421.9/423.9 (M+H)

**Preparation of Starting Materials**

The starting materials for the Examples above are either commercially available or are readily prepared by standard methods from known materials. For example the following reactions are illustrations but not limitations of the preparation of some of the starting materials used in the above reactions.

**Method #1**

3-Chloro-5-methoxycarbonyl-4*H*-thieno[3,2-*b*]pyrrole

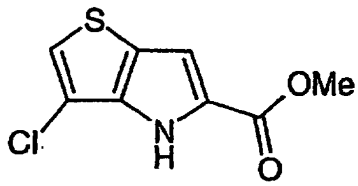

Methanolic sodium methoxide solution (28%) (5 ml, 25.9 mmol) was diluted with MeOH (5 ml) and was cooled to -25°C under nitrogen. A solution of 4-chloro-2-thiophenecarboxaldehyde (J Heterocyclic Chem, 1976, 13, 393; 1.1 g; 7.5 mmol) and methyl azidoacetate (3.0 g, 26.1 mmol) in MeOH (20 ml) was added dropwise, maintaining the temperature at -25°C. On completion of addition the solution was allowed to warm to 5°C . over a period of approximately 16 hours. The solution was added to saturated aqueous ammonium chloride (250 ml) and the mixture was extracted using DCM. The combined organic layers were concentrated at 0°C. The residue was taken up in xylene (30 ml) and this solution was added dropwise to xylene (120 ml) under reflux. The solution was heated under reflux for 30 minutes before being cooled and concentrated. The title compound was purified by a mixture of crystallisation (EtOAc/isohexane) and chromatography on a Bond Elut column eluting with a graduated solvent of 5-50% EtOAc in isohexane (640 mg, 40%). NMR ($CDCl_3$) 9.1 (1H, br), 7.1 (2H, s), 3.9 (3H, s); m/z 214.3.

**Methods #2 - #4**

The following compounds were made by the process of Method #1 using the appropriate starting materials

| Meth | Compound | NMR ($CDCl_3$) | M/z |
|---|---|---|---|
| #2 | 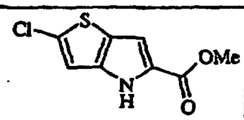 | 9.1 (1H, br), 7.0 (1H, s), 6.9 (1H, s), 3.9 (3H, s) | 214.2 |
| #3 [1] | 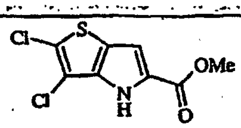 | 9.2 (1H, br), 7.0 (1H, s), 3.9 (3H, s) | 248.2 |
| #4 [2] | 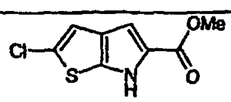 | 9.4-9.2 (1H, br), 7.0 (1H, s), 6.9(1H, s), 3.9 (3H, s) | 214 |

1 Aldehyde: DE 2814798

2 Aldehyde: Aldehyde ref. Gronowitz *et al.* Tetrahedron Vol.32 1976 p.1403

**Method #5**

N-Benzyl-2-(*tert*-butoxycarbonylamino)acetamide

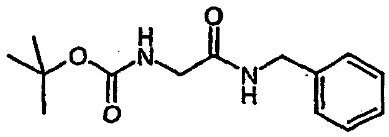

*N*-(*tert*-Butoxycarbonyl)glycine (875mg, 5mmol) was dissolved in DMF (7ml) containing DIPEA (3.5ml, 20mmol) and benzylamine (536mg, 5mmol). The mixture was allowed to stand for one minute before addition of *O*-(7-Azabenzotriazol-1-yl*)-N,N,N;N* '-tetramethyluronium hexafluorophosphate (HATU) (2.09g, 5.5mmol). The solution was allowed to stand for approximately 18 hours before being partitioned between ethyl acetate (50ml) and water (50ml). The layers were separated and the organic phase dried using magnesium sulphate, filtered, concentrated and purified using bond-elute silica column chromatography (eluent: dichloromethane-dichloromethane/methanol 5% gradient) to afford the *title compound* as an oil (1.32g, quantitative).
NMR: ($CDCl_3$): 7.2 (5H, m), 6.3(1H, br), 5.0(1H, br), 4.4(2H, d), 3.8(2H, d), 1.4(9H, s); m/z 265.4

**Method #6**

2-Amino-*N*-benzylacetamide

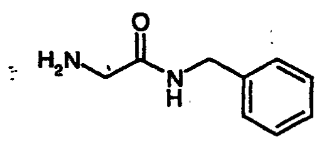

To a solution of *N*-benzyl-2-(*tert*-butoxycarbonylamino)acetamide (Method #5, 1.18g, 4.47mmol) in dichloromethane (6ml) at 0°C was added dropwise trifluoroacetic acid (24ml) and the resulting solution allowed to stir warming to room temperature overnight. The reaction mixture was neutralised by addition of saturated sodium bicarbonate solution and extracted with dichloromethane. The combined organic phases were dried over magnesium sulphate, filtered, concentrated and purified by bond-elute SCX column chromatography (eluent: methanol/dichloromethane (1:1) then methanol/dichloromethane (1:1)/ammonia5%) to afford the *title compound* as an oil (215mg, 29%).
NMR: ($CDCl_3$): 7.2(6H, m), 4.4(1.4H,d), 4.3(0.6H,d), 3.4(2H, br); m/z 165.17

**Method #7**

5-Carboxy-3-chloro-4*H*-thieno[3,2-*b*]pyrrole

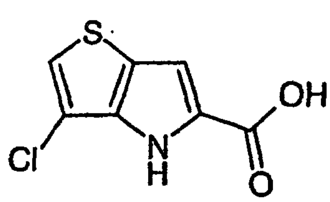

3-Chloro-5-methoxycarbonyl-4*H* thieno[3,2-*b*]pyrrole (Method #1; 0.61 g, 2.83 mmol) was taken up in MeOH (10 ml) and was heated under reflux. Aqueous lithium hydroxide (2.0 M, 3.0 ml, 6.0 mmol) was added portionwise over 45 minutes. The mixture was heated under reflux for 30 minutes before being cooled and concentrated. Water (20 ml) was added and the solution was neutralised using aqueous hydrochloric acid (2.0 M, 3.0 ml). The solution was extracted using EtOAc, and the combined organic layers were concentrated to afford the title compound as a yellow solid (0.57 g, 100%). NMR: 12.4 (1H, br), 7.4 (1H, s), 7.0 (1H, s); m/z 200.3.

<u>Methods #8 - #10</u>

The following compounds were made by the process of Method #7 using the appropriate starting materials.

| Meth | Compound | NMR | M/z | SM |
|---|---|---|---|---|
| #8 | 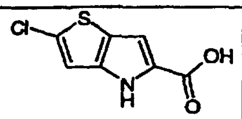 | 11.9 (1H, br), 7.0 (1H, s), 6.9 (1H, s) | 200.1 | Method #2 |
| #9 | 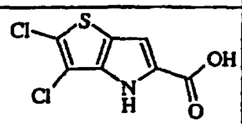 | 7.0 (1H, s) | 234.2 | Method #3 |
| #10 | 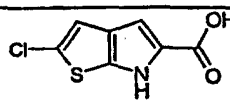 | NMR 12.6-12.7 (1H, b), 12.0-12.1 (1H, b), 7.15(1H, s), 6.9(1H, s) | 183 | Method #4 |

<u>Method #11</u>

<u>2,3-Dichloro-5-[*N*-(ethoxycarbonylmethyl)carbamoyl]-4*H* -thieno[3,2-*b*]pyrrole</u>

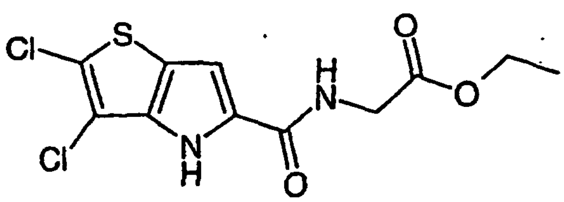

5-Carboxy-2,3-dichloro-4*H*-thieno[3,2-*b*]pyrrole (Method #9,4.0g 16.95mmol) was added to a solution of glycine ethyl ester hydrochloride (2.60g, 18.64mmol) and DIPEA in dichloromethane (200ml) followed by HOBT (2.29g, 16.95mmol). The solution was stirred under nitrogen for 15 minutes before the addition of EDAC (3.89g, 22.03mmol). The mixture was stirred at ambient temperature for approximately 16 hours. The resultant white precipitate was isolated by filtration, washed with water and ether and dried. (4.79g, 88%).
NMR: 12.45 (1H,br), 8.75 (1H, t), 7.1 (1H,s), 4.1 (2H, q), 4.0 (2H, d), 1.2 (3H, t); m/z 321.2

**Method #12**

5-(*N*-Carboxymethylcarbamoyl)-2,3-dichloro-4*H*-thieno[3,2-*b*]pyrrole

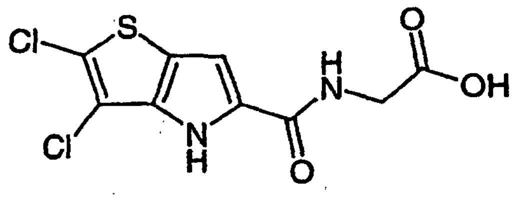

2N Sodium hydroxide solution (14.3ml, 28.7mmol) was added to a suspension of 2,3-dichloro-5-[*N*-(ethoxycarbonylmethyl)carbamoyl]-4*H*-thieno[3,2-*b*]pyrrole(Method#11 4.60g, 14.33mmol) in tetrahydrofuran (THF) (100ml). The resultant solution was stirred at room temperature for one hour. The reaction mixture was concentrated *'in vacuo',* the residue diluted with water (250ml), the solution adjusted to pH=2 by addition of 2N Hydrochloric acid, and then extracted with ethyl acetate (3x150ml). The organic extracts were combined, dried over sodium sulphate then filtered and concentrated to give a white powder (3.34g, 80%); NMR: 12.6 (1H,br), 12.4 (1H, br), 8.6 (1H, t), 7.1 (1H, s), 3.9. (2H, d); m/z 291.17

**Method #13**

*N-(2*-Cyanoethyl)-*N*-phenyl-2-(*tert*-butoxycarbonylamino)acetamide

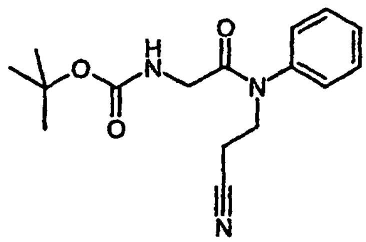

By a similar procedure to Method #5 the *title compound* was prepared using anilinopropionitrile to give a clear oil (598mg, 39%); NMR: ($CDCl_3$): 7.4(3H, m), 7.2(2H, m), 5.2(1H, br), 3.9(2H, t), 3.6(2H, d), 2.6(2H, t), 1.3(9H, s); m/z 304.51

**Method #14**

2-Amino-*N*-(2-cyanoethyl)-*N*-phenylacetamide

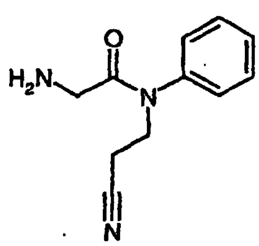

By a similar procedure to Method #6 the *title compound* was prepared using *N-(2*-cyanoethyl)-*N*-phenyl-2-(*tert*-butoxycarbonylamino)acetamide (Method #13) to give a clear oil (222mg, 60%); NMR: ($CDCl_3$): 7.4(3H, m), 7.2 (1H, d), 7.1(1H, d), 3.9(2H, t), 3. 1( 1.33H,s), 3.0(0.67H, s). 2.65(1.33H,t), (0.67H, t); m/z 204.31

**Method #15**

*N*-[(2-Amino-2-(2-thiazolyl)]ethyl-*N*-methylmethanesulphonamide

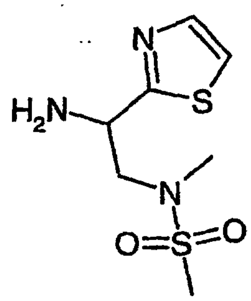

2-Bromothiazole (6.9 g, 42.0 mmol) was dissolved in dry diethyl ether (15 ml) and was added dropwise to butyl lithium (1.6 M, 29.7 ml, 47.5 mmol) in diethyl ether (40 ml) at -70°C. The mixture was stirred at -70°C for 30 minutes before the addition of a cold solution of ethyl 2-(*N*-methylmethanesulphonamido)acetate (Ger Offen,1976, 27pp; 7.6 g, 39.0 mmol) in dry THF (70 ml). The solution was stirred for a further 30 minutes at -70°C before being allowed to warm to ambient temperature. Aqueous ammonium chloride (10%, 200 ml) was added and the solution was extracted using diethyl ether. The aqueous layer was acidified and re-extracted using diethyl ether. The combined organic layers were dried, filtered and concentrated under reduced pressure. The residue (5.30 g, 22.6 mmol) was dissolved in ethanol (100 ml) containing pyridine (18 ml) and hydroxylamine hydrochloride (1.88 g, 27.1 mmol). The mixture was heated under reflux in an inert atmosphere for 2.5 hours before being cooled and concentrated under reduced pressure. The residue was suspended in water and cooled to 0°C. The mixture was acidified to pH 4 using aqueous hydrochloric acid (2.0 M). A solid precipitated out of solution and was filtered off and washed with water. The product was dried under reduced pressure in the presence of phosphorous pentoxide. The dry solid (4.34 g, 17.4 mmol) was dissolved in acetic acid and 5% rhodium on carbon (40% *w/w,* 1.7 g) was added. The solution was agitated under an atmosphere of hydrogen at 5 bar for 48 hours. The reaction vessel was flushed free of hydrogen using inert gas and the solution was filtered through celite. The filtrate was concentrated under reduced pressure. The residue was suspended in ethanol and cooled to 0°C. Aqueous hydrochloric acid (5.0 M) and ethanol (50 ml, 1:1) was added and the mixture was stirred for 30 minutes. A white precipitate was filtered off to afford the title compound (3.16 g) as the hydrochloride salt.

**Method #16**

Ethyl (*S*)-5-(□-aminophenethyl)-1,3,4-oxadiazole-2-carboxylate trifluoroacetate

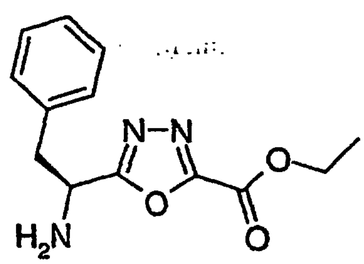

Ethyl (*S*)-5-[□-[(*tert*-butoxycarbonylamino)phenethyl]-1,3,4-oxadiazole-2-carboxylate, (Borg et al. J. Org.

Chem. 1995, 60, 3112; 350mg) was dissolved in trifluoroacetic acid (5ml.) and allowed to stand at ambient temperature for 1 hour. The reaction mixture was concentrated and dried under vacuum to give a glassy solid (322mg) NMR 1.75 (3H,t); 3.2-3.4(2H,m); 4.4(2H,q); 5.2(1H,t); 7.1-7.4(5H,m); 8.8-9.2(3H,bs)

**Method #17**

2.3-Dichloro-5-{*N*-[(2-phenyl-1,3-dioxolan-2-yl)methyl]carbamoy}-4*H*-thieno[3,2-*b*]pyrrole

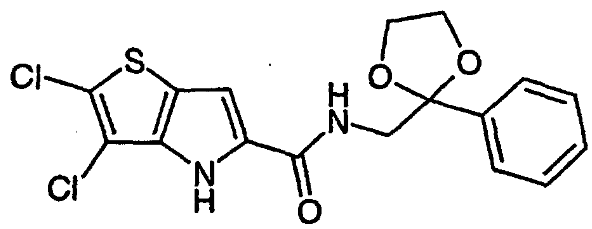

2-Phenyl-1,3-dioxolan-2-ylmethylamine hydrochloride (65 mg, 0.3 mmol) and 5-carboxy-2,3-dichloro-4*H* thieno[3,2-*b*]pyrrole (Method #9; 71 mg, 0.3 mmol) were dissolved in DCM (10 ml) containing DIPEA (175 ml, 1.0 mmol) and HOBT (40 mg, 0.3 mmol). The mixture was stirred for one minute before the addition of EDAC (75 mg, 0.39 mmol). The solution was stirred at room temperature for approximately 18 hours. The mixture was concentrated and chromatographed on a Bond Elut column eluting with 20% - 40% EtOAc in isohexane. The title compound was isolated as a white solid (100 mg). NMR: 12.4 (1H, s), 8.2 (1H, s), 7.2 (1H, s), 4.0 (2H, m), 3.7 (2H, m), 3.6 (2H, d); m/z 395.2.

**Method #18**

(*S*)-5-(□-Aminophenethyl)-3-phenyl-1,2,4-oxadiazole trifluoroacetate

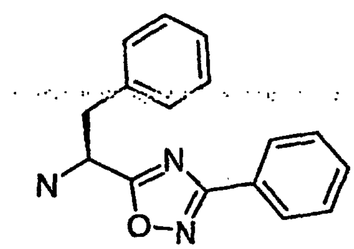

BOC-Phenylalanine (614mg, 2.32mmol) was dissolved in DCM (20ml) cooled with ice/water and dicyclohexyl carbodiimide (239mg, 1.16mmol) added. After stirring at 0-5°C for 1 hour the reaction mixture was filtered and concentrated in vacuo. Phenylamidoxime (104mg,0.77mmol) and pyridine (10ml) were added and the mixture heated to reflux for 2 hours. The reaction mixture was then evaporated to small volume, dissolved in ethyl acetate, washed with dilute citric acid, saturated sodium bicarbonate, water and brine, dried with magnesium sulphate and evaporated to give a crude product which was purified by chromatography on silica gel (eluted with Hexane/ ethyl acetate 4:1) to (*S*)-5-[□-(*tert*-butoxycarbonylamino)phenethyl]-3-phenyl-1,2,4-oxadiazole (274mg).
NMR 1.2-1.4 (9H,s), 3.1-3.3 (2H,m); 5.1-5.2(1H,m); 7.2-7.3(5H,m);7.5-7.6(3H,m); 7.8(1H,d); 7.95-8.05(2H,m); m/z 364

(*S*)-5-[□-(*tert*-Butoxycarbonylamino)phenethyl]-3-phenyl-1,2,4-oxadiazole (274mg) was dissolved in trifluoroacetic acid (5ml) and stirred at ambient temperature for 2 hours. After evaporation and drying under vacuum the title compound was obtained as a pale yellow solid (232mg). NMR 3.2-3.5(2H,m); 5.15(1H,t); 7.1-7.3(5H,m); 7.4-7.6(3H, m); 7.9(2H,d); 9.0(3H,s); m/z 266

**Method #19**

3-(1*H* Tetrazol-5-yl)propylamine

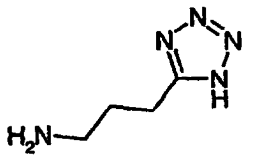

4-{[(Phenylacetyl)oxy]amino}butanamide (*J. Biol. Chem,* 1971, **246**, 6683) (16 g) was dissolved in dry pyridine (150 ml) and the solution was cooled to -10°C. A solution of $POCl_3$ (8.1 ml) in DCM (16.5 ml) was added dropwise over 30 minutes. The mixture was stirred at ambient temperature for 1 hour before being diluted with water until the pH reached 5. The solution was extracted using EtOAc. The combined organic layers were washed with dilute aqueous hydrochloric acid and water before being dried, filtered and concentrated. The resulting solid (9.2 g, 42.8 mmol) was dissolved in dry DMF (30 ml) and the solution was heated on a steam bath for 2 hours. Ammonium chloride (2.22 g, 41.5 mmol) was added to the hot solution along with sodium azide (2.68 g, 56.3 mmol). The mixture was cooled and left to stir for over 48 hours. The solution was filtered and basified to pH 8 using aqueous potassium bicarbonate. The aqueous layer was washed using EtOAc before being acidified using dilute aqueous hydrochloric acid. A white solid was filtered off. This solid (3.0 g, 11.5 mmol) was dissolved in acetic acid (50 ml) and water 5 ml. Palladium on charcoal (5%, 400 mg) was added and the solution was shaken under an atmosphere of hydrogen for 6.5 hours. The suspension was filtered and washed with acetic acid. The filtrate was concentrated and dried. The residue was treated with iso-propanol and the title compound was filtered off as a white solid (1.37 g, 94%).

**Method #20**

2-Amino-*N*-(2-hydroxy-3 phenoxypropyl)acetamide

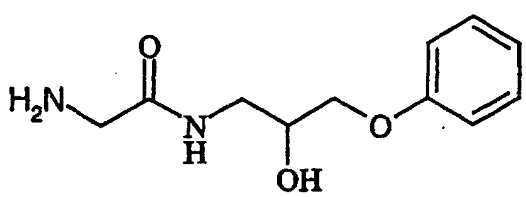

*N*-Benzyloxycarbonylglycine (2.09 g) was dissolved in toluene (40 ml) and DMF (5 drops). Oxalyl chloride (1.3 ml) was added and the mixture was stirred at ambient temperature for 2 hours. The solution was diluted with diethyl ether (25 ml) and was added dropwise to a solution of 1-amino-3-phenoxy-2-propanol (1.67 g) in diethyl ether (25 ml). Sodium hydroxide (0.4 g) was dissolved in water (1.5 ml) and was added to the mixture. The solution was stirred for greater than 48 hours before being filtered. The sticky solid isolated was added to saturated aqueous sodium bicarbonate (100 ml) and was stirred for 15 minutes before being filtered. The solid was recrystallized from an EtOAc/petrol mixture. This resulting compound (650 mg) was dissolved in MeOH (10 ml) and palladium on charcoal (5%, 50 mg) was added along with acetic acid (1ml). The solution was stirred under an atmosphere of hydrogen for 4 hours before being filtered. The filtrate was concentrated to afford an oily residue. The residue was recrystallized from EtOH/diethyl ether mixture to afford the title compound (200 mg).

**Method #21**

2-Amino-*N*-(3-methylisothiazol-5-yl)acetamide

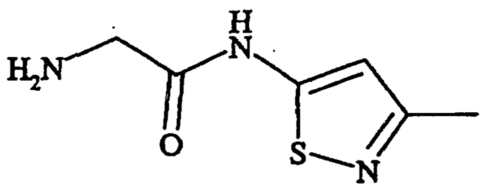

*N-t*-Butoxycarboylglycine (1.92 g, 1.1 mmol) was dissolved in dry EtOAc (20 ml) and the solution was cooled to -25°C. *N*-Methyl morpholine (1.1 g, 1.1 mmol) was added and the mixture was stirred for 2 minutes before the addition of ethyl chloroformate (1.08 g, 1.0 mmol). A white solid precipitated from solution. A suspension of 5-amino-3-methylisotbiazole hydrochloride (1.5 g, 1.0 mmol) was added in triethylamine (1.01 g, 1.0 mmol) and dry EtOAc (10 ml). The mixture was stirred at ambient temperature for 17 hours before being filtered. The filtrate was concentrated and the residue was purified by flash column chromatography, using diethyl ether as eluent. The residue (271 mg, 0.1 mmol) was dissolved in EtOAc (3 ml). Ethanolic HCl (1 ml) was added and the mixture was stirred at ambient temperature for 2.5 hours. The title compound precipitated as a white solid and was isolated by filtration (220 mg, 92%).

**Method #22**

2-(Pyrridazin-3-yloxy)ethylamine

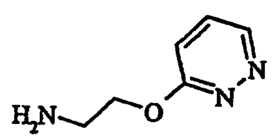

Ethanolamine (6.0 ml) was suspended in dry xylene (100 ml) and the mixture was stirred at ambient temperature with sodium hydride (3 g). After 20 minutes the solution was cooled to 0°C. 3,6-Dichloropyridazine (15.0 g) was added. The solution was warmed to ambient temperature and was stirred for 15 hours before being extracted with chloroform. The chloroform was concentrated and the residue was taken up in EtOAc before being filtered. Ethanolic HCl was added and a white solid was filtered off. This compound (550 mg) was dissolved in MeOH (130 ml) and palladium on charcoal (5%, 200 mg) was added. The suspension was stirred under an atmosphere of hydrogen for 6 hours before being filtered. The filtrate was concentrated and the residue was triturated using diethyl ether. The title compound was isolated as a white solid (340 mg).

**Method #23**

1-(2-Aminoethyl)imidazolidine-2,4-dione

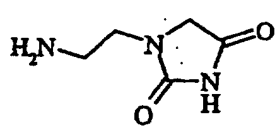

Ethylene diamine (30 ml) was dissolved in DCM (130 ml) with di-*t*-butyl dicarbonate (13.5 g). The mixture was stirred at ambient temperature for 30 minutes before the solvent was decanted off. The residue was washed with water

and dried. The product (6.1 g) was dissolved in ethanol (15 ml). Chloroacetic acid (5.77 g) solution in aqueous sodium hydroxide (1 M, 61 ml) was added dropwise, followed by more aqueous sodium hydroxide (1 M, 31 ml). The mixture was stirred for 17 hours before being washed with diethyl ether. Benzyl chloroformate (5 g) was added to the aqueous phase and the mixture was stirred at ambient temperature for 4 hours. The solution was washed with diethyl ether and the aqueous layer was acidified using aqueous citric acid (30%). The solution was extracted using EtOAc. The combined organic layers were washed with brine, then water before being dried, filtered and concentrated. The residue was triturated using diethyl ether/petrol mixture and the required intermediate was isolated as a white solid. Following repeated procedure on a larger scale, this solid (28 g) was dissolved in ethanol (250 ml) containing palladium on charcoal (10%, 4.6 g). The suspension was stirred under an atmosphere of hydrogen until the reaction was complete. The mixture was filtered and the filtrate was concentrated. The residue (3 g, was treated with potassium cyanate (1.3 g) in water (30 ml) and the mixture was heated under reflux for 2 hours. An excess of concentrated hydrochloric acid was added and the mixture was heated for a short time before being concentrated. The residue was taken up in water (50 ml) and was loaded onto basic Amberlite® IRA resin. The resin was washed with water until the eluent was found to be neutral. Dilute aqueous hydrochloric acid was used to elute the crude product from the resin. The acidic fractions were concentrated and the residue was triturated with ethanol. The title compound was isolated as orange crystals (950 mg).

**Method #24**

*N*-(4-Aminobutyryl)methanesulphonamide

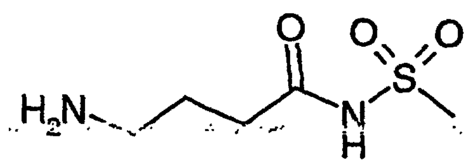

4-Phthalimidobutyryl chloride *(J. Am. Chem. Soc.,* 1981, **103**, 6750) (1.27 g) was heated with methane sulphonamide (480 mg) at 100°C for 15 min under argon. The mixture was cooled and left to stand for 17 hours. The residue was triturated with ethanol to afford the required intermediate. The solid (4 g) was treated with potassium hydroxide (75 g) in water (100 ml) at ambient temperature for 2 hours. Concentrated hydrochloric acid was added until the pH of the solution reached 9. The solution was extracted using EtOAc. The combined organic layers were concentrated and the residue was triturated using diethyl ether/petrol mixture and filtered. The residue (5.7 g) was taken up in water (150 ml) and dilute hydrochloric acid (1 M) was added until the pH reached 1. The mixture was heated on a steam bath for 1 hour before being cooled and extracted using EtOAc. The aqueous phase was concentrated and the residue was triturated using EtOAc/ethanol mixture to afford the title compound (1.5 g).

**Method #25**

2-[3-(2-Aminoethoxy)phenyl]acetamide

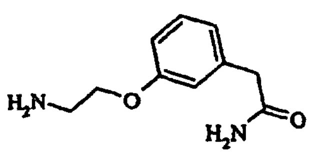

3-Hydroxyphenylacetic acid (30.4 ml) was dissolved in MeOH (160 ml). Concentrated sulphuric acid (1.6 ml) was added and the mixture was heated under reflux for 6 hours. The mixture was concentrated to low bulk before the addition of toluene (120 ml). The mixture was washed with water, saturated aqueous sodium bicarbonate and brine. The combined organic layers were reduced in volume by one half before being stirred with ammonia solution (180 ml) for 16 hours. The mixture was concentrated and filtered. The solid was washed with water and was dried. The residue

(24 g) was dissolved in MeOH (400 ml) with 1,2-dibromoethane (20 ml) and sodium hydroxide (6 g). The solution was heated under reflux for 36 hours before being concentrated. The residue was partitioned between water and EtOAc. The organic phase was separated and concentrated. This residue was purified by medium pressure liquid chromatography, using 5% MeOH in DCM as eluent. The purified intermediate (7.5 g) was dissolved in ethanol (300 ml) with ammonia solution (500 ml). The reaction vessel was sealed and the mixture was stirred at ambient temperature for 6 hours before being allowed to stand for 2.5 days. The solution was concentrated and the residue was purified using medium pressure liquid chromatography, using 25% MeOH in DCM as eluent, to afford the title compound (4.1 g).

**Method #26**

(*trans*)-4-Amino-5-hydroxy-1,3,4,5-tetrahydro-2*H*-1-benzazepin-2-one

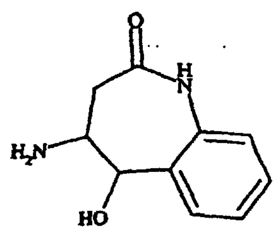

Anhydrous toluene (33 ml) and ethanol (2.2 ml) were heated with potassium (0.3 g) until all the metal has dissolved. 3,4-Dihydro-1*H*-1-benzazepine-2,5-dione (*J. Org. Chem.,* 1972, 37, 208) (11.6 g) was added and the mixture was heated under reflux for 2 minutes before being cooled to ambient temperature. Butyl nitrite (1.7 ml) was added and the mixture was stirred for 4 hours before being left to stand for 3 days. The solvent was decanted and the residue was passed down a Fluorisil® column, using 5% MeOH in chloroform as eluent. The product (1.0 g) was dissolved in water (40 ml) and MeOH (14 ml) containing palladium on charcoal (5%, 500 mg). The mixture was stirred under an atmosphere of hydrogen for 1 hour. The mixture was filtered and the filtrate was concentrated. The residue was recrystallized using ethanol to afford the title compound.

**Method #27**

(2*R,5S*)-2-Aminomethyl-5-(1,3-benzodioxol-5yl)-4-methylmorpholine

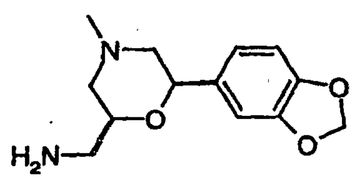

A solution of 1-(1,3-benzodioxol-5-yl}-2-(methylamino)ethanol *(Synthesis,* 1979, 423) (28.65 g) and oxirane-2-carboxamide *(Bull. Chem. Soc. Jpn,* 1989, **62**, 3202) (14.0 g) in ethanol (500 ml) was heated under reflux for 1.5 hours. The solution was concentrated and the residue was triturated using diethyl ether. A white powder was filtered off. This product (3.0 g) was added portionwise to trifluoroacetic acid (30 ml) at ambient temperature. The solution was stirred for 45 minutes before being cencentrated. The residue was basified using satured aqueous sodium bicarbonate. The mixture was filtered and the filtrate was concentrated. The residual oil was purified by flash column chromatography, using a gradient of MeOH in EtOAc as eluent, to afford both *cis-* and *trans-* isomers of the required intermediate. The *trans-* isomer (1.6 g) was dissolved in dry diethyl ether (180 ml). The solution was heated under reflux using Soxhlet equipment with lithium aluminium hydride (2.0 g) for 16 hours. The solution was cooled and water (2 ml) was added with sodium hydroxide solution (3 M, 2 ml), followed by more water (6 ml). The metal residues were removed by filtration and the filtrate was extracted using DCM. The combined organic layers were dried, filtered and concentrated. The

residue was triturated using ethanol to afford the title compound as a white solid.

**Method #28**

*N*-Methyl-*N*-phenyl-2-(*tert*-butoxycarbonylamino)acetamide

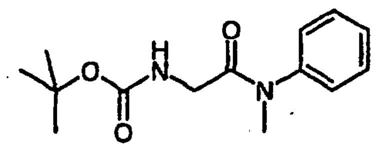

By a similar procedure to Method #5 the *title compound* was prepared using *N*-methylaniline to give a pale orange oil (771mg, 58%); NMR: ($CDCl_3$): 7.4 (3H, m), 7.1(2H, m), 5.3(1H, br), 3.6(2H, br), 3.2(3H, s), 1.4(9H, s); m/z 265.42

**Method #29**

*N*-Benzyl-*N*-phenyl-2-(*tert*-butoxycarbonylamino)acetamide

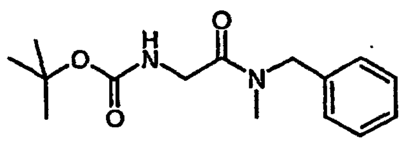

By a similar procedure to Method #5 the *title compound* was prepared using *N*-benzylmethylamine to give a yellow oil (670mg, 48%); NMR: $CDCl_3$): 7.2 (5H, m), 5.5(1H, br), 4.6 (1.33H, s), 4.4(0.67H, s), 3.9(2H, br), 2.9(1H, s), 2.8(2H, s), 1.4(9H, s); m/z 279.50

**Method #30**

2-Amino-*N*-methyl-*N*-phenylacetamide

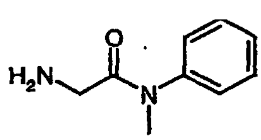

By a similar procedure to Method #6 using *N*-methyl-*N*-phenyl-2-(*tert*-butoxycarbonylamino)acetamide (Method #28) the *title compound* was prepared to give the *title compound* as a clear oil (231mg, 56%); NMR: ($CDCl_3$): 7.3 (3H, m), 7.1(2H, d), 3.2(3H, s), (2H, br); m/z 165.17

**Method #31**

2-Amino-*N*-methyl-*N*-benzylacetamide

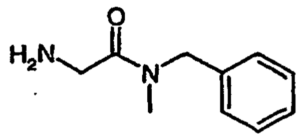

By a similar procedure to Method #6 using *N*-benzyl-*N*-phenyl-2-(*tert*-butoxycarbonylamino)acetamide (Method #29) the *title compound* was prepared to give the *title compound* as a clear oil (256mg, 73%); NMR: ($CDCl_3$): 7.3 (5H, m), 4.6(1.33H, s), 4.4(0.67H, s), 3.4(2H, br), 2.95(1.33H, s), 2.8(2.34H, s), (1.33H, s); m/z 179.23

**Method #32**

Methyl (*S*)-5-(□-aminophenethyl)oxazole-4-carboxylate trifluoroacetate

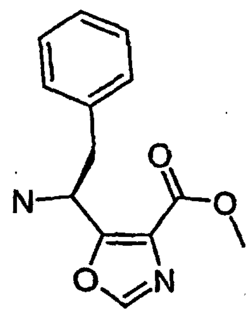

Methyl (*S*)-5-[□-(*tert*-butoxycarbonylamino)phenethyl]-oxazole-4-carboxylate, (Tett. Lett., 1982, 23, 235; 417mg) was dissolved in trifluoroacetic acid (3ml) and stood at ambient temperature for 1 hour and concentrated to give an oil. Trituration with diethyl ether gave the title compound as a white solid. (281mg); NMR 3.1-3.25(1H,m); 3.3-3.4(1H,m); 3.7(3H,s); 5.2-5.3(1H,m); 7.05(2H,d); 7.2-7.3(3H,m); 8.7(1H,s); 8.75-8.85(3H,bs)

**Method #33**

3-Aminomethyl-4-phenylisoxazole

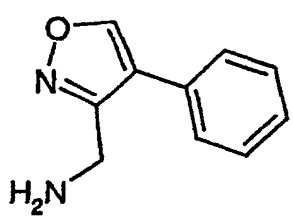

4-Phenyhsoxazole-3-carboxylic acid ethyl ester (JOC 50 13 2372 1983; 404mg, 1.86mmol) dissolved in THF 10ml under nitrogen was treated with 2M lithium borohydride in THF (1.86ml, 3.72mmol). The resultant mixture was

stirred at 0C for 5hours, then allowed to warm to room temperature overnight. 1M acetic acid was added dropwise till effervesence ceased, a further 20 ml of water was added and the resultant solution extracted with ethyl acetate (3x20 ml), the organic extracts were washed with saturated sodium bicarbonate solution (10ml) and saturated brine (10ml), dried with anhydrous magnesium sulphate and evaporated to dryness under reduced pressure to give 3-hydroxymethyl-4-phenylisoxazole (295mg); NMR (CDCl3): 4.9(2H,d), 7.3-7.5(5H,m), 8.5(1H,s) m/z 176 (M+H)

3-Hydroxymethyl-4-phenylisoxazole (295mg, 1.68mmol) was dissolved in THF 5ml under nitrogen together with phthalimide (292mg, 1.68mmol) and triphenylphosphine (440mg, 1.68mmol), the resultant stirred solution was cooled to 0C and treated with DIAD (330ul,1.68mmol) dropwise over 30 mins, then allowed to warm to room temperature overnight. After evaporation to dryness the mixture was purified by chromatography on a 20g Bond Elute silica column eluting with 1:1 DCM:hexane to give 4-phenyl-3-phthalimidomethylisoxazole (316mg): m/z 305 (M+H). This was dissolved in methanol 3ml, treated with hydrazine hydrate (114ul, 2.4mmol) and refluxed for 30 mins. The resultant suspension was filtered, the solid washed with ethanol (2x5ml) and the filtrate then evaporated to dryness, this residue was taken up in 2N HCl and any insoluble material again removed by filtration. The filtrate was basified with saturated sodium bicarbonate and extracted with ethyl acetate (3x10ml), the combined organic extracts were washed with water (5ml) and saturated brine (5ml), then dried over magnesium sulphate and evaporated under reduced pressure to give 3-aminomethyl-4-phenylisoxazole (130mg); NMR (CDCl3) : 4.1(2H,s), 7.3-7.5(5H,m), 8.4(1H,s); m/z 175 (M+H)

**Method #34**

2-[2-(2-morpholinoethoxyphenyl]ethylamine

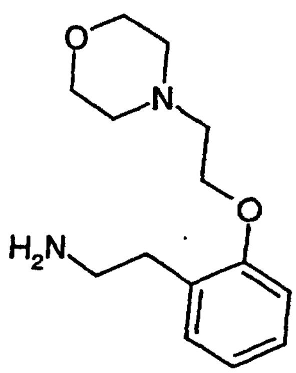

2-(2-Hydroxyphenyl)ethylamine (1.73g, 10mmol) dissolved in DCM 60ml was treated with a solution of sodium bicarbonate (1.68g, 20mmol) in water, the emulsion was stirred vigorously in an ice bath and benzyl chloroformate (1.785g, 10.5mmol) was added dropwise over 10 mins and the mixture was stirred overnght at room temperature. The DCM phase was removed and the aqueous phase extracted with DCM (2x20ml). The combined organics were then dried over magnesium sulphate and evaporated under reduced pressure, the product was purified by chromatography on 20g silica bond elute, eluting with DCM to give *N*-(benzyloxycarbonyl)-2-(2-hydroxyphenyl)ethylamine (1.4g); NMR (CDCl3): 2.8(2H,q), 3.4(2H,q), 5.1(3H,m), 6.4(1H,s), 6.8(2H,m), 7.1(2Hm), 7.3-7.4(5H,m)

*N*-(Benzyloxycarbonyl)-2-(2-hydroxyphenyl)ethylamine (542mg, 2mmol) dissolved in DMF 5ml was treated with potassium carbonate (325 mesh, 350mg, 2.5mmol) and 2-chloroethyl p-toluenesulphonate (484ul,2.6mmol) and the mixture was stirred at 60C overnight. After cooling, water 10ml was added and the mixture extracted with diethyl ether (3x20ml) dried over magnesium sulphate and evaporated under reduced pressure, the product was purified by chromatography on 20g silica bond elute, eluting with DCM to give *N*-(benzyloxycarbonyl)-2-[2-(2-chloroethoxy)phenyl] ethylamine(450mg); NMR (CDCl3) : 2.9(2H,q), 3.5(2H,m), 3.8(2H,m), 4.2(2H,m), 4.9(1H,b), 5.1(2H,s), 6.8(1H,d), 6.9 (1H,t), 7.1(2H,m), 7.3-7.4(5H,m) HPLC Hichrome C18 column Acetonitnle/water/0.1%TFA 5-95% over 7.5 min Rt 4.98min

*N*-(Benzyloxycarbonil)-2-[2-(2-chloroethoxy)phenyl]ethylamine(110mg, 0.09mmol) dissolved in NMP 3ml was heated with morpholine (174ul, 2mmol) at 80C overnight. Water (5ml) was added and the mixture extracted with ethyl acetate (30ml), the organic phase was washed with water (2x5m1) dried over magnesium sulphate and evaporated under reduced pressure to give *N*-(benzyloxycarbonyl)-2-[2-(2-morpholinoethoxy)phenyl]ethylamine (143mg); NMR (CDCl3): 2.5(4H,q), 2.8(4H,m), 3.4(2H,q), 3.7(4H,q), 4.1(2H,t), 5.0(2H,s), 5.3(1H,b), 6.8(2H,m), 7.1(1H,d), 7.2(1H,t), 7.3-7.4(5H,m) HPLC Hichrome C18 column Acetonitrile/water/0.1 %TFA 5-95% over 7.5 min Rt 3.73min m/z 385 (M+H)

*N*-(Benzyloxycarbonyl)-2-[2-(2-morpholinoethoxy)phenyl]ethylamine (143mg) dissolved in methanol was treated with 10% palladium on carbon and hydrogenated for 2 hours. The solid was removed by filtration, washed with methanol (2x2ml), the filtrate evaporated under reduced pressure and azeotroped twice with toluene to give the title compound (83mg) m/z 251 (M+H), which was used directly in the next step.

**Method #35**

Methyl 2-[2-(2-aminoethyl)phenoxy]acetate

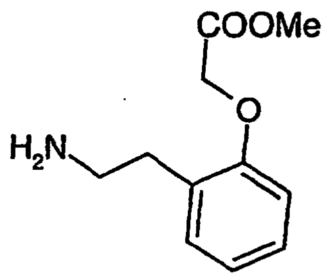

*N*-(Benzyloxycarbanyl)-2-(2-hydroxyphenyl)ethylamine (see Method #34: 718mg, 2.65mmol) dissolved in DMF 6ml was treated with potassium carbonate (-325 mesh, 397mg, 4.0mmol) methyl bromoacetate (362ul, 3.7mmol) and the mixture was stirred overnight at room temperature. After cooling, water 10ml was added and the mixture extracted with diethyl ether (3x20ml) dried over magnesium sulphate and evaporated under reduced pressure to give methyl 2-{2-[2-(benzyloxycarbonyl(amino)ethyl)]phenoxy) acetate (847mg); NMR (CDCl3) : 2.8(2H,q), 3.5(2H,m), 3.8 (3H,s), 4.6(2H,s), 5.1(3H,m), 6.7(1H,d), 6.9(1H,t) 7.1-7.4(7H,m)

Methyl 2-{2-[2-(benzyloxycarbonylamino)ethyl]phenoxy}acetate (343mg, 1mmol) dissolved in methanol together with p-toluenesulphonic acid (190mg,1mmol was treated with 10% palladium on carbon and hydrogenated for 2 hours. The solid was removed by filtration, washed with methanol (2x2ml), the filtrate evaporated under reduced pressure and azeotroped twice with toluene to give methyl 2-[2-(2-aminoethyl)phenoxy]acetate (403mg), m/z 210 (M+H) which was used without further purification.

**Method #36**

2-Aminoindan-1-ol

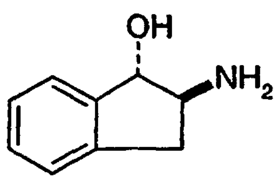

Isoamyl nitrite (15 ml, 108 mmol) was added to a solution of indan-1,2-dione (12 g, 90 mmol) in methanol (380 ml) at 45°C followed by concentrated HCl (12 ml) dropwise over 5 minutes. The reaction mixture was stirred for 3 hours at room temperature. Excess isoamyl nitrite (1 ml) and concentrated HCl (1 ml) was added and the suspension stirred for a further 15 minutes. On cooling to room temperature a white precipitate formed. The precipitate was filtered off and washed with cold methanol (40 ml) followed by diethyl ether (40 ml) to afford indan-1,2-dione-2-oxime as a white solid (6.2 g, 43%).

NMR: 3.8(2H,s), 7.4(1H,t), 7.6(1H,d), 7.7(2H,t); m/z 162 (M+H)

A solution of indan-1,2-dione-2-oxime (6.2 g, 39 mmol) in ethanol (470 ml) and 4MHC1/Dioxane (36 ml) was hydrogenated at room temperature and 40 psi. The reaction mixture was filtered through celite, washed with ethanol (30 ml) and concentrated under reduced pressure to give 10 g of an off-white solid which was recrystallised from ethanol to give the title compound as a white solid (5 g, 86%). NMR: 2.8(1H,dd), 3.2(1H,dd), to give the title compound

as a white solid (5 g, 86%). NMR: 2.8(1H,dd), 3.2( 1H,dd), 3.7(1H,q), 5.1(1H,t), 6.0(1H,d), 7.1-7.3(4H,m), 8.6(2H,s)

**Method #37**

2-Amino-*N*-methyl-*N*-(1-oxo-1,2,3,4-tetrahydronaphth-2-yl)methylacetamide

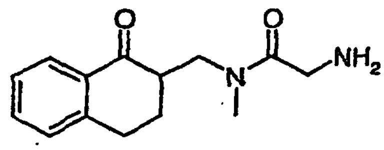

Tetralone (1 eq, 100 mmol, 15 g) and methylamine hydrochloride (2.5 eq, 250 mmol, 15 g) and paraformaldehyde (1eq, 100 mmol, 3 g) in ethanol (100 ml) were heated under reflux for 16 hours. The reaction mixture was cooled and filtered to afford 2-(methylaminomethyl)-3,4,dihydro-1-(2H)-naphthalenone as a solid (14 g).

2-(Methylaminomethyl)-3,4,dihydro-1-(2*H*)-naphthalenone (1 eq, 20 mmol, 4.5 g), HOBT (1 eq, 20 mmol, 2.7 g),*N*-benzyloxycarbonylglycine (1 eq, 20 mmol, 4.18 g), triethylamine (1.25 eq, 25 mmol, 3 ml) and dicyclohexylcarbodiimide (1 eq, 20 mmol, 4.12 g) were dissolved in dichloromethane (130 ml) and were stirred at ambient temperature for 18 hours before being cooled to 0 °C and filtered The filtrate was concentrated and the residue was taken up in ethyl acetate before being washed with aqueous sodium bicarbonate, dried, filtered and concentrated. The residue was left to stand in a solution of hydrogen bromide in acetic acid for 30 minutes. The mixture was triturated with diethyl ether and the solvent was decanted off. The residue was treated with ammonia solution and this was extracted with chloroform. The chloroform was concentrated and the residue was recrystallised from ethanol to afford the title compound.

**Method #38**

2-Amino-6-fluoro-1-indanol

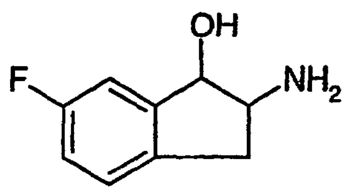

To a solution of sodium carbonate (0.8 g, 7.2 mmol) in water (13 ml) was added 4-fluorophenylalanine (1.3 g, 7.2 mmol) followed by a solution of *N*-ethoxycarbonylphthalamide (1.6 g, 7.2 mmol) in ethyl acetate (10 ml). The two-phase reaction mixture was stirred for 24 hours at room temperature. The organic phase was separated and discarded and the aqueous phase was acidified with concentrated HCl to pH 2. The aqueous phase was then extracted with ethyl acetate (3 x 20 ml) and the combined extracts dried over magnesium sulphate then concentrated under reduced pressure to give 3-(4-fluorophenyl)-2-phthalimidopropanoic acid as a white solid (2g, 89%); NMR 3.3(1H,dd), 3.5(1H,dd), 5.1(1H,dd), 6.3(1H,brs), 7.0(2H,t), 7.1(2H,dd), 7.8(4H,s).

To a solution of 3-(4-fluorophenyl)-2-phthalimidopropanoic acid (2,0g, 64 mmol) in DCM (20 ml) was added thionylchloride (0.6 ml, 6.4 mmol) and I drop of DMF. The reaction mixture was stirred at room temperature for 1 hour. Aluminium chloride (2.6 g, 19.2 mmol) was added and the reaction mixture stirred for 3 hours at room temperature. The resulting mixture was poured into ice and concentrated HCl (20 ml) and stirred for 10 minutes. This mixture was extracted with DCM (3 x 20 ml) and the combined organic extracts were washed with water (2 x 20 ml), saturated sodium hydrogen carbonate (1 x 20 ml) and water (1 x 20 ml). The organic phase was then dried over magnesium sulphate and concentrated under reduced pressure to give 6-fluoro-2 phthalamido-indan-1-one as a white solid (1g, 53%). NMR 3.3(1H,dd), 3.5(1H,dd), 5.1(1H,dd), 6.8(1H,t), 7.0(1H,dd), 7.7-8.0(5H,m); m/z 296 (M+H)

Sodium borohydride (512 mg, 14 mmol) was added to a solution 6-fluoro-2-phthalamido-indan-1-one (800 mg, 2.7 mmol) in isopropanol/water (6:1,10.5 ml) and the solution stirred at room temperature for 24 hours. Excess

acetic acid was then added to the reaction mixture and the resulting solution heated at 60°C for 6 hours. The reaction mixture was then cooled to room temperature and concentrated under reduced pressure to give a white solid which was purified by ion-exchange chromatography using Dowex 50wx2 (water:methanol 1:1 containing 3% ammonia) to give the title compound as a white solid (141 mg, 31 %); NMR 3.3(1H,dd), 3.5(1H,dd), 4.8(1H,m), 4.9(1H,d), 6.8-7.4 (3H,m); m/Z 168 (M+H)

**Method #39**

2-Amino-1,2,3,4-tetrahydronaphth-1-ol

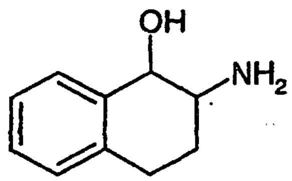

To a solution of sodium carbonate (0.8 g, 7.2 mmol) in water (13 ml) was added homophenylalanine (1.3 g, 7.2 mmol) followed by a solution of *N*-ethoxycarbonylphthalamide (1.6 g, 7.2 mmol) in ethyl acetate (10 ml). The two-phase reaction mixture was stirred for 24 hours at room temperature. The organic phase was separated and discarded and the aqueous phase was acidified with concentrated HCl to pH 2. The aqueous phase was then extracted with ethyl acetate (3 x 20 ml) and the combined extracts dried over magnesium sulphate then concentrated under reduced pressure to give 4-phenyl-2-phthalimidobutanoic acid as a white solid (1.5 g, 67%); m/z 308 (M-H).

To a solution of 4-phenyl-2-phthalimidobutanoic acid (1.5 g, 4.9 mmol) in DCM (20 ml) was added thionylchloride (0.4 ml, 4.9 mmol) and 1 drop of DMF. The reaction mixture was stirred at room temperature for 1 hour. Aluminium chloride (2.0 g, 14.7 mmol) was added and the reaction mixture stirred for 3 hours at room temperature. The resulting mixture was poured into ice and concentrated HCl (20 ml) and stirred for 10 minutes. This mixture was extracted with DCM (3 x 20 ml) and the combined organic extracts were washed with water (2 x 20 ml), saturated sodium hydrogen carbonate (1 x 20 ml) and water (1 x 20 ml). The organic phase was then dried over magnesium sulphate and concentrated under reduced pressure to give 2-phthalamido-3,4-dihydro-(2*H*)-naphthalen-1-one as a white solid (880 mg, 62%). NMR 2.3(1H,m), 2.7(1H,m), 3.1(1H,dt), 3.3(1H,m), 5.2(1H,dd), 7.3-8.0(8H,m); m/z 292 (M+H)

To a solution of 2-phthalamido-3,4-dihydro-(2*H*)-naphthalen-1-one (880 mg, 3 mmol) in isopropanol/water (6: 1,11.5 ml) was added sodium borohydride (567 mg, 15 mmol) and the solution stirred at room temperature for 24 hours. Excess acetic acid was then added to the reaction mixture and the resulting solution heated at 60°C for 6 hours. The reaction mixture was then cooled to room temperature and concentrated under reduced pressure to give a white solid which was purified by ion-exchange chromatography using Dowex 50wx2 (water:methanol 1:1 containing 3% ammonia) to give the title compound as a white solid (150 mg, 30 %). NMR 2.8(1H,dd), 3.0(1H,dd), 3.3(1H,m), 3.5(1H,m), 4.5 (1H,m), 4.8(1H,d), 7.1-7.4(4H,m); m/z 164 (M+H)

**Method #40**

(+/-) *Trans*-2-Amino-1-methoxyindan

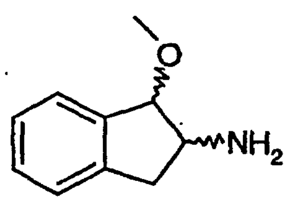

A solution of (+/-) *trans*-2-bromo-1-hydroxyindan (21.0g, 0.1mol) and potassium phthalimide (42.0g, 0.22 mol) in dry DMF (120ml) was heated at 100°C for 5 hours. The reaction mixture was cooled and evaporated to an oil which

was triturated with ethyl acetate and filtered. The filtrates were evaporated and purified by chromatography on silica with 2: 1 iso-hexane:ethyl acetate as eluent to give (+/-)-*trans* -1-hydroxy-2-phthalimido indan as a pale yellow amorphous powder (17.7g, 63%). NMR 2.8(1H, dd), 3.15(1Hdd), 4.8-5.0(1H, m), 5.4(1H, d), 5.45(1H, d), 7.0-7.3(4H, m), 7.75-8.95(4H, m).

To a solution of (+/-) *trans*-1-hydroxy-2-phthalimidoindan (1.4g, 5.0mmol) in dry tetrahydrofuran (20ml) was added 60% sodium hydride (300mg, 7.5mmol). The mixture was stirred at room temperature for 2 hours and then methyl iodide (0.62ml, 10.0mmol) added. The mixture was stirred for a further 2 hours and then 5% water in tetrahydrofuran (10ml) and ethyl acetate (75ml) added. The solution was washed with water, dried over magnesium sulphate and evaporated to give (+/-)-*trans*-1-methoxy-2-phthalimidoindan as a white solid (1.2g, 82%). NMR 2.85(1H, dd), 3.25 (3H, s), 3.5(1H, dd), 4.5-4.65(1H, m), 5.55(1H, d), 7.05-7.3(4H, m), 7.85(4H, s).

A mixture of (+/-)-*trans*-1-methoxy-2-phtalimidoindan (850mg, 2.9mmol) and hydrazine hydrate (5ml) in ethanol was stirred at room temperature for 24 hours: The mixture was evaporated and purified by ion exchange chromatography (Dowex 50W X2 $H^+$ form) and the title compound eluted with 50% aqueous methanol containing 3% ammonium hydroxide to give the title compound a pale yellow solid (400mg, 85%). NMR 2.8(1H, dd), 3.35(3H, s), 3.38 (1H, dd),4.05-4.15(1H, m), 4.5-4.6(1H, m), 7.2-7.35(3H, m), 7.45-7.55(1H, m); m/z 164 (M+H).

**Method #41**

(1*R*,2*S*)-1-[(1,1-dimethylethoxy)carbonylamino]-2 hydroxyindan

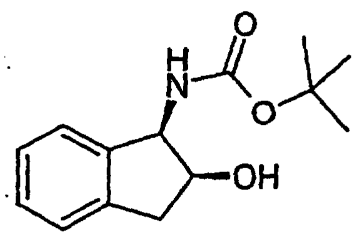

(1*R*,2*S*)-1-Amino-2-hydroxyindan(10.0g, 67,1mmol) was dissolved in dichloromethane (550ml) and triethylamine (18.7ml, 134.2mmol). Di-*tert*-butyl dicarbonate (18.3g, 83,9mmol) in dichloromethane (50ml) was added and the mixture stirred at room temperature for 20 hours then evaporated. Ethyl acetate (200ml) was added, the solution washed with water, dried over magnesium sulphate and evaporated. The crude product was purified by chromatography on silica with 4:1 *iso*-hexane:ethyl acetate as eluent to give the title compound as a white solid (16.1g, 96%); NMR 1.42(9H, s), 2.78(1H, dd), 3.0(1H, dd), 4.3-4.42(1H, m), 4.78-4.9(1H, m), 4.9-5.0(1H, m), 6.3(1H, d), 7.0-7.25(4H, m).

**Method #42**

(1*R*,2*R*)-2-Amino-1-methanesulphonamidoindan

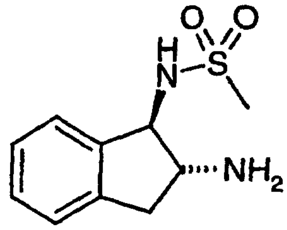

(1*R*,2*S*)-1-Amino-2-hydroxyindan (3.0g, 20mmol) was dissolved in dry tetrahydrofuran (40ml) and triethylamine (8.4ml, 60.0mmol) at 10°C. Methane sulphonyl chloride (5.0g, 44.0mmol) dissolved in tetrahydrofuran (10ml) was added at such a rate that the internal temperature remained below 15°C. Following the addition the mixture was stirred at room temperature for 20hours and then evaporated. To the residue was added ethyl acetate (100ml) and the

mixture washed with saturated aqueous sodium bicarbonate and then water. The organic solution was dried over magnesium sulphate and evaporated to give (1*R*,2*S*)-1-methanesulphonamido-2-methylsulphonyloxyindan as a pale yellow solid (5.7g, 93%).

NMR: 3.0-3.35(2H, m), 3.1(3H, s), 3.25(3H, s), 5.05-5.2(1H, m), 5.3-5.4(1H, m), 7.2-7.4(4H, m), 7.85-8.0(1H, m). m/z 304.2 (M-H).

(1*R,2S)*-1-Methanesulphonamido-2-methylsulphonyloxyindan (2.0g, 6.56mmol) was dissolved in dry dimethyl acetamide (20ml). Sodium azide (1.7g, 26.2mmol) was added and the mixture heated to 90°C for 1 hour. The reaction was cooled, diluted with ethyl acetate (100ml), washed with water (6 x 50ml), dried over magnesium sulphate and filtered. 10% Palladium on activated carbon was added and the mixture stirred under a hydrogen atmosphere for 3 hours. Filtration through celite followed by evaporation gave the title compound as a pale green solid (1.25g, 83%). NMR ($CDCl_3$):1.68(2H, broad s), 2.67(1H, dd), 3.2(3H, s), 3.23(1H, dd), 4.5-4.6(1H, m), 4.6-4.8(1H, m), 7.15-7.35(4H, m); m/z 227.4 (M+H).

**Method #43**

(1*R,2R*)-2-Amino-1-[(1,1-dimethylethoxy)carbonylamino]indan

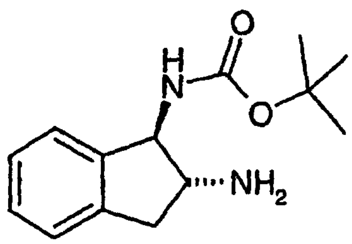

(1*R*,2*S*)-1-[(1,1-Dimethylethoxy)carbonylamino]-2-hydroxyindan (Method #41, 7.5g, 30.1mmo1) was dissolved in dry tetrahydrofuran (90ml) and triethylamine (6.3ml, 45.0mmol). Methanesulfonyl chloride (3.78g, 33.0mmol) dissolved in dry tetrahydrofuran (10ml) was added and the mixture stirred at room temperature for 20 hours. The mixture was evaporated and ethyl acetate (250ml) added. After washing with water and drying over magnesium sulphate the organic solution was evaporated to (1*R*,2*S*)-1-((1,1-dimethylethoxy)carbonylamino]-2-methanesulphonyloxyindan as white solid (9.7g, 98%).

NMR1.45(9H, s), 3.05-3.35(2H, m), 3.18(3H, s), 5.15-5.25(1H, m), 5.28-5.38(1H, m), 7.15-7.22(4H, m), 7.45(1H, d).

(1*R*,2*S*)-1-[(1,1-Dimethylethoxy)carbonylamino]-2-methanesulphonyloxyindan (3.5g, 10.7mmol) was dissolved in dry dimethyl acetamide (50ml). Sodium azide (3.5g; 53.9mmol) was added and the mixture heated to 90°C for 3 hours. The reaction was cooled, diluted with ethyl acetate (150ml), washed with water (6 x 50ml) and dried over magnesium sulphate. 10% Palladium on activated carbon was added and the mixture stirred under a hydrogen atmosphere for 4 hours. Filtration through celite followed by evaporation gave the title compound as a white solid (2.6g, 98%). NMR: 1.45(9H, s), 2.5(1H, dd), 3.0(1H, dd), 3.2-3.45(3H, m), 4.5-4.6(1H, m), 7.0-7.25(5H, m).

**Method #44**

2,3-Dichloro-5-[*N*-(1-{*N*-[(1,1-dimethylethoxy)carbonyl]-*N*-methylamino}indan-2-yl)carbamoyl]-4*H*-thieno[3.2-*b*] pyrrole

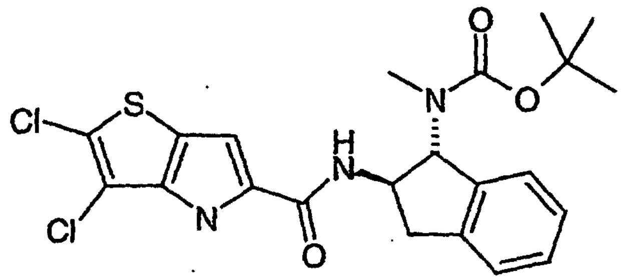

To a solution of (1*R*,2S)-1-[(1,1-dimethylethoxy)carbonylamino]-2-hydroxyindan (Method #41, 7.0g, 28.1mmol) in dichloromethane (50ml) was added 3,4-dihydro-2*H*-pyran (4.7g, 56.2mmol) and pyridinium toluene-4-sulphonate (100mg). The mixture was stirred for 4 hours, diluted with ethyl acetate (200ml), washed with water (2 x 50ml), dried over magnesium sulphate and evaporated to give (1*R*,2*S*-1-[(1,1-dimethylethoxy)carbonylamino]-2-[(tetrahydropyran-2-yl)oxy]indan as a white solid (8.9g, 96%). NMR1.25-1.85(6H, m), 1.45(9H, d), 2.85-3.1(2H, m), 3.35-3.5(1H, m), 3.68-3.9(1H, m), 4.35-5.1(3H, m), 6.8(1H, dd), 7.1-7.3(4H, m).

(1*R*, 2*S*)-1-[(1,1-Dimethylethoxy)carbonylamino]-2-[(tetrahydropyran-2-yl)oxy]indan (4.0g, 12.0mmol) was dissolved in dry DMA (25ml) at 10°C and 60% sodium hydride (575mg, 14.4mmol) added. The mixture was stirred at room temperature for 30 minutes and then methyl iodide (2.0g, 14.4mmol) added after which the reaction was stirred for a further 3 hours at room temperature. The mixture was diluted with ethyl acetate (50ml), washed with water (6 x 50ml), dried over magnesium sulphate and evaporated to give (1*R*,2*S*)-1-{*N*-[(1,1-dimethylethoxy)]carbonyl-*N*-methylamino}-2-(tetrahydropyran-2-yl)oxyindan as an oil (4.1g, 98%). NMR 1.4-1.9(6H, m), 1.5(9H, d), 2.7(3H, dd), 2.85-3.3 (2H, m), 3.47-3.6(1H, m), 3.72-4.0(1H, m), 4.7-5.0(2H, m), 5.5-5.84(1H, m), 7.15-7.35(4H, m); m/z 348.6 (M+H).

To a solution of (1*R*,2*S*)-1-{*N*-[(1,1-dimethylethoxy)carbonyl]-*N*-methylamino}-2-(tetrahydropyran-2-yl)oxyindan (4.0g, 11.5mmol) in methanol (50ml) was added toluene-4-sulphonic acid (100mg) and the mixture stirred at room temperature for 2 hours. Saturated aqueous sodium bicarbonate (50ml) and water (10ml) was added and the mixture extracted with ethyl acetate. The organic extract was washed with water, dried over magnesium sulphate and evaporated to give (1*R*,*2S*}-1-{*N*-[(1,1-dimethylethoxy)carbonyl]-*N*-metylamino}-2-hydroxyindan as an oil (3.0g, 100%). NMR 1.45(9H, s), 2.6(3H, s), 2.75(1H, dd), 3.05(1H, dd), 4.4-4.57(1H,m), 5.0-5.12(1H, m), 5.34(1H, dd), 7.03-7.33 (4H, m).

To a solution give (1*R*,*2S*)-1-{*N*-[(1,1-dimethylethoxy)carbonyl]-*N*-methylamino}-2-hydroxyindan (3.0g, 11.4mmol) in dry tetrahydrofuran (40mmol) was added triethylamine (2.4ml, 17.1mmol) and methane sulphonyl chloride (1.44g, 12.55mmol). The mixture was stirred at room temperature for 1 hour, evaporated and diluted with ethyl acetate (100ml). The organic solution was washed with saturated aqueous sodium bicarbonate and then water, dried over magnesium sulphate and evaporated to give a pale yellow syrup. The crude material was purified by silica chromatography with 4:1 *iso*-hexane:ethyl acetate as eluent to (1*R*,2*S*)-1-{*N*-[(1,1-dimethylethoxy)carbonyl]-*N*-methyl-amino}-2-methanesulphonyloxyindan as a clear colourless syrup (3.1g, 80%). NMR ($CDCl_3$): 1.54(9H, s), 2.7(3H, d), 3.0(3H, s), 3.16-3.42(2H, m),5.35-551(1H, m), 5.78(1H, dd), 7.2-7.35(4H, m); m/z 342.5 (M+H).

(1*R*,2*S*)-1-{*N*-[(1,1-Dimethylethoxy)carbonyl]-*N*-methyl-amino}-2-methanesulphonyloxyindan (3.0g, 8.8mmol) was dissolved in dry DMA (30ml) and sodium azide (2.3g, 35.2mmol) added. The mixture was heated to 90°C for 6 hours, cooled and diluted with ethyl acetate (100ml). The solution was washed with water (6 x 50ml), dried over magnesium sulphate and filtered. 10% Palladium on activated carbon was added and the mixture stirred under a hydrogen atmosphere for 4 hours. The mixture was filtered, evaporated and purified by silica chromatography with 10% methanol in dichloromethane to give (1*R*,2*S*)-2-amino-1-{*N*-[(1,1-dimethylethoxy)]carbonyl-*N*-methyl-amino}indan as an oil (1.2g, 55%). NMR 1.36-1.56(9H, m), 2.6(3H, s), 2.7-2.87(1H, m), 3.2-3.35(1H, m), 4.37-4.54(1H, m), 5.4-5.7(1H, m), 6.93-7.1 (1H, m), 7.12-7.5(4H, m); m/z 263.48 (M+H).

5-Carboxy-2,3-Dichloro-4*H*-thieno[3,2-*b*]pyrrole (Method #9, 472mg, 2.0 mmol), (1*R*,2*S*)-2-amino-1-{*N*-[(1,1-dimethylethoxy)]carbonyl-*N*-methyl-amino}indan (524mg, 2.0mmol), DIPEA(0.348ml, 2.0mmol) and HOBT (270mg,2.0mmol) was sulfur in dichloromethane (10ml) at room temperature for 2 minutes. EDAC (480mg, 2.5mmol)

was added and the mixture stirred at room temperature for 20 hours. The reaction was evaporated, ethyl acetate (50ml) added and washed with water. The organic phase was dried over magnesium sulphate and evaporated to give the title compound as a pale brown foam (900mg, 94%). NMR 1.2-1.45(9H, m), 2.77(3H, s), 2.9-3.26(2H, m), 4.7-4.94(1H, m), 5.5-5.8(1H, m), 6.9-7.34(5H, m), 8.55-8.73(1H, m), 12.25(1H, broad s); m/z 480.3/482.1 (M+H).

**Example 155**

The following illustrate representative pharmaceutical dosage forms containing the compound of formula (**I**), or a pharmaceutically acceptable salt or *in vivo* hydrolysable ester thereof (hereafter compound X), for therapeutic or prophylactic use in humans:-

| **(a): Tablet I** | **mg/tablet** |
|---|---|
| Compound X | 100 |
| Lactose Ph.Eur | 182.75 |
| Croscarmellose sodium | 12.0 |
| Maize starch paste (5% w/v paste) | 2.25 |
| Magnesium stearate | 3.0 |
| | |
| **(b): Tablet II** | **mg/taNet** |
| Compound X | 50 |
| Lactose Ph.Eur | 223.75 |
| Croscarmellose sodium | 6.0 |
| Maize starch | 15.0 |
| Polyvinylpyrrolidone (5% w/v paste) | 2.25 |
| Magnesium stearate | 3.0 |
| | |
| **(c): Tablet III** | **mg/tablet** |
| Compound X | 1.0 |
| Lactose Ph.Eur | 93.25 |
| Croscarmellose sodium | 4.0 |
| Maize starch paste (5% w/v paste) | 0.75 |
| Magnesium stearate | 1.0 |
| | |
| **(d): Capsule** | **mg/capsule** |
| Compound X | 10 |
| Lactose Ph.Eur | 488.5 |
| Magnesium stearate | 1.5 |
| | |
| **(e): Injection I** | **(50 mg/ml)** |
| Compound X | 5.0% w/v |
| 1M Sodium hydroxide solution | 15.0% v/v |
| 0.1M Hydrochloric acid | (to adjust pH to 7.6) |
| Polyethylene glycol 400 | 4.5% w/v |

(continued)

| (e): Injection I | (50 mg/ml) |
|---|---|
| Water for injection | to 100% |
| | |
| **(f): Injection II** | **10 mg/ml** |
| Compound X | 1.0% w/v |
| Sodium phosphate BP | 3.6% w/v |
| 0.1M Sodium hydroxide solution | 15.0% v/v |
| Water for injection | to 100% |
| | |
| **(g): Injection III** | **(1mg/ml,buffered to pH6)** |
| Compound X | 0.1% w/v |
| Sodium phosphate BP | 2.26% w/v |
| Citric acid | 0.38% w/v |
| Polyethylene glycol 400 | 3.5% w/v |
| Water for injection | to 100% |

Note

The above formulations may be obtained by conventional procedures well known in the pharmaceutical art. The tablets (a)-(c) may be enteric coated by conventional means, for example to provide a coating of cellulose acetate phthalate.

## Claims

**1.** A compound of formula (**I**):

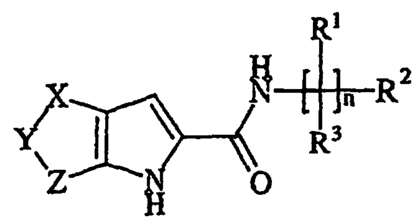

**(I)**

wherein:

**-X-Y-Z-** is selected from -S-$CR^4$=$CR^5$-, -$CR^4$=$CR^5$-S-, O-$CR^4$=$CR^5$ , -$CR^4CR^5$-O-, -N=$CR^4$-S-,-S-$CR^4$N-, -$NR^6$-$CR^4$=$CR^5$-and-$CR^4$=$CR^5$-$NR^6$-;

wherein **$R^4$** and **$R^5$** are independently selected from hydrogen, halo, nitro, cyano, hydroxy, fluoromethyl, difluoromethyl, trifluoromethyl, trifluoromethoxy, amino, carboxy, carbamoyl, mercapto, sulphamoyl, ureido, $C_{1-6}$alkyl, $C_{2-6}$alkenyl, $C_{2-6}$alkynyl, $C_{1-6}$alkoxy, $C_{1-6}$alkanoyl, $C_{1-6}$alkanoyloxy, *N*-($C_{1-6}$alkyl)amino, *N,N*-($C_{1-6}$alkyl)$_2$amino, $C_{1-6}$alkanoylamino, *N*-($C_{1-6}$alkyl)carbamoyl, *N,N*-($C_{1-6}$alkyl)$_2$carbamoyl, $C_{1-6}$alkylS(O)$_a$ wherein a is 0 to 2, $C_{1-6}$alkoxycarbonyl, $C_{1-6}$alkaxycarbonylamino, *N*-($C_{1-6}$alkyl)sulphamoyl, *N,N*-($C_{1-6}$alkyl)$_2$sulphamoyl, $C_{1-6}$alkylsulphonylamino and $C_{1-6}$alkylsulphonyl-*N*-($C_{1-6}$alkyl)amino;

**$R^6$** is hydrogen or $C_{1-6}$alkyl;

**$R^1$** is selected from hydrogen, halo, nitro, cyano, hydroxy, amino, carboxy, carbamoyl, mercapto, sulphamoyl,

ureido, $C_{1-6}$alkyl, $C_{2-6}$alkenyl, $C_{1-6}$alkynyl, $C_{1-6}$alkoxy, $C_{1-6}$alkanoyl, $C_{1-6}$alkanoyloxy, *N*-($C_{1-6}$alkyl)amino, *N,N*-($C_{1-6}$alkyl)$_2$amino, $C_{1-6}$alkanoylamino, *N*-($C_{1-6}$alkyl)carbamoyl, *N,N*-($C_{1-4}$alkyl)$_2$carbamoyl, $C_{1-6}$alkylS(O)$_a$ wherein a is 0 to 2, $C_{1-6}$alkoxycarbonyl, $C_{1-6}$alkoxycarbonylamino, *N*-($C_{1-6}$alkyl)sulphamoyl, *N,N*-($C_{1-6}$alkyl)$_2$sulphamoyl, $C_{1-6}$alkylsulphonylamino, $C_{1-6}$alkylsulphonyl-*N*-($C_{1-6}$alkyl)amino, $C_{3-8}$cycloalkyl, $C_{3-8}$cycloalkyl$C_{1-6}$alkyl, aryl, aryl$C_{1-6}$alkyl, heterocyclic group and (heterocyclic group)$C_{1-6}$alkyl; wherein $R^1$ may be optionally substituted on carbon by one or more groups selected from P and wherein if said heterocyclic group contains an -NH- moiety that nitrogen may be optionally substituted by a group selected from R;

**$R^2$** is selected from hydrogen; halo, nitro, cyano, hydroxy, fluoromethyl, difluoromethyl, trifluoromethyl, trifluoromethoxy, amino, carboxy, carbamoyl, mercapto, sulphamoyl, ureido, $C_{1-6}$alkyl, $C_{2-6}$alkenyl, $C_{2-6}$alkynyl, $C_{1-6}$alkoxy, $C_{1-6}$alkanoyl, $C_{1-6}$alkanoyloxy, *N*-($C_{1-6}$alkyl)amino, *N,N*-($C_{1-6}$alkyl)$_2$amino, $C_{1-6}$alkanoylamino, *N*-($C_{1-6}$alkyl)carbamoyl, *N,N*-($C_{1-4}$alkyl)$_2$carbamoyl, *N*-($C_{1-6}$alkyl)-*N*-($C_{1-6}$alkoxy)carbamoyl, $C_{1-6}$alkylS(O)$_a$ wherein a is 0 to 2, $C_{1-6}$alkoxycarbonyl, $C_{1-6}$alkoxycarbonylamino, *N*-($C_{1-6}$alkyl)sulphamoyl, *N,N*-($C_{1-6}$alkyl)$_2$sulphamoyl, sulphamoylamino, *N*-($C_{1-6}$alkyl)sulphamoylamino, *N,N*-($C_{1-6}$alkyl)$_2$sulphamoylamino, $C_{1-6}$akylsulphonylamino, $C_{1-6}$alkylsulphonylaminocarbonyl, $C_{1-6}$alkylsulphonyl-*N*-($C_{1-6}$alkyl)amino and a group -E-F-G-H;

wherein **E** and **G** are independently selected from a direct bond, -O-, -S-, -SO-, -$SO_2$-, -OC(O)-, -C(O)O-, -C(O)-, -$NR^a$-, -$NR^a$C(O)-, -C(O)$NR^a$-, -$SO_2NR^a$-, -$NR^aSO_2$-, -$NR^a$C(O)$NR^b$-, -OC(O)$NR^a$ , -$NR^a$C(O)O-, -$NR^aSO_2NR^b$-, -$SO_2NR^a$C(O)- and -C(O)$NR^aSO_2$-; wherein $R^a$ and $R^b$ are independently selected from hydrogen or $C_{1-6}$alkyl which is optionally substituted by a group V ;

**F** is $C_{1-6}$alkrylene optionally substituted by one or more Q or a direct bond;

**H is** selected from aryl, $C_{3-8}$cycloalkyl and heterocyclic group; wherein H may be optionally substituted on carbon by one or more groups selected from S and wherein if said heterocyclic group contains an -NH- moiety that nitrogen may be optionally substituted by a group selected from T;

**$R^3$** is hydrogen or $C_{1-6}$alkyl;

**n** is selected from 0-4; wherein the values of **$R^1$** be the same or different; and wherein the values of $R^3$ may be the same or different;

**P**, **S** and **Q** are independently selected from halo, nitro, cyano, hydroxy, trifluoromethyl, trifluoromethoxy, amino, carboxy, carbamoyl, mercapto, sulphamoyl, ureido, $C_{1-6}$alkyl, $C_{2-6}$alkenyl, $C_{2-6}$alkynyl, $C_{1-6}$alkoxy, $C_{1-6}$alkanoyl, $C_{1-6}$alkanoyloxy, *N*-($C_{1-6}$alkyl)amino. *N,N*-($C_{1-6}$alkyl)$_2$amino, $C_{1-6}$alkanoylamino, *N*-($C_{1-6}$alkyl)carbamoyl, *N,N*-($C_{1-6}$alkyl)$_2$carbamoyl, *N*-($C_{1-6}$alkyl)-*N*-($C_{1-6}$alkoxy)carbamoyl, $C_{1-6}$alkylS(O)a wherein a is 0 to 2, $C_{1-6}$alkoxycarbonyl, $C_{1-6}$alkoxycarbonylamino, *N*-($C_{1-6}$alkyl)sulphamoyl, *N,N*-($C_{1-6}$alkyl)$_2$sulphamoyl, $C_{1-6}$alkylsulphonylamino, $C_{1-6}$alkylsulphonyl-*N*-($C_{1-6}$alkyl)amino, $C_{3-8}$cycloalkyl, aryl and heterocyclic group; wherein P, S and Q may be optionally and independently substituted on carbon by one or more groups selected from V and wherein if said heterocyclic group contains an -NH- moiety that nitrogen may be optionally substituted by a group selected from U;

**V** is selected from halo, nitro, cyano, hydroxy, trifluoromethoxy, trifluoromethyl, amino, carboxy, carbamoyl, mercapto, sulphamoyl, methyl, ethyl, methoxy, ethoxy, acetyl, acetoxy, methylamino, ethylamino, dimethylamino, diethylamino, *N*-methyl-*N*-ethylamino, acetylamino, *N*-methylcarbamoyl, *N*-ethylcarbamoyl, *N,N*-dimethylcarbamoyl, *N,N*-diethylcarbamoyl, *N*-methyl-*N*-ethylcarbamoyl, methylthio, ethylthio, methylsulphinyl, ethylsulphinyl, mesyl, ethylsulphonyl, methoxycarbonyl, ethoxycarbonyl, *N*-methylsulphamoyl, *N*-ethylsulphamoyl, *N,N*-dimethylsulphamoyl, *N,N*-diethylsulphamoyl, *N*-methyl-*N*-ethylsulphamoyl, morpholino, morpholinocarbonyl, *N*-benzylcarbamoyl, and 4-hydroxypiperidinocarbonyl;

**R, T** and **U** are independently selected from $C_{1-4}$alkyl, $C_{1-4}$alkanoyl, $C_{1-4}$alkylsulphonyl, $C_{1-4}$alkoxycarbonyl, carbamoyl,*N*,($C_{1-4}$alkyl)carbamoyl, *N,N*-($C_{1-4}$alkyl)carbamoyl, phenyl, benzyl, benzyloxycarbonyl, benzoyl and phenylsulphonyl wherein R, T and U may be optionally and independently substituted on carbon by one or more groups selected from V;

or a pharmaceutically acceptable salt or an *in vivo* hydrolysable ester thereof; with the provisos:

i) when -X-Y-Z- is -S-CH=CH-, $R^2$-($CR^1R^3$)$_n$- cannot be amino, 1-phenyl-5-methyl-1H-1,5-benzodiazepine-2,4(3H,5H)dion-3-yl, 1-methyl-5 phenyl-2-oxo-2,3-dihydro-1H-benzo(E)(1,4)diazepin-3-yl, 2-(4-phenyl-1,2,5,6-tetrahydropyrid-1-yl)ethyl, 3-(4-phenyl-1,2,3,6-tetrahydropyrid-1-yl)propyl,2-(4-phenylpiperazin-1yl)ethyl,2-(*N*-methylamino)ethyl, 2-morpholinoethyl or 2-(*N*-methyl-*N*-benzylamino)ethy);

ii) when -X-Y-Z- is -CH=CH-S-, $R^2$-($CR^1R^3$)$_n$- cannot be amino or 1-methyl-5-phenyl-2-oxo-2,3-dihydro-1H-benzo(E)(1,4)diazepin-3-yl;

iii) when -X-Y-Z- is -CH=C($SO_2NH_2$)-S-, $R^2$-($CR^1R^3$)$_n$- cannot be methyl or isobutyl; and

iv) when -X-Y-Z- is as initially defined, n is 1, $R^1$ is arylmethyl, substituted arylmethyl, (heterocyclic group) methyl and substituted (heterocyclic group)methyl and $R^3$ is hydrogen then $R^2$ is not a group -C(=O)-A or a

group -CH(OH)-C(=O)-A in which A is $NR^dR^d$, $-NR^aCH_2CH_2OR^a$, or

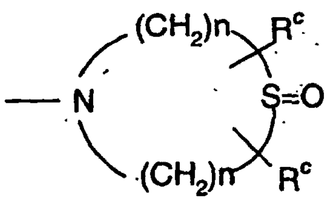 , 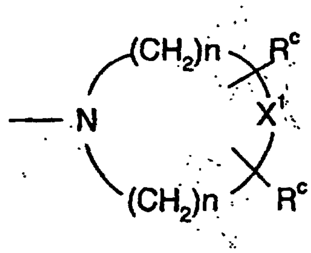 , or 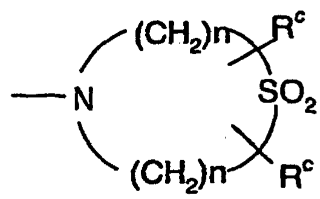 ;

each $R^a$ and $R^b$ is independently hydrogen or $-C_1$-$C_8$alkyl;

each $R^d$ is independently hydrogen, $C_1$-$C_8$alkyl, $C_1$-$C_8$alkoxy, aryl, substituted aryl, heteroaryl, or substituted heteroaryl;

each $R^c$ is independently hydrogen, $-C(=O)OR^a$, $-OR^a$, $-SR^a$, or $-NR^aR^a$; and each n is independently 1-3, and $X^1$ is $NR^a$, $-CH_2$-, O or S.

**2.** A compound of formula (**I**) according to claim1 wherein:

-X-Y-Z- is selected from $-S-CR^4=CR^5$- or $-CR^4=CR^5-S$-;
wherein $R^4$ and $R^5$ are independently selected from hydrogen, halo, nitro, cyano, hydroxy, fluoromethyl, difluoromethyl, trifludromethyl, triflupromethoxy, amino, carboxy, carbamoyl, mercapto, sulphamoyl, ureido, $C_{1-6}$alkyl, $C_{2-6}$alkenyl, $C_{2-6}$alkynyl, $C_{1-6}$alkoxy, $C_{1-6}$alkanoyl, $C_{1-6}$alkanoyloxy, *N*-($C_{1-6}$alkyl)amino,*N,N*-($C_{1-6}$akyl)$_2$amino, $C_{1-6}$alkanoylamino, *N*-($C_{1-6}$alkyl)carbamoyl, *N,N*-($C_{1-6}$alkyl)$_2$carbamoyl; $C_{1-6}$alkylS(O)$_a$ wherein a is 0 to 2, $C_{1-6}$alkoxycarbonyl, $C_{1-6}$alkoxycarbonylamino, *N*-($C_{1-6}$alkyl)sulphamoyl, *N,N*-($C_{1-6}$ alkyl)$_2$sulphamoyl,$C_{1-6}$alkylsuphonylamino and $C_{1-6}$alkylsulphonyl-*N*-($C_{1-6}$alkyl)amino;
n is 0;
$R^2$ is a group -E-F-G-H;
wherein E, F and G are each a direct bond;
H is a $C_{3-12}$cycloalkyl which is optionally fused to a benz ring wherein H may be optionally substituted on carbon by one or more groups S which are independently selected from halo, nitro, cyano, hydroxy, trifluoromethyl, trifluoromethoxy, amino, carboxy, carbamoyl, mercapto, sulphamoyl, ureido, $C_{1-6}$alkyl, $C_{2-6}$alkenyl, $C_{2-6}$alkynyl, $C_{1-6}$alkoxy, $C_{1-6}$alkanoyl, $C_{1-6}$alkanoyloxy, *N*-($C_{1-6}$alkyl)amino, *N,N*-($C_{1-6}$alkyl)$_2$amino, $C_{1-6}$alkanoylamino, *N*-($C_{1-6}$alkyl)carbamoyl, *N,N*-($C_{1-6}$alkyl)$_2$carbamoyl, *N*-($C_{1-6}$alkyl)-*N*-($C_{1-6}$alkoxy)carbamoyl, $C_{1-6}$alkylS(O)$_a$ wherein a is 0 to 2, $C_{1-6}$alkoxycarbonyl, $C_{1-6}$alkoxycarbonylamino, *N*-($C_{1-6}$akyl)sulphamoyl, *N,N*-($C_{1-6}$alkyl)$_2$sulphamoyl, $C_{1-6}$alkylsulphonylamino, $C_{1-6}$alkylsulphonyl-*N*-($C_{1-6}$alkyl)amino, $C_{3-8}$cycloalkyl, aryl and heterocyclic groups; wherein S may be optionally substituted on carbon by one or more groups selected from V;
V is selected from halo, nitro, cyano, hydroxy, trifluoromethoxy, trifluoromethyl, amino, carboxy, carbamoyl, mercapto, sulphamoyl, methyl, ethyl, methoxy, ethoxy, acetyl, acetoxy, methylamino, ethylamino, dimethylamino, diethylamino, *N*-methyl, *N*-ethylamino, acetylamino, *N*-methylcarbamoyl; *N*-ethylcarbamoyl, *N,N*-dimethylcarbamoyl, *N,N*-diethylcarbamoyl, N-methyl-*N*-ethylcarbamoyl, methylthio, ethylthio, methylsulphinyl, ethylsulphinyl, mesyl, ethylsulphonyl, methoxycarbonyl, ethoxycarbonyl, *N*-methylsulphamoyl, *N*-ethylsulphamoyl, *N,N*-dimethylsulphamoyl, *N,N*-diethylsulphamoyl, *N*-methyl-*N*-ethylsulphamoyl, morpholino , morpholinocarbonyl ,*N*-benzylcarbamoyl, and 4-hydroxypiperidinocarbonyl;

or a pharmaceutically acceptable salt thereof.

**3.** A compound of formula (**I**) as claimed in claim 1 wherein:

-X-Y-Z- is selected from $-S-CR^4=CR^5$- or $-CR^4=CR^5-S$-;
wherein $R^4$ and $R^5$ are independently selected from hydrogen, halo, nitro, cyano, hydroxy, fluoromethyl, difluoromethyl, trifluoromethyl, trifluoromethoxy, amino, carboxy, carbamoyl, mercapto, sulphamoyl, ureido, $C_{1-6}$alkyl, $C_{2-6}$alkenyl, $C_{2-6}$alkynyl, $C_{1-6}$alkoxy, $C_{1-6}$alkanoyl, $C_{1-6}$alkanoyloxy,*N*-($C_{1-6}$alkyl)amino,*N,N*-($C_{1-6}$alkyl)$_2$amino, $C_{1-6}$alkanoylamino, *N*-($C_{1-6}$alkyl)carbamoyl, *N,N*-($C_{1-6}$alkyl)$_2$carbamoyl, $C_{1-6}$alkylS(O)$_n$ wherein a is 0 to 2, $C_{1-6}$alkoxycarbonyl, $C_{1-6}$alkoxycarbonylamino, *N*-($C_{1-6}$alkyl)sulphamoyl, *N,N*-($C_{1-6}$alkyl) sulphamoyl, $C_{1-6}$alkylsulphonylamino and $C_{1-6}$alkylsulphonyl-*N*-($C_{1-6}$alkyl)amino:

n is 0;
$R^2$ is a group -E-F-G-H;
wherein E, F and G are each a direct bond; and
H is a cyclic amide of formula

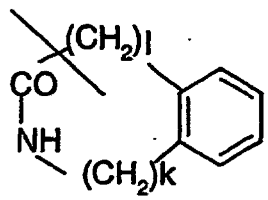

in which k is 0, 1, 2 or 3 and l is 0, 1, 2 or 3 such that the sum of k and l is 2 or 3 and wherein one of the carbon atoms governed by k or l may be replaced by sulphur and wherein H is optionally substituted on carbon by one or more groups selected from S and may be independently optionally substituted on nitrogen by a group selected from T;
S is selected from halo, nitro, cyano, hydroxy, trifluoromethyl, trifluoromethoxy, amino, carboxy, carbamoyl, mercapto, sulphamoyl, ureido, $C_{1-6}$alkyl, $C_{2-6}$alkenyl, $C_{2-6}$ alkynyl, $C_{1-6}$alkoxy, $C_{1-6}$alkanoyl, $C_{1-6}$alkanoyloxy, *N*-($C_{1-6}$alkyl)amino, *N,N*-($C_{1-6}$alkyl) amino, $C_{1-6}$alkanoylamino, *N*-($C_{1-6}$alkyl)carbamoyl, *N, N*-($C_{1-6}$alkyl)$_2$carbamoyl, *N*-($C_{1-6}$alkyl)-*N*-($C_{1-6}$alkoxy)carbamoyl, $C_{1-6}$alkylS(O)$_n$ wherein a is 0 to 2, $C_{1-6}$alkoxycarbonyl, $C_{1-6}$alkoxycarbonylamino, *N*-($C_{1-6}$alkyl)sulphamoyl, *N,N*-($C_{1-6}$alkyl)$_2$sulphamoyl, $C_{1-6}$alkylsulphonylamino, $C_{1-6}$alkylsulphonyl-*N*-($C_{1-6}$alkyl)amino, $C_{3-8}$cycloalkyl, aryl and heterocyclic group; wherein S may be optionally and independently substituted on carbon by one or more groups selected from V and wherein if said heterocyclic group contains an -NH- moiety that nitrogen may be optionally substituted by a group selected from U;
T and U are independently selected from $C_{1-4}$alkyl, $C_{1-4}$alkanoyl, $C_{1-4}$alkylsulphonyl, $C_{1-4}$alkoxycarbonyl, carbamoyl, *N*-($C_{1-4}$alkyl)carbamoyl,*N,N*-($C_{1-4}$alkyl)carbamoyl, phenyl, benzyl, benzyloxycarbonyl, benzoyl and phenylsulphonyl wherein R, T and U may be optionally and independently substituted on carbon by one or more groups selected from V,
V is selected from halo, nitro, cyano, hydroxy, trifluoromethoxy, trifluoromethyl, amino, carboxy, carbamoyl, mercapto, sulphamoyl, methyl, ethyl, methoxy, ethoxy, acetyl, acetoxy, methylamino, ethylamino, dimethylamino, diethylamino, *N*-methyl-*N*-ethylamino, acetylamino, *N*-methylcarbamoyl, *N*-ethylcarbamoyl, *N,N*-dimethylcarbamoyl, *N*,*N*-diethylcarbamoyl, *N*-methyl-*N*-ethylcarbamoyl, methylthio, ethylthio, methylsulphinyl, ethylsulphinyl, mesyl, ethylsulphonyl, methoxycarbonyl, ethoxycarbonyl, *N*-methylsulphamoyl, *N*-ethylsulphamoyl, *N*,*N*-dimethylsulphamoyl, *N*,*N*-diethylsulphamoyl, *N*-methyl-*N*-ethylsulphamoyl, morpholino, morpholinocarbonyl, *N*-benzylcarbamoyl and 4-hydroxypiperidinocarbonyl;

or a pharmaceutically acceptable salt or an *in vivo* hydrolysable ester thereof.

**4.** A compound of formula (**I**) as claimed in claim 1 wherein:

-X-Y-Z- is selected from -S-CR$^4$=CR$^5$ - or-CR$^4$=CR$^5$-S-;
wherein $R^4$ and $R^5$ are independently selected from hydrogen, halo or $C_{1-6}$alkyl.
n is 1;
$R^1$ is hydrogen or aryl$C_{1-6}$alkyl;
$R^2$ is selected from a group -E-F-G-H;
wherein E,F and G are each a direct bond;
H is an unsaturated five membered heterocyclic group containing at least one nitrogen atom and one or two ring atoms selected from oxygen and sulphur and wherein H may be optionally substituted on carbon by one or more groups S which are independently selected from halo, nitro, cyano, hydroxy, trifluoromethyl, trifluoromethoxy, amino, carboxy, carbamoyl, mercapto, sulphamoyl, ureido, $C_{1-6}$alkyl, $C_{2-6}$alkenyl, $C_{2-6}$alkynyl, $C_{1-6}$alkoxy, $C_{1-6}$alkanoyl, $C_{1-6}$alkanoyloxy, *N*-($C_{1-6}$alkyl)amino,*N,N*-($C_{1-6}$alkyl)$_2$amino, $C_{1-6}$alkanoylamino, *N*-($C_{1-6}$alkyl)$_2$carbamoyl,*N,N*-($C_{1-6}$alkyl)$_2$carbamoyl, *N*-($C_{1-6}$alkyl)-*N*-($C_{1-6}$alkoxy)carbamoyl, $C_{1-6}$alkylS(O)$_a$ wherein a is 0 to 2, $C_{1-6}$alkoxycarbonyl, $C_{1-6}$alkoxycarbonylamino,*N*-($C_{1-6}$alkyl)sulphamoyl, *N,N*-($C_{1-6}$ alkyl)$_2$sulphamoyl, $C_{1-6}$alkylsulphonylamino, $C_{1-6}$alkylsulphonyl-*N*-($C_{1-6}$alkyl)amino, $C_{3-8}$cycloalkyl and aryl

groups;
$R^3$ is hydrogen or $C_{1-6}$alkyl;

or a pharmaceutically acceptable salt thereof.

5. A compound of formula (**I**) as claimed in claim 1 wherein:

-X-Y-Z- is selected from -S-$CR^4$-$CR^5$- or -$CR^4$=$CR^5$-S-;
wherein $R^4$ and $R^5$ are independently selected from hydrogen, halo or $C_{1-6}$alkyl.
n is 0;
$R^2$ is a group -E-F-G-H;
wherein E is a direct bond;
F is methylene;
wherein G is -C(O)$NR^a$-, wherein $R^a$ is selected from hydrogen or $C_{1-6}$alkyl which is optionally substituted by a group V ;
H is aryl which may be optionally substituted on carbon by one or more groups selected from S;
S is selected from halo, nitro, cyano, hydroxy, trifluoromethyl, trifluoromethoxy, amino, carboxy, carbamoyl, mercapto, sulphamoyl, ureido, $C_{1-6}$alkyl, $C_{2-6}$alkenyl, $C_{2-6}$alkynyl, $C_{1-6}$alkoxy, $C_{1-6}$alkanoyl, $C_{1-6}$alkanoyloxy, *N*-($C_{1-6}$alkyl)amino,*N,N*-($C_{1-6}$alkyl)$_2$amino, $C_{1-6}$alkanoylamino; *N*-($C_{1-6}$alkyl)carbamoyl, *N*,*N*-($C_{1-6}$ alkyl)$_2$carbamoyl, *N*-($C_{1-6}$alkyl)-*N*-($C_{1-6}$alkoxy)carbamoyl, $C_{1-6}$alkylS(O)$_a$ wherein a is 0 to 2, $C_{1-6}$alkoxycarbonyl, $C_{1-6}$alkoxycarbonylamino, *N*-($C_{1-6}$alkyl)sulphamoyl, *N*,*N*-($C_{1-6}$alkyl)$_2$sulphamoyl, $C_{1-6}$alkylsulphonylamino, $C_{1-6}$alkylsulphonyl-*N*-($C_{1-6}$alkyl)amino, $C_{3-8}$cycloalkyl, aryl and heterocyclic group; wherein S may be optionally and independently substituted on carbon by one or more groups selected from V ;
V is selected from halo, nitro, cyano, hydroxy, trifluoromethoxy, trifluoromethyl, amino, carboxy, carbamoyl, mercapto, sulphamoyl, methyl, ethyl, methoxy, ethoxy, acetyl, acetoxy, methylamino, ethylamino, dimethylamino, diethylamino, *N*-methyl-*N*-ethylamino, acetylamino, *N*-methylcarbamoyl, *N*-ethylcarbamoyl, *N,N* dimethylcarbamoyl, *N,N*-diethylcarbamoyl, *N*-methyl-*N*-ethylcarbamoyl, methylthio, ethylthio, methylsulphinyl, ethylsulphinyl, mesyl, ethylsulphonyl, methoxycarbonyl, ethoxycarbonyl, *N*-methylsulphamoyl, *N*-ethylsulphamoyl, *N*,*N*-dimethylsulphamoyl, *N,N*-diethylsulphamoyl, *N*-methyl-*N*-ethylsulphamoyl, morpholino, morpholinocarbonyl, *N*-benzylcarbamoyl, and 4-hydroxypiperidinocarbonyl;

or a pharmaceutically acceptable salt thereof.

6. A compound selected from
2,3-dichloro-5-[*N* -(2-phenoxyethyl)carbamoyl]-4*H*-thieno[3,2-*b*]pyrrole;
2,3-dichloro-5-{*N*-(2-thienyl)ethyl]carbamoyl}-4*H*-thieno [5,2-*b*]pyrrole;
2,3-dichloro-5-{*N* [2-(2-methoxyphenyl)ethyl]carbamoyl]-4*H*-thieno[3,2-*b*]pyrrole;
2,3-dichloro-5-[*N*-(2-phenyl-1-cyclopropyl)carbamoyl]-4*H*-thieno[3,2-*b*]pyrrole;
2,3-dichloro-5-{*N*-[2-(4-fluorophenyl)ethyl]carbamoyl}-4*H*-thieno[3,2-*b*]pyrrole;
2,3-dichloro-5-[*N*-(*N*-phenylcarbamoylmethyl)carbamoyl]-4*H*-thieno[3,2-*b*]pyrrole;
2,3-dichloro-5-(*N*-{2-[(2-pyridyl)amino]ethyl}carbamoyl)-4*H*-thieno[3,2-*b*]pyrrole;
2,3-dichloro-5-{*N*-[2-(*N*-methylmethanesulphonamido)-1-(thiazol-2-yl)ethyl]carbamoyl}-4*H* -thieno[3,2-*b*]pyrrole;
2,3-dichloro-5-{*N*-[2-(thiomorpholino)ethyl]carbamoyl}-4*H*-thieno[3,2-*b*]pyrrole;
5-[*N*-(benzoylmethyl)carbamoyl]-2,3-dichloro-4*H*-thieno[3,2-*b*]pyrrole;
3-chloro-5-[*N*-(*N*-phenylcarbamoylmethyl)carbamoyl]-4*H*-thieno[3,2-*b*]pyrrole;
3-chloro-5-{*N*-[2-(thiomorpholino)ethyl)carbamoyl}-4*H*-thieno[3,2-*b*]pyrrole;
3-chloro-5-{*N*-[2-(*N*-methylmethanesulphonamido)-1-(thiazol-2-yl)ethyl]carbamoyl}-4*H* -thieno[3,2-*b*]pyrrole;
3-chloro-5-[*N*-(benzoylmethyl)carbamoyl]-4*H*-thieno[3,2-*b*]pyrrole;
3-chloro-5-{*N*-[2-(2-methoxyphenyl)ethyl]carbamoyl)-4*H*-thieno[3,2-*b*]pyrrole;
3-chloro-5-{*N*-[2-(2-thienyl)ethyl]carbamoyl}-4*H*-thieno[3,2-*b*]pyrrole;
3-chloro-5-[*N*-(2-phenyl-1-cyclopropyl)carbamoyl]-4*H*-thieno[3,2-*b*]pyrrole;
3-chloro-5-{*N*-[2-(4-fluorophenyl)ethyl]carbamoyl}-4*H*-thieno[3,2-*b*]pyrrole;
3-chloro-5-[*N*-(2-phenoxyethyl)carbamoyl]-4*H*-thieno[3,2-*b*]pyrole;
3-chloro-5-{*N*-[2-(1-phenylmethanesulphonamido)ethyl]carbamoyl}-4*H*-thieno[3,2-*b*]pyrrole;
3-chloro-5-[*N*-(4-oxo-2,3,4,5-tetrahydrobenz[1,5]thiazepin-3-yl)carbamoyl]-4H-thieno[3,2-*b*]pyrrole;
2-chloro-5-[*N*-(benzoylmethyl)carbamoyl]-4*H*-thieno[3,2-*b*]pyrrole;
2-chloro-5-[*N*-(2-phenyl-1-cyclopropyl)carbamoyl]-4*H*-thieno[3,2-*b*]pyrrole;
2-chloro-5-[*N*-(*N*-phenylcarbamoylmethyl)carbamoyl]-4*H*-thieno[3,2-*b*]pyrrole;

2-chloro-5-(*N*-{2-[(2-pyridyl)amino]ethyl}carbamoyl)-4*H*-thieno[3,2-*b*]pyrrole;
2-chloro-5-{*N*-[2-(2-methoxyphenyl)ethyl]carbamoyl}-4*H*-thieno[3,2-*b*]pyrrole;
2-chloro-5-[*N*-(2-phenoxyethyl)carbamoyl]-4*H*-thieno[3,2-*b*]pyrrole;
2-chloro-5-{*N*-[2-(2-thienyl)ethyl]carbamoyl}-4*H*-thieno[3,2-*b*]pyrrole;
2-chloro-5-{*N*-[2-(4-fluorophenyl)ethyl]carbamoyl}-4*H*-thieno[3,2-*b*]pyrrole;
2-chloro-5-{*N*-[2-(*N*-methylmethanesulphonamido)-1-(thiazol-2-yl)ethyl]carbamoyl}-4*H*-thieno[3,2-*b*]Pyrrole;
2-chloro-5-{*N*-[2-(thiomorpholino)ethyl]carbamoyl]-4*H*-thieno[3,2*b*]pyrrole,
2,3-dichloro-5-[*N*-(2,3-dimethyl-5-oxo-1-phenyl-2,5-dihydro-1*H* pyrazol-4-yl)carbamoyl]-4*H*-thieno[3,2-*b*]pyrrole;
2,3-dichloro-5-[*N*-(4-sulphamoylphenylmethyl)carbamoyl]-4*H*-thieno[3,2-*b*]pyrrole;
2,3-dichloro-5-[*N*-(2-hydroxy-1-phenethyl)carbamoyl]-4*H*-thieno[3,2-*b*]pyrrole;
2,3-dichloro-5-{*N*-(2-[(3-trifluoromethylpyrid-2-yl)amino]ethyl)carbamoyl}-4*H*-thieno[3,2-*b*]pyrrole;
2,3-dichloro-5-{*N*-[3-(5-tetrazolyl)propyl]carbamoyl}-4*H*-thieno[3,2-*b*]pyrrole;
2,3-dichloro-5-[*N*-(5-oxo-3-phenyl-4,5-dihydroisoxazol-4-yl)carbamoyl]-4*H*-thieno[3,2-*b*]pyrrole;
2,3-dichloro-5-[*N*-(5-hydroxy-2-oxo-2,3,4,5-tetrahydro-1H-benz[*b*]azepin-4-yl)carbamoyl]-4*H*-thieno[3,2-*b*]pyrrole;
2-chloro-5-{*N*-[3-(benzyloxycarbonylamino)propyl]carbamoyl}-4*H*-thieno[3,2-*b*]pyrrole;
2,3-dichloro-5-{*N*-[(4-dimethylaminophenyl)methyl]carbamoyl}-4*H*-thieno[3,2-*b*]pyrrole;
5-[*N*-(1-benzyl-2-hydroxyethyl)carbamoyl]-2,3-dichloro-4*H* thieno[3,2-*b*]pyrrole;
2,3-dichloro-5-{*N*-[2-(phenylamino)ethyl]carbamoyl}-4*H*-thieno[3,2-*b*]pyrrole;
2,3-dichloro-5-[*N*-β-*(R)*-hydroxy-α-methylphenethyl)carbamoyl]-4*H* thieno[3,2-*b*]pyrrole;
2,3-dichloro-5-[*N*-(β-hydroxyphenethyl)carbamoyl}-4*H*-thieno[3,2-*b*]pyrrole;
2,3-dichloro-5-{*N*-[2-(4-hydroxyphenyl)ethyl]carbamoyl}4*H*-thieno[3,2-*b*]pyrrole;
2,3-dichloro-5-{*N*-[(benzimidazol-2-yl)methyl]carbamoyl}-4*H*-thieno[3,2-*b*]pyirole;
2,3-dichloro-5-{*N*-[2-(4-chlorophenyl)-2-hydroxy-1-(methoxycarbonyl)ethyl]carbamoyl}-4*H* -thieno[3,2-*b*]pyrrole;
2,3-dichloro-5-(*N*-(imidazo[1,2-*a*]pyrid-2-yl)carbamoyl}-4*H*-thieno[3,2-*b*]pyrrole;
5-{*N*-[(benzthiazol-2-yl)methyl]carbamoyl}-2,3-dichloro-4*H*-thieno[3,2-*b*]pyrrole;
2,3-dichloro-5-{*N*-[(6-trifluoromethylpyrid-3-yl)methyl]carbamoyl}-4*H*-thieno[3,2-*b*]pyrrole;
2,3-dichloro-5-{*N*-(2-[(2-pyridazinyl)methyl]carbamoyl}-4*H*-thieno[3,2-*b*]pyrrole;
2,3-dichloro-5-{*N*-[*N*-(2-hydroxy-3-phenoxypropyl)carbamoylmethyl] carbamoyl}-4*H*-thieno[3,2-*b*]pyrrole;
2,3-dichloro-5-{*N*-[*N*-(3-methylisothiazol-5-yl)carbamoylmethyl]carbamoyl}-4*H*-thieno[3,2-*b*]pyrrole;
2,3-dichloro-5-{*N*-[2-(pyridazin-3-yloxy)ethyl]carbamoyl}-4H-thieno[3,2-*b*]pyrrole;
2-chloro-5-(*N*-{2-[(3-triffuoromethylpyrid-2-yl)amino]ethyl}carbamoyl)-4*H*-thieno[3,2-*b*]pyrrole;
2,3-dichloro-5-{*N*-[2-(4-sulphamoylphenyl)ethyl]carbamoyl}-4*H*-thieno[3,2-*b*]pyrrole,
2,3-dichloro-5-{*N*-[2-(2-pyridyl)ethyl]carbamoyl}-4*H*-thieno[3,2-*b*]pyrrole;
2,3-dichloro-5-{*N*-(2-[1-hydroxymethyl-2-(4-imidazolyl)ethyl]carbamoyl}-4*H*-thieno[3,2-*b*]pyrrole;
2,3-dichloro-5-{*N*-(2-[(3-quinolyl)methyl]carbamoyl}-4*H*-thieno[3,2-*b*]pyrrole;
5-{*N*-[3-(4-acetamidophenoxy)-2-hydroxypropyl]carbamoyl)-2,3-dichloro-4*H*-thieno[3,2-*b*]pyrrole;
2,3-dichloro-5-{*N*-[3-(*N*-methylsulphonylcarbamoyl)propyl]carbamoyl}-4*H* thieno[3,2-*b*]pyrrole;
2,3-dichloro-5-[*N*-(2-{[2-(guanidino)thiazol-4-yl]methylthio}ethyl)carbamoyl]-4*H*-thieno[3,2-*b*]pyrrole;
2,3-dichlom-5-{*N*-[2-(2,4-dioxoimidazolidin-1-yl)ethyl]carbamoyl}-4*H*-thieno[3,2-*b*]pyrrole;
5-{*N* [2-benzylthio-1-(hydroxymethyl)ethyl]carbamoyl}-2,3-dichloro-4*H*-thieno[3,2-*b*]pyrrole;
2,3-dichloro-5-{*N-[2-(*dimethyaminosulphonylamino)ethyl]carbamoyl}-4*H*-thieno[3,2-*b*]pyrrole;
2,3-dichloro-5-{*N*-[ (6-methoxypyrid-3-yl)methyl]carbamoyl}-4*H*-thieno[3*,2-b* ]pyrrole;
(*S*)-2,3-dichloro-5-{*N*-[(2-oxo-3-phenyl-2,3,4,5-tetrahydrooxazol-5-yl)methyl]carbamoyl}-4*H*-thieno[3,2-*b*]pyrrole;
2,3-dichloro-5-(*N*-{2-[3-(carbamoylmethyl)phenoxy]ethyl}carbamoyl)-4*H*-thieno[3,2-*b*]pyrrole;
5-(*N*-{[6-(benzo[1,3]dioxol-5-yl)-4-methylmorpholin-2-yl]methyl}carbamoyl)-2,3-dichloro-4*H*-thieno[3,2-*b*]pyrrole;
*5-(N* benzylcarbamoyl)-2,3-dichloro-4*H* thieno(3,2-*b*]pyrrole;
2,3-dichloro-5-(*N*-phenethylcarbamoyl)-4*H*-thieno[3,2-*b*]pyrrole;
2,3-dichloro-5-[*N*-(3-phenylpropyl)carbanoyl)-4*H* thieno(3,2-*b*]pyrrole;
2,3-dichloro-5-{*N*-[2-(2-hydroxyphenyl)ethyl]carbamoyl}-4*H*-thieno[3,2-*b*]pyrrole;
2,3-dichloro-5-[*N* (α,α-dimethylphenethyl)carbamoyl]-4*H*-thieno[3,2-*b*]pyrrole;
2,3-dichloro-5-[*N*-(1-phenylcyclobutyl)methyl)carbamoyl]-4*H*-thieno[3,2-*b*]pyrrole;
2,3-dichloro-5-[*N*-(β-methylphenethyl)carbamoyl]-4*H*-thieno[3,2-*b*]pyrrole;
2,3-dichloro-5-[*N*-(1,2,3,4-tetrahydronaphth-2-yl)carbamoyl]-4*H*-thieno[3,2-*b*]pyrrole;
5-[*N*-(*N*-benzylcarbamoylmethyl)carbomoyl]-2,3-dichloro-4*H*-thieno[3,2-*b*]Pyrrole;
5-[*N*-(*N*-benzyl-*N*-methylcarbamoylmethyl)carbamoyl]-2,3-dichloro-4*H*-thieno[3,2-*b*]pyrrole;
2,3-dichloro-5-[*N*-(*N*-methyl-*N*-phenylcarbamoylmethyl)carbamoyl]-4*H*-thieno[3,2-*b*]pyrrole;
2,3-dichloro-5-{*N*-[*N*-(2-cyanoethyl)-*N*-phenylcarbamoylmethyl]carbamoyl}-4*H*-thieno[3,2-*b*]pyrrole;

2,3-dichloro-5-{*N*-[*N*-(4-methoxyphenyl)carbamoylmethyl]carbamoyl}-4*H*-thieno[3,2-*b*]pyrrole;
2,3-dichloro-5-{*N*-[*N*-(4-fluorophenyl)carbamoylmethyl]carbamoyl}-4*H*-thieno[3,2-*b*]pyrrole;
2,3-dichloro-5-{*N*-[*N*-(4-nitrophenyl)carbamoylmethyl]carbamoyl}-4*H*-thieno[3,2-*b*]pyrrole;
2,3-dichloro-5-{*N*-[*N*-(2,6-dimethylphenyl)carbamoylmethyl]carbamoyl}-4*H*-thieno[3,2-*b*]pyrrole;
2,3-dichloro-5-{*N*-[*N*-methyl-*N*-(4-methylphenyl)carbamoylmethyl]carbamoyl}-4*H*-thieno[3,2-*b*]pyrrole;
2,3-dichloro-5-{*N*-[*N*-methyl-*N*-(3-methylphenyl)carbamoylmethyl]carbamoyl}-4*H* -thieno[3,2-*b*]pyrrole;
2,3-dichloro-5-{*N*-[*N*-(3-chlorophenyl) *N*-methylcarbamoylmethyl]carbamoyl}-4*H* thieno [3,2-*b*]pyrrole;
2,3-dichloro-5-{*N*-[*N*-(2-hydroxyethyl)-*N*-phenylcarbamoylmethyl]carbamoyl}-4*H* -thieno[3,2-*b*]pyrrole;
2,3-dichloro-5-{*N* [*N*-(1,1-dimethyl-2-hydroxyethyl)carbamoylmethyl]carbamoyl}-4*H* -thieno[3,2-*b*]pyrrole;
2,3-dichloro-5-{*N*-[*N*-(2-hydroxyethyl)-*N*-methylcarbamoylmethyl]carbamoyl}-4*H*-thieno[3,2-*b*]pyrrole;
2,3-dichloro-5-{*N*-[*N*-(2-hydroxyethyl)carbamoylmethyl]carbamoyl}-4*H* thieno[3,2-*b*]pyrrole;
2,3-dichloro-5-{*N*-[*N*-(3-hydroxypropyl)carbamoylmethyl]carbamoyl}-4*H*-thieno[3,2-*b*]pyrrole;
2,3-dichloro-5-{*N*-[*N*-(4-hydroxybutyl)carbamoylmethyl]carbamoyl}-4*H*-thieno[3,2-*b*]pyrrole;
2,3-dichloro-5-(*N*-{*N*-[bis(hydroxymethyl)methyl]carbamoylmethyl}carbamoyl)-4*H*-thieno[3,2-*b*]pyrrole;
2,3-dichloro-5-{*N*-[*N*-(2,3-dihydroxypropyl)carbamoylmethyl]carbamoyl}-4*H*-thieno[3,2-*b*]pyrrole,
2,3-dichloro-5-{*N*-[*N*-(4-hydroxymethylphenyl)carbamoylmethyl]carbamoyl}-4*H*-thieno[3,2-*b*]pyrrole;
2,3-dichloro-5-{*N*-[*N*-(5-isoquinolyl)carbamoylmethyl]carbamoyl}-4*H* thieno[3,2-*b*]pyrrole;
2,3-dichloro-5-{*N*-[*N*-(3-hydroxymethyl)phenyl)carbamoylmethyl]carbamoyl)-4*H*-thieno[3,2-*b*]pyrrole
2,3-dichloro-5-*(N*-{*N*-[4-(2-hydroxyethyl)phenyl]carbamoylmethyl}carbamoyl)-4*H*-thieno[3,2-*b*] pyrrole;
2,3-dichloro-5-{*N*-[*N*-(2,4-difluorophenyl) *N*-methyl-carbamoylmethyl]carbamoyl}-4*H* -thieno[3,2-*b*]pyrrole;
2,3-dichloro-5-{*N*-[(1,2,3,4-tetrahydro-1-quinolyl)carbonylmethyl]carbamoyl}-4*H*-thieno[3,2-*b*]pyrrole;
2,3-dichloro-5-{*N*-[*N*-(2-cyanoethyl)-*N*-methylcarbamoylmethyl]carbamoyl}-4*H*-thieno[3,2-*b*]pyrrole;
2,3-dichloro-5-{*N*-*[N*-{4-hydroxypiperidino)carbamoylmethyl]carbamoyl}-4*H*-thieno[3,2-*b*]pyrrole;
2,3-dichloro-5-[*N*-(*N*-cyclopentylcarbamoylmethyl)carbamoyl]-4*H*-thieno[3,2-*b*]pyrrole;
2,3-dichloro-5-[*N*-(*N*-isopropylcarbamoylmethyl)carbamoyl]-4*H*-thieno[3,2-*b*]pyrrole;
2,3-dichloro-5-[*N*-(*N*-isopropyl-*N*-methylcarbamoylmethyl)carbamoyl]-4*H*-thieno[3,2-*b*]pyrrole;
2,3-dichloro-5-[*N*-(thiomorpholinocarbonylmethyl)carbamoyl]-4*H*-thieno[3,2-*b*]pyrrole;
2,3-dichloro-5-[*N*-(morpholinocarbonylmethyl)carbamoyl]-4*H*-thieno[3,2-*b*]pyrrole;
2,3-dichloro-5-{*N*-[(1,1-dioxothiomorpholino)carbonylmethyl]carbamoyl}-4*H*-thieno[3,2*b*]-pyrrole;
2,3-dichloro-5-{*N*-[(1-oxothiamorpholino)carbonylmethyl]carbamoyl}-4*H*-thieno[3,2-*b*]pyrrole;
2-chloro-5-[*N*-(2-indanyl)carbamoyl]-6*H*-thieno[2,3-*b*]pyrrole; 5-[*N*-(benz[1,2]oxazol-3-ylmethyl)carbamoyl]-2,3-dichloro-4*H*-thieno[3,2-*b*]pyrrole;
2,3-dichloro-5-(*N*-{2-[2-(hydroxymethyl)phenyl]ethyl}carbamoyl)-4*H*-thieno[3,2-*b*]pyrrole;
2,3-dichloro-5-[*N*-(4-phenylisoxazol-3-ylmethyl)carbamoyl]-4*H*-thieno[3,2-*b*]pyrrole;
2,3-dichloro-5-(*N*-{2-[2-(2-morpholinoethoxy)phenyl)ethyl}carbamoyl)-4*H*-thieno[3,2-*b*]pyrrole;
2,3-dichloro-5-(*N*-{2-[2-(methoxycarbonylmethoxy)phenyl]ethyl}carbamoyl)-4*H*-thieno[3,2-*b*]pyrrole;
5-(*N*-{2-[2-(carboxymethoxy)phenyl]ethyl}carbamoyl)-2,3-dichloro-4*H*-thieno[3,2-*b*]pyrrole;
2,3-dichloro-5-{*N*-[2-(3-methoxyphenyl)ethyl]carbamoyl}-4*H*-thieno[3,2-*b*]pyrrole;
2,3 dichloro-5-(*N*-(2-oxo-1,2,3,4-tetrahydroquinol-3-yl)carbamoyl]-4*H*-thieno[3,2-*b*]pyrrole;
2,3-dichloro-5-(*N*-{2-[2-(2-methoxyethoxy)phenyl]ethyl}carbamoyl)-4*H*-thieno[3,2-*b*]pyrrole;
5-(*N*-{2-[2-(carbamoylmethoxy)phenyl]ethyl}carbamoyl)-2,3-dichloro-4*H*-thieno[3,2*b*]pyrrole;
2,3-dichloro-5-(*N*-{2-[2-(*N*-methylcarbamoylmethoxy)phenyl]ethyl)carbamoyl)-4*H* -thieno[3,2-*b*]pyrrole;
2,3-dichloro-5-(*N*-(2-[2-(*N,N*-dimethylcarbamoylmethoxy)phenyl]ethyl)carbamoyl)-4*H* -thieno[3,2-*b*]pyrrole;
2,3-dichloro-5-(*N*-[2-[2-(morpholinocarbonylmethoxy)phenyl]ethyl)carbamoyl)-4*H* -thieno[3,2-*b*]pyrrole;
*5*-(*N*-{2-[2-*(N*-benzylcarbamoylamethoxy)phenyl]ethyl}carbamoyl)2,3-dichloro-4*H* -thieno[3,2-*b*]pyrrole;
2,3-dichloro-5-(*N*-{2-[2-(4-hydroxypiperidinocarbonylmethoxy)phenyl]ethyl}carbamoyl)-4*H* -thieno[3,2-*b*]pyrrole;
(*S*)-2-chloro-5-{*N*-[α-(5-ethoxycarbonyl-1,3,4-oxadiazol-2-yl)phenethyl]carbamoyl}-6*H* -thieno[2,3-*b*]pyrrole;
(S)-2-chloro-5-{*N*-[α-(4-methoxycarbonyloxazol-5-yl)phenethyl]carbamoyl)-6*H*-thieno[2,3-*b*]pyrrole;
2-chloro-5-{*N*-(α-(3-pyridyl)phenethyl]carbamoyl}-6*H*-thieno[2,3-*b*]pyrrole;
2,3-dichloro-5-{*N*-(α-{3-pyridyl)phenethyl)]carbamoyl}-4*H*-thieno[3,2-*b*]pyrrole;
(*S*)-2-chloro-5-{*N*-[α-(3-phenyl-1,2,4-oxadiazol-5-yl)phenethyl]carbamoyl}-6*H*-thieno[2,3-*b*]pyrrole;
2,3-dichloro-5-[*N*-(1-hydroxyindan-2-yl)carbamoyl]-4*H*-thieno[3,2-*b*]pyrrole;
2,3-dichloro-5-[*N*-((*1S,2S*)-1-hydroxyindan-2-yl)carbamoyl]-4*H*-thieno[3,2-*b*]pyrrole;
2,3-dichloro-5-[*N*-((*1R,2R*)-1-hydroxyindan-2-yl)carbamoyl]-4*H*-thieno[3,2-*b*]pyrrole;
2-chloro-5-[*N*-(1-hydroxyindan-2-yl)carbamoyl]-6*H*-thieno[2,3-*b*]pyrrole;
2,3-dichloro-5-[*N*-(1-hydroxy-1,2,3,4-tetrahydronaphth-2-yl)carbamoyl]-4*H*-thieno[3,2-*b*]pyrrole;
2,3-dichloro-5-[*N*-(6-fluoro-1-hydroxyindan-2-yl)carbamoyl]-4*H*-thieno[3,2-*b*]pyrrole;
2,3-dichloro-5-[*N*-(7-methoxy-1-oxo-1,2,3,4-tetrahydronaphth-2-yl)carbamoyl)-4*H*

thieno[3,2-*b*]pyrrole;
2,3-dichloro-5-[*N*-(2-indanyl)carbamoyl]-4*H*-thieno[3,2-*b*]pyrrole;
2,3-dichloro-5-[*N*-(3-methylisoxazol-5-yl)methyl]carbamoyl]-4H thieno[3,2-*b*]pyrrole;
2,3-dichloro-5-[*N*-(4-hydroxy-1,1-dioxotetrahydrothiophen-3-yl)carbamoyl]-4*H*-thieno[3,2-*b*]pyrrole;
2,3-dichloro-5-(*N*-{*N*-methyl-*N*[(1-oxo-1,2,3,4-tetrahydronaphth-2-yl)methyl]carbamoylmethyl}carbamoyl)-4*H*-thieno[3,2-*b*]pyrrole;
2-chloro-5-[*N*-(2-oxo-1,2,3,4-tetrahydroquinol-3-yl)carbamoyl]-6H-thieno[2,3-*b*]pyrrole;
2-chloro-5-[*N*-(1,2,3,4-tetrahydroquinol-3-yl)carbamoyl]-6*H*-thieno[2,3-*b*]pyrrole;
2-chloro-5-[*N*-(1-methyl-2-oxo-1,2,3,4-tetrahydroquinol-3-yl)carbamoyl]-6*H*-thieno[2,3-*b*]pyrrole;
2-chloro-5-[*N*-(3-oxo-2,3,4,5-tetrahydro-1*H*-benz[2]azepin-4-yl)carbamoyl]-6*H* thieno[2,3-*b*]pyrrole;
2,3-dichloro-5-[*N*-(1-methoxyindan-2-yl)carbamoyl]-4*H* thieno[3,2-*b*]pyrrole;
2,3-dichloro-5-(*N*-{1-[(*N*-(1,1-dimethylethoxy)carbonylamino]indan-2-yl}carbamoyl)-4*H* -thieno[3,2-*b*]pyrrole;
5-[*N*-(1-aminoindan-2-yl)carbamoyl]-2,3-dichloro-4*H*-thieno[3,2-*b*]pyrrole;
5-[*N*-(1-acetamidoindan-2-yl)carbamoyl]-2,3-dichloro-4*H* thieno[3,2-*b*]pyrrole;
2,3-dichloro-5-{*N*-[1-(methanesulphonamido)indan-2-yl]carbamoyl}-4*H*-thieno[3,2-*b*]pyrrole;
2,3-dichloro-5-{*N*-[1-(methylamino)indan-2-yl]carbamoyl}-4*H*-thieno[3,2-*b*]pyrrole; and
2,3-dichloro-5-{*N*-[1-(*N*-methylacetamido)indan-2-yl]carbamoyl}-4*H*-thieno[3,2-*b*]pyrrole or a pharmaceutically acceptable salt thereof.

**7.** A pharmaceutical composition which comprises a compound of the formula (I) as claimed in any one of claims 1-6, or a pharmaceutically acceptable salt or *in vivo* hydrolysable ester thereof, as defined hereinbefore in association with a pharmaceutically-acceptable diluent or carrier.

**8.** A compound of the formula as claimed in any one of claims 1 to 6 or a pharmaceutically acceptable salt or *in vivo* hydrolysable ester thereof as a medicament.

**9.** The use of a compound of the formula as claimed in any one of claims 1 to 6 or a pharmaceutically acceptable salt or *in vivo* hydrolysable ester thereof, as defined hereinbefore in the manufacture of a medicament for use in the production of a glycogen phosphorylase inhibitory effect in a warm-blooded animal such as man.

**Patentansprüche**

**1.** Verbindungen der Formel (I):

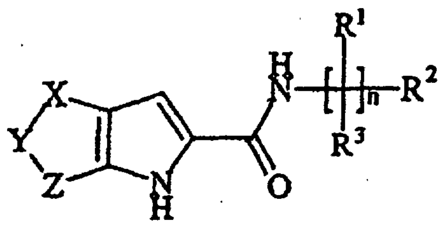

(I)

wobei:

**- X-Y-Z-** aus -S-$CR^4$=$CR^5$-, -$CR^4$=$CR^5$-S-, -O-$CR^4$=$CR^5$-, -$CR^4$=$CR^5$-O-, -N=$CR^4$-S-, -S-$CR^4$=N-, -$NR^6$-$CR^4$=$CR^5$- und -$CR^4$=$CR^5$-$NR^6$- ausgewählt ist;
wobei $\mathbf{R^4}$ und $\mathbf{R^5}$ unabhängig voneinander aus Wasserstoff, Halogen, Nitro, Cyano, Hydroxy, Fluormethyl, Difluormethyl, Trifluormethyl, Trifluormethoxy, Amino, Carboxy, Carbamoyl, Mercapto, Sulfamoyl, Ureido, $C_{1-6}$-Alkyl, $C_{2-6}$-Alkenyl, $C_{2-6}$-Alkinyl, $C_{1-6}$-Alkoxy, $C_{1-6}$-Alkanoyl, $C_{1-6}$-Alkanoyloxy, *N*-($C_{1-6}$-Alkyl)amino, *N,N*-($C_{1-6}$-Alkyl)$_2$amino, . $C_{1-6}$-Alkanoylamino, *N*-($C_{1-6}$-Alkyl)carbamoyl, *N,N*-($C_{1-6}$-Alkyl)$_2$carbamoyl, $C_{1-6}$-Alkyl-S(O)$_a$, wobei a für 0 bis 2 steht, $C_{1-6}$-Alkoxycarbonyl, $C_{1-6}$-Alkoxycarbonylamino, N-($C_{1-6}$-Alkyl) sulfamoyl, N,N-($C_{1-6}$-Alkyl)$_2$Sulfamoyl, $C_{1-6}$-Alkylsulfonylamino und $C_{1-6}$-Alkylsulfonyl-N-($C_{1-6}$-Alkyl)amino ausgewählt sind;
$\mathbf{R^6}$ für Wasserstoff oder $C_{1-6}$-Alkyl steht;
$\mathbf{R^1}$ aus Wasserstoff, Halogen, Nitro, Cyano, Hydroxy, Amino, Carboxy, Carbamoyl, Mercapto, Sulfamoyl, Urei-

do, $C_{1-6}$-Alkyl, $C_{2-6}$-Alkenyl, $C_{2-6}$-Alkinyl, $C_{1-6}$-Alkoxy, $C_{1-6}$-Alkanoyl, $C_{1-6}$-Alkanoyloxy, *N*-($C_{1-6}$-Alkyl) amino, N, N-($C_{1-6}$-Alkyl)$_2$amino, $C_{1-6}$-Alkanoylamino, *N*-($C_{1-6}$-Alkyl)carbamoyl, *N,N*-($C_{1-4}$-Alkyl)$_2$carbamoyl, $C_{1-6}$-Alkyl-S(O)$_a$, wobei a für 0 bis 2 steht, $C_{1-6}$-Alkoxycarbonyl, $C_{1-6}$-Alkoxycarbonylamino, *N*-($C_{1-6}$-Alkyl)sulfamoyl, N,N-($C_{1-6}$-Alkyl)$_2$sulfamoyl, $C_{1-6}$-Alkylsulfonylamino, $C_{1-6}$-Alkylsulfonyl-*N*-($C_{1-6}$-Alkyl)amino, $C_{3-8}$-Cycloalkyl, $C_{3-8}$-Cycloalkyl-$C_{1-6}$-Alkyl, Aryl, Aryl-$C_{1-6}$-Alkyl, heterocyclische Gruppe und (heterocyclische Gruppe)-$C_{1-6}$-Alkyl ausgewählt ist; wobei $R_1$ gegebenenfalls an Kohlenstoff durch eine oder mehrere aus P ausgewählte Gruppen substituiert sein kann und wobei, wenn die heterocyclische Gruppe eine -NH-Einheit enthält, dieser Stickstoff gegebenenfalls durch eine aus R ausgewählte Gruppe substituiert sein kann;

**$R^2$** aus Wasserstoff, Halogen, Nitro, Cyano, Hydroxy, Fluormethyl, Difluormethyl, Trifluormethyl, Trifluormethoxy, Amino, Carboxy, Carbamoyl, Mercapto, Sulfamoyl, Ureido, $C_{1-6}$-Alkyl, $C_{2-6}$-Alkenyl, $C_{2-6}$-Alkinyl, $C_{1-6}$-Alkoxy, $C_{1-6}$-Alkanoyl, $C_{1-6}$-Alkanoyloxy, *N*-($C_{1-6}$-Alkyl) amino, *N, N*-($C_{1-6}$-Alkyl)$_2$amino, $C_{1-6}$-Alkanoylamino, *N*-($C_{1-6}$-Alkyl) carbamoyl, *N,N*-($C_{1-4}$-Alkyl)$_2$carbamoyl, *N*-($C_{1-6}$-Alkyl) -*N*-($C_{1-6}$-Alkoxy) carbamoyl, $C_{1-6}$-Alkyl-S(O)$_a$, wobei a für 0 bis 2 steht, $C_{1-6}$-Alkoxycarbonyl, $C_{1-6}$-Alkoxycarbonylamino, *N*-($C_{1-6}$-Alkyl)sulfamoyl, *N, N*-($C_{1-6}$-Alkyl)$_2$sulfamoyl, Sulfamoylamino, *N*-($C_{1-6}$-Alkyl) sulfamoylamino, *N,N*-($C_{1-6}$-Alkyl)$_2$sulfamoylamino, $C_{1-6}$-Alkylsulfonylamino, $C_{1-6}$-Alkylsulfonylaminocarbonyl, $C_{1-6}$-Alkylsulfonyl-*N*-($C_{1-6}$-Alkyl)amino und einer Gruppe -E-F-G-H ausgewählt ist;

wobei **E** und **G** unabhängig voneinander aus einer direkten Bindung, -O-, -S-, -SO-, -$SO_2$-, -OC(O)-, -C(O)O-, -C (O)-, -$NR^a$-, -$NR^a$C (O) -, -C (O) $NR^a$-, -$NR^aSO_2$-, -$NR^a$C(O)$NR^b$-, -OC(O)$NR^a$-, -$NR^a$C(O)O-, -$NR^aSO_2NR^b$-, -$SO_2NR^a$C(O)- und -C(O)$NR^aSO_2$- ausgewählt sind; wobei $R^a$ und $R^b$ unabhängig voneinander aus Wasserstoff oder $C_{1-6}$-Alkyl, das gegebenenfalls durch eine Gruppe V substituiert ist, ausgewählt sind;

**F** für $C_{1-6}$-Alkylen, gegebenenfalls substituiert durch ein oder mehrere Q, oder eine direkte Bindung steht;

**H** aus Aryl, $C_{3-8}$-Cycloalkyl und heterocyclische Gruppe ausgewählt ist; wobei H gegebenenfalls an Kohlenstoff durch eine oder mehrere Gruppen ausgewählt aus S substituiert sein kann und wobei, wenn die heterocyclische Gruppe eine -NH-Einheit enthält, dieser Stickstoff gegebenenfalls durch eine aus T ausgewählte Gruppe substituiert sein kann;

**$R^3$** für Wasserstoff oder $C_{1-6}$-Alkyl steht;

**n** aus 0-4 ausgewählt ist; wobei die Werte von $R_1$ gleich oder verschieden sein können; und wobei die Werte von $R^3$ gleich oder verschieden sein können;

**P, S** und **Q** unabhängig voneinander aus Halogen, Nitro, Cyano, Hydroxy, Trifluormethyl, Trifluormethoxy, Amino, Carboxy, Carbamoyl, Mercapto, Sulfamoyl, Ureido, $C_{1-6}$-Alkyl, $C_{2-6}$-Alkenyl, $C_{2-6}$-Alkinyl, $C_{1-6}$-Alkoxy, $C_{1-6}$-Alkanoyl, $C_{1-6}$-Alkanoyloxy, *N*-($C_{1-6}$-Alkyl)amino, *N, N* ( $C_{1-6}$-Alkyl)$_2$amino, $C_{1-6}$-Alkanoylamino, N-($C_{1-6}$-Alkyl) carbamoyl, *N, N*-( $C_{1-6}$-Alkyl)$_2$carbamoyl, *N*-($C_{1-6}$-Alkyl)-*N*-($C_{1-6}$-Alkoxy)carbamoyl, $C_{1-6}$-Alkyl-S(O)$_a$, wobei a für 0 bis 2 steht, $C_{1-6}$-Alkoxycarbonyl, $C_{1-6}$-Alkoxycarbonylamino, *N*-($C_{1-6}$-Alkyl)sulfamoyl, *N, N*-($C_{1-6}$-Alkyl)$_2$sulfamoyl, $C_{1-6}$-Alkylsulfonylamino, $C_{1-6}$-Alkylsulfonyl-*N*-($C_{1-6}$-Alkyl ) amino, $C_{3-8}$-Cycloalkyl, Aryl und heterocyclische Gruppe ausgewählt sind; wobei P, S und Q gegebenenfalls und unabhängig voneinander an Kohlenstoff durch eine oder mehrere Gruppen ausgewählt aus V substituiert sein können und wobei, wenn die heterocyclische Gruppe eine -NH-Einheit enthält, dieser Stickstoff gegebenenfalls durch eine aus U ausgewählte Gruppe substituiert sein kann;

**V** aus Halogen, Nitro, Cyano, Hydroxy, Trifluormethoxy, Trifluormethyl, Amino, Carboxy, Carbamoyl, Mercapto, Sulfamoyl, Methyl, Ethyl, Methoxy, Ethoxy, Acetyl, Acetoxy, Methylamino, Ethylamino, Dimethylamino, Diethylamino, *N*-Methyl-N-ethylamino, Acetylamino, *N*-Methylcarbamoyl, *N*-Ethylcarbamoyl, *N*, *N*-Dimethylcarbamoyl, *N*, *N*-Diethylcarbamoyl, *N*-Methyl-*N*-ethylcarbamoyl, Methylthio, Ethylthio, Methylsulfinyl, Ethylsufinyl, Mesyl, Ethylsulfonyl, Methoxycarbonyl, Ethoxycarbonyl, *N*-Methylsulfamoyl, N-Ethylsulfamoyl, *N,N*-Dimethylsulfamoyl, N,N-Diethylsulfamoyl, N-Methyl-*N*-ethylsulfamoyl, Morpholino, Morpholinocarbonyl, N-Benzylcarbamoyl und 4-Hydroxypiperidinocarbonyl ausgewählt ist;

**R, T** und **U** unabhängig voneinander aus $C_{1-4}$-Alkyl, $C_{1-4}$-Alkanoyl, $C_{1-4}$-Alkylsufonyl, $C_{1-4}$-Alkoxycarbonyl, Carbamoyl, N-($C_{1-4}$-Alkyl)carbamoyl, *N, N*-($C_{1-4}$-Alkyl)$_2$carbamoyl, Phenyl, Benzyl, Benzyloxycarbonyl, Benzoyl und Phenylsulfonyl ausgewählt sind, wobei R, T und U gegebenenfalls und unabhängig voneinander an Kohlenstoff durch eine oder mehrere Gruppen ausgewählt aus V substituiert sein können;

und deren pharmazeutisch unbedenkliche Salze und in vivo hydrolisierbare Ester; mit den Maßgaben, daß:

i) wenn -X-Y-Z- für -S-CH=CH- steht, $R^2$-($CR^1R^3$)$_n$ nicht für Amino, 1-Phenyl-5-methyl-1H-1,5-benzodiazepin-2,4(3H,5H)dion-3-yl, 1-Methyl-5-phenyl-2-oxo-2,3-dihydro-1H-benzo(E)(1,4)diazepin-3-yl, 2-(4-Phenyl-1,2,5,6-tetrahydropyrid-1-yl)ethyl, 3-(4-Phenyl-1,2,5,6-tetrahydropyrid-1-yl)propyl, 2-(4-Phenylpiperazin-1-yl)ethyl, 2-(*N*-Methylamino)ethyl, 2-Morpholinoethyl oder 2-(*N* Methyl-N-benzylamino) ethyl steht;

ii) wenn -X-Y-Z- für -CH=CH-S- steht, $R^2$-($CR^1R^3$)$_n$nicht für Amino oder 1-Methyl-5-phenyl-2-oxo-2,3-dihydro-1H-benzo(E)(1,4)diazepin-3-yl steht;

iii) wenn -X-Y-Z- für $-CH=C(SO_2NH_2)-S-$ steht, $R^2-(CR^1R^3)_n-$ nicht für Methyl oder Isobutyl steht; und
iv) wenn -X-Y-Z- wie ursprünglich definiert ist, n für 1 steht, $R^1$ für Arylmethyl, substituiertes Arylmethyl, (heterocyclische Gruppe)Methyl oder substituiertes (heterocyclische Gruppe)Methyl steht und $R^3$ für Wasserstoff steht, $R^2$ nicht für eine Gruppe -C(=O)-A oder eine Gruppe -CH(OH)-C(=O)-A steht, in welcher A für $NR^dR^d$, $-NR^aCH_2CH_2OR^a$ oder

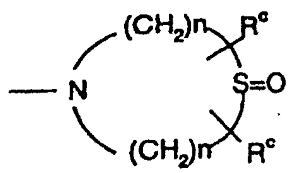 , 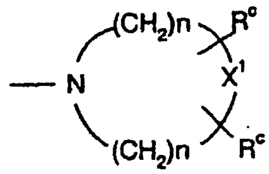 , oder 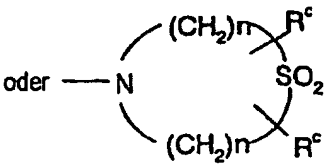

steht;

$R^a$ und $R^b$ jeweils unabhängig voneinander für Wasserstoff oder $-C_1-C_8$-Alkyl stehen;
die Reste $R^d$ jeweils unabhängig voneinander für Wasserstoff, $C_{1-8}$-Alkyl, $C_1$-$C_8$-Alkoxy, Aryl, substituiertes Aryl, Heteroaryl oder substituiertes Heteroaryl stehen;
die Reste $R^c$ jeweils unabhängig voneinander für Wasserstoff, $-C(=O)OR^a$, $-OR^a$, $-SR^a$ oder $-NR^aR^a$ stehen;
und die Indizes n jeweils unabhängig voneinander für 1-3 stehen, und
$X^1$ für $NR^a$, $-CH_2-$, O oder S steht.

**2.** Verbindungen der Formel (I) nach Anspruch 1, wobei:

-X-Y-Z- aus $-S-CR^4=CR^5-$ und $-CR^4=CR^5-S-$ ausgewählt ist;
wobei $R^4$ und $R^5$ unabhängig voneinander aus Wasserstoff, Halogen, Nitro, Cyano, Hydroxy, Fluormethyl, Difluormethyl, Trifluormethyl, Trifluormethoxy, Amino, Carboxy, Carbamoyl, Mercapto, Sulfamoyl, Ureido, $C_{1-6}$-Alkyl, $C_{2-6}$-Alkenyl, $C_{2-6}$-Alkinyl, $C_{1-6}$-Alkoxy, $C_{1-6}$-Alkanoyl, $C_{1-6}$-Alkanoyloxy, *N*-($C_{1-6}$-Alkyl) amino, *N,N*-($C_{1-6}$-Alkyl)$_2$amino, $C_{1-6}$-Alkanoylamino, *N*-($C_{1-6}$-Alkyl)carbamoyl, *N, N*-( $C_{1-6}$-Alkyl )$_2$carbamoyl , $C_{1-6}$-Alkyl-S(O)$_a$, wobei a für 0 bis 2 steht, $C_{1-6}$-Alkoxycarbonyl, $C_{1-6}$-Alkoxycarbonylamino, *N*-($C_{1-6}$-Alkyl)sulfamoyl, *N,N*-($C_{1-6}$-Alkyl)$_2$sulfamoyl, $C_{1-6}$-Alkylsulfonylamino und $C_{1-6}$-Alkylsulfonyl-*N*-($C_{1-6}$-Alkyl)amino ausgewählt sind;
n für 0 steht;
$R^2$ für eine Gruppe -E-F-G-H-steht;
wobei E, F und G jeweils für eine direkte Bindung stehen;
H für $C_{3-12}$-Cycloalkyl steht, das gegebenenfalls mit einem Benzolring kondensiert ist, wobei H an Kohlenstoff durch eine oder mehrere Gruppen S substituiert sein kann, die unabhängig voneinander aus Halogen, Nitro, Cyano, Hydroxy, Trifluormethyl, Trifluormethoxy, Amino, Carboxy, Carbamoyl, Mercapto, Sulfamoyl, Ureido, $C_{1-6}$-Alkyl, $C_{2-6}$-Alkenyl, $C_{2-6}$-Alkinyl, $C_{1-6}$-Alkoxy, $C_{1-6}$-Alkanoyl, $C_{1-6}$-Alkanoyloxy, *N*-($C_{1-6}$-Alkyl)amino, *N, N*-($C_{1-6}$-Alkyl) $_2$amino, $C_{1-6}$-Alkanoylamino, *N*-($C_{1-6}$-Alkyl) carbamoyl, *N, N*-($C_{1-6}$-Alkyl )$_2$carbamoyl , *N*-($C_{1-6}$-Alkyl)-N-($C_{1-6}$-Alkoxy)carbamoyl, $C_{1-6}$-Alkyl-S(O)$_a$, wobei a für 0 bis 2 steht, $C_{1-6}$-Alkoxycarbonyl, $C_{1-6}$-Alkoxycarbonylamino, *N*-($C_{1-6}$-Alkyl)sulfamoyl, *N, N*-($C_{1-6}$-Alkyl)$_2$sulfamoyl, $C_{1-6}$-Alkylsulfonylamino, $C_{1-6}$-Alkylsulfonyl-N-($C_{1-6}$-Alkyl) amino, $C_{3-8}$-Cycloalkyl, Aryl und heterocyclische Gruppen ausgewählt sind; wobei S gegebenenfalls an Kohlenstoff durch eine oder mehrere Gruppen ausgewählt aus V substituiert sein kann;
V aus Halogen, Nitro, Cyano, Hydroxy, Trifluormethoxy, Trifluormethyl, Amino, Carboxy, Carbamoyl, Mercapto, Sulfamoyl, Methyl, Ethyl, Methoxy, Ethoxy, Acetyl, Acetoxy, Methylamino, Ethylamino, Dimethylamino, Diethylamino, *N*-Methyl-*N*-ethylamino, Acetylamino, *N*-Methylcarbamoyl, *N*-Ethylcarbamoyl, *N,N*-Dimethylcarbamoyl, *N,N*-Diethylcarbamoyl, *N*-Methyl-*N*-ethylcarbamoyl, Methylthio, Ethylthio, Methylsulfinyl, Ethylsufinyl, Mesyl, Ethylsulfonyl, Methyoxycarbonyl, Ethoxycarbonyl, *N*-Methylsulfamoyl, *N*-Ethylsulfamoyl, *N,N*-Dimethylsulfamoyl, *N,N*-Diethylsulfamoyl, *N*-Methyl-*N*-ethylsulfamoyl, Morpholino, Morpholinocarbonyl, *N*-Benzylcarbamoyl und 4-Hydroxypiperidinocarbonyl ausgewählt ist; und deren pharmazeutisch unbedenkliche Salze.

**3.** Verbindungen der Formel (I) nach Anspruch 1, wobei:

-X-Y-Z- aus $-S-CR^4=CR^5-$ und $-CR^4=CR^5-S-$ ausgewählt ist;
wobei $R^4$ und $R^5$ unabhängig voneinander aus Wasserstoff, Halogen, Nitro, Cyano, Hydroxy, Fluormethyl, Difluormethyl, Trifluormethyl, Trifluormethoxy, Amino, Carboxy, Carbamoyl, Mercapto, Sulfamoyl, Ureido, $C_{1-6}$-

Alkyl, $C_{2-6}$-Alkenyl, $C_{2-6}$-Alkinyl, $C_{1-6}$-Alkoxy, $C_{1-6}$-Alkanoyl, $C_{1-6}$-Alkanoyloxy, *N*-($C_{1-6}$-Alkyl) amino, *N, N*-($C_{1-6}$-Alkyl)$_2$amino, $C_{1-6}$-Alkanoylamino, *N*-($C_{1-6}$-Alkyl) carbamoyl, *N, N*-($C_{1-6}$-Alkyl) $_2$carbamoyl, $C_{1-6}$-Alkyl-S(O)$_a$, wobei a für 0 bis 2 steht, $C_{1-6}$-Alkoxycarbonyl, $C_{1-6}$-Alkoxycarbonylamino, *N*-($C_{1-6}$-Alkyl)sulfamoyl, *N, N*-($C_{1-6}$-Alkyl)$_2$sulfamoyl, $C_{1-6}$-Alkylsulfonylamino und $C_{1-6}$-Alkylsulfonyl-*N*-($C_{1-6}$-Alkyl)amino ausgewählt sind;
n für 0 steht;
$R^2$ für eine Gruppe -E-F-G-H-steht;
wobei E, F und G jeweils für eine direkte Bindung stehen; und
H für ein cyclisches Amid der Formel

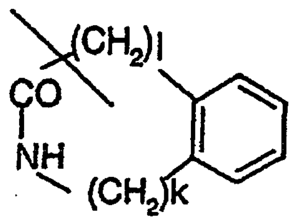

steht, in welcher k für 0, 1, 2 oder 3 steht und 1 für 0, 1, 2 oder 3 steht, so daß die Summe von k und 1 2 oder 3 beträgt, und wobei eines der von k oder 1 bezeichneten Kohlenstoffatome durch Schwefel ersetzt sein kann und wobei H gegebenenfalls an Kohlenstoff durch eine oder mehrere Gruppen ausgewählt aus S substituiert ist und unabhängig davon gegebenenfalls an Stickstoff durch eine aus T ausgewählte Gruppe substituiert sein kann;
S aus Halogen, Nitro, Cyano, Hydroxy, Trifluormethyl, Trifluormethoxy, Amino, Carboxy, Carbamoyl, Mercapto, Sulfamoyl, Ureido, $C_{1-6}$-Alkyl, $C_{2-6}$-Alkenyl, $C_{2-6}$-Alkinyl, $C_{1-6}$-Alkoxy, $C_{1-6}$-Alkanoyl, $C_{1-6}$-Alkanoyloxy, *N*-($C_{1-6}$-Alkyl)amino, *N,N*-($C_{1-6}$-Alkyl)$_2$amino, $C_{1-6}$-Alkanoylamino, N-($C_{1-6}$-Alkyl)carbamoyl, *N,N*-($C_{1-6}$-Alkyl)$_2$carbamoyl, *N-(C$_{1-6}$*-Alkyl) -*N*-($C_{1-6}$-Alkoxy)carbamoyl, $C_{1-6}$-Alkyl-S(O)$_a$, wobei a für 0 bis 2 steht, $C_{1-6}$-Alkoxycarbonyl, $C_{1-6}$-Alkoxycarbonylamino, *N*-($C_{1-6}$-Alkyl)sulfamoyl, *N,N*-($C_{1-6}$-Alkyl)$_2$sulfamoyl, $C_{1-6}$-Alkylsulfonylamino, $C_{1-6}$-Alkylsulfonyl-*N*-($C_{1-6}$-Alkyl) amino, $C_{3-8}$-Cycloalkyl, Aryl und heterocyclische Gruppe ausgewählt ist; wobei S gegebenenfalls und unabhängig an Kohlenstoff durch eine oder mehrere Gruppen ausgewählt aus V substituiert sein kann und wobei, wenn die heterocyclische Gruppe eine-NH-Einheit enthält, dieser Stickstoff gegebenenfalls durch eine aus U ausgewählte Gruppe substituiert sein kann;
T und U unabhängig voneinander aus $C_{1-4}$-Alkyl, $C_{1-4}$-Alkanoyl, $C_{1-4}$-Alkylsulfonyl, $C_{1-4}$-Alkoxycarbonyl, Carbamoyl, N-($C_{1-4}$-Alkyl)carbamoyl, N,N-($C_{1-4}$-Alkyl)$_2$carbamoyl, Phenyl, Benzyl, Benzyloxycarbonyl, Benzoyl und Phenylsulfonyl ausgewählt sind, wobei R, T und U gegebenenfalls und unabhängig voneinander an Kohlenstoff durch eine oder mehrere Gruppen ausgewählt aus V substituiert sein können;
V aus Halogen, Nitro, Cyano, Hydroxy, Trifluormethoxy, Trifluormethyl, Amino, Carboxy, Carbamoyl, Mercapto, Sulfamoyl, Methyl, Ethyl, Methoxy, Ethoxy, Acetyl, Acetoxy, Methylamino, Ethylamino, Dimethylamino, Diethylamino, *N*-Methyl-N-ethylamino, Acetylamino, *N*-Methylcarbamoyl, *N*-Ethylcarbamoyl, *N,N*-Dimethylcarbamoyl, *N,N*-Diethylcarbamoyl, *N*-Methyl-*N*-ethylcarbamoyl, Methylthio, Ethylthio, Methylsulfinyl, Ethylsufinyl, Mesyl, Ethylsulfonyl, Methoxycarbonyl, Ethoxycarbonyl, *N*-Methylsulfamoyl, *N*-Ethylsulfamoyl, *N,N*-Dimethylsulfamoyl, *N,N*-Diethylsulfamoyl, *N*-Methyl-*N*-ethylsulfamoyl, Morpholino, Morpholinocarbonyl, *N*-Benzylcarbamoyl und 4-Hydroxypiperidinocarbonyl ausgewählt ist;

und deren pharmazeutisch unbedenkliche Salze und in vivo hydrolisierbare Ester.

**4.** Verbindungen der Formel (I) nach Anspruch 1, wobei:

-X-Y-Z- aus -S-CR$^4$=CR$^5$- und -CR$^4$=CR$^5$-S- ausgewählt ist;
wobei $R^4$ und $R^5$ unabhängig voneinander aus Wasserstoff, Halogen und $C_{1-6}$-Alkyl ausgewählt sind;
n für 1 steht;
$R^1$ für Wasserstoff oder Aryl-$C_{1-6}$-alkyl steht;
$R^2$ aus einer Gruppe -E-F-G-H ausgewählt ist;
wobei E, F und G jeweils für eine direkte Bindung stehen;
H für eine ungesättigte, fünfgliedrige heterocyclische Gruppe mit wenigstens einem Stickstoffatom und einem oder zwei Ringatomen ausgewählt aus Sauerstoff und Schwefel steht, wobei H gegebenenfalls an Kohlenstoff durch eine oder mehrere Gruppen S substituiert sein kann, die unabhängig voneinander aus Halogen, Nitro,

Cyano, Hydroxy, Trifluormethyl, Trifluormethoxy, Amino, Carboxy, Carbamoyl, Mercapto, Sulfamoyl, Ureido, $C_{1-6}$-Alkyl, $C_{2-6}$-Alkenyl, $C_{2-6}$-Alkinyl, $C_{1-6}$-Alkoxy, $C_{1-6}$-Alkanoyl, -$C_{1-6}$-Alkanoyloxy, *N*-($C_{1-6}$-Alkyl)amino, *N,N*-($C_{1-6}$-Alkyl)$_2$amino, $C_{1-6}$-Alkanoylamino, *N*-($C_{1-6}$-Alkyl)carbamoyl, *N,N*-($C_{1-6}$-Alkyl)$_2$carbamoyl, *N*-($C_{1-6}$-Alkyl)-*N*-($C_{1-6}$-Alkoxy)carbamoyl, $C_{1-6}$-Alkyl-S(O)$_a$, wobei a für 0 bis 2 steht, $C_{1-6}$-Alkoxycarbonyl, $C_{1-6}$-Alkoxycarbonylamino, *N*-($C_{1-6}$-Alkyl)sulfamoyl, *N, N*-($C_{1-6}$-Alkyl) $_2$sulfamoyl, $C_{1-6}$-Alkylsulfonylamino, $C_{1-6}$-Alkylsulfonyl-N-($C_{1-6}$-Alkyl) amino, $C_{3-8}$-Cycloalkyl und Arylgruppen ausgewählt sind;
$R^3$ für Wasserstoff oder $C_{1-6}$-Alkyl steht;
und deren pharmazeutisch unbedenkliche Salze.

**5.** Verbindungen der Formel (I) nach Anspruch 1, wobei:

-X-Y-Z- aus -S-$CR^4$=$CR^5$- und -$CR^4$=$CR^5$-S- ausgewählt ist;
wobei $R^4$ und $R^5$ unabhängig voneinander aus Wasserstoff, Halogen und $C_{1-6}$-Alkyl ausgewählt sind;
n für 0 steht;
$R^2$ für eine Gruppe -E-F-G-H steht;
wobei E, für eine direkte Bindung steht;
F für Methylen steht;
wobei G für -C(O)$NR^a$- steht, wobei $R^a$ aus Wasserstoff und $C_{1-6}$-Alkyl, das gegebenenfalls durch eine Gruppe V substituiert ist, ausgewählt ist;
H für Aryl steht, das gegebenenfalls an Kohlenstoff durch eine oder mehrere Gruppen ausgewählt aus S substituiert sein kann;
S aus Halogen, Nitro, Cyano, Hydroxy, Trifluormethyl, Trifluormethoxy, Amino, Carboxy, Carbamoyl, Mercapto, Sulfamoyl, Ureido, $C_{1-6}$-Alkyl, $C_{2-6}$-Alkenyl, $C_{2-6}$-Alkinyl, $C_{1-6}$-Alkoxy, $C_{1-6}$-Alkanoyl, $C_{1-6}$-Alkanoyloxy, *N*-($C_{1-6}$-Alkyl) amino, *N,N*-($C_{1-6}$-Alkyl)$_2$amino, $C_{1-6}$-Alkanoylamino, *N*-($C_{1-6}$-Alkyl ) carbamoyl, *N, N*-($C_{1-6}$-Alkyl ) $_2$carbamoyl, *N*-($C_{1-6}$-Alkyl)-*N*-($C_{1-6}$-Alkoxy)carbamoyl, $C_{1-6}$-Alkyl-S (O)$_a$, wobei a für 0 bis 2 steht, $C_{1-6}$-Alkoxycarbonyl, $C_{1-6}$-Alkoxycarbonylamino, *N*-($C_{1-6}$-Alkyl)sulfamoyl, N, N-($C_{1-6}$-Alkyl)$_2$sulfamoyl, $C_{1-6}$-Alkylsulfonylamino, $C_{1-6}$-Alkylsulfonyl-N-($C_{1-6}$-Alkyl ) amino, $C_{3-8}$-Cycloalkyl, Aryl und heterocyclische Gruppe ausgewählt ist; wobei S gegebenenfalls und unabhängig an Kohlenstoff durch eine oder mehrere Gruppen ausgewählt aus V substituiert sein kann;
V aus Halogen, Nitro, Cyano, Hydroxy, Trifluormethoxy, Trifluormethyl, Amino, Carboxy, Carbamoyl, Mercapto, Sulfamoyl, Methyl, Ethyl, Methoxy, Ethoxy, Acetyl, Acetoxy, Methylamino, Ethylamino, Dimethylamino, Diethylamino, *N*-Methyl-*N*-ethylamino, Acetylamino, *N*-Methylcarbamoyl, *N*-Ethylcarbamoyl, *N,N*-Dimethylcarbamoyl , *N,N*-Diethylcarbamoyl, *N*-Methyl-*N*-ethylcarbamoyl, Methylthio, Ethylthio, Methylsulfinyl, Ethylsulfinyl, Mesyl, Ethylsulfonyl, Methyoxycarbonyl, Ethyoxycarbonyl, *N*-Methylsulfamoyl, *N*-Ethylsulfamoyl, N, N-Dimethylsulfamoyl, *N,N*-Diethylsulfamoyl, N-Methyl-N-ethylsulfamoyl, Morpholino, Morpholinocarbonyl, *N*-Benzylcarbamoyl und 4-Hydroxypiperidinocarbonyl ausgewählt ist;

und deren pharmazeutisch unbedenkliche Salze.

**6.** Verbindungen, ausgewählt aus
2,3-Dichlor-5-[*N*-(2-phenoxyethyl)carbamoyl]-4*H*thieno[3,2-*b*]pyrrol;
2,3-Dichlor-5-{*N*-[2-(2-thienyl)ethyl]carbamoyl}-4H-thieno[3,2-*b*]pyrrol;
2,3-Dichlor-5-{*N*-[2-(2-methoxyphenyl)ethyl]carbamoyl)-4*H*-thieno [3,2-*b*]pyrrol;
2,3-Dichlor-5-[*N*-(2-phenyl-1-cyclopropyl)carbamoyl]-4*H*-thieno[3,2-*b*]pyrrol;
2,3-Dichlor-5-{*N*-[2-(4-fluorphenyl)ethyl]carbamoyl}-4*H*-thieno(3,2-*b*]pyrrol;
2,3-Dichlor-5-[*N*-[*N*-phenylcarbamoylmethyl)carbamoyl]-4*H*-thieno[3,2-*b*]pyrrol;
2,3-Dichlor-5-(*N*-{2-[(2-pyridyl)amino]ethyl}carbamoyl)-4*H*-thieno[3,2-*b*]pyrrol;
2,3-Dichlor-5-{*N*-[2-(*N*-methylmethansulfonamid)-1-(thiazol-2-yl)ethyl]carbamoyl}-4*H*-thieno[3,2-*b*]pyrrol ;
2,3-Dichlor-5-{*N*-[2-(thiomorpholino)ethyl]carbamoyl}-4*H*-thieno[3,2-*b*]pyrrol;
5-[*N*-(Benzoylmethyl)carbamoyl]-2,3-dichlor-4*H*thieno[3,2-*b*]pyrrol;
3-Chlor-5-[*N*-(*N*-phenylcarbamoylmethyl)carbamoyl]-4H-thieno[3,2-*b*]pyrrol;
3-Chlor-5-(*N*-[2-(thiomorpholino)ethyl]carbamoyl}-4H-thieno[3,2-*b*]pyrrol;
3-Chlor-5-{*N*-[2-(*N*-methylmethansulfonamid)-1-(thiazol-2-yl)ethyl]carbamoyl}-4*H*-thieno[3,2-*b*]pyrrol;
3-Chlor-5-[*N*-(Benzoylmethyl)carbamoyl]-4*H*-thieno[3,2-*b*]pyrrol;
3-Chlor-5-{*N*-[2-(2-methoxyphenyl)ethyl]carbamoyl}-4*H*-thieno[3,2-*b*]pyrrol;
3-Chlor-5-{*N*-[2-(2-thienyl) ethyl] carbamoyl)-4*H*-thieno[3,2-*b*] pyrrol;
3-Chlor-5-[*N*-(2-phenyl-1-cyclopropyl)carbamoyl]-4H-thieno [3,2-*b*]pyrrol;
3-Chlor-5-{*N*-[2-(4-fluorphenyl)ethyl]carbamoyl}-4*H*-thieno[3,2-*b*]pyrrol;

3-Chlor-5-[*N*-(2-phenoxyethyl)carbamoyl]-4*H*-thieno[3,2-*b*]pyrrol;
3-Chlor-5-{*N*-[2-(1-phenylmethansulfonamid)-ethyl]carbamoyl}-4*H*-thieno[3,2-*b*]pyrrol;
3-Chlor-5-[*N*-(4-oxo-2,3,4,5-tetrahydro-benz[1,5]thiazepin-3-yl)carbamoyl]-4*H*-thieno[3,2-*b*]pyrrol;
2-Chlor-5-[*N*-(benzoylmethyl)carbamoyl]-4*H*-thieno[3,2-*b*]pyrrol;
2-Chlor-5-[*N*-(2-phenyl-1-cyclopropyl)carbamoyl]-4*H*-thieno[3,2-*b*]pyrrol;
2-Chlor-5-[*N*-(*N*-phenylcarbamoylmethyl)carbamoyl]-4*H*-thieno[3,2-*b*]pyrrol;
2-Chlor-5-{*N*-{2-[(2-pyridyl)amino]ethyl}carbamoyl)-4*H*-thieno[3,2-**b**]pyrrol;
2-Chlor-5-{*N*-[2-(2-methoxyphenyl)ethyl]carbamoyl}-4*H*-thieno[3,2-*b*]pyrrol;
2-Chlor-5-[*N*-(2-phenoxyethyl)carbamoyl]-4*H*-thieno[3,2-*b*]pyrrol;
2-Chlor-5-{*N*-[2-(2-thienyl)ethyl]carbamoyl}-4*H* thieno[3,2-**b**]pyrrol;
2-Chlor-5-{*N*-[2-(4-fluorphenyl)ethyl]carbamoyl}-4*H*-thieno [3,2-*b*] pyrrol;
2-Chlor-5-{*N*-[2-(*N*-methylmethansulfonamid)-1-(thiazol-2-yl)ethyl]carbamoyl}-4*H*-thieno[3,2-**b**]pyrrol;
2-Chlor-5-{*N*-[2-(thiomorpholino)ethyllcarbamoyl}-4*H*-thieno[3,2-*b*]pyrrol;
2,3-Dichlor-5-[*N*-(2,3-dimethyl-5-oxo-1-phenyl-2,5-dihydro-1*H*-pyrazol-4-yl)carbamoyl]-4*H*-thieno[3,2-**b**]pyrrol;
2,3-Dichlor-5-[*N*-(4-sulfamoylphenylmethyl)carbamoyl]-4**H**-thieno[3,2-*b*]pyrrol;
2,3-Dichlor-5-[*N*-(2-hydroxy-1-phenethyl)carbamoyl]-4**H**-thieno[3,2-*b*]pyrrol;
2,3-Dichlor-5-{*N*-(2-[(3-trifluormethylpyrid-2-yl)amino]ethyl)carbamoyl}-4*H*-thieno[3,2-**b**]pyrrol;
2,3-Dichlor-5-{*N*-[3-(5-tetrazolyl)propyl]carbamoyl}-4*H*-thieno[3,2-*b*]pyrrol;
2,3-Dichlor-5-[*N*-(5-oxo-3-phenyl-4,5-dihydroisoxazol-4-yl)carbamoyl]-4*H*-thieno[3,2-*b*]pyrrol;
2,3-Dichlor-5-[*N*-(5-hydroxy-2-oxo-2,3,4,5-tetrahydro-1H-benz[*b*]azepin-4-yl)carbamoyl]-4*H*-thieno[3,2-*b*]pyrrol;
2-Chlor-5-{*N*-[3-(benzyloxycarbonylamino)propyl]-carbamoyl}-4*H*-thieno[3,2-*b*]pyrrol;
2,3-Dichlor-5-{*N*-[(4-dimethylaminophenyl)methyl]carbamoyl}-4*H*-thieno[3,2-*b*]pyrrol;
5-[*N*-(1-Benzyl-2-hydroxyethyl)carbamoyl)-2,3-dichlor-4*H*-thieno[3,2-*b*]pyrrol;
2,3-Dichlor-5-{*N*-[2-(phenylamino)ethyl]carbamoyl}-4*H*-thieno[3,2-*b*]pyrrol;
2,3-Dichlor-5-[*N*-(β-(*R*)-hydroxy-α-methylphenethyl)carbamoyl]-4*H*-thieno[3,2-*b*]pyrrol;
2,3-Dichlor-5-[*N*-(β-hydroxyphenethyl)carbamoyl]-4*H*-thieno[3,2-*b*]pyrrol;
2,3-Dichlor-5-{*N*-[2-(4-hydroxyphenyl)ethyl]carbamoyl}-4*H*-thieno[3,2-*b*]pyrrol;
2,3-Dichlor-5-{*N*-[(benzimidazol-2-yl)methyl]carbamoyl}-4*H*-thieno[3,2-*b*]pyrrol;
2,3-Dichlor-5-{*N*-[2-(4-clorphenyl)-2-hydroxy-1-(methoxycarbonyl)ethyl]carbamoyl}-4*H*-thieno[3,2-*b*]pyrrol;
2,3-Dichlor-5-{*N*-(imidazo[1,2-α]pyrid-2-yl) carbamoyl}-4*H*-thieno[3,2-*b*]pyrrol;
5-{*N*-[(Benzthiazol-2-yl)methyl]carbamoyl}-2,3-dichlor-4*H*-thieno[3,2-*b*]pyrrol;
2,3-Dichlor-5-{*N*-[(6-trifluormethylpyrid-3-yl)methyl]carbamoyl}-4*H*-thieno[3,2-*b*]pyrrol;
2,3-Dichlar-5-{*N*-(2-[(2-pyridazinyl)methyl]carbamoyl}-4*H*-thieno[3,2-*b*]pyrrol;
2,3-Dichlor-5-{*N*-[*N*-(2-hydroxy-3-phenoxypropyl)carbamoylmethyl]carbamoyl}-4*H*-thieno[3,2-*b*]Pyrrol;
2,3-Dichlor-5-{*N*-[*N*-(3-methylisothiazol-5-yl)carbamoylmethyl] carbamoyl)-4*H*-thieno[3,2 -*b*]pyrrol;
2,3-Dichlor-5-{*N*-[2-(pyridazin-3-yloxy)ethyl]carbamoyl)-4*H*-thieno [3,2-*b*]pyrrol;
2-Chlor-5-(*N*-{2-[(3-trifluormethylpyrid-2-yl)amino]ethyl}carbamoyl)-4*H*-thieno[3,2-*b*]pyrrol;
2,3-Dichlor-5-{*N*-[2-(4-sulfamoylphenyl)ethyl]carbamoyl)-4*H*-thieno[3,2-*b*]pyrrol;
2,3-Dichlor-5-{*N*-[2-(2-pyridyl)ethyl]carbamoyl}-4*H*-thieno[3,2-*b*]pyrrol;
2,3-Dichlor-5-{*N*-(2-[1-hydroxymethyl-2-(4-imidazoyl)ethyl]carbamoyl}-4*H*-thieno[3,2-*b*]pyrrol;
2,3-Dichlor-5-{*N*-(2-[(3-chinolyl)methyl]carbamoyl}-4*H*-thieno[3,2-*b*]pyrrol;
5-{*N*-[3-(4-Acetamidophenoxy)-2-hydroxypropyl]carbamoyl}-2,3-dichlor-4*H*-thieno[3,2-*b*]pyrrol;
2,3-Dichlor-5-{*N*-[3-(*N*-methylsulfonylcarbamoyl)propyl]carbamoyl}-4*H*-thieno[3,2-*b*]pyrrol;
2,3-Dichlor-5-[*N*-(2-{[2-[guanidino)thiazol-4-yl)methylthio}ethyl)carbamoyl]-4*H*-thieno[3,2-*b*]pyrrol;
2,3-Dichlor-5-{*N*-[2-(2,4-dioxoimidazolidin-1-yl)ethyl]carbamoyl}-4*H*-thieno[3,2-*b*]pyrrol;
5-{*N*-[2-Benzylthio-1-(hydroxymethyl)ethyl]carbamoyl}-2,3-dichlor-4*H*-thieno[3,2-*b*]pyrrol;
2,3-Dichlor-5-{*N*-[2-(dimethylaminosulfonylamino)ethyl]carbamoyl}-4*H*-thieno[3,2-*b*]pyrrol;
2,3-Dichlor-5-{*N*-[(6-methoxypyrid-3-yl)methyl]carbamoyl} -4*H*-thieno [3,2-*b*] pyrrol;
(*S*)-2,3-Dichlor-5-{*N*-[(2-oxo-3-phenyl-2,3,4,5-tetrahydrooxazol-5-yl)methyl]carbamoyl}-4*H*-thieno [3,2-*b*]pyrrol;
2,3-Dichlor-5-(*N*-{2-[3-(carbamoylmethyl)phenoxy]ethyl}carbamoyl)-4*H*-thieno[3,2-*b*]pyrrol;
5-(N-{[6-(benzo[1,3]dioxol-5-yl)-4-methylmorpholin-2-yl]methyl}carbamoyl)-2,3-dichlor-4*H*-thieno[3,2-*b*]pyrrol;
5-(*N*-Benzylcarbamoyl-2,3-dichlor-4*H*-thieno[3,2-*b*]pyrrol;
2,3-Dichlor-5-(*N*-phenethylcarbamoyl)-4*H*-thieno[3,2-*b*]pyrrol;
2,3-Dichlor-5-[*N*-phenylpropyl)carbamoyl]-4*H*-thieno[3,2-*b*]pyrrol;
2,3-Dichlor-5-{*N*-[2-(2-hydroxyphenyl)ethyl]carbamoyl}-4*H*-thieno[3,2-*b*]pyrrol;
2,3-Dichlor-5-[*N*-(α,α-dimethylphenethyl)carbamoyl]-4*H*-thieno[3,2-*b*]pyrrol;
2,3-Dichlor-5-[*N*-(1-phenylcyclobutyl)methyl)carbamoyl]-4*H*-thieno[3,2-*b*]pyrrol;
2,3-Dichlor-5-[*N*-(β-methylphenethyl)carbamoyl]-4*H*thieno[3,2-*b*]pyrrol;

2,3-Dichlor-5-[*N*-(1,2,3,4-tetrahydronaphth-2-yl)carbamoyl]-4*H*-thieno[3,2-*b*]pyrrol;
5-[*N*-(*N*-Benzylcarbamoylmethyl)carbamoyl]-2,3-dichlor-4*H*-thieno[3,2-*b*]pyrrol;.
5-[*N*-(*N*-Benzyl-*N*-methylcarbamoylmethyl)carbamoyl]-2,3-dichlor-4*H*-thieno[3,2-*b*]pyrrol;
2,3-Dichlor-5-[*N*-(*N*-methyl-*N*-phenylcarbamoylmethyl)carbamoyl]-4*H*-thieno[3,2-*b*]pyrrol;
2,3-Dichlor-5-{*N*-[*N*-(2-cyanoethyl)-*N*-phenylcarbamoylmethyl]carbamoyl)-4*H*-thieno[3,2-*b*]pyrrol;
2,3-Dichlor-5-{*N*-[*N*-(4-methoxyphenyl}carbamoylmethyl]carbamoyl}-4*H*-thieno[3,-2-*b*)pyrrol;
2,3-Dichlor-5-{*N*-[*N*-(4-fluorphenyl)carbamoylmethyl)carbamoyl)-4*H*-thieno[3,2-*b*)pyrrol;
2,3-Dichlor-5-{*N*-[*N*-(4-nitrophenyl)carbamoylmethyl]carbamoyl}-4*H*-thieno[3,2-*b*]pyrrol;
2,3-Dichlor-5-{*N*-[*N*-(2,6-dimethylphenyl)carbamoylmethyl]carbamoyl)-4*H*-thieno[3,2-*b*]pyrrol;
2,3-Dichlor-5-{*N*-[*N*-methyl-*N*-(4-methylphenyl)carbamoylmethyl]carbamoyl}-4*H*-thieno[3,2-*b*]pyrrol;
2,3-Dichlor-5-{*N*-[*N*-(3-chlorphenyl)-*N*-methylcarbamoylmethyl]carbamoyl]-4*H*-thieno[3,2-*b*]pyrrol;
2,3-Dichlor-5-{*N*-[*N*-(2-hydroxyethyl)-*N*-phenylcarbamoylmethyl]carbamoyl}-4*H*-thieno[3,2-*b*]pyrrol;
2,3-Dichlor-5-{*N*-[*N*-(1,1-dimethyl-2-hydroxyethyl)carbamoylmethyl]carbamoyl}-4*H*-thieno[3,2-*b*]pyrrol;
2,3-Dichlor-5-(*N*-[*N*-(2-hydroxyethyl)-*N*-methylcarbamoylmethyl]carbamoyl}-4*H*-thieno[3,2-*b*]pyrrol;
2,3-Dichlor-5-{*N*-{*N*-(2-hydroxyethyl)carbamoylmethyl]carbamoyl}-4*H*-thieno[3,2-*b*]pyrrol;
2,3-Dichlor-5-{*N*-[*N*-(3-hydroxyprapyl)carbamoylmethyl]carbamoyl}-4*H*-thieno[3,2-*b*]pyrrol;
2,3-Dichlor-5-{*N*-[*N*-(4-hydroxybutyl)carbamoylmethyl]carbamoyl}-4*H*-thieno[3,2-*b*]pyrrol;
2,3-Dichlor-5-(*N*-{*N*-[bis(hydroxymethyl)methyl]carbamoylmethyl]carbamoyl}-4*H*-thieno[3,2-*b*]pyrrol;
2,3-Dichlor-5-{*N*-[*N*-(2,3-dihydroxypropyl)carbamoylmethyl]carbamoyl}-4*H*-thieno[3,2-*b*]pyrrol;
2,3-Dichlor-5-{*N*-[*N*-(4-hydroxymethylphenyl)carbamoylmethyl]carbamoyl}-4*H*-thieno[3,2-*b*]pyrrol;
2,3-Dichlor-5-{*N*-[*N*-(5-isochinolyl)carbamoylmethyl]carbamoyl)-4*H*-thieno[3,2-*b*]pyrrol;
2,3-Dichlor-5-[*N*-[*N*-(3-hydroxymethyl)phenyl]carbamoylmethyl)carbamoyl}-4*H*-thieno[3,2-*b*]pyrrol;
2,3-Dichlor-5-(*N*-{(*N*-[4-(2-hydroxyethyl)phenyl]carbamoylmethyl]carbamoyl}-4*H*-thieno[3,2-*b*]pyrrol;
2,3-Dichlor-5-(*N*-[*N*-(2,4-difluorphenyl)-*N*-methylcarbamoylmethyl]carbamoyl}-4*H*-thieno[3,2-*b*]pyrrol;
2,3-Dichlor-5-{*N*-[(1,2,3,4-tetrahydro-1-chinolyl)carbamoylmethyl]carbamoyl}-4*H*-thieno[3,2-*b*]pyrrol;
2,3-Dichlor-5-{*N*-[*N*-(2-cyanoethyl)-*N*-methylcarbamoylmethyl]carbamoyl}-4*H*-thieno[3,2-*b*]pyrrol;
2,3-Dichlor-5-{*N*-[*N*-(4-hydroxypiperidino)carbamoylmethyl]carbamoyl}-4*H*-thieno[3,2-*b*]pyrrol;
2,3-Dichlor-5-[*N*-(*N*-cyclopentylcarbamoylmethyl)carbamoyl]-4*H*-thieno[3,2-*b*]pyrrol;
2,3-Dichlor-5-[*N*-(*N*-isopropylcarbamoylmethyl)carbamoyl]-4*H*-thieno[3,2-*b*]pyrrol;
2,3-Dichlor-5-[*N*-(*N*-isopropyl-*N*-methylcarbamoylmethyl)carbamoyl]-4*H*-thieno[3,2-*b*]pyrrol;
2,3-Dichlor-5-[*N*-thiomorpholinocarbonylmethyl)carbamoyl]-4*H*-thieno[3,2-*b*]pyrrol;
2,3-Dichlor-5-[*N*-(morpholinocarbonylmethyl)carbamoyl}-4*H*-thieno[3,2-*b*]pyrrol;
2,3.-Dichlor-5-{*N*-[(1,1-dioxothiomorpholino)carbonylmethyl]carbamoyl}-4*H*-thieno[3,2-*b*]pyrrol;
2,3-Dichlor-5-{*N*-[(1-oxothiomorpholino)carbonylmethyl]carbamoyl]-4*H*-thieno[3,2-*b*]pyrrol;
2-Chlor-5-[*N*-(2-indanyl)carbamoyl]6*H*-thieno[2,3-*b*] pyrrol;
5-[*N*-Benz[1,2]oxazol-3-ylmethyl)carbamoyl]-2,3-dichlor-4*H*-thieno[3,2-*b*]pyrrol;
2,3-Dichlor-5-(*N*-(2-[2-hydroxymethyl)phenyl]-ethyl}carbamoyl)-4*H*-thieno[3,2-*b*]pyrrol;
2,3-Dichlor-5-[*N*-(4-phenylisooxazol-3-ylmethyl)carbamoyl]}-4*H*-thieno[3,2-*b*]pyrrol;
2,3-Dichlor-5-(*N*-{2-[2-(2-morpholinoethoxy)phenyl]ethyl}carbamoyl)}-4*H*-thieno[3,2-*b*]pyrrol;
2,3-Dichlor-5-(*N*-{2-[2-(methoxycarbonylmethoxy)-phenyl]ethyl}carbamoyl)-4*H*-thieno[3,2-*b*]pyrrol;
5-(*N*-{2-[2-(carboxymethoxy)phenyl]ethyl}carbamoyl)-2,3-dichlor-4*H*-thieno[3,2-*b*]pyrrol;
2,3-Dichlor-5-{*N*-[2-(3-methoxyphenyl)ethyl]carbamoyl]-4*H*-thieno[3,2-*b*]pyrrol;
2,3-Dichlor-5-[*N*-(2-oxo-1,2,3,4-tetrahydrochinol-3-yl)carbamoyl]-4*H*-thieno[3,2-*b*]pyrrol;
2,3-Dichlor-5-(*N*-{2-[2-(2-methoxyethoxy)phenyl]-ethyl)carbamoyl)-4*H*-thieno[3,2-*b*]pyrrol;
5-(*N*-{2-[2-(carbamoylmethoxy)phenyl]ethyl}carbamoyl)-2,3-dichlor-4*H*-thieno[3,2-*b*]pyrrol;
2,3-Dichlor-5-(*N*-{2-[2-(*N* methylcarbamoylmethoxy)phenyl]ethyl}carbamoyl)-4*H*thieno[3,2-*b*]pyrrol;
2,3-Dichlor-5-(*N*-{2-[2-(*N*,*N*-dimethylcarbamoylmethoxy)phenyl]ethyl)carbamoyl))-4*H*-thieno13,2-*b*]pyrrol;
2,3-Dichlor-5-(*N*-{2-[2-(morpholinocarbonylmethoxy)phenyl]ethyl}carbamoyl)-4*H*-thieno[3,2-*b*]pyrrol;
5-(*N*-{2-[2-(*N*-Benzylcarbamoylmethoxy)phenyl]-ethyl}carbamoyl)-2,3-dichlor-4*H*-thieno[3,2-*b*]pyrrol;
2,3-Dichlor-5-(*N*-{2-[2-(4-hydroxypiperidinocarbonylmethoxy)phenyl]ethyl]carbamoyl)-4*H*-thieno[3,2-*b*]pyrrol;
*(S)*-2-Chlor-5-{*N*-[(α-(5-ethoxycarbonyl-1,3,4-oxadiazol-2-yl)phenethyl]carbamoyl}-6*H*-thieno[2,3-*b*]pyrrol;
(*S*)-2-Chlor-5-{*N*-[α-(4-methoxycarbonyloxazol-5-yl)phenethyl]carbamoyl}-6*H*-thieno[2,3-*b*]pyrrol;
2-Chlor-5-{*N*-[α-(3-pyridyl)phenethyl]carbamoyl}-6*H*-thieno[2,3-*b*]pyrrol;
2,3-Dichlor-5-{*N*-[α-(3-pyridyl)phenethyl)]carbamoyl}-4*H*-thieno[3,2-*b*]pyrrol;
*(S)*-2-Chlor-5-{*N*-[α-(3-phenyl-1,2,4-oxadiazol-5-yl)phenethyl]carbamoyl}-6*H*-thieno[2,3-*b*]pyrrol;
2,3-Dichlor-5-[*N*-(1-hydroxyindan-2-yl)carbamoyl]-4*H*-thieno[3,2-*b*]pyrrol;
2,3-Dichlor-5-[*N*-((*1S,2S*)-1-hydroxyindan-2-yl)carbamoyl]-4*H*-thieno[3,2-*b*]pyrrol;
2,3-Dichlor-5-[*N*-((*1R,2R*)-1-hydroxyindan-2-yl)carbamoyl]-4*H*-thieno[3,2-*b*]pyrrol;

2-Chlor-5-[*N*-(1-hydroxyinda-2-yl)carbamoyl]-6*H* thieno[2,3-*b*]pyrrol;
2,3-Dichlor-5-[*N*-(1-hydroxy-1,2,3,4-tetrahydronaphth-2-yl)carbamoyl]-4*H*-thieno[3,2-*b*]pyrrol;
2,3-Dichlor-5-[*N*-(6-fluor-1-hydroxyindan-2-yl)carbamoyl]-4*H*-thieno[3,2-*b*]pyrrol;
2,3-Dichlor-5-[*N*-(7-methoxy-1-oxo-1,2,3,4-tetrahydronaphth-2-yl)carbamoyl]-4*H*-thieno[3,2-*b*]pyrrol;
2,3-Dichlor-5-[*N*-(2-indanyl)carbamoyl)-4*H*-thieno[3,2-*b*]pyrrol;
2,3-Dichlor-5-[*N*-(3-methylisooxazol-5-yl)methyl]carbamoyl]-4*H*-thieno[3,2-*b*]pyrrol;
2,3-Dichlor-5-[*N*-(4-hydroxy-1,1-dioxotetrahydrothiophen-3-yl)carbamoyl]-4*H*-thieno[3,2-*b*]pyrrol;
2,3-Dichlor-5-(*N*-(*N*-methyl-*N*-[(1-oxo-1,2,3,4-tetrahydronaphth-2-yl)methyl]carbamoylmethyl}carbamoyl)-4*H*-thieno[3,2-*b*]pyrrol;
2-Chlor-5-[*N*-(2-oxo-1,2,3,4-tetrahydrochinlol-3-yl)carbamoyl]-6*H*-thieno[2,3-*b*]pyrrol;
2-Chlor-5-[*N*-(2,2,3,4-tetrahydrochinlol-3-yl)carbamoyl]}-6*H*-thieno[2,3-*b*]pyrrol;
2-Chlor-5-[*N*-(1-methyl-2-oxo-1,2,3,4-tetrahydrochinlol-3-yl)carbamoyl]-6*H*-thieno[2,3-*b*]pyrrol;
2-Chlor-5-[*N*-(3-oxo-2,3,4,5-tetrahydro-1*H*-benz[2]azepin-4-yl)carbamoyl]-6*H*-thieno[2,3-*b*] pyrrol ;
2,3-Dichlor-5-[*N*-(1-methoxyindan-2-yl)carbamoyl]-4*H*-thieno[3,2-*b*]pyrrol;
2,3-Dichlor-5-(*N*-{1-[*N*-(1,1-dimethylethoxy)carbonylamino]indan-2-yl)carbamoyl)-4*H*-thieno[3,2-*b*]pyrrol;
5-[*N*-(1-Aminoinda-2-yl)carbamoyl]-2,3-dichlor-4*H*-thieno[3,2-*b*]pyrrol;
5-[*N*-(1-Acetamidoindan-2-yl)carbamoyl]-2,3-dichlor-4*H*-thieno[3,2-*b*]pyrrol;
2,3-Dichlor-5-{*N*-[1-(methansulfonamido)indan-2-yl]carbamoyl)-4*H*-thieno[3,2-*b*]pyrrol;
2,3-Dichlor-5-{*N*-[1-(methylarnino)indan-2-yl]carbamoyl}-4*H*-thieno[3,2-*b*]pyrrol; und
2,3-Dichlor-5-{*N*-[1-(*N*-methylacetamido)indan-2-yl]carbamoyl}-4*H*-thieno[3,2-*b*]pyrrol und deren pharmazeutisch unbedenklichen Salze.

**7.** Pharmazeutische Zusammensetzung, enthaltend eine Verbindung der Formel (I) nach einem der Ansprüche 1-6 oder ein pharmazeutisch unbedenkliches Salz oder einen in vivo hydrolisierbaren Ester davon, wie oben definiert, zusammen mit einem pharmazeutisch unbedenklichen Verdünnungsmittel oder Träger.

**8.** Verbindung der Formel (I) nach einem der Ansprüche 1 bis 6 und deren pharmazeutisch unbedenkliche Salze und in vivo hydrolisierbare Ester als Medikament.

**9.** Verwendung einer Verbindung der Formel (I) nach einem der Ansprüche 1 bis 6 oder eines pharmazeutisch unbedenklichen Salzes oder eines in vivo hydrolisierbaren Esters davon, wie oben definiert, zur Herstellung eines Medikaments zum Hervorrufen einer Glykogenphosphorylase hemmenden Wirkung in einem Warmblüter wie dem Menschen.

## Revendications

**1.** Composé de formule (**I**) :

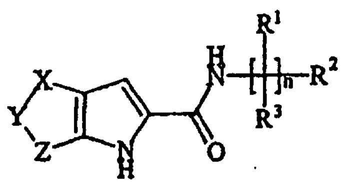

(I)

dans laquelle:

**X-Y-Z-** est choisi parmi -S-CR$^4$=CR$^5$-, -CR$^4$=CR$^5$S-, -O-CR$^4$=CR$^5$-, -CR$^4$=CR$^5$-O-, -N=CR$^4$-S-, -S-CR$^4$=N-, -NR$^6$-CR$^4$=CR$^5$- et -CR$^4$=CR$^5$-NR$^6$-;
où **R$^4$** et **R$^5$** sont choisis indépendamment parmi hydrogène, halogéno, nitro, cyano, hydroxy, fluorométhyle, difluorométhyle, trifluorométhyle, trifluorométhoxy, amino, carboxy, carbamoyle, mercapto, sulfamoyle, uréido, alkyle en $C_{1-6}$, alcényle en $C_{2-6}$, alkynyle en $C_{2-6}$, alcoxy en $C_{1-6}$, alkanoyle en $C_{1-6}$, alkanoyloxy en $C_{1-6}$, *N*-(alkyle en $C_{1-6}$)amino, *N, N*-(alkyle en $C_{1-6}$)$_2$amino, alkanoylamino en $C_{1-6}$, *N*-(alkyle en $C_{1-6}$)carbamoyle, *N,N*-(alkyle en $C_{1-6}$)$_2$carbamoyle, alkylS(O)$_a$ en $C_{1-6}$ où a va de 0 à 2, alcoxy ($C_{1-6}$) carbonyle, alcoxy ($C_{1-6}$)

carbonylamino, *N*-(alkyle en $C_{1-6}$) sulfamoyle, *N,N*-(alkyle en $C_{1-6}$)$_2$-sulfamoyle, alkyl($C_{1-6}$)sulfonylamino et alkyl ($C_{1-6}$) -sulfonyl-N-(alkyle en $C_{1-6}$)amino;

**$R^6$** est hydrogène or alkyle en $C_{1-6}$;

**$R^1$** est choisi parmi hydrogène, halogéno, nitro, cyano, hydroxy, amino, carboxy, carbamoyle, mercapto, sulfamoyle, uréido, alkyle en $C_{1-6}$, alcényle en $C_{2-6}$, alkynyle en $C_{2-6}$, alcoxy en $C_{1-6}$, alkanoyle en $C_{1-6}$, alkanoyloxy en $C_{1-6}$, *N*-(alkyle en $C_{1-6}$)amino, *N, N*-(alkyle en $C_{1-6}$)$_2$amino, alkanoylamino en $C_{1-6}$, *N*-(alkyle en $C_{1-6}$) carbamoyle, *N, N-(alkyle* en $C_{1-4}$)$_2$carbamoyle, alkylS(O)$_a$ en $C_{1-6}$ où a va de 0 à 2, alcoxy ($C_{1-6}$)carbonyle, alcoxy ($C_{1-6}$)carbonylamino, *N*-(alkyle en $C_{1-6}$)sulfamoyle, *N,N*-(alkyle en $C_{1-6}$)$_2$-sulfamoyle, alkyl($C_{1-6}$)sulfonylamino, alkyl ($C_{1-6}$) sulfonyl-*N*-(alkyle en $C_{1-6}$) amino, cycloalkyle en $C_{3-8}$, cycloalkyle($C_{3-8}$) alkyl ($C_{1-6}$), aryle, arylalkyl($C_{1-6}$), un groupement hétérocyclique et un (groupement hétérocyclique) alkyl ($C_{1-6}$); où $R^1$ peut éventuellement être substitué sur le carbone par un ou plusieurs groupements choisis parmi P et où si ledit groupement hétérocyclique contient un motif -NH-, cet azote peut éventuellement être substitué par un groupement choisi parmi R ;

**$R^2$** est choisi parmi hydrogène, halogéno, nitro, cyano, hydroxy, fluorométhyle, difluorométhyle, trifluorométhyle, trifluorométhoxy, amino, carboxy, carbamoyle, mercapto, sulfamoyle, uréido, alkyle en $C_{1-6}$, alcényle en $C_{2-6}$, alkynyle en $C_{2-6}$, alcoxy en $C_{1-6}$, alkanoyle en $C_{1-6}$, alkanoyloxy en $C_{1-6}$, *N*-(alkyle en $C_{1-6}$)amino, *N, N*-(alkyle en $C_{1-6}$)$_2$amino, alkanoylamino en $C_{1-6}$, N-(alkyle en $C_{1-6}$) carbamoyle, *N, N*-(alkyle en $C_{1-4}$)$_2$carbamoyle, *N*-(alkyle en $C_{1-6}$)-*N*-(alcoxy en $C_{1-6}$) carbamoyle, alkylS(O)$_a$ en $C_{1-6}$ où a va de 0 à 2, alcoxy($C_{1-6}$) carbonyle, alcoxy ($C_{1-6}$) carbonylamino, *N*-(alkyle en $C_{1-6}$) sulfamoyle, *N,N*-(alkyle en $C_{1-6}$)$_2$-sulfamoyle, sulfamoylamino, *N*-(alkyle en $C_{1-6}$)sulfamoylamino, *N,N*-(alkyle en $C_{1-6}$)$_2$-sulfamoylamino, alkyl ($C_{1-6}$)sulfonylamino, alkyl ($C_{1-6}$) sulfonylaminocarbonyle, alkyl($C_{1-6}$)sulfonyl-*N*-(alkyle en $C_{1-6}$)amino et un groupement -E-F-G-H-; où **E** et **G** sont choisis indépendamment parmi une liaison directe, -O-, -S-, -SO-, -$SO_2$-, -OC(O)-, -C(O)O-, -C(O)-, -$NR^a$-, $NR^aC(O)$-, -C(O)$NR^a$-, -$SO_2NR^a$-, -$NR^aSO_2$- , -$NR^aC(O)NR^b$- , -OC(O)$NR^a$-, -$NR^aC(O)O$-,-$NR^aSO_2NR^b$-, -$SO_2NR^aC(O)$- et -C(O)$NR^aSO_2$- ; où $R^a$ et $R^b$ sont choisis indépendamment parmi hydrogène ou alkyle en $C_{1-6}$ qui est éventuellement substitué par un groupement V ;

**F** est alkylène en $C_{1-6}$ éventuellement substitué par un ou plusieurs Q ou une liaison directe ;

**H** est choisi parmi aryle, cycloalkyle en $C_{3-8}$ et un groupement hétérocyclique ; où H peut éventuellement être substitué sur le carbone par un ou plusieurs groupements choisis parmi S et où si ledit groupement hétérocyclique contient un motif -NH-, cet azote peut éventuellement être substitué par un groupement choisi parmi T ;

**$R^3$** est hydrogène ou alkyle en $C_{1-6}$ ;

**n** est choisi parmi 0-4 ; où les valeurs de **$R^1$** peuvent être identiques ou différentes ; et où les valeurs de $R^3$ peuvent être identiques ou différentes ;

**P**, **S** et **Q** sont choisis indépendamment parmi halogéno, nitro, cyano, hydroxy, trifluorométhyle, trifluorométhoxy, amino, carboxy, carbamoyle, mercapto, sulfamoyle, uréido, alkyle en $C_{1-6}$, alcényle en $C_{2-6}$, alkynyle en $C_{2-6}$, alcoxy en $C_{1-6}$, alkanoyle en $C_{1-6}$, alkanoyloxy en $C_{1-6}$, *N*-(alkyle en $C_{1-6}$)amino, *N,N*-(alkyle en $C_{1-6}$)$_2$amino, alkanoylamino en Ci-6, *N*-(alkyle en $C_{1-6}$)carbamoyle, *N, N*-(alkyle en $C_{1-6}$)$_2$carbamoyle, *N*-(alkyle en $C_{1-6}$)-*N*-(alcoxy en $C_{1-6}$)carbamoyle, alkylS (O)$_a$ en $C_{1-6}$ où a va de 0 à 2, alcoxy ($C_{1-6}$) carbonyle, alcoxy ($C_{1-6}$) carbonylamino, N-(alkyle en $C_{1-6}$) sulfamoyle, *N,N*-(alkyle en $C_{1-6}$)$_2$sulfamoyle, alkyl($C_{1-6}$)sulfonylamino, alkyl($C_{1-6}$)sulfonyl-*N*-(alkyle en $C_{1-6}$)amino, cycloalkyle en $C_{3-8}$, aryle et un groupement hétérocyclique ; où P, S et Q peuvent éventuellement et indépendamment être substitués sur le carbone par un ou plusieurs groupements choisis parmi V et où si ledit groupement hétérocyclique contient un motif -NH-, cet azote peut éventuellement être substitué par un groupement choisi parmi U;

**V** est choisi parmi halogéno, nitro, cyano, hydroxy, trifluorométhoxy, trifluorométhyle, amino, carboxy, carbamoyle, mercapto, sulfamoyle, méthyle, éthyle, méthoxy, éthoxy, acétyle, acétoxy, méthylamino, éthylamino, diméthylamino, diéthylamino, *N*-méthyl-*N*-éthylamino, acétylamino, *N*-méthylcarbamoyle, *N*-éthylcarbamoyle, *N,N*-diméthylcarbamoyle, *N,N*-diéthylcarbamoyle, *N*-méthyl-*N*-éthylcarbamoyle, méthylthio, éthylthio, méthylsulfinyle, éthylsulfinyle, mésyle, éthylsulfonyle, méthoxycarbonyle, éthoxycarbonyle, *N*-méthylsulfamoyle, *N*-éthylsulfamoyle, *N,N*-diméthylsulfamoyle, *N,N*-diéthylsulfamoyle, *N*-méthyl-*N*-éthylsulfamoyle, morpholino, morpholinocarbonyle, *N*-benzylcarbamoyle, et 4-hydroxypipéridinocarbonyle ;

**R, T** et **U** sont choisis indépendamment parmi alkyle en $C_{1-4}$, alcanoyle en $C_{1-4}$, alkylsulfonyle en $C_{1-4}$, alcoxy ($C_{1-4}$)carbonyle, carbamoyle, N-(alkyle en $C_{1-4}$)carbamoyle, *N, N*-(alkyle en $C_{1-4}$)$_2$carbamoyle, phényle, benzyle, benzyloxycarbonyle, benzoyle et phénylsulfonyle où R, T et U peuvent être éventuellement et indépendamment substitués sur le carbone par un ou plusieurs groupements choisis parmi V ;

ou un sel pharmaceutiquement acceptable ou un ester hydrolysable *in vivo* de celui-ci ; avec les conditions :

i) lorsque -X-Y-Z- est -S-CH=CH-, $R^2$-($CR^1R^3$)$_n$- ne peut être amino, 1-phényl-5-méthyl-1H-1,5-benzodiazépine-2,4(3H,5H)dion-3-yle, 1-méthyl-5-phényl-2-oxo-2,3-dihydro-1H-benzo(E)(1,4)diazépin-3-yle, 2-(4-phényl-1,2,5,6-tétrahydropyrid-1-yl)éthyle, 3-(4-phényl-1,2,5,6-tétrahydropyrid-1-yl)propyle, 2-(4-phénylpipérazin-1-yl)éthyle, 2-(*N*-méthylamino) éthyle, 2-morpholinoéthyle ou 2-(*N*-méthyl-*N*-benzylamino) éthyle ;

ii) lorsque -X-Y-Z- est -CH=CH-S-, $R^2$-$(CR^1R^3)_{n}$- ne peut être amino ou 1-méthyl-5-phényl-2-oxo-2,3-dihydro-1H-benzo(E)(1,4)diazépin-3-yle ;

iii) lorsque -X-Y-Z- est -CH=C $(SO_2NH_2)$ -S-, $R^2$-$(CR^1R^3)_n$- ne peut être méthyle ou isobutyle ; et

iv) lorsque -X-Y-Z- est tel que défini initialement, n vaut 1, $R^1$ est arylméthyle, arylméthyle substitué, (groupement hétérocyclique)méthyle et (groupement hétérocyclique)méthyle substitué et $R^3$ est hydrogène alors $R^2$ n'est pas un groupement -C(=O)-A ou un groupement -CH(OH)-C(=O)-A dans lequel A est $NR^dR^d$, $-NR^aCH_2CH_2OR^a$, ou

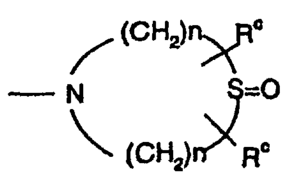 , 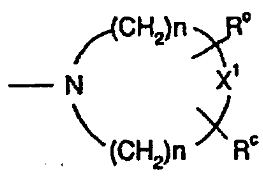 , ou 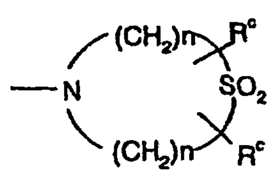

chaque $R^a$ et $R^b$ est indépendamment hydrogène ou alkyle en $C_{1-8}$;
chaque $R^d$ est indépendamment hydrogène, alkyle en $C_{1-8}$, alcoxy en $C_{1-8}$, aryle, aryle substitué, hétéroaryle, ou hétéroaryle substitué ;
chaque $R^c$ est indépendamment hydrogène, $-C(=O)OR^a$, $-OR^a$, $-SR^a$, ou $-NR^aR^a$ ; et chaque n vaut indépendamment 1-3, et
$X^1$ est $NR^a$, $-CH_2$-, O ou S.

**2.** Composé de formule (**I**) selon la revendication 1, dans laquelle :

-X-Y-Z- est choisi parmi -S-$CR^4$=$CR^5$- ou -$CR^4$=$CR^5$-S- ;
où $R^4$ et $R^5$ sont choisis indépendamment parmi hydrogène, halogéno, nitro, cyano, hydroxy, fluorométhyle, difluorométhyle, trifluorométhyle, trifluorométhoxy, amino, carboxy, carbamoyle, mercapto, sulfamoyle, uréido, alkyle en $C_{1-6}$, alcényle en $C_{2-6}$, alkynyle en $C_{2-6}$, alcoxy en $C_{1-6}$, alkanoyle en $C_{1-6}$, alkanoyloxy en $C_{1-6}$, *N*-(alkyle en $C_{1-6}$) amino, *N*,*N*-(alkyle en $C_{1-6}$)$_2$amino, alkanoylamino en $C_{1-6}$, *N*-(alkyle en $C_{1-6}$) carbamoyle, *N, N*-(alkyle en $C_{1-6}$)$_2$carbamoyle, alkylS$(O)_a$ en $C_{1-6}$ où a va de 0 à 2, alcoxy ($C_{1-6}$)carbonyle, alcoxy ($C_{1-6}$) carbonylamino, *N*-(alkyle en $C_{1-6}$) sulfamoyle, *N,N*-(alkyle en $C_{1-6}$)$_2$-sulfamoyle, alkyl($C_{1-6}$)sulfonylamino et alkyl ($C_{1-6}$)-sulfonyl-*N*-(alkyle en $C_{1-6}$) amino ;
n vaut 0 ;
$R^2$ est un groupement -E-F-G-H-;
où E, F et G sont chacun une liaison directe ;
H est cycloalkyle en $C_{3-12}$ qui est éventuellement condensé avec un cycle benzénique où H peut éventuellement être substitué sur le carbone par un ou plusieurs groupements S qui sont choisis indépendamment parmi halogéno, nitro, cyano, hydroxy, trifluorométhyle, trifluorométhoxy, amino, carboxy, carbamoyle, mercapto, sulfamoyle, uréido, alkyle en $C_{1-6}$, alcényle en $C_{2-6}$, alkynyle en $C_{2-6}$, alcoxy en $C_{1-6}$, alkanoyle en $C_{1-6}$, alkanoyloxy en $C_{1-6}$, *N*-(alkyle en $C_{1-6}$) amino, *N,N*-(alkyle en $C_{1-6}$)$_2$amino, alkanoylamino en $C_{1-6}$, *N*-(alkyle en $C_{1-6}$)carbamoyle, *N,N*-(alkyle en $C_{1-6}$)$_2$carbamoyle, *N*-(alkyle en $C_{1-6}$)-*N*-(alcoxy en $C_{1-6}$) carbamoyle, alkylS $(O)_a$ en $C_{1-6}$ où a va de 0 à 2, alcoxy ($C_{1-6}$) carbonyle, alcoxy ($C_{1-6}$) carbonylamino, *N*-(alkyle en $C_{1-6}$) sulfamoyle, *N,N*-(alkyle en $C_{1-6}$)$_2$sulfamoyle, alkyl($C_{1-6}$)sulfonylamino, alkyl ($C_{1-6}$) sulfonyl-*N*-(alkyle en $C_{1-6}$)amino, cycloalkyle en $C_{3-8}$, aryle et des groupements hétérocycliques ; où S peut éventuellement être substitué sur le carbone par un ou plusieurs groupements choisis parmi V ;
V est choisi parmi halogéno, nitro, cyano, hydroxy, trifluorométhoxy, trifluorométhyle, amino, carboxy, carbamoyle, mercapto, sulfamoyle, méthyle, éthyle, méthoxy, éthoxy, acétyle, acétoxy, méthylamino, éthylamino, diméthylamino, diéthylamino, *N*-méthyl-*N*-éthylamino, acétylamino, *N*-méthylcarbamoyle, *N*-éthylcarbamoyle, *N,N*-diméthylcarbamoyle, *N,N*-diéthylcarbamoyle, N-méthyl-*N*-éthylcarbamoyle, méthylthio, éthylthio, méthylsulfinyle, éthylsulfinyle, mésyle, éthylsulfonyle, méthoxycarbonyle, éthoxycarbonyle, *N*-méthylsulfamoyle, *N*-éthylsulfamoyle, *N,N*-diméthylsulfamoyle, *N,N*-diéthylsulfamoyle, *N*-méthyl-*N*-éthylsulfamoyle, morpholino, morpholinocarbonyle, *N*-benzylcarbamoyle, et 4-hydroxypipéridinocarbonyle;

ou un sel pharmaceutiquement acceptable de celui-ci.

**3.** Composé de formule (**I**) selon la revendication 1, dans laquelle :

-X-Y-Z- est choisi parmi -S-CR$^4$=CR$^5$- ou -CR$^4$=CR$^5$-S- ;
où $R^4$ et $R^5$ sont choisis indépendamment parmi hydrogène, halogéno, nitro, cyano, hydroxy, fluorométhyle, difluorométhyle, trifluorométhyle, trifluorométhoxy, amino, carboxy, carbamoyle, mercapto, sulfamoyle, uréido, alkyle en $C_{1-6}$, alcényle en $C_{2-6}$, alkynyle en $C_{2-6}$, alcoxy en $C_{1-6}$, alkanoyle en $C_{1-6}$, alkanoyloxy en $C_{1-6}$, *N*-(alkyle en $C_{1-6}$)amino, *N,N*-(alkyle en $C_{1-6}$)$_2$amino, alkanoylamino en $C_{1-6}$, *N*-(alkyle en $C_{1-6}$) carbamoyle, *N,N*-(alkyle en $C_{1-6}$)$_2$carbamoyle, alkylS(O)$_a$ en $C_{1-6}$ où a va de 0 à 2, alcoxy ($C_{1-6}$)carbonyle, alcoxy ($C_{1-6}$) carbonylamino, *N*-(alkyle en $C_{1-6}$) sulfamoyle, *N,N*-(alkyle en $C_{1-6}$)$_2$-sulfamoyle, alkyl($C_{1-6}$)sulfonylamino et alkyl($C_{1-6}$)-sulfonyl-*N*-(alkyle en $C_{1-6}$) amino ;
n vaut 0 ;
$R^2$ est un groupement -E-F-G-H-;
où E, F et G sont chacun une liaison directe ;
H est un amide cyclique de formule

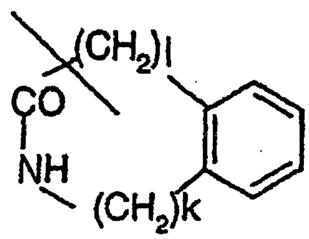

dans laquelle k vaut 0, 1, 2 ou 3 et 1 vaut 0, 1, 2 ou 3, tel que la somme de k et de 1 vaille 2 ou 3 et dans laquelle l'un des atomes de carbone gouverné par k ou 1 peut être remplacé par soufre et dans laquelle H est éventuellement substitué sur le carbone par un ou plusieurs groupements choisis parmi S et peut éventuellement et indépendamment être substitué sur l'azote par un groupement choisi parmi T;
S est choisi parmi halogéno, nitro, cyano, hydroxy, trifluorométhyle, trifluorométhoxy, amino, carboxy, carbamoyle, mercapto, sulfamoyle, uréido, alkyle en $C_{1-6}$, alcényle en $C_{2-6}$, alkynyle en $C_{2-6}$, alcoxy en $C_{1-6}$, alkanoyle en $C_{1-6}$, alkanoyloxy en $C_{1-6}$, *N*-(alkyle en $C_{1-6}$)amino, *N,N*-(alkyle en $C_{1-6}$)$_2$amino, alkanoylamino en $C_{1-6}$, *N*-(alkyle ,en $C_{1-6}$)carbamoyle, *N,N*-(alkyle en $C_{1-6}$)$_2$carbamoyle, *N*-(alkyle en $C_{1-6}$) -*N*-(alcoxy en $C_{1-6}$) carbamoyle, alkylS(O)$_a$ en $C_{1-6}$ où a va de 0 à 2, alcoxy ($C_{1-6}$) carbonyle, alcoxy ($C_{1-6}$) carbonylamino, *N*-(alkyle en $C_{1-6}$) sulfamoyle, *N,N*-(alkyle en $C_{1-6}$)$_2$-sulfamoyle, alkyl($C_{1-6}$)sulfonylamino, alkyl($C_{1-6}$)-sulfonyl-N-(alkyle en $C_{1-6}$)amino, cycloalkyle en $C_{3-8}$, aryle et un groupement hétérocyclique ;où S peut éventuellement et indépendamment être substitué sur le carbone par un ou plusieurs groupements choisis parmi V et où si ledit groupement hétérocyclique contient un motif -NH-, cet azote peut éventuellement être substitué par un groupement choisi parmi U ;
T et U sont choisis indépendamment parmi alkyle en $C_{1-4}$, alcanoyle en $C_{1-4}$, alkylsulfonyle en $C_{1-4}$, alcoxy ($C_{1-4}$)carbonyle, carbamoyle, *N*-(alkyle en $C_{1-4}$)carbamoyle, *N, N*-(alkyle en $C_{1-4}$ )$_2$carbamoyle, phényle, benzyle, benzyloxycarbonyle, benzoyle et phénylsulfonyle où R, T et U peuvent être éventuellement et indépendamment substitués sur le carbone par un ou plusieurs groupements choisis parmi V ;
V est choisi parmi halogéno, nitro, cyano, hydroxy, trifluorométhoxy, trifluorométhyle, amino, carboxy, carbamoyle, mercapto, sulfamoyle, méthyle, éthyle, méthoxy, éthoxy, acétyle, acétoxy, méthylamino, éthylamino, diméthylamino, diéthylamino, *N*-méthyl-*N*-éthylamino, acétylamino, *N*-méthylcarbamoyle, *N*-éthylcarbamoyle, *N,N*-diméthylcarbamoyle, *N,N*-diéthylcarbamoyle, *N*-méthyl-*N*-éthylcarbamoyle, méthylthio, éthylthio, méthylsulfinyle, éthylsulfinyle, mésyle, éthylsulfonyle, méthoxycarbonyle, éthoxycarbonyle, *N*-méthylsulfamoyle, *N*-éthylsulfamoyle, *N,N*-diméthylsulfamoyle, *N,N*-diéthylsulfamoyle, *N*-méthyl-*N*-éthylsulfamoyle, morpholino, morpholinocarbonyle, *N*-benzylcarbamoyle, et 4-hydroxypipéridinocarbonyle ;
ou un sel pharmaceutiquement acceptable ou un ester hydrolysable *in vivo* de celui-ci.

**4.** Composé de formule (**I**) selon la revendication 1, dans laquelle :

-X-Y-Z- est choisi parmi -S-CR$^4$=CR$^5$- ou -CR$^4$=CR$^5$-S- ;
où $R^4$ et $R^5$ sont choisis indépendamment parmi hydrogène, halogéno ou alkyle en $C_{1-6}$ ;

n vaut 1 ;
$R^1$ est hydrogène ou arylalkyl($C_{1-6}$) ;
$R^2$ est choisi parmi un groupement -E-F-G-H- ; où E, F et G sont chacun une liaison directe ;
H est un groupement hétérocyclique insaturé à 5 chaînons contenant au moins un atome d'azote et un ou deux atomes de cycle choisis parmi oxygène et soufre et où H peut éventuellement être substitué sur le carbone par un ou plusieurs groupements S qui sont choisis indépendamment parmi halogéno, nitro, cyano, hydroxy, trifluorométhyle, trifluorométhoxy, amino, carboxy, carbamoyle, mercapto, sulfamoyle, uréido, alkyle en $C_{1-6}$, alcényle en $C_{2-6}$, alkynyle en $C_{2-6}$, alcoxy en $C_{1-6}$, alkanoyle en $C_{1-6}$, alkanoyloxy en $C_{1-6}$, *N*-(alkyle en $C_{1-6}$)amino, *N,N*-(alkyle en $C_{1-6}$)$_2$amino, alkanoylamino en $C_{1-6}$, *N*-(alkyle en $C_{1-6}$)carbamoyle, *N,N*-(alkyle en $C_{1-6}$)$_2$carbamoyle, *N*-(alkyle en $C_{1-6}$)-*N*-(alcoxy en $C_{1-6}$) carbamoyle, alkylS(O)$_a$ en $C_{1-6}$ où a va de 0 à 2, alcoxy ($C_{1-6}$) carbonyle, alcoxy ($C_{1-6}$)carbonylamino, *N*-(alkyle en $C_{1-6}$) sulfamoyle, *N,N*-(alkyle en $C_{1-6}$)$_2$-sulfamoyle, alkyl($C_{1-6}$)sulfonylamino, alkyl($C_{1-6}$)sulfonyl-*N*-(alkyle en $C_{1-6}$) amino, cycloalkyle en $C_{3-8}$ et des groupements aryle ;
$R^3$ est hydrogène ou alkyle en $C_{1-6}$ ;

ou un sel pharmaceutiquement acceptable de celui-ci.

**5.** Composé de formule (I) selon la revendication 1, dans laquelle :

-X-Y-Z- est choisi parmi -S-$CR^4$=$CR^5$- ou -$CR^4$=$CR^5$-S- ;
où $R^4$ et $R^5$ sont choisis indépendamment parmi hydrogène, halogéno ou alkyle en $C_{1-6}$ ;
n vaut 0;
$R^2$ est un groupement -E-F-G-H- ;
où E est une liaison directe ;
F est méthylène ;
où G est -C (O) $NR^a$, où $R^a$ est choisi parmi hydrogène ou alkyle en $C_{1-6}$ qui est éventuellement substitué par un groupement V ;
H est aryle pouvant éventuellement être substitué sur le carbone par un ou plusieurs groupements choisis parmi S;
S est choisi parmi halogéno, nitro, cyano, hydroxy, trifluorométhyle, trifluorométhoxy, amino, carboxy, carbamoyle, mercapto, sulfamoyle, uréido, alkyle en $C_{1-6}$, alcényle en $C_{2-6}$, alkynyle en $C_{2-6}$, alcoxy en $C_{1-6}$, alkanoyle en $C_{1-6}$, alkanoyloxy en $C_{1-6}$, *N*-(alkyle en $C_{1-6}$)amino, *N, N*-(alkyle en $C_{1-6}$)$_2$amino, alkanoylamino en $C_{1-6}$, *N*-(alkyle en $C_{1-6}$)carbamoyle, N, N-(alkyle en $C_{1-6}$)$_2$carbamoyle, N-(alkyle en $C_{1-6}$)-*N*-(alcoxy en $C_{1-6}$) carbamoyle, alkylS(O)$_a$ en $C_{1-6}$ où a va de 0 à 2, alcoxy ($C_{1-6}$)carbonyle, alcoxy ($C_{1-6}$) carbonylamino, N-(alkyle en $C_{1-6}$) sulfamoyle, *N,N*-(alkyle en $C_{1-6}$)$_2$-sulfamoyle, alkyl($C_{1-6}$)sulfonylamino, alkyl($C_{1-6}$)sulfonyl-*N*-(alkyle en $C_{1-6}$)amino, cycloalkyle en $C_{3-8}$, aryle et un groupement hétérocyclique ; où S peut éventuellement et indépendamment être substitué sur le carbone par un ou plusieurs groupements choisis parmi V ;
V est choisi parmi halogéno, nitro, cyano, hydroxy, trifluorométhoxy, trifluorométhyle, amino, carboxy, carbamoyle, mercapto, sulfamoyle, méthyle, éthyle, méthoxy, éthoxy, acétyle, acétoxy, méthylamino, éthylamino, diméthylamino, diéthylamino, *N*-méthyl-*N*-éthylamino, acétylamino, *N*-méthylcarbamoyle, *N*-éthylcarbamoyle, *N,N*-diméthylcarbamoyle, *N,N*-diéthylcarbamoyle, *N*-méthyl-*N*-éthylcarbamoyle, méthylthio, éthylthio, méthylsulfinyle, éthylsulfinyle, mésyle, éthylsulfonyle, méthoxycarbonyle, éthoxycarbonyle, *N*-méthylsulfamoyle, *N*-éthylsulfamoyle, *N,N*-diméthylsulfamoyle, *N,N*-diéthylsulfamoyle, *N*-méthyl-*N*-éthylsulfamoyle, morpholino, morpholinocarbonyle, *N*-benzylcarbamoyle, et 4-hydroxypipéridinocarbonyle ;

ou un sel pharmaceutiquement acceptable de celui-ci.

**6.** Composé choisi parmi
le 2,3-dichloro-5-[*N*-(2-phénoxyéthyl)carbamoyl]-4*H*-thiéno[3,2-*b*]pyrrole ;
le 2,3-dichloro-5-{*N*-[2-(2-thiényl)éthyl]carbamoyl}-4*H*-thiéno[3,2-*b*]pyrrole ;
le 2,3-dichloro-5-{*N*-[2-(2-méthoxyphényl)éthyl]carbamoyl}-4*H*-thiéno(3,2-*b*]pyrrole ;
le 2,3-dichloro-5-[*N*-(2-phényl-1-cyclopropyl)carbamoyl}-4*H*-thiéno[3,2-*b*]pyrrole ;
le 2,3-dichloro-5-{*N*-[2-(4-fluorophényl)éthyl]carbamoyl}-4*H*-thiéno[3,2-*b*]pyrrole ;
le 2,3-dichloro-5-[*N*-(*N*-phénylcarbamoylméthyl)-carbamoyl]-4*H*-thiéno[3,2-*b*]-pyrrole ;
le 2,3-dichloro-5-(*N*-(2-[(2-pyridyl)amino]éthyl}-carbamoyl)-4*H*-thiéno[3,2-*b*]pyrrole ;
le 2,3-dichloro-5-{*N*-[2-(*N*-méthylméthanesulfonamido)-1-(thiazol-2-yl)éthyl]carbamoyl}-4*H*-thiéno[3,2-*b*)-pyrrole;
le 2,3-dichloro-5-(*N*-[2-(thiomorpholino)éthyl]carbamoyl}-4*H*-thiéno[3,2-*b*]pyrrole ;
le 5-[*N*-(benzoylméthyl)carbamoyl]-2,3-dichloro-4*H*-thiéno[3,2-*b*]pyrrole ;

le 3-chloro-5-[*N*-(*N*-phénylcarbamoylméthyl)carbamoyl]-4*H*-thiéno[3,2-*b*]pyrrole ;
le 3-chloro-5-{*N*-[2-(thiomorpholino)éthyl]carbamoyl}-4H-thiéno[3,2-*b*]pyrrole ;
le 3-chloro-5-{*N*-[2-(*N*-méthylméthanesulfonamido)-1-(thiazol-2-yl)éthyl]carbamoyl}-4*H*-thiéno[3,2-*b*]pyrrole;
le 3-chloro-5-[*N*-(benzoylméthyl)carbamoyl]-4*H*-thiéno-[3,2-*b*]pyrrole ;
le 3-chloro-5-{*N*-[2-(2-méthoxyphényl)éthyl]carbamoyl}-4*H*-thiéno[3,2-*b*]pyrrole ;
le 3-chloro-5-{*N*-[2-(2-thiényl)éthyl]carbamoyl}-4*H*-thiéno[3,2-*b*]pyrrole ;
le 3-chloro-5-[*N*-(2-phényl-1-cyclopropyl)carbamoyl]-4*H*-thiéno[3,2-*b*]pyrrole ;
le 3-chloro-5-{*N*-[2-(4-fluorophényl)éthyl]carbamoyl}-4*H*-thiéno[3,2-*b*]pyrrole ;
le 3-chloro-5-[*N*-(2-phénoxyéthyl)carbamoyl]-4*H*-thiéno[3,2-*b*]pyrrole ;
le 3-chloro-5-{*N*-[2-(1-phénylméthanesulfonamido)-éthyl]carbamoyl}-4*H*-thiéno [3,2-*b*]pyrrole ;
le 3-chloro-5-[*N*-(4-oxo-2,3,4,5-tétrahydrobenz[1,5]-thiazépin-3-yl)carbamoyl]-4*H*-thiéno[3,2-*b*]pyrrole ;
le 2-chloro-5-[*N*-(benzoylméthyl)carbamoyl)-4*H*-thiéno[3,2-*b*]pyrrole ;
le 2-chloro-5-[*N*-(2-phényl-1-cyclopropyl)carbamoyl]-4*H*-thiéno[3,2-*b*]pyrrole ;
le 2-chloro-5-[*N*-(*N*-phénylcarbamoylméthyl)carbamoyl]-4H-thiéno[3,2-*b*]pyrrole ;
le 2-chloro-5-(*N*-{2-[(2-pyridyl)amino]éthyl}carbamoyl)-4*H*-thiéno[3,2-*b*]pyrrole ;
le 2-chloro-5-{*N*-[2-(2-méthoxyphényl)éthyl]carbamoyl}-4*H*-thiéno[3,2-*b*]pyrrole ;
le 2-chloro-5-[*N*-(2-phénoxyéthyl)carbamoyl]-4H-thiéno[3,2-*b*]pyrrole ;
le 2-chloro-5-{*N*-[2-(2-thiényl)éthyl]carbamoyl}-4*H*-thiéno [3,2-*b*] pyrrole ;
le 2-chloro-5-{*N*-[2-(4-fluorophényl)éthyl]carbamoyl}-4*H*-thiéno[3,2-*b*]pyrrole ;
le 2-chloro-5-{*N*-[2-(*N*-méthylméthanesulfonamido)-1-(thiazol-2-yl)éthyl]carbamoyl}-4*H*-thiéno[3,2-*b*]pyrrole;
le 2-chloro-5-{*N*-[2-(thiomorpholino)éthyl]carbamoyl}-4*H*-thiéno[3,2-*b*]pyrrole
le 2,3-dichloro-5-[*N*-(2,3-diméthyl-5-oxo-1-phényl-2,5-dihydro-1*H*-pyrazol-4-yl)carbamoyl]-4*H*-thiéno[3,2-*b*]pyrrole;
le 2,3-dichloro-5-[*N*-(4-sulfamoylphénylméthyl)-carbamoyl]-4*H*-thiéno[3,2-*b*]pyrrole ;
le 2,3-dichloro-5-[*N*-(2-hydroxy-1-phénéthyl)carbamoyl]-4H-thiéno[3,2-*b*]pyrrole;
le 2,3-dichloro-5-{*N*-(2-[(3-trifluorométhylpyrid-2-yl)amino]éthyl)carbamoyl}-4*H*-thiéno[3,2-*b*] pyrrole ;
le 2,3-dichloro-5-{*N*-[3-(5-tétrazolyl)propyl]-carbamoyl)-4*H*-thiéno [3,2-*b*]pyrrole ;
le 2,3-dichloro-5-[*N*-(5-oxo-3-phényl-4,5-dihydroisoxazol-4-yl)carbamoyl]-4*H*-thiéno[3,2-*b*]pyrrole ;
le 2,3-dichloro-5-[*N*-(5-hydroxy-2-oxo-2,3,4,5-tétrahydro-1*H*-benz[*b*]azépin-4-yl)carbamoyl]-4*H*thiéno[3,2-*b*] pyrrole ;
le 2-chloro-5-{*N*-[3-(benzyloxycarbonylamino)-propyl]carbamoyl}-4*H*-thiéno[3,2-*b*]pyrrole ;
le 2,3-dichloro-5-{*N*-[(4-diméthylaminophényl)méthyl]carbamoyl}-4*H*-thiéno[3,2-*b*]pyrrole ;
le 5-[*N*-(1-benzyl-2-hydroxyéthyl)carbamoyl]-2,3-dichloro-4*H*-thiéno[3,2-*b*]pyrrole ;
le 2,3-dichloro-5-{*N*-[2-(phénylamino)éthyl)carbamoyl}-4*H*-thiéno[3,2-*b*]pyrrole ;
le 2,3-dichloro-5-[*N*-(β-(*R*)-hydroxy-α-méthyl-phénéthyl)carbamoyl]-4*H*-thiéno[3,2-*b*]pyrrole ;
le 2,3-dichloro-5-[*N*-(β-hydroxyphénéthyl)carbamoyl)-4H-thiéno[3,2-*b*]pyrrole ;
le 2,3-dichloro-5-{*N*-[2-(4-hydroxyphényl)éthyl]carbamoyl}4*H*-thiéno[3,2-*b*]pyrrole ;
le 2,3-dichloro-5-{*N*-[(benzimidazol-2-yl)méthyl]-carbamoyl}-4*H*-thiéno[3,2-*b*)pyrrole ;
le 2,3-dichloro-5-{*N*-[2-(4-chlorophényl)-2-hydroxy-1-(méthoxycarbonyl)éthyl]carbamoyl}-4*H*-thiéno[3,2-*b*]-pyrrole ;
le 2,3-dichloro-5-{*N*-(imidazo[1,2-*a*]pyrid-2-yl)carbamoyl}-4*H*-thiéno[3,2-*b*]pyrrole ;
le 5-{*N*-[(benzthiazol-2-yl)méthyl]carbamoyl}-2,3-di-chloro-4*H*-thiéno[3,2-*b*]pyrrole ;
le 2,3-dichloro-5-{*N*-[(6-trifluorométhylpyrid-3-yl)-méthyl]carbamoyl)-4*H*-thiéno[3,2-*b*]pyrrole ;
le 2,3-dichloro-5-{*N*-(2-[(2-pyridazinyl)méthyl]carbamoyl}-4*H*-thiéno[3,2-*b*]pyrrole ;
le 2,3-dichloro-5-{*N*-[*N*-(2-hydroxy-3-phénoxypropyl)-carbamoylméthyl]carbamoyl}-4*H*-thiéno[3,2-*b*]pyrrole ;
le 2,3-dichloro-5-{*N*-[*N*-(3-méthylisothiazol-5-yl)carbamoylméthyl]carbamoyl}-4*H*-thiéno[3,2-*b*]-pyrrole ;
le 2,3-dichloro-5-(*N*-(2-(pyridazin-3-yloxy)éthyl]-carbamoyl}-4*H*-thiéno[3,2-*b*]pyrrole ;
le 2-chloro-5-(*N*-{2-[(3-trifluorométhylpyrid-2-yl)amino]éthyl}carbamoyl}-4*H*-thiéno[3,2-*b*)pyrrole ;
le 2,3-dichloro-5-(*N*-[2-(4-sulfamoylphényl)éthyl)-carbamoyl} -4*H*-thiéno-[3,2-*b*] pyrrole
le 2,3-dichloro-5-{*N*-[2-(2-pyridyl)éthyl]carbamoyl}-4*H*-thiéno[3,2-*b*]pyrrole ;
le 2,3-dichloro-5-(*N*-(2-[1-hydroxyméthyl-2-(4-imidazolyl)éthyl]carbamoyl}-4*H*-thiéno[3,2-*b*]pyrrole ;
le 2,3-dichloro-5-{*N*-(2-[(3-quinolyl)méthyl]carbamoyl}-4*H*-thiéno[3,2-*b*]pyrrole ;
le 5-{*N*-[3-(4-acétamidophénoxy)-2-hydroxypropyl]-carbamoyl}-2,3-dichloro-4*H*-thiéno[3,2-*b*]pyrrole ;
le 2,3-dichloro-5-{*N*-[3-(*N*-méthylsulfonylcarbamoyl)-propyl]carbamoyl}-4*H*-thiéno[3,2-*b*]pyrrole ;
le 2,3-dichloro-5-[*N*-(2-{[2-(guanidino)thiazol-4-yl]-méthylthio}éthyl)carbamoyl]-4*H*-thiéno[3,2-*b*]pyrrole ;
le 2,3-dichloro-5-{*N*-[2-(2,4-dioxoimidazolidin-1-yl)éthyl] carbamoyl)-4*H*-thiéno[3,2-*b*]pyrrole ;
le 5-{*N*-[2-benzylthio-1-(hydroxyméthyl)éthyl]carbamoyl)-2,3-dichloro-4*H*-thiéno[3,2-*b*]pyrrole ;
le 2,3-dichloro-5-{*N*-[2-(diméthylaminosulfonylamino)-éthyl]carbamoyl)-4*H*-thiéno[3,2-*b*]pyrrole ;

le 2,3-dichloro-5-{*N*-[(6-méthoxypyrid-3-yl)méthyl]-carbamoyl}-4*H*-thiéno[3,2-*b*]pyrrole ;
le (S)-2,3-dichloro-5-{*N*-[(2-oxo-3-phényl-2,3,4,5-tétrahydrooxazol-5-y1)méthyl)carbamoyl}-4*H*-thiéno[3,2-*b*] pyrrole ;
le 2,3-dichloro-5-(*N*-{2-[3-(carbamoylméthyl)phénoxy]-éthyl}carbamoyl)-4*H*-thiéno[3,2-*b*]pyrrole ;
le 5-(*N*-{[6-(benzo[1,3]dioxol-5-yl)-4-méthyl-morpholin-2-yl]méthyl)carbamoyl)-2, 3-dichloro-4*H*-thiéno[3,2-*b*] pyrrole ;
le 5-(*N*-benzylcarbamoyl)-2,3-dichloro-4*H*-thiéno[3,2-*b*]pyrrole ;
le 2,3-dichloro-5-(*N*-phénéthylcarbamoyl)-4*H*-thiéno[3,2-*b*]pyrrole ;
le 2,3-dichloro-5-[*N*-(3-phénylpropyl)carbamoyl]-4*H*-thiéno[3,2-*b*]pyrrole ;
le 2,3-dichloro-5-{*N*-[2-(2-hydroxyphényl)éthyl]carbamoyl}-4*H*-thiéno[3,2-*b*]pyrrole ;
le 2,3-dichloro-5-[*N*-(α,α-diméthylphénéthyl)carbamoyl]-4*H*-thiéno[3,2-*b*]pyrrole ;
le 2,3-dichloro-5-[*N*-(1-phénylcyclobutyl)méthyl)-carbamoyl]-4*H*-thiéno[3,2-*b*]pyrrole;
le 2,3-dichloro-5-[*N*-(β-méthylphénéthyl)carbamoyl]-4*H*-thiéno[3,2-*b*]pyrrole;
le 2,3-dichloro-5-[*N*-(1,2,3,4-tétrahydronapht-2-yl)carbamoyl]-4*H*-thiéno[3,2-*b*]pyrrole;
le 5-[*N*-(*N*-benzylcarbamoylméthyl)carbamoyl]-2,3-dichloro-4*H*-thiéno[3,2-*b*]pyrrole;
le 5-[*N*-(*N*-benzyl-*N*-méthylcarbamoylméthyl)carbamoyl]-2,3-dichloro-4*H*-thiéno[3,2-*b*]pyrrole;
le 2,3-dichloro-5-[*N*-(*N*-méthyl-*N*-phénylcarbamoyl-méthyl)carbamoyl)-4*H*-thiéno[3,2-*b*]pyrrole ;
le 2,3-dichloro-5-{*N*-[*N*-(2-cyanoéthyl)-*N*-phényl-carbamoylméthyl]carbamoyl}-4*H*-thiéno[3,2-*b*]-pyrrole ;
le 2,3-dichloro-5-{*N*-[*N*-(4-méthoxyphényl)carbamoylméthyl]carbamoyl}-4H-thiéno[3,2-*b*]pyrrole ;
le 2,3-dichloro-5-{*N*-[*N*-(4-fluorophényl)carbamoyl-méthyl]carbamoyl}-4H-thiéno[3,2-*b*]pyrrole ;
le 2,3-dichloro-5-{*N*-[*N*-(4-nitrophényl)carbamoyl-méthyl]carbamoyl}-4*H*-thiéno(3,2-*b*]pyrrole ;
le 2,3-dichloro-5-{*N*-[*N*-(2,6-diméthylphényl)carbamoylméthyl]carbamoyl}-4*H*-thiéno[3,2-*b*]pyrrole ;
le 2,3-dichloro-5-(*N*-[*N*-méthyl-N-(4-méthylphényl)-carbamoylméthyl]carbamoyl}-4*H*-thiéno[3,2-*b*]-pyrrole ;
le 2,3-dichloro-5-{*N*-[*N*-méthyl-*N*-(3-méthylphényl)-carbamoylméthyl)carbamoyl}-4*H*-thiéno[3,2-*b*]-pyrrole ;
le 2,3-dichloro-5-{*N*-[*N*-(3-chlorophényl)-N-méthylcarbamoylméthyl]carbamoyl}-4*H*-thiéno[3,2-*b*]-pyrrole ;
le 2,3-dichloro-5-{*N*-[*N*-(2-hydroxyéthyl)-*N*-phényl-carbamoylméthyl)carbamoyl}-4*H*-thiéno[3,2-*b*]-pyrrole ;
le 2,3-dichloro-5-{*N*-[*N*-(1,1-diméthyl-2-hydroxy-éthyl)carbamoylméthyl]carbamoyl}-4*H*-thiéno[3,2-*b*]-pyrrole ;
le 2,3-dichloro-5-{*N*-[*N*-(2-hydroxyéthyl)-*N*-méthylcarbamoylméthyl]carbamoyl}-4*H*-thiéno[3,2-*b*]-pyrrole ;
le 2,3-dichloro-5-{*N*-[*N*-(2-hydroxyéthyl)carbamoyl-méthyl]carbamoyl}-4*H*-thiéno[3,2-*b*]pyrrole ;
le 2,3-dichloro-5-{*N*-[*N*-(3-hydroxypropyl)carbamoylméthyl]carbamoyl}-4*H*-thiéno[3,2-*b*]pyrrole;
le 2,3-dichloro-5-{*N*-[*N*-(4-hydroxybutyl)carbamoylméthyl]carbamoyl}-4*H*-thiéno[3,2-*b*]pyrrole;
le 2,3-dichloro-5-(*N*-{*N*-[bis(hydroxyméthyl)-méthyl] carbamoylméthyl}carbamoyl) -4*H* -thiéno [3,2-*b*] pyrrole ;
le 2,3-dichloro-5-{*N*-[*N*-(2,3-dihydroxypropyl)-carbamoylméthyl]carbamoyl}-4*H*-thiéno[3,2-*b*]pyrrole ;
le 2,3-dichloro-5-{*N*-[*N*-(4-hydroxyméthylphényl)-carbamoylméthyl]carbamoyl}-4*H*-thiéno[3,2-*b*]-pyrrole ;
le 2,3-dichloro-5-{*N*-[*N*-(5-isoquinolyl)carbamoylméthyl] carbamoyl}-4*H*-thiéno[3,2-*b*]pyrrole ;
le 2,3-dichloro-5-{*N*-[*N*-(3-hydroxyméthyl)phényl]carbamoylméthyl]carbamoyl}-4*H*-thiéno[3,2-*b*]pyrrole ;
le 2,3-dichloro-5-(*N*-{*N*-[4-(2-hydroxyéthyl)phényl]-carbamoylméthyl}carbamoyl)-4*H*-thiéno[3,2-*b*]pyrrole ;
le 2,3-dichloro-5-{*N*-[*N*-(2,4-difluorophényl)-*N*-méthyl-carbamoylméthyl]carbamoyl}-4*H* -thiéno[3,2-*b*] pyrrole ;
le 2,3-dichloro-5-{*N*-[(1,2,3,4-tétrahydro-1-quinolyl)carbonylméthyl]carbamoyl}-4*H* -thiéno[3,2-*b*]pyrrole ;
le 2,3-dichloro-5-{*N*-[*N*-(2-cyanoéthyl)-*N*-méthylcarbamoylméthyl]carbamoyl)-4*H*-thiéno[3,2-*b*]-pyrrole ;
le 2,3-dichloro-5-{*N*-[*N*-(4-hydroxypipéridino)-carbamoylméthyl]carbamoyl}-4*H*-thiéno[3,2-*b*]-pyrrole ;
le 2,3-dichloro-5-[*N*-(*N*-cyclopentylcarbamoylméthyl)carbamoyl]-4*H*-thiéno[3,2-*b*]pyrrole ;
le 2,3-dichloro-5-[*N*-(*N*-isopropylcarbamoylméthyl)-carbamoyl]-4*H*-thiéno[3,2-*b*]pyrrole ;
le 2,3-dichloro-5-[*N*-(*N*-isopropyl-*N*-méthylcarbamoylméthyl)carbamoyl]-4*H*-thiéno[3,2-*b*]-pyrrole ;
le 2,3-dichloro-5-[*N*-(thiomorpholinocarbonylméthyl)carbamoyl]-4*H*-thiéno[3,2-*b*]pyrrole ;
le 2,3-dichloro-5-[*N*-(morpholinocarbonylméthyl)-carbamoyl]-4*H*-thiéno[3,2-*b*]pyrrole ;
le 2,3-dichloro-5-{*N*-[(1,1-dioxothiomorpholino)-carbonylméthyl]carbamoyl]-4*H*-thiéno[3,2-*b*]-pyrrole;
le 2,3-dichloro-5-{*N*-[(1-oxothiomorpholino)-carbonylméthyl]carbamoyl]-4*H*-thiéno[3,2-*b*]pyrrole ;
le 2-chloro-5-[*N*-(2-indanyl)carbamoyl]-6*H* -thiéno[2,3-*b*]pyrrole;
le 5-[*N*-(benz[1,2]oxazol-3-ylméthyl)carbamoyl]-2,3-dichloro-4*H*-thiéno[3,2-*b*]pyrrole;
le 2,3-dichloro-5-(*N*-{2-[2-(hydroxyméthyl)phényl]-éthyl}carbamoyl)-4*H*-thiéno[3,2-*b*]pyrrole ;
le 2,3-dichloro-5-[*N*-(4-phénylisoxazol-3-ylméthyl) carbamoyl]-4*H*-thiéno [3,2-*b*] pyrrole ;
le 2,3-dichloro-5-(*N*-(2-[2-(2-mopholinoéthoxy)-phényl]éthyl}carbamoyl)-4*H*-thiéno[3,2-*b*]pyrrole ;
le 2,3-dichloro-5-(*N*-{2-[2-(méthoxycarbonylméthoxy)phényl]éthyl}carbamoyl)-4*H*-thiéno[3,2-*b*]-pyrrole ;
le 5-(*N*-{2-[2-(carboxyméthoxy)phényl]éthyl}carbamoyl)-2,3-dichloro-4*H*-thiéno[3,2-*b*]pyrrole ;
le 2,3-dichloro-5-{*N*-[2-(3-méthoxyphényl)éthyl)-carbamoyl}-4*H*-thiéno[3,2-*b*]pyrrole ;
le 2,3-dichloro-5-[*N*-(2-oxo-1,2,3,4-tétrahydroquinol-3-yl)carbamoyl]-4*H*-thiéno[3,2-*b*]pyrrole;
le 2,3-dichloro-5-(*N*-(2-[2-(2-méthoxyéthoxy)phényl]éthyl)carbamoyl)-4*H*-thiéno[3,2-*b*]pyrrole;

le 5-(*N*-{2-[2-(carbamoylméthoxy)phényl]éthyl)-carbamoyl)-2,3-dichloro-4*H*-thiéno[3,2-*b*]pyrrole;
le 2,3-dichloro-5-(*N*-{2-[2-(*N*-méthylcarbamoyl-méthoxy)phényl]éthyl}carbamoyl)-4*H*-thiéno[3,2-*b*]-pyrrole ;
le 2,3-dichloro-5-(*N*-{2-[2-(*N*,*N*-diméthylcarbamoylméthoxy)phényl]éthyl}carbamoyl)-4*H*-thiéno[3,2-*b*]-pyrrole ;
le 2,3-dichloro-5-(*N*-{2-[2-(morpholinocarbonylméthoxy)phényl]éthyl}carbamoyl)-4*H*-thiéno[3,2-*b*]-pyrrole ;
le 5-(*N*-{2-[2-(*N*-benzylcarbamoylméthoxy)phényl]éthyl}carbamoyl)-2,3-dichloro-4*H* -thiéno[3,2-*b*]pyrrole ;
le 2,3-dichloro-5-(*N*-{2-[2-(4-hydroxypipéridinocarbonylméthoxy)phényl)éthyl}carbamoyl-4*H*-thiéno[3,2-*b*] pyrrole ;
le (*S*)-2-chloro-5-{*N*-[α-(5-éthoxycarbonyl-1,3,4-oxadiazol-2-yl)phénéthyl]carbamoyl}-6*H* -thiéno[2,3-*b*]pyrrole ;
le (*S*)-2-chloro-5-(*N*-[α-(4-méthoxycarbonyloxazol-5-yl)phénéthyl]carbamoyl}-6*H*-thiéno[2,3-*b*]-pyrrole ;
le 2-chloro-5-{*N*-[α-(3-pyridyl)phénéthyl]carbamoyl}-6*H*-thiéno[2,3-*b*]pyrrole ;
le 2,3-dichloro-5-{*N*-[α-(3-pyridyl)phénéthyl)]-carbamoyl}-4*H*-thiéno[3,2-*b*]pyrrole;
le (S)-2-chloro-5-{*N*-[α-(3-phényl-1,2,4-oxadiazol-5-yl)phénéthyl]carbamoyl)-6*H*-thiéno[2,3-*b*]-pyrrole;
le 2,3-dichloro-5-[*N*-(1-hydroxyindan-2-yl)carbamoyl)-4*H*-thiéno(3,2-*b*)pyrrole ;
le 2,3-dichloro-5-[*N*-((1*S*,2*S*)-1-hydroxyindan-2-yl)carbamoyl]-4*H*-thiéno[3,2-*b*]pyrrole ;
le 2,3-dichloro-5-[*N*-((*1R*,*2R*)-1-hydroxyindan-2-yl)carbamoyl]-4*H*-thiéno[3,2-*b*]pyrrole;
le 2-chloro-5-[(*N*-(1-hydroxyindan-2-yl)carbamoyl]-6*H*-thiéno(2,3-*b*]pyrrole ;
le 2,3-dichloro-5-(N-(1-hydroxy-2,2,3,4-tétrahydronapht-2-yl)carbamoyl]-4*H*-thiéno[3,2-*b*]-pyrrole ;
le 2,3-dichloro-5-[*N*-(6-fluoro-1-hydroxyindan-2-yl)carbamoyl]-4*H*-thiéno[3,2-*b*]pyrrole ;
le 2,3-dichloro-5-(*N*-(7-méthoxy-1-oxo-2,2,3,4-tétrahydronapht-2-yl)carbamoyl]-4*H*-thiéno[3,2-*b*]-pyrrole ;
le 2,3-dichloro-5-[*N*-(2-indanyl)carbamoyl]-4*H*-thiéno[3,2-*b*]pyrrole ;
le 2,3-dichloro-5-[*N*-(3-méthylisoxazol-5-yl)-méthyl]carbamoyl)-4*H*-thiéno[3,2-*b*]pyrrole ;
le 2,3-dichloro-5-[*N*-(4-hydroxy-1,1-dioxotétrahydrothiophén-3-yl)carbamoyl]-4*H*-thiéno[3,2-*b*]-pyrrole ;
le 2,3-dichloro-5-(*N*-{*N*-méthyl-N-[1-oxo-1,2,3,4-tétrahydronapht-2-yl)méthyl]carbamoylméthyl}carbamoyl)-4*H*-thiéno[3,2-*b*]pyrrole ;
le 2-chloro-5-[*N*-(2-oxo-1,2,3,4-tétrahydroquinol-3-yl)carbamoyl]-6*H*-thiéno[2,3-*b*]pyrrole ;
le 2-chloro-5-[*N*-(1,2,3,4-tétrahydroquinol-3-yl)carbamoyl]-6*H*-thiéno[2,3-*b*]pyrrole ;
le 2-chloro-5-[*N*-(1-méthyl-2-oxo-1,2,3,4-tétrahydroquinol-3-yl)carbamoyl]-6*H*-thiéno[2,3-*b*)-pyrrole;
le 2-chloro-5-[*N*-(3-oxo-2,3,4,5-tétrahydro-1*H*-benz[2]azépin-4-yl)carbamoyl]-6*H*-thiéno[2,3-*b*]-pyrrole;
le 2,3-dichloro-5-[*N*-(1-méthoxyindan-2-yl)carbamoyl]-4*H*-thiéno[3,2-*b*]pyrrole;
le 2,3-dichloro-5-(*N*-(1-[*N*-(1,1-diméthyléthoxy)-carbonylamino]indan-2-yl}carbamoyl)-4*H*-thiéno[3,2-*b*]pyrrole ;
le 5-[*N*-(1-aminoindan-2-yl)carbamoyl]-2,3-dichloro-4*H*-thiéno[3,2-*b*]pyrrole ;
le 5-[*N*-(1-acétamidoindan-2-yl)carbamoyl)-2,3-dichloro-4*H*-thiéno[3,2-*b*]pyrrole ;
le 2,3-dichloro-5-{*N*-[1-(méthanesulfonamido)indan-2-yl]carbamoyl}-4*H*-thiéno[3,2-*b*]pyrrole ;
le 2,3-dichloro-5-{*N*-[1-(méthylamino)indan-2-yl]carbamoyl}-4*H*-thiéno[3,2-*b*]pyrrole ; et
le 2,3-dichloro-5-{*N*-[1-(*N*-méthylacétamido)indan-2-yl]carbamoyl}-4*H*-thiéno[3,2-*b*]pyrrole
ou un sel pharmaceutiquement acceptable de celui-ci.

**7.** Composition pharmaceutique comprenant un composé de formule (**I**) selon l'une quelconque des revendications 1 à 6, ou un sel pharmaceutiquement acceptable ou un ester hydrolysable in vivo de celui-ci, tel que défini dans le texte ci-dessus, en association avec un diluant ou véhicule pharmaceutiquement acceptable.

**8.** Composé répondant à la formule (I) selon l'une quelconque des revendications 1 à 6, ou un sel pharmaceutiquement acceptable ou un ester hydrolysable *in vivo* de celui-ci, en tant que médicament.

**9.** Utilisation d'un composé répondant à la formule (I) selon l'une quelconque des revendications 1 à 6, ou d'un sel pharmaceutiquement acceptable ou d'un ester hydrolysable *in vivo* de celui-ci, tel que défini dans le texte ci-dessus, dans la fabrication d'un médicament pour une utilisation dans la production d'un effet d'inhibition de la glycogène phosphorylase chez un animal à sang chaud tel que l'homme.